(19) Europäisches Patentamt
European Patent Office
Office européen des brevets

(11) EP 4 385 980 A1

(12) **EUROPEAN PATENT APPLICATION**

(43) Date of publication:
**19.06.2024 Bulletin 2024/25**

(21) Application number: **22213327.4**

(22) Date of filing: **14.12.2022**

(51) International Patent Classification (IPC):
***C07D 231/56*** (2006.01) ***C07D 401/12*** (2006.01)
***C07D 403/12*** (2006.01) ***C07D 405/06*** (2006.01)
***C07D 405/14*** (2006.01) ***C07D 409/12*** (2006.01)
***C07D 417/12*** (2006.01) ***C07D 417/14*** (2006.01)
***A61P 29/00*** (2006.01) ***C07D 471/04*** (2006.01)
***A61K 31/416*** (2006.01)

(52) Cooperative Patent Classification (CPC):
**C07D 401/12; A61P 29/00; C07D 231/56; C07D 403/12; C07D 405/06; C07D 405/14; C07D 409/12; C07D 417/12; C07D 417/14; C07D 471/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Grünenthal GmbH**
**52078 Aachen (DE)**

(72) Inventors:
- **Krüger, Sebastian**
**52078 Achen (DE)**
- **Peil, Sebastian**
**52078 Achen (DE)**
- **Sitnikov, Nikolay**
**52078 Achen (DE)**
- **Konetzki, Ingo**
**52078 Achen (DE)**
- **Dialer, Clemens**
**52078 Achen (DE)**
- **Alen, Jo**
**52078 Achen (DE)**
- **Young, Clint**
**52078 Achen (DE)**
- **Hagendorf, Silke**
**52078 Achen (DE)**

# (54) INDAZOLES AS INHIBITORS OF NAV1.8

(57) The invention relates to compounds according to general formula (I)

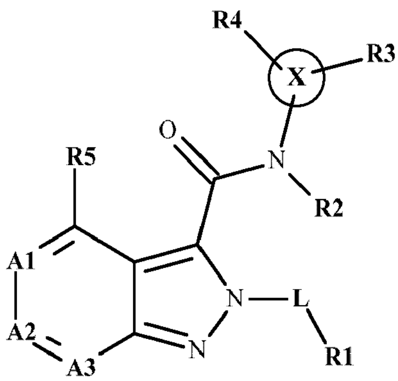

(I)

which act as inhibitors of Navl.8 and can be used in the treatment of pain.

EP 4 385 980 A1

## Description

The invention relates to compounds according to general formula (I)

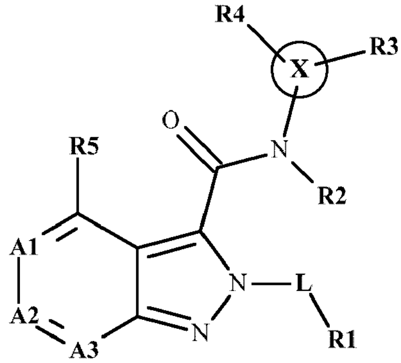

(I)

which act as inhibitors of $Na_V1.8$ and can be used in the treatment of pain.

Normal pain sensation (nociception) serves primarily as a survival mechanism, the body's way of self-protection, alerting against (further) tissue damage and disease from noxious stimuli. For instance, acute pain can arise when the external environment (temperature, pressure, chemicals) activates modality specific receptors (nociceptors) and ion channels within the skin. The peripheral terminals of pain-signaling neurons - whose cell bodies are found in the dorsal root ganglia [DRG] and trigeminal ganglia [TG] - convert the external stimuli into electrochemical generator potentials. Specific voltage-gated sodium channels ($Na_Vs$) integrate and amplify these generator signals until the threshold for an action potential [AP] is reached. Thus, what starts as a noxious stimuli in the periphery eventually leads to action potential firing that travels towards the central nervous system, synapsing first onto neurons in the spinal cord and then towards the brain. $Na_Vs$ also function to support propagation of action potentials to the central terminals within the spinal cord. At the end of its travels along the somatosensory pathway, the action potential signal is interpreted as pain by the brain (Lumpkin and Caterina, Nature (2007), Vol.445 pp 858-865; and Crawford and Caterina Toxicologic Pathology (2020) 48(1)-174; Goodwin G and McMahon S.B. Nature Reviews Neuroscience (2021) Vol 22 pp 263-274; Bennett D.L. et al. Physiol Rev 99 (2019) Vol 99 pp 1079-1151).

Abnormal persistent neuropathic pain arises as a consequence of a lesion or disease of this somatosensory pathway. In response to nerve injury or inflammation, abnormal changes in ion channel expression can cause hyper-excitability of pain-signaling neurons and their nerves/axons, thus resulting in pathological pain.

The voltage-gated $Na_V1.8$ sodium channel is a therapeutic target for analgesia because of its restricted expression profile (almost exclusive to peripheral sensory tissues), its placement along the pain pathway (free nerve endings, sciatic nerve, and DRG), prominent physiological role in pain signaling (supports upstroke of AP and facilitates repetitive AP firing), and supporting genetic/pharmaco-phenotypic evidence (human/animal studies showing changes in $Na_V1.8$ function cause parallel changes in pain sensitivity).

Regarding its expression profile along the pain pathway, because $Na_V1.8$ was first found predominantly in peripheral sensory neurons of the dorsal root ganglia (DRG) and trigeminal ganglia (TG), it was originally termed SNS (sensory neuron specific) (Akopian A.N. et al Nature (1996) Vol 379 pp 257-261) or PN3 (peripheral nerve 3) (Sangameswaran L. et al J. Biol. Chem. (1996) Vol 271 pp 5953-5956). As well, Nav1.8 is localized at free nerve endings, where pain signaling is initiated in the skin (Persson A.K. et al Mol. Pain. (2010) 6:84) and is diffusely localized along the entire length of non-myelinated axons of sciatic nerve (Rush A.M. et al. Eur.J.Neurosci (2005) Vol 22 pp 39-49).

In contrast, $Na_V1.8$ has minimal expression in nonneuronal tissue, such as heart and skeletal muscle, and in the CNS, including brain and spinal cord (9, 10, 338, 406) (Akopian A.N. op. cit.; Akopian A.N. et al. Nat. Neurosci (1999) Vol 2 pp 541-548; Novakovic S.D. et al. J. Neurosci. (1998) Vol 18, pp 2184-2187; and Sagameswaran L. op. cit.).

Regarding its physiological role, $Na_V1.8$ contributes the majority of the inward current during the rising phase of an all-or-none action potential in nociceptive sensory neurons (Blair N.T. et al. J. Neurosci. (2003) Vol 23 pp 10338-10350 and Renganathan M et al. J. Neurophysiol (2001) Vol 86 pp 629-640) - and also contributes most of the current in subsequent spikes during repetitive firing in DRG neurons (Choi J.S. J. Neurophysiol (2011) Vol 106 pp 3173-3184; and Tan Z.Y. et al. J. Neurosci. (2014) Vol 34 pp 7190-7197).

Regarding genetic and pharmacology studies, gain-of-function mutations in $Na_V1.8$ were found in patients with chronic neuropathic pain such as small fiber neuropathy (Faber C.G. et al. (2012) Ann. Neurol Vol 71 pp 26-39; Han C

et al. J. Neurol Neurosurg Psychiatry (2014) Vol 85 pp 499-505; and, Kist A.M. et al. PLoS One (2016) Vol 11 e0161789); Eijkenboom I. et al J. Neurol Neurosurg Psychiatry (2019) 90 (3) pp 342-352); loss-of-function (gene knockout) studies in mice reduced pain sensitivity, notably in nociception (Laird J.M. et al. J. Neurosci (2002) J. Neurosci Vol 22 pp 8352-8356; Jarvis M.F. et al Proc Natl Acad Sci USA (2007) Vol 104 pp 8520-8525; Joshi S.K. et al Pain (2006) Vol 123 pp 75-82) and in neuropathic models (Roza C. et al J Physiol (2003) Vol 550 pp 921-926); $Na_V1.8$-selective small molecule inhibitors reduced pain in rodents, specifically in inflammatory and neuropathic models (Jarvis et al. op. cit.; Kort M.E. et al Bioorg Med Chem Lett (2010) Vol 20 pp 6812-6815; Scanio M.J. et al Bioorg Med Chem (2010) Vol 18 pp 7816-7825; Payne C.E. et al Br J Pharmacol (2015) Vol 172 pp 2654-2670).

Currently, non-selective $Na_V$ channel inhibitors are used to treat epilepsy, cardiac arrhythmia, and chronic pain (Hille, B. J. Gen. Physiol. (1977) Vol. 69 pp. 497-515; Hille. B, Ion Channels of Excitable Membranes (1992) pp. 391-421; Sunderland, Mass., Sinauer Associates, Inc. 3rd ed.; Hondeghem L.M. and Katzung B.G. Annu. Rev. Pharmacol. Toxicol. (1984) Vol. 24. Pp. 387-423; Catterall W.A. Trends Pharmacol. Sci. (1987) Vol. 8 pp.57-65) - however, all of these analgesics have limited efficacy owing to dose-limiting adverse side-effects related to inhibiting $Na_V1.1/Na_V1.2/1.6$ (seizure liability), inhibiting $Na_V1.4$ (muscle weakness/paralysis), inhibiting $Na_V1.5$ (arrhythmia risk).

There is a need to develop a $Na_V1.8$-selective small molecule inhibitor as an effective and safe analgesic.

$Na_V1.8$ inhibitors are also known from WO 2020/092187, WO 2020/092667, WO 2009/049180, WO 2009/049183, WO 2009/049181 and WO 2021/113627.

It was an object of the invention to provide novel compounds which are inhibitors, preferably selective inhibitors, of $Na_V1.8$, and which preferably have advantages over the compounds of the prior art. The novel compounds should in particular be suitable for use in the treatment of pain.

This object has been achieved by the subject-matter of the patent claims.

It was surprisingly found that the compounds according to the invention are highly potent and selective inhibitors of the $Na_V1.8$ channel.

The invention relates to a compound according to general formula (I)

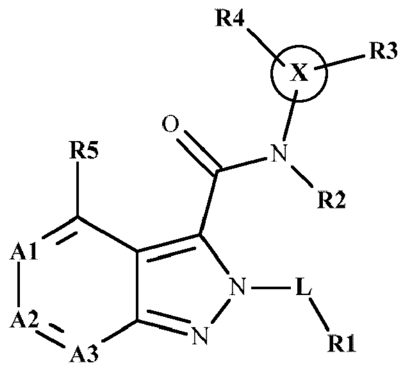

(I)

wherein

**L** represents $CH_2$, $CH(CH_3)$ or $CH(CH_2CH_3)$;

**R1** represents $C_{3-10}$-cycloalkyl, 4 to 10-membered heterocycloalkyl, 5 to 11-membered spiroalkyl, 5 to 11-membered heterospiroalkyl, 4 to 11-membered bicycloalkyl, or 5 to 11-membered heterobicycloalkyl;

**R2** represents H or $C_{1-6}$-alkyl;

**X** represents phenyl, or 5 to 9-membered heteroaryl;

**R3** represents S(O)-$C_{1-6}$-alkyl, S(O)-($C_{3-6}$-cycloalkyl), S(O)-(4 to 6-membered heterocycloalkyl), S(O)-phenyl, S(O)-(5 or 6-membered heteroaryl), $S(O)_2$-$C_{1-6}$-alkyl, $S(O)_2$-($C_{3-6}$-cycloalkyl), $S(O)_2$-(4 to 6-membered heterocycloalkyl), $S(O)_2$-phenyl, $S(O)_2$-(5 or 6-membered heteroaryl), S(O)-$NH_2$, S(O)-N(H)($C_{1-6}$-alkyl), S(O)N(H)($C_{3-6}$-cycloalkyl), S(O)N(H)(4 to 6-membered heterocycloalkyl), $S(O)_2$-$NH_2$, $S(O)_2$-N(H)($C_{1-6}$-alkyl), $S(O)_2$N(H)($C_{3-6}$-cycloalkyl), $S(O)_2$N(H)(4 to 6-membered heterocycloalkyl), S(O)-N($C_{1-6}$-alkyl)$_2$, $S(O)_2$-N($C_{1-6}$-alkyl)$_2$, C(O)-$C_{1-6}$-alkyl, C(O)-$NH_2$, C(O)-N(H)($C_{1-6}$-alkyl), C(O)-N(H)($C_{3-6}$-cycloalkyl), C(O)-N($C_{1-6}$-alkyl)$_2$, C(O)-N($C_{1-6}$-alkyl)($C_{3-6}$-cycloalkyl), CN, $C_{1-6}$-alkylene-OH, OH, =O, O-$C_{1-6}$-alkyl, $OCF_3$, $OCF_2H$, O-$C_{3-6}$-cycloalkyl, O-(4 to 6-membered het-

erocycloalkyl), $NH_2$, N(H)($C_{1-6}$-alkyl), N(H)(C(O)$C_{1-6}$-alkyl), N(H)(C(O)$C_{3-6}$-cycloalkyl), N(H)(C(O)$NH_2$), N(H)($S(O)_2$-$C_{1-6}$-alkyl), N(H)($S(O)_2$-(4 to 6-membered heterocycloalkyl), or S(O)(N**R3a**)**R3b**;

**R3a** represents H, $C_{1-6}$-alkyl, C(O)$C_{1-5}$-alkyl, C(O)$C_{1-5}$-alkyl-$NH_2$, C(O)$C_{1-5}$-alkyl-NH-$CH_3$, C(O)$C_{1-5}$-alkyl-N($CH_3$)$_2$, $C_{3-10}$-cycloalkyl, $C_{1-6}$-alkylene-$C_{3-10}$-cycloalkyl, 4 to 10-membered heterocycloalkyl, or $C_{1-6}$-alkylene-(4 to 10-membered heterocycloalkyl);

**R3b** represents $NH_2$, $C_{1-6}$-alkyl, $C_{3-10}$-cycloalkyl, $C_{1-6}$-alkylene-$C_{3-10}$-cycloalkyl, 4 to 10-membered heterocycloalkyl, or $C_{1-6}$-alkylene-(4 to 10-membered heterocycloalkyl);

or **R3a** and **R3b** mean $(CH_2)_{3-5}$ and together with the atoms to which they are attached form a ring;

**R4** represents H, F, Cl, Br, $C_{1-6}$-alkyl, $CF_3$, $CF_2H$, $CFH_2$ or $C_{3-6}$-cycloalkyl;

or **R3** and **R4** together with the atoms to which they are attached form a 5 to 6-membered heterocycloalkyl;

**R5** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl;

**A1** represents N or C**R6,** wherein **R6** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl;

**A2** represents N or C**R7,** wherein **R7** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl;

**A3** represents N or C**R8**, wherein **R8** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl;

wherein $C_{1-6}$-alkyl and $C_{1-6}$-alkylene in each case independently from one another is linear or branched, saturated or unsaturated;

wherein $C_{1-6}$-alkyl, $C_{1-6}$-alkylene, $C_{3-10}$-cycloalkyl, $C_{3-6}$-cycloalkyl, 4 to 10-membered heterocycloalkyl, 4 to 6-membered heterocycloalkyl, 4 to 11-membered bicycloalkyl, 5 to 11-membered heterobicycloalkyl, 5 to 11-membered spiroalkyl, and 5 to 11-membered heterospiroalkyl, in each case independently from one another are unsubstituted or substituted with one, two, three, four or more substituents independently from one another selected from the group consisting of F, Cl, CN, $C_{1-6}$-alkyl, $C_{1-6}$-alkylene-OH, $C_{1-6}$-alkylene-$OCH_3$, $CF_3$, $CF_2H$, $CFH_2$, C(O)-$C_{1-6}$-alkyl, OH, =O, $OCF_3$, $OCF_2H$, $OCFH_2$, O-$C_{1-6}$-alkyl, $C_{1-4}$-alkylene-O-$C_{1-4}$-alkylene-O-$CH_3$, $C_{0-4}$-alkylene-O-($C_{1-4}$-alkylene-O)$_{1-4}$-$CH_3$, $NH_2$, NH-$C_{1-6}$-alkyl, or N($C_{1-6}$-alkyl)$_2$;

wherein phenyl, 5 to 10-membered heteroaryl and 5 or 6-membered heteroaryl in each case independently from one another are unsubstituted or mono- or polysubstituted with one or more substituents selected from the group consisting of F, Cl, Br, CN, $C_{1-6}$-alkyl, $C_{3-6}$-cycloalkyl, $CF_3$, $CF_2H$, $CFH_2$, $C_{1-6}$-alkylene-$CF_3$, $C_{1-6}$-alkylene-$CF_2H$, $C_{1-6}$-alkylene-$CFH_2$, $C_{1-6}$-alkylene-OH, $C_{1-6}$-alkylene-$OCH_3$, C(O)-$C_{1-6}$-alkyl, $OCF_3$, $OCF_2H$, $OCFH_2$, and O-$C_{1-6}$-alkyl;

in the form of the free compound or a physiologically acceptable salt thereof.

In a preferred embodiment, the compound according to the invention is present in form of the free compound. For the purpose of specification, "free compound" preferably means that the compound according to the invention is not present in form of a salt. Methods to determine whether a chemical substance is present as the free compound or as a salt are known to the skilled artisan such as $^{14}N$ or $^{15}N$ solid state NMR, x-ray diffraction, x-ray powder diffraction, IR, Raman, XPS. $^{1}H$-NMR recorded in solution may also be used to consider the presence of protonation.

In another preferred embodiment, the compound according to the invention is present in form of a physiologically acceptable salt. For the purposes of this specification, the term "physiologically acceptable salt" preferably refers to a salt obtained from a compound according to the invention and a physiologically acceptable acid or base.

According to the invention, the compound according to the invention may be present in any possible form including solvates, cocrystals and polymorphs. For the purposes of this specification, the term "solvate" preferably refers to an adduct of (i) a compound according to the invention and/or a physiologically acceptable salt thereof with (ii) distinct molecular equivalents of one or more solvents.

Further, the compound according to the invention may be present in form of the racemate, enantiomers, diastereomers, tautomers or any mixtures thereof.

The compounds according to the invention may have one or more stereocenter. The person skilled in art knows by looking at a chemical structure whether the depicted compound has one or more stereocenters or not.

For some compounds according to the invention that have one or more stereocenters and which chemical structures are disclosed in the examples of the present application, the chemical structure includes bold bonds and/or hashed bonds to indicate the relative structural orientation of those substituents connected by the bold bonds and/or hashed bonds to the superior structure. If the bold bonds and/or hashed bonds are depicted in form of a wedge, the absolute stereochemical configuration of the compound is known and thereby indicated. If the bold bonds and/or hashed bonds are depicted as a straight bond (i.e. no wedge), the absolute stereochemical configuration of the compound has not been determined. In that case, the bold bonds and/or hashed bonds merely serve to indicate that this particular compound is present as one enantiomer or one diastereomer (e.g. cis-diastereomer (i.e. mixture of two cis-enantiomers) or trans-diastereomer (i.e. mixture of two trans-enantiomers)). All compounds according to the invention that have one or more stereocenters but which chemical structures disclosed in the examples of the present application do not include bold bonds and/or hashed bonds, are present as a mixture of the respective stereoisomers.

The invention also includes isotopic isomers of a compound of the invention, wherein at least one atom of the compound is replaced by an isotope of the respective atom which is different from the naturally predominantly occurring isotope, as well as any mixtures of isotopic isomers of such a compound. Preferred isotopes are $^{2}H$ (deuterium), $^{3}H$ (tritium), $^{13}C$ and $^{14}C$. Isotopic isomers of a compound of the invention can generally be prepared by conventional procedures known to a person skilled in the art.

According to the invention, the terms "$C_{1-6}$-alkyl" and "$C_{1-4}$-alkyl" preferably mean acyclic and preferably saturated hydrocarbon residues, which can be linear (i.e. unbranched) or branched and which can be unsubstituted or mono- or polysubstituted (e.g. di- or trisubstituted), and which contain 1 to 6 (i.e. 1, 2, 3, 4, 5 or 6) and 1 to 4 (i.e. 1, 2, 3 or 4) carbon atoms, respectively. Preferably, $C_{1-6}$-alkyl and $C_{1-4}$-alkyl are saturated. Preferred $C_{1-6}$-alkyl groups are selected from the group consisting of methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl, n-hexyl, 2-hexyl, 3-hexyl, 2-methylpentyl, 4-methylpentyl, 4-methylpent-2-yl, 2-methylpent-2-yl, 3,3-dimethylbutyl, 3,3-dimethylbut-2-yl, 3-methylpentyl, 3-methylpent-2-yl and 3-methylpent-3-yl; more preferably methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl, n-hexyl. Particularly preferred $C_{1-6}$-alkyl groups are selected from $C_{1-4}$-alkyl groups. Preferred $C_{1-4}$-alkyl groups are selected from the group consisting of methyl, ethyl, n-propyl, 2-propyl, n-butyl, isobutyl, sec-butyl and tert-butyl.

According to the invention, the terms "$C_{1-6}$-alkylene" and "$C_{1-4}$-alkylene" relate to linear or branched and preferably saturated aliphatic residues which can be unsubstituted or mono- or polysubstituted (e.g. di- or trisubstituted) and which are preferably selected from the group consisting of $CH_2$, $CH(CH_3)$, $CH_2CH_2$, $CH_2CH(CH_3)$, $CH(CH_3)CH_2$, $CH_2CH_2CH_2$, $CH(CH_3)CH_2CH_2$, $CH_2CH(CH_3)CH_2$, $CH_2CH_2CH(CH_3)$, $CH_2CH_2CH_2CH_2$, $CH(CH_3)CH_2CH_2CH_2$, $CH_2CH(CH_3)CH_2CH_2$, $CH_2CH_2CH(CH_3)CH_2$, $CH_2CH_2CH_2CH(CH_3)$, $CH_2CH_2CH_2CH_2CH_2$, $CH(CH_3)CH_2CH_2CH_2CH_2$, $CH_2CH(CH_3)CH_2CH_2CH_2$, $CH_2CH_2CH(CH_3)CH_2CH_2$, $CH_2CH_2CH_2CH(CH_3)CH_2$, $CH_2CH_2CH_2CH_2CH(CH_3)$, and $CH_2CH_2CH_2CH_2CH_2CH_2$; more preferably $CH_2$, $CH(CH_3)$, $CH_2CH_2$, $CH_2CH(CH_3)$ and $CH(CH_3)CH_2$, most preferably $CH_2$ and $CH(CH_3)$, and in particular $CH_2$. Preferably, $C_{1-6}$-alkylene is selected from $C_{1-4}$-alkylene.

According to the invention, the terms "$C_{3-10}$-cycloalkyl" and "$C_{3-6}$-cycloalkyl" preferably mean monocyclic aliphatic hydrocarbons containing 3, 4, 5, 6, 7, 8, 9 or 10 carbon atoms as ring members and 3, 4, 5 or 6 carbon atoms as ring members, respectively, wherein the hydrocarbons in each case can be saturated or unsaturated (but not aromatic), unsubstituted or mono- or polysubstituted.

Preferably, $C_{3-10}$-cycloalkyl and $C_{3-6}$-cycloalkyl are saturated. The $C_{3-10}$-cycloalkyl and $C_{3-6}$-cycloalkyl can be bound to the respective superordinate general structure via any desired and possible ring member of the cycloalkyl group. The $C_{3-10}$-cycloalkyl and $C_{3-6}$-cycloalkyl are not condensed with further ring systems and are not bridged.

Preferred $C_{3-10}$-cycloalkyl groups are selected from the group consisting of cyclopropyl, cyclobutyl, cyclopentyl, cyclohexyl, cycloheptyl, cyclooctyl, cyclononyl, and cyclodecyl. Preferred $C_{3-10}$-cycloalkyl groups are selected from $C_{3-6}$-cycloalkyl groups.

According to the invention, the terms "4 to 10-membered heterocycloalkyl" and "4 to 6-membered heterocycloalkyl" preferably mean monocyclic, heterocycloaliphatic saturated or unsaturated (but not aromatic) residues having 4 to 10, i.e. 4, 5, 6, 7, 8, 9 or 10 ring members and 4 to 6, i.e. 4, 5 or 6 ring members, respectively, wherein in each case at least one, if appropriate also two or three carbon atoms are replaced by a heteroatom or a heteroatom group each selected independently of one another from the group consisting of O, S, S(=O), $S(=O)_2$, N, NH and N($C_{1-4}$-alkyl) such as $N(CH_3)$, wherein the carbon atoms of the ring can be unsubstituted or mono- or polysubstituted. Preferably, the 4 to 10-membered heterocycloalkyl and the 4 to 6-membered heterocycloalkyl contain only one heteroatom or heteroatom group within the ring.

Preferably, 4 to 10-membered heterocycloalkyl and 4 to 6-membered heterocycloalkyl are saturated. The 4 to 10-membered heterocycloalkyl and 4 to 6-membered heterocycloalkyl are not condensed with further ring systems and are not bridged.

The 4 to 10-membered heterocycloalkyl and the 4 to 6-membered heterocycloalkyl group can be bound to the superordinate general structure via any desired and possible ring member of the heterocycloaliphatic residue if not indicated otherwise. In a preferred embodiment, 4 to 10-membered heterocycloalkyl and 4 to 6-membered heterocycloalkyl are bound to the superordinate general structure via a carbon atom.

Preferred 4 to 10-membered heterocycloalkyl groups are selected from the group consisting of oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, azepanyl, dioxepanyl, oxazepanyl, diazepanyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, piperazinyl, N-methylpyridinonyl, pyrazolidinyl, pyranyl; dihydroquinolinyl, dihydroisoquinolinyl, dihydroindolinyl, dihydroisoindolyl, tetrahydroquinolinyl, tetrahydroisoquinolinyl and tetrahydroindolinyl; more preferably oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, and tetrahydropyrrolyl.

Preferred 4 to 6-membered heterocycloalkyl groups are selected from the group consisting of oxetanyl, tetrahydrofuranyl, tetrahydropyranyl, morpholinyl, thiomorpholinyl, tetrahydrothiopyranyl, tetrahydrothiopyranyl 1,1-dioxide, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, N-methylpyridinonyl, pyrazolidinyl, and pyranyl; more preferably oxetanyl, tetrahydrofuranyl, tetrahydropyranyl, morpholinyl, thiomorpholinyl, tetrahydrothiopyranyl, tetrahydrothiopyranyl 1,1-dioxide, piperidinyl, piperidinonyl, azetidinyl, pyrrolidinyl, pyrrolidinonyl, 4-methylpiperazinyl, morpholinonyl, dioxanyl, piperazinyl, and tetrahydropyrrolyl.

According to the invention, the term "5 to 11-membered spiroalkyl" means spirocyclic aliphatic hydrocarbons containing 5, 6, 7, 8, 9, 10 or 11 carbon atoms as ring members, wherein the hydrocarbons in each case can be saturated or unsaturated (but not aromatic), unsubstituted or mono- or polysubstituted. The spirocyclic system shares a common carbon atom connecting the rings. For the purpose of the specification, the term "spiroalkyl" encompasses dispirocyclic systems, i.e. three rings, whereas two of these three rings share a common carbon atom connecting these rings.

Preferably, 5 to 11-membered spiroalkyl is saturated. The 5 to 11-membered spiroalkyl can be bound to the respective superordinate general structure via any desired and possible ring member of the 5 to 11-membered spiroalkyl group.

Preferred 5 to 11-membered spiroalkyl groups are selected from the group consisting of spiro[2.2]pentyl, spiro[2.3]hexyl, spiro[2.4]heptyl, spiro[3.3]heptyl, spiro[2.5]octyl, spiro[3.4]octyl, spiro[4.4]nonyl, spiro[3.5]nonyl, spiro[4.5]decyl, and dispiro[2.0.2.1]heptyl.

According to the invention, the term "5 to 11-membered heterospiroalkyl" preferably means spirocyclic heteroaliphatic hydrocarbons containing 5, 6, 7, 8, 9, 10 or 11 ring members, wherein in each case at least one, if appropriate also two or three carbon atoms are replaced by a heteroatom or a heteroatom group each selected independently of one another from the group consisting of O, S, S(=O), $S(=O)_2$, N, NH and $N(C_{1-4}$-alkyl) such as $N(CH_3)$, wherein the carbon atoms of the ring can be unsubstituted or mono- or polysubstituted. Preferably, the 5 to 11-membered heterospiroalkyl contains only one heteroatom or heteroatom group within the ring.

Preferably, 5 to 11-membered heterospiroalkyl is saturated. The 5 to 11-membered heterospiroalkyl can be bound to the respective superordinate general structure via any desired and possible ring member of the 5 to 11-membered heterospiroalkyl group.

Preferred 5 to 11-membered heterospiroalkyl groups are selected from the group consisting of oxaspiro[3.2]hexyl, azaspiro[3.2]hexyl, oxaspiro[3.3]heptyl, azaspiro[3.3]heptyl, oxaspiro[4.2]heptyl, azaspiro[4.2]heptyl, oxaspiro[3.4]octyl, azaspiro[3.4]octyl, oxaspiro[4.3]octyl, azaspiro[4.3]octyl, oxaspiro[5.2]octyl, azaspiro[5.2]octyl, oxaspiro[3.5]nonyl, azaspiro[3.5]nonyl, oxaspiro[4.4]nonyl, azaspiro[4.4]nonyl, oxaspiro[5.3]nonyl, and azaspiro[5.3]nonyl.

According to the invention, the term "4 to 11-membered bicycloalkyl" preferably means bicyclic aliphatic hydrocarbons containing 4, 5, 6, 7, 8, 9, 10 or 11 carbon atoms as ring members, wherein the hydrocarbons in each case can be saturated or unsaturated (but not aromatic), unsubstituted or mono- or polysubstituted. The bicyclic system may share a common bond (annealed rings) or may be bridged.

Preferably, 4 to 11-membered bicycloalkyl is saturated. The 4 to 11-membered bicycloalkyl can be bound to the respective superordinate general structure via any desired and possible ring member of the 4 to 11-membered bicycloalkyl group.

Preferred 4 to 11-membered bicycloalkyl groups are selected from the group consisting of bicycle [1.1.0]butyl, bicylo[1.1.1]pentyl, bicyclo[2.1.0]pentyl, bicyclo[3.1.0]hexyl, bicyclo[2.2.0]hexyl, bicyclo[2.1.1]hexyl, bicyclo[3.1.1]heptyl,

bicyclo[4.1.0]heptyl, bicyclo[2.2.1]heptyl, and bicyclo[3.3.0]octyl.

According to the invention, the term "5 to 11-membered heterobicycloalkyl" preferably means bicyclic heteroaliphatic saturated or unsaturated (but not aromatic) hydrocarbons containing 5, 6, 7, 8, 9, 10 or 11 ring members, wherein in each case at least one, if appropriate also two or three carbon atoms are replaced by a heteroatom or a heteroatom group each selected independently of one another from the group consisting of O, S, S(=O), $S(=O)_2$, N, NH and $N(C_{1-4}$-alkyl) such as $N(CH_3)$, wherein the carbon atoms of the ring can be unsubstituted or mono- or polysubstituted. The bicyclic system may share a common bond (annealed rings) or may be bridged.

Preferably, 5 to 11-membered heterobicycloalkyl is saturated. The 5 to 11-membered heterobicycloalkyl can be bound to the respective superordinate general structure via any desired and possible ring member of the 5 to 11-membered heterobicycloalkyl group.

Preferred 5 to 11-membered heterobicycloalkyl group are selected from the group consisting of oxabicyclo[2.1.1]hexyl, oxabicyclo[2.2.1]heptyl, oxabicyclo[3.1.1]heptyl, and oxabicyclo[3.2.1]octyl.

According to the invention, the term "5- to 10-membered heteroaryl" and "5- or 6-membered heteroaryl", respectively, preferably means a 5 to 10-membered or 5- or 6-membered cyclic aromatic residue containing at least 1, if appropriate also 2, 3, 4 or 5 heteroatoms, wherein the heteroatoms are each selected independently of one another from the group S, N and O and the heteroaryl residue can be unsubstituted or mono- or polysubstituted, if not indicated otherwise. In the case of substitution on the heteroaryl, the substituents can be the same or different and be in any desired and possible position of the heteroaryl. The binding to the superordinate general structure can be carried out via any desired and possible ring member of the heteroaryl residue if not indicated otherwise. Preferably, the 5- to 10-membered heteroaryl and 5- or 6-membered heteroaryl is bound to the suprordinate general structure via a carbon atom of the heterocycle. The heteroaryl can also be condensed with a further ring system, e.g. saturated or (partially) unsaturated (hetero)cyclic, aromatic or heteroaromatic ring systems, which can in turn be unsubstituted or mono- or polysubstituted, if not indicated otherwise.

Preferably, the 5- to 10-membered heteroaryl is selected from the group consisting of pyridyl (i.e. 2-pyridyl, 3-pyridyl, 4-pyridyl), pyrazolyl, pyrrolo[2,3-b]pyridyl, pyridonyl (pyridinonyl), thienyl (thiophenyl), thiazolyl, 2,3-dihydrobenzo[d]isothiazolyl 1,1-dioxide, isoindolinonyl, pyrimidinyl, pyrazinyl, pyridazinyl, isoxazolyl, triazolyl, oxazolyl, oxadiazolyl, indazolyl, pyrazolopyridyl, pyrrolyl, imidazolyl, isothiazolyl, furanyl, thiadiazolyl, N-methylpyridinonyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indolizinyl, indolyl, isoquinolinyl, naphthyridinyl, phenazinyl, phenothiazinyl, phthalazinyl, purinyl, phenazinyl, tetrazolyl and triazinyl; more preferably pyridyl, pyrazolyl, pyrrolo[2,3-b]pyridyl, pyridonyl, thienyl, thiazolyl, 2,3-dihydrobenzo[d]isothiazolyl 1,1-dioxide, isoindolinonyl, pyrimidinyl, pyrazinyl, pyridazinyl, isoxazolyl, triazolyl, oxazolyl, oxadiazolyl, indazolyl, pyrazolopyridyl, pyrrolyl, imidazolyl, isothiazolyl, furanyl, and thiadiazolyl. Particularly preferably, 5 to 10-membered heteroaryl is selected from 5- or 6-membered heteroaryl.

Preferably, the 5- or 6-membered heteroaryl is selected from the group consisting of pyridyl, pyrazolyl, pyridonyl, thienyl, thiazolyl, pyrimidinyl, pyrazinyl, pyridazinyl, isoxazolyl, triazolyl, oxazolyl, oxadiazolyl, pyrrolyl, imidazolyl, isothiazolyl, furanyl, thiadiazolyl, N-methylpyridinonyl, tetrazolyl and triazinyl; more preferably pyridyl, pyrazolyl, pyridonyl, thienyl, thiazolyl, pyrimidinyl, pyrazinyl, pyridazinyl, isoxazolyl, triazolyl, oxazolyl, oxadiazolyl, pyrrolyl, imidazolyl, isothiazolyl, furanyl, and thiadiazolyl.

As pyridones and 2-hydroxypyridine are tautomers, for the purpose of the specification the definition of pyridines that may optionally be substituted with OH covers pyridones.

In connection with the terms "$C_{1-6}$-alkyl", "$C_{1-4}$-alkyl", "$C_{1-6}$-alkylene", "$C_{1-4}$-alkylene", "$C_{3-10}$-cycloalkyl" "$C_{3-6}$-cycloalkyl", "4 to 11-membered bicycloalkyl", "5 to 11-membered spiroalkyl or dispiroalkyl", "4 to 10-membered heterocycloalkyl", "4 to 6-membered heterocycloalkyl", and "5 to 11-membered heterobicycloalkyl", the term "substituted" refers in the sense of the invention, with respect to the corresponding residues or groups, to the single substitution (monosubstitution) or multiple substitution (polysubstitution), e.g. disubstitution, trisubstitution or tetrasubstitution; more preferably to monosubstitution, disubstitution or trisubstitution; of one or more hydrogen atoms each independently of one another by at least one substituent. In case of a multiple substitution, i.e. in case of polysubstituted residues, such as di- or trisubstituted residues, these residues may be polysubstituted either on different or on the same atoms, for example trisubstituted on the same carbon atom, as in the case of $CF_3$, $CH_2CF_3$ or disubstituted as in the case of 1,1-difluorocyclohexyl, or at various points, as in the case of 1-chloro-3-fluorocyclohexyl. The multiple substitution can be carried out using the same or using different substituents.

If a residue occurs multiply within a molecule, then this residue can have respectively different meanings for various substituents: if, for example, both R4 and R5 denote $C_{1-6}$-alkyl, then $C_{1-6}$-alkyl can e.g. represent methyl for R4 and can represent 2-propyl for R5.

According to the invention, $C_{1-6}$-alkyl, $C_{1-6}$-alkylene, $C_{3-10}$-cycloalkyl, $C_{3-6}$-cycloalkyl, 4 to 10-membered heterocycloalkyl, 4 to 6-membered heterocycloalkyl, 4 to 11-membered bicycloalkyl, 5 to 11-membered heterobicycloalkyl, 5 to 11-membered spiroalkyl, and 5 to 11-membered heterospiroalkyl, in each case independently from one another are

unsubstituted or substituted with one, two, three, four or more substituents independently from one another selected from the group consisting of F, Cl, CN, $C_{1-6}$-alkyl, $C_{1-6}$-alkylene-OH, $C_{1-6}$-alkylene-$OCH_3$, $CF_3$, $CF_2H$, $CFH_2$, C(O)-$C_{1-6}$-alkyl, OH, =O, $OCF_3$, $OCF_2H$, $OCFH_2$, O-$C_{1-6}$-alkyl, $C_{1-4}$-alkylene-O-$C_{1-4}$-alkylene-O-$CH_3$, $C_{0-4}$-alkylene-O-($C_{1-4}$-alkylene-O)$_{1-4}$-$CH_3$, $NH_2$, NH-$C_{1-6}$-alkyl, or N($C_{1-6}$-alkyl)$_2$; preferably $CH_3$, $CF_3$, $CF_2H$, $CFH_2$, and $OCH_3$; more preferably $CF_3$, $CF_2H$, and $CFH_2$; and most preferably $CF_3$.

Preferably, $C_{1-6}$-alkylene groups are unsubstituted.

According to the invention, phenyl, 5 to 10-membered heteroaryl and 5 or 6-membered heteroaryl in each case independently from one another are unsubstituted or mono- or polysubstituted with one or more substituents selected from the group consisting of F, Cl, Br, CN, $C_{1-6}$-alkyl, $C_{3-6}$-cycloalkyl, $CF_3$, $CF_2H$, $CFH_2$, $C_{1-6}$-alkylene-$CF_3$, $C_{1-6}$-alkylene-$CF_2H$, $C_{1-6}$-alkylene-$CFH_2$, $C_{1-6}$-alkylene-OH, $C_{1-6}$-alkylene-$OCH_3$, C(O)-$C_{1-6}$-alkyl, $OCF_3$, $OCF_2H$, $OCFH_2$, and O-$C_{1-6}$-alkyl; preferably F, $CH_3$, $CF_3$, $CF_2H$, $CFH_2$, and $OCH_3$; more preferably F, $CF_3$, $CF_2H$, and $CFH_2$; and most preferably F.

According to the invention, **L** represents $CH_2$, $CH(CH_3)$ or $CH(CH_2CH_3)$.

In preferred embodiments, **L** represents $CH_2$ or $CH(CH_3)$.

According to the invention, **R1** represents $C_{3-10}$-cycloalkyl, 4 to 10-membered heterocycloalkyl, 5 to 11-membered spiroalkyl, 5 to 11-membered heterospiroalkyl, 4 to 11-membered bicycloalkyl, or 5 to 11-membered heterobicycloalkyl.

In preferred embodiments, **R1** represents $C_{3-10}$-cycloalkyl, unsubstituted or substituted with one, two, three, four, or more substituents independently from one another selected from F, CN, $CH_3$, $CHF_2$, $CF_3$, OH, and $OCH_3$.

Preferably, **R1** represents cyclobutyl, cyclopentyl, cyclohexyl, or cycloheptyl, in each case optionally substituted with one, two or three substituents independently from one another selected from F, CN, $CH_3$, $CF_3$, OH, and $OCF_3$.

More preferably, **R1** is selected from cyclopropyl, fluoro cyclopropyl, cyano cyclopropyl, methyl cyclopropyl, trifluoromethyl cyclopropyl, trifluoromethoxy cyclopropyl, hydroxy cyclopropyl, difluoro cyclopropyl, dimethyl cyclopropyl, methyl hydroxy cyclopropyl, methyl difluoro cyclopropyl, cyclobutyl, fluoro cyclobutyl, cyano cyclobutyl, methyl cyclobutyl, trifluoromethyl cyclobutyl, trifluoromethoxy cyclobutyl, hydroxy cyclobutyl, difluoro cyclobutyl, dimethyl cyclobutyl, methyl hydroxy cyclobutyl, methyl difluoro cyclobutyl, cyclopentyl, fluoro cyclopentyl, cyano cyclopentyl, methyl cyclopentyl, trifluoromethyl cyclopentyl, trifluoromethoxy cyclopentyl, hydroxy cyclopentyl, difluoro cyclopentyl, dimethyl cyclopentyl, methyl hydroxy cyclopentyl, methyl difluoro cyclopentyl, cyclohexyl, fluoro cyclohexyl, cyano cyclohexyl, methyl cyclohexyl, trifluoromethyl cyclohexyl, trifluoromethoxy cyclohexyl, hydroxy cyclohexyl, difluoro cyclohexyl, dimethyl cyclohexyl, methyl hydroxy cyclohexyl, methyl difluoro cyclohexyl, cycloheptyl, fluoro cycloheptyl, cyano cycloheptyl, methyl cycloheptyl, trifluoromethyl cycloheptyl, trifluoromethoxy cycloheptyl, hydroxy cycloheptyl, difluoro cycloheptyl, dimethyl cycloheptyl, methyl hydroxy cycloheptyl, and methyl difluoro cycloheptyl.

Still more preferably, **R1** represents cyclobutyl, dimethyl cyclobutyl, difluoro cyclobutyl, trifluoromethyl cyclobutyl, trifluoromethoxy cyclobutyl, methyl difluoro cyclobutyl, cyclopentyl, fluoro cyclopentyl, difluoro cyclopentyl, methyl difluoro cyclopentyl, trifluoromethyl cyclopentyl, cyclohexyl, methyl cyclohexyl, fluoro cyclohexyl, difluoro cyclohexyl, or cycloheptyl.

In preferred embodiments, **R1** represents $C_{3-10}$-cycloalkyl, substituted with $CH_3$ and optionally substituted with one, two, three, four, or more substituents independently from one another selected from F, $OCH_3$, CN, $CHF_2$ and $CF_3$, wherein the carbon atom of $C_{3-10}$-cycloalkyl, which is substituted with $CH_3$, is linked to L. Preferred representatives include but are not limited to methyl difluoro cyclobutyl and methyl difluoro cyclopentyl.

In further preferred embodiments, **R1** represents 4 to 10-membered heterocycloalkyl, unsubstituted or substituted with one, two, three, four, or more substituents independently from one another selected from F, CN, $CH_3$, $CHF_2$, $CF_3$, OH, and $OCH_3$.

Preferably, **R1** is selected from oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, azepanyl, dioxepanyl, oxazepanyl, diazepanyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, piperazinyl, N-methylpyridinonyl, pyrazolidinyl, pyranyl; dihydroquinolinyl, dihydroisoquinolinyl, dihydroindolinyl, dihydroisoindolyl, tetrahydroquinolinyl, tetrahydroisoquinolinyl and tetrahydroindolinyl, in each case unsubstituted or substituted with one, two, three, four, or more substituents independently from one another selected from F, and $CH_3$.

More preferably, **R1** is selected from tetrahydrofuranyl, methyl tetrahydrofuranyl, dimethyl tetrahydrofuranyl, fluoro tetrahydrofuranyl, difluoro tetrahydrofuranyl, tetrahydropyranyl, methyl tetrahydropyranyl, dimethyl tetrahydropyranyl, fluoro tetrahydropyranyl, difluoro tetrahydropyranyl, oxepanyl, methyl oxepanyl, dimethyl oxepanyl, fluoro oxepanyl, and difluoro oxepanyl.

Still more preferably, **R1** represents tetrahydrofuranyl, tetrahydropyranyl, methyl tetrahydropyranyl, dimethyl tetrahydropyranyl, difluoro tetrahydropyranyl, or oxepanyl.

In other preferred embodiments, **R1** represents 5 to 11-membered spiroalkyl or dispiroalkyl, unsubstituted or

substituted with one, two, three, four, or more substituents independently from one another selected from F, $CH_3$, $OCH_3$, CN, $CHF_2$ and $CF_3$.

Preferably, **R1** is selected from spiro[2.2]pentyl, methyl spiro[2.2]pentyl, fluoro spiro[2.2]pentyl, difluoro spiro[2.2]pentyl, methyl difluoro spiro[2.2]pentyl, spiro[2.3]hexyl, methyl spiro[2.3]hexyl, fluoro spiro[2.3]hexyl, difluoro spiro[2.3]hexyl, methyl difluoro spiro[2.3]hexyl, spiro[3.3]heptyl, methyl spiro[3.3]heptyl, fluoro spiro[3.3]heptyl, difluoro spiro[3.3]heptyl, methyl difluoro spiro[3.3]heptyl, and dispiro[2.0.2.1]heptyl.

More preferably, **R1** is selected from spiro[2.2]pentyl, difluoro spiro[2.2]pentyl, spiro[2.3]hexyl, methyl spiro[2.3]hexyl, fluoro spiro[2.3]hexyl, methyl difluoro spiro[2.3]hexyl, spiro[3.3]heptyl, and dispiro[2.02.1]heptyl.

In still further preferred embodiments, **R1** represents 5 to 11-membered heterospiroalkyl, unsubstituted or substituted with one, two, three, four, or more substituents independently from one another selected from F, CN, $CH_3$, $CHF_2$, $CF_3$, OH, and $OCH_3$.

Preferably, **R1** is selected from oxaspiro[3.2]hexyl, azaspiro[3.2]hexyl, oxaspiro[3.3]heptyl, azaspiro[3.3]heptyl, oxaspiro[4.2]heptyl, azaspiro[4.2]heptyl, oxaspiro[3.4]octyl, azaspiro[3.4]octyl, oxaspiro[4.3]octyl, azaspiro[4.3]octyl, oxaspiro[5.2]octyl, azaspiro[5.2]octyl, oxaspiro[3.5]nonyl, azaspiro[3.5]nonyl, oxaspiro[4.4]nonyl, azaspiro[4.4]nonyl, oxaspiro[5.3]nonyl, and azaspiro[5.3]nonyl.

More preferably, **R1** represents oxaspiro[3.3]heptyl.

In yet further preferred embodiments, **R1** represents 4 to 11-membered bicycloalkyl, unsubstituted or substituted with one, two, three, four, or more substituents independently from one another selected from F, $CH_3$, $OCH_3$, CN, $CHF_2$ and $CF_3$.

Preferably, **R1** is selected from bicylo[1.1.1]pentyl, methyl bicylo[1.1.1]pentyl, methoxy bicylo[1.1.1]pentyl, difluoromethyl bicylo[1.1.1]pentyl, trifluoromethyl bicylo[1.1.1]pentyl, fluoro bicylo[1.1.1]pentyl, difluoro bicylo[1.1.1]pentyl, methyl difluoro bicylo[1.1.1]pentyl, trifluoromethyl difluoro bicylo[1.1.1]pentyl, bicyclo[2.1.0]pentyl, methyl bicyclo[2.1.0]pentyl, trifluoromethyl bicyclo[2.1.0]pentyl, fluoro bicyclo[2.1.0]pentyl, difluoro bicyclo [2.1.0]pentyl, methyl difluoro bicyclo[2.1.0]pentyl, trifluoromethyl difluoro bicyclo[2.1.0]pentyl, bicyclo[3.1.0]hexyl, methylbicyclo[3.1.0]hexyl, trifluoromethylbicyclo[3.1.0]hexyl, fluoro bicyclo[3.1.0]hexyl, difluoro bicyclo[3.1.0]hexyl, methyl difluoro bicyclo[3.1.0]hexyl, trifluoromethyl difluoro icyclo[3.1.0]hexyl, bicyclo[2.2.0]hexyl, methyl bicyclo[2.2.0]hexyl, trifluoromethyl bicyclo[2.2.0]hexyl, fluoro bicyclo[2.2.0]hexyl, difluoro bicyclo[2.2.0]hexyl, methyl difluoro bicyclo[2.2.0]hexyl, trifluoromethyl difluoro bicyclo[2.2.0]hexyl, bicyclo[2.1.1]hexyl, methyl bicyclo[2.1.1]hexyl, trifluoromethyl bicyclo[2.1.1]hexyl, fluoro bicyclo[2.1.1]hexyl, difluoro bicyclo[2.1.1]hexyl, fluoro bicyclo[2.1.1]hexyl, difluoro bicyclo[2.1.1] hexyl, methyl difluoro bicyclo[2.1.1]hexyl, trifluoromethyl difluoro bicyclo[2.1.1]hexyl, bicyclo[4.1.0]heptyl, methyl bicyclo[4.1.0]heptyl, trifluoromethyl bicyclo[4.1.0]heptyl, fluoro bicyclo[4.1.0]heptyl, difluoro bicyclo[4.1.0]heptyl, methyl difluoro bicyclo[4.1.0]heptyl, trifluoromethyl difluoro bicyclo[4.1.0]heptyl, bicyclo[2.2.1]heptyl, methyl bicyclo[2.2.1]heptyl, trifluoromethyl bicyclo[2.2.1]heptyl, fluoro bicyclo[2.2.1]heptyl, difluoro bicyclo[2.2.1]heptyl, methyl difluoro bicyclo[2.2.1]heptyl, trifluoromethyl difluoro bicyclo[2.2.1]heptyl, bicyclo[3.3.0]octyl, methyl bicyclo[3.3.0]octyl, trifluoromethyl bicyclo[3.3.0]octyl, fluoro bicyclo [3.3.0]octyl, difluoro bicyclo [3.3.0]octyl, methyl difluoro bicyclo[3.3.0]octyl, and trifluoromethyl difluoro bicyclo[3.3.0]octyl.

More preferably, **R1** is selected from bicylo[1.1.1]pentyl, trifluoromethyl-bicylo[1.1.1]pentyl, bicyclo[2.1.0]pentyl, difluoro bicyclo[2.1.0]pentyl, bicyclo[3.1.0]hexyl, difluoro bicyclo[3.1.0]hexyl, methyl difluoro bicyclo[3.1.0]hexyl, trifluoromethyl difluoro bicyclo[3.1.0]hexyl, bicyclo[2.2.0]hexyl, methyl bicyclo[2.2.0]hexyl, bicyclo[2.1.1]hexyl, fluoro bicyclo[2.1.1]hexyl, difluoro bicyclo[2.1.1]hexyl, bicyclo[4.1.0]heptyl, difluoro bicyclo[4.1.0]heptyl, bicyclo[2.2.1]heptyl, fluoro bicyclo[2.2.1]heptyl, bicyclo[3.3.0]octyl, and fluoro bicyclo [3.3.0]octyl.

In other preferred embodiments, **R1** represents 5 to 11-membered heterobicycloalkyl, unsubstituted or substituted with one, two, three, four, or more substituents independently from one another selected from F, $CH_3$, $OCH_3$, CN, $CHF_2$ and $CF_3$.

Preferably, **R1** represents 2-oxa-bicyclo[2.1.1]hexyl or methyl 2-oxa-bicyclo[2.1.1]hexyl.

According to the invention, R2 represents H or $C_{1-6}$-alkyl.

In preferred emboidments, R2 represents H.

According to the invention, **X** represents phenyl, or 5 to 9-membered heteroaryl.

In preferred embodiments, **X** represents phenyl, fluorophenyl, pyridyl, pyrazolyl, pyrrolo[2,3-b]pyridyl, pyridonyl, thienyl, thiazolyl, 2,3-dihydrobenzo[d]isothiazolyl 1,1-dioxide, isoindolinonyl, pyrimidinyl, pyrazinyl, pyridazinyl, isoxazolyl, triazolyl, oxazolyl, oxadiazolyl, indazolyl, pyrazolopyridyl, pyrrolyl, imidazolyl, isothiazolyl, furanyl, thiadiazolyl, N-methylpyridinonyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indolizinyl, indolyl, isoquinolinyl, naphthyridinyl, phenazinyl, phenothiazinyl, phthalazinyl, purinyl, phenazinyl, tetrazolyl or triazinyl.

Preferably, **X** represents phenyl, thienyl, thiazolyl, pyrazolyl, pyridyl, or pyrimidinyl.

According to the invention, **R3** represents S(O)-$C_{1-6}$-alkyl, S(O)-($C_{3-6}$-cycloalkyl), S(O)-(4 to 6-membered heterocycloalkyl), S(O)-phenyl, S(O)-(5 or 6-membered heteroaryl), $S(O)_2$-$C_{1-6}$-alkyl, $S(O)_2$-($C_{3-6}$-cycloalkyl), $S(O)_2$-(4 to

6-membered heterocycloalkyl), $S(O)_2$-phenyl, $S(O)_2$-(5 or 6-membered heteroaryl), S(O)-$NH_2$, S(O)-N(H)($C_{1-6}$-alkyl), S(O)N(H)($C_{3-6}$-cycloalkyl), S(O)N(H)(4 to 6-membered heterocycloalkyl), $S(O)_2$-$NH_2$, $S(O)_2$-N(H)($C_{1-6}$-alkyl), $S(O)_2$N(H)($C_{3-6}$-cycloalkyl), $S(O)_2$N(H)(4 to 6-membered heterocycloalkyl), S(O)-N($C_{1-6}$-alkyl)$_2$, $S(O)_2$-N($C_{1-6}$-alkyl)$_2$, C(O)-$C_{1-6}$-alkyl, C(O)-$NH_2$, C(O)-N(H)($C_{1-6}$-alkyl), C(O)-N(H)($C_{3-6}$-cycloalkyl), C(O)-N($C_{1-6}$-alkyl)$_2$, C(O)-N($C_{1-6}$-alkyl)($C_{3-6}$-cycloalkyl), CN, $C_{1-6}$-alkylene-OH, OH, =O, O-$C_{1-6}$-alkyl, $OCF_3$, $OCF_2H$, O-$C_{3-6}$-cycloalkyl, O-(4 to 6-membered heterocycloalkyl), $NH_2$, N(H)($C_{1-6}$-alkyl), N(H)(C(O)$C_{1-6}$-alkyl), N(H)(C(O)$C_{3-6}$-cycloalkyl), N(H)(C(O)$NH_2$), N(H)($S(O)_2$-$C_{1-6}$-alkyl), N(H)($S(O)_2$-(4 to 6-membered heterocycloalkyl), or S(O)(N**R3a**)**R3b**.

In preferred embodiments, **R3** represents S(O)-$C_{1-6}$-alkyl, $S(O)_2$-$C_{1-6}$-alkyl, S(O)-$NH_2$, S(O)-N(H)($C_{1-6}$-alkyl), S(O)N(H)($C_{3-6}$-cycloalkyl), C(O)-$NH_2$, CN, $C_{1-6}$-alkylene-OH, OH, =O, O-$C_{1-6}$-alkyl, $NH_2$, or S(O)(N**R3a**)**R3b**, wherein **R3a** represents H or $C_{1-6}$-alkyl, and **R3b** represents $C_{1-6}$-alkyl.

Preferably, **R3** represents $SOCH_3$, $SO_2CH_3$, $SO_2NH_2$, $SO_2NHCH_3$, $SO_2NH$-cyclopropyl, S(O)(NH)$CH_3$; S(O)($NCH_3$)$CH_3$, CN, $CONH_2$, $C(CH_3)_2OH$, OH, $OCH_3$, =O, or $NH_2$.

According to the invention, **R4** represents H, F, Cl, Br, $C_{1-6}$-alkyl, $CF_3$, $CF_2H$, $CFH_2$ or $C_{3-6}$-cycloalkyl.

In preferred embodiments, **R4** represents H, F, $C_{1-6}$-alkyl, $CFH_2$, $CF_2H$, $CF_3$, or $C_{3-6}$-cycloalkyl; preferably H, F, or $CH_3$

According to the invention, **R3** and **R4** may alternatively together with the atoms to which they are attached form a 5 to 6-membered heterocycloalkyl.

Preferably, **X** represents phenyl and **R3** and **R4** form a 5 to 6-membered heterocycloalkyl, more preferably dihydroisoindolyl or 2,3-dihydro-1,2-benzothiazolyl.

According to the invention, **R5** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl.

In preferred embodiments, **R5** represents H, F, Cl, CN, $C_{1-6}$-alkyl, $CHF_2$, $CH_2F$, $CF_3$, O-$C_{1-6}$-alkyl, or $C_{3-10}$-cycloalkyl.

Preferably, R5 represents H, F, Cl, $CH_3$, $CHF_2$, $CF_3$, $OCH_3$, or cyclopropyl.

According to the invention, **A1** represents N or C**R6**, wherein **R6** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl.

In preferred embodiments, **A1** represents N or C**R6**; wherein **R6** represents H, F, Cl, CN, $C_{1-6}$-alkyl, $CHF_2$, $CH_2F$, $CF_3$, O-$C_{1-6}$-alkyl, or $C_{3-10}$-cycloalkyl.

Preferably, R6 represents H, $CH_3$, or $CF_3$.

According to the invention, **A2** represents N or C**R7**, wherein **R7** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl.

In preferred embodiments, **A2** represents N or C**R7**; wherein **R7** represents H, F, Cl, CN, $C_{1-6}$-alkyl, $CHF_2$, $CH_2F$, $CF_3$, O-$C_{1-6}$-alkyl, or $C_{3-10}$-cycloalkyl.

Preferably, **R7** represents H, F, Cl, $CH_3$, or $CF_3$.

According to the invention, **A3** represents N or C**R8**, wherein **R8** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl.

In preferred embodiments, **A3** represents C**R8**; wherein **R8** represents H, F, Cl, CN, $C_{1-6}$-alkyl, $CHF_2$, $CH_2F$, $CF_3$, O-$C_{1-6}$-alkyl, or $C_{3-10}$-cycloalkyl.

Preferably, **R8** represents H, F, Cl, CN, $CH_3$, $CHF_2$, $CF_3$, or cyclopropyl.

In a particularly preferred embodiment, the compound according to the invention is selected from the group consisting of

| Ex. | Name |
|---|---|
| **SC-05** | 2-(cyclohexylmethyl)-6-fluoro-*N*-(2-hydroxypyridin-4-yl)indazole-3-carboxamide |
| **SC-06** | 2-[(4,4-difluorocyclohexyl)methyl]-6-fluoro-*N*-(2-hydroxypyridin-4-yl)indazole-3-carboxamide |
| **SC-10** | 2-(cyclohexylmethyl)-*N*-(2-hydroxypyridin-4-yl)indazole-3-carboxamide |
| **SC-11** | 2-(cyclohexylmethyl)-6-fluoro-*N*-(3-methylsulfonylphenyl)indazole-3-carboxamide |
| **SC-12** | 2-(cyclobutylmethyl)-*N*-(2-hydroxypyridin-4-yl)indazole-3-carboxamide |
| **SC-13** | 2-(cyclohcptylmethyl)-*N*-(2-hydroxypyridin-4-yl)indazole-3-carboxamide |
| **SC-14** | *N*-(3-methylsulfonylphenyl)-2-(oxan-4-ylmethyl)indazole-3-carboxamide |
| **SC-15** | 2-(oxan-4-ylmethyl)-N-(3-sulfamoylphenyl)indazole-3-carboxamide |
| **SC-16** | 2-(oxolan-2-ylmethyl)-*N*-(3-sulfamoylphenyl)indazole-3-carboxamide |
| **SC-17** | *N*-(3-carbamoylphenyl)-2-(cyclohexylmethyl)indazole-3-carboxamide |
| **SC-18** | 2-(cyclohexylmethyl)-*N*-(3-sulfamoylphenyl)indazole-3-carboxamide |
| **SC-19** | 2-(cyclohexylmethyl)-6-fluoro-*N*-[3-[(methylsulfinyl]phenyl]indazole-3-carboxamide |
| **SC-20** | 2-(cyclohexylmethyl)-7-fluoro-*N*-(2-hydroxypyridin-4-yl)indazole-3-carboxamide |

(continued)

| Ex. | Name |
|---|---|
| **SC-21** | *N*-(3-carbamoyl-4-fluorophenyl)-2-(oxan-4-ylmethyl)indazole-3-carboxamide |
| **SC-22** | 2-[(4,4-difluorocyclohexyl)methyl]-*N*-(3-methylsulfonylphenyl)indazole-3-carboxamide |
| **SC-23** | 2-[(4,4-difluorocyclohexyl)methyl]-*N*-(3-sulfamoylphenyl)indazole-3-carboxamide |
| **SC-24** | 2-[(4,4-difluorocyclohexyl)methyl]-*N*-(2-hydroxypyridin-4-yl)indazole-3-carboxamide |
| **SC-25** | *N*-(3-carbamoyl-4-fluorophenyl)-2-[(4,4-difluorocyclohexyl)methyl]indazole-3-carboxamide |
| **SC-26** | 2-(cyclohexylmethyl)-*N*-[3-(2-hydroxypropan-2-yl)phenyl]indazole-3-carboxamide |
| **SC-27** | 2-[(4,4-difluorocyclohexyl)methyl]-6-fluoro-*N*-(3-methylsulfonylphenyl)indazole-3-carboxamide |
| **SC-28** | 2-(cyclohexylmethyl)-6-fluoro-*N*-(3-sulfamoylphenyl)indazole-3-carboxamide |
| **SC-29** | 2-(cyclohaxylmethyl)-7-fluoro-*N*-(3-methylsulfonylphenyl)indazole-3-carboxamide |
| **SC-32** | 2-(cyclohexylmethyl)-5-fluoro-*N*-(2-hydroxypyridin-4-yl)indazole-3-carboxamide |
| **SC-34** | 2-(cyclohexylmethyl)-*N*-(3-fluoro-5-methylsulfonylphenyl)indazole-3-carboxamide |
| **SC-36** | 2-(cyclohexylmethyl)-*N*-(1-methylsulfonylpyrazol-4-yl)indazole-3-carboxamide |
| **SC-37** | 2-(cyclohexylmethyl)-*N*-(3-methylsulfonylphenyl)indazole-3-carboxamide |
| **SC-38** | 2-[(4,4-difluorocyclohexyl)methyl]-6-fluoro-*N*-(3-sulfamoylphenyl)indazole-3-carboxamide |
| **SC-39** | 2-(cyclohexylmethyl)-*N*-(1-methyl-2-oxopyridin-4-yl)indazole-3-carboxamide |
| **SC-42** | 2-(cyclohexylmethyl)-*N*-(2-hydroxypyridin-4-yl)-7-(trifluoromethyl)indazole-3-carboxamide |
| **SC-43** | 2-(cyclohexylmethyl)-*N*-(2-hydroxypyndin-4-yl)-7-methylindazole-3-carboxamide |
| **SC-44** | 2-(cyclohexylmethyl)-*N*-(3-methylsulfonylphenyl)-7-(trifluoromethyl)indazole-3-carboxamide |
| **SC-45** | 2-(cyclohexylmethyl)-7-methyl-*N*-(3-methylsulfonylphenyl)indazole-3-carboxamide |
| **SC-46** | 2-(cyclohexylmethyl)-5-methyl-*N*-(3-methylsulfonylphenyl)indazole-3-carboxamide |
| **SC-47** | 2-(cyclohexylmethyl)-4-fluoro-*N*-(3-methylsulfonylphenyl)indazole-3-carboxamide |
| **SC-48** | 2-(cyclohexylmethyl)-*N*-(2-hydroxypyndin-4-yl)-5-methylmdazole-3-carboxamide |
| **SC-49** | 2-(cyclohexylmethyl)-4-fluoro-*N*-(2-hydroxypyridin-4-yl)indazole-3-carboxamide |
| **SC-50** | *N*-(2-hydroxypyridin-4-yl)-2-(oxolan-3-ylmethyl)indazole-3-carboxamide |
| **SC-53** | *N*-(2-hydroxypyridin-4-yl)-2-(oxan-2-ylmethyl)indazole-3-carboxamide |
| **SC-56** | *N*-(3-methylsulfonylphenyl)-2-(oxolan-3-ylmethyl)indazole-3-carboxamide |
| **SC-58** | *N*-(3-methylsulfonylphenyl)-2-(oxan-2-ylmethyl)indazole-3-carboxamide |
| **SC-60** | 6-fluoro-*N*-(3-methylsulfonylphenyl)-2-(oxan-4-ylmethyl)indazole-3-carboxamide |
| **SC-61** | 2-(2-cyclopentylethyl)-6-fluoro-*N*-(3-methylsulfonylphenyl)indazole-3-carboxamide |
| **SC-62** | 2-[(3,3-difluorocyclobutyl)methyl]-6-fluoro-*N*-(3-methylsulfonylphenyl)indazole-3-carboxamide |
| **SC-64** | 2-(cyclobutylmethyl)-N-(3-methylsulfonylphenyl)indazole-3-carboxamide |
| **SC-65** | *N*-(3-methylsulfonylphenyl)-2-(oxan-3-ylmethyl)indazole-3-carboxamide |
| **SC-67** | 2-(cyclopentylmethyl)-7V-(3-methylsulfonylphenyl)indazole-3-carboxamide |
| **SC-68** | 2-[(3,3-dimethylcyclobutyl)methyl]-6-fluoro-*N*-(3-methylsulfonylphenyl)indazole-3-carboxamide |
| **SC-69** | 2-(cyclohexylmethyl)-*N*-(5-methylsulfonylthiophen-2-yl)indazole-3-carboxamide |
| **SC-70** | 6-fluoro-*N*-(3-methylsulfonylphenyl)-2-(spiro[2.5]octan-6-ylmethyl)indazole-3-carboxamide |
| **SC-71** | 6-fluoro-*N*-(2-hydroxypyridin-4-yl)-2-[[oxan-2-yl]methyl]indazole-3-carboxamide |
| **SC-73** | 2-(cyclopentylmethyl)-*N*-(2-hydroxypyridin-4-yl)indazole-3-carboxamide |
| **SC-74** | 2-(1-cyclohexylethyl)-*N*-(3-methylsulfonylphenyl)indazole-3-carboxamide |
| **SC-76** | *N*-(3-carbamoylphenyl)-2-[(4,4-difluorocyclohexyl)methyl]-6-fluoroindazole-3-carboxamide |
| **SC-77** | 2-(cyclohexylmethyl)-*N*-(4-fluoro-3-methylsulfonylphenyl)indazole-3-carboxamide |
| **SC-78** | 2-(cyclohexylmethyl)-*N*-(2-methylsulfonylpyridin-4-yl)indazole-3-carboxamide |
| **SC-79** | 2-(cyclohexylmethyl)-*N*-(2-fluoro-3-methylsulfonylphenyl)indazole-3-carboxamide |
| **SC-80** | 2-((3,3-difluorocyclopentyl)methyl)-7-methyl-N-(3-sulfamoylphenyl)-2H-indazole-3-carboxamide |
| **SC-81** | 2-((3,3-difluorocyclohexyl)methyl)-7-fluoro-N-(3-sulfamoylphenyl)-2H-indazole-3-carboxamide |
| **SC-82** | N-(2-carbamoylpyridin-4-yl)-4-chloro-2-((3,3-difluorocyclopentyl)methyl)-7-methyl-2H-indazole-3 -carboxamide |
| **SC-83** | 2-(cyclohexylmethyl)-4-methyl-N-(3-(methylsulfonyl)phenyl)-2H-indazole-3 -carboxamide |
| **SC-84** | 4-chloro-7-methyl-N-(3-sulfamoylphenyl)-2-((tetrahydro-2H-pyran-2-yl)methyl)-2H-indazole-3-carboxamide |

(continued)

| Ex. | Name |
|---|---|
| **SC-85** | 7-cyclopropyl-N-(3-sulfamoylphenyl)-2-((tetrahydro-2H-pyran-4-yl)methyl)-2H-indazole-3-carboxamide |
| **SC-86** | N-(2-carbamoylpyridin-4 -yl)-2-(cyclohexylmethyl)-6-fluoro-2H-indazole-3-carboxamide |
| **SC-87** | 2-(cyclohexylmethyl)-6-fluoro-N-(2-sulfamoylpyridin-4-yl)-2H-indazole-3-carboxamide |
| **SC-88** | 7-methyl-N-(2-sulfamoylpyridin-4-yl)-2-((tetrahydro-2H-pyran-2-yl)methyl)-2H-indazole-3-carboxamide |
| **SC-89** | 7-(difluoromethyl)-N-(2-sulfamoylpyridin-4-yl)-2-((tetrahydro-2H-pyran-2-yl)methyl)-2H-indazole-3 -carboxamide |
| **SC-90** | 2-(1-bicyclo[2.1.1]hexanylmethyl)-*N*-(3-methylsulfonylphenyl)indazole-3-carboxamide |
| **SC-91** | 2-(1-bicyclo[1.1.1]pentanylmethyl)-*N*-(3-methylsulfonylphenyl)indazole-3-carboxamide |
| **SC-92** | 2-(cyclobutylmethyl)-N-(3 -sulfamoylphenyl)indazole-3 -carboxamide |
| **SC-93** | 2-(oxolan-3 -ylmethyl)-N-(3 -sulfamoylphenyl)indazole-3 -carboxamide |
| **SC-94** | *N*-(3-cyano-4-fluorophenyl)-2-[(4,4-difluorocyclohexyl)methyl]-6-fluoroindazole-3-carboxamide |
| **SC-95** | 2-[(4,4-difluorocyclohexyl)methyl]-6-fluoro-*N*-(2-sulfamoylpyridin-4-yl)indazole-3-carboxamide |
| **SC-96** | 2-[(4,4-difluorocyclohexyl)methyl]-6-fluoro-*N*-(4-fluoro-3-methylsulfonylphenyl)indazole-3-carboxamide |
| **SC-97** | *N*-(3-carbamoyl-5-fluorophenyl)-2-[(4,4-difluorocyclohexyl)methyl]-6-fluoroindazole-3-carboxamide |
| **SC-98** | *N*-(3-carbamoyl-4-fluorophenyl)-2-[(4,4-difluorocyclohexyl)methyl]-6-fluoroindazole-3-carboxamide |
| **SC-99** | 2-(cyclohexylmethyl)-*N*-[4-[*(R)*-methylsulfinyl]pyridin-2-yl]indazole-3-carboxamide |
| **SC-100** | 6-fluoro-*N*-(3-methylsulfonylphenyl)-2-[[3-(trifluoromethyl)cyclobutyl]methyl]indazole-3-carboxamide |
| **SC-101** | 6-fluoro-2-[(4-methylcycloheayl)methyl]-N (3-methylsulfonylphenyl)indazole-3-carboxamide |
| **SC-102** | 2-(cyclohexylmethyl)-*N*-(2-hydroxypyridin-4-yl)pyrazolo[3,4-c]pyridine-3-carboxamide |
| **SC-103** | 7-methyl-2-(oxan-2-ylmethyl)-N-(3 -sulfamoylphenyl)indazole-3 -carboxamide |
| **SC-104** | 2-(cyclohexylmethyl)-6-fluoro-*N*-(2-methylsulfonylpyridin-4-yl)indazole-3-carboxamide |
| **SC-105** | 2-[(4,4-difluorocyclohexyl)methyl]-*N*-[4-[*(R)*-methylsulfinyl]pyridin-2-yl]indazole-3-carboxamide |
| **SC-106** | 2-(cyclohexylmethyl)-*N*-(4-methylsulfonylpyridin-2-yl)indazole-3-carboxamide |
| **SC-107** | 2-[(4,4-difluorocyclohexyl)methyl]-6-fluoro-*N*-(2-methylsulfonylpyridin-4-yl)indazole-3-carboxamide |
| **SC-108** | 2-(cyclopentylmethyl)-6-fluoro-*N*-(3-sulfamoylphenyl)indazole-3-carboxamide |
| **SC-109** | *N*-(3-carbamoylphenyl)-2-(cyclopentylmethyl)-6-fluoroindazole-3-carboxamide |
| **SC-110** | 2-(cyclopentylmethyl)-*N*-(3-sulfamoylphenyl)indazole-3-carboxamide |
| **SC-110** | 2-(cyclopentylmethyl)-6-fluoro-*N*-(3-methylsulfonylphenyl)indazole-3-carboxamide |
| **SC-111** | 2-(oxan-2-ylmethyl)-N-(3 -sulfamoylphenyl)-7-(trifluoromethyl)indazole-3-carboxamide |
| **SC-112** | *N*-(1,1-dioxo-2,3-dihydro-1,2-benzothiazol-6-yl)-2-(oxan-4-ylmethyl)indazole-3-carboxamide |
| **SC-113** | 6-fluoro-*N*-(3-methylsulfonylphenyl)-2-(2-oxaspiro[3.3]heptan-6-ylmethyl)indazole-3- carboxamide |
| **SC-114** | 6-fluoro-*N*-(3-methylsulfonylphenyl)-2-[[rac-(3R)-oxolan-3-yl]methyl]indazole-3-carboxamide |
| **SC-115** | 2-[[2-(cyclohexylmethyl)-6-fluoroindazole-3-carbonyl]amino]-1,3-thiazole-5-carboxamide |
| **SC-116** | 2-(cyclohexylmethyl)-6-fluoro-*N*-(3-oxo-1,2-dihydroisoindol-5-yl)indazole-3-carboxamide |
| **SC-117** | 2-[(4,4-difluorocyclohexyl)methyl]-*N*-(2-methylsulfonylpyridin-4-yl)indazole-3-carboxamide |
| **SC-118** | *N*-(5-carbamoylthiophen-2-yl)-2-[(4,4-difluorocyclohexyl)methyl]-6-fluoroindazole-3-carboxamide |
| **SC-119** | 6-methyl-2-(oxan-2-ylmethyl)-*N*-(3-sulfamoylphenyl)indazole-3-carboxamide |
| **SC-120** | 2-(cyclohexylmethyl)-*N*-(5-methylsulfonylpyridin-3-yl)indazole-3-carboxamide |
| **SC-121** | 2-(cyclohexylmethyl)-*N*-(5-methylsulfonylpyridin-3-yl)indazole-3-carboxamide |
| **SC-122** | 2-[(4,4-difluorocyclohexyl)methyl]-6-fluoro-N-(3-sulfamoylphenyl)indazole-3-carboxamide |
| **SC-123** | 2-(cycloheptylmethyl)-6-fluoro-N-(3-methylsulfonylphenyl)indazole-3-carboxamide |
| **SC-124** | N-(5-carbamoylthiophen-3-yl)-2-((4,4-difluorocyclohexyl)methyl)-6-fluoro-2H-indazole-3-carboxamide |
| **SC-125** | N-(3-cyanophenyl)-2-((4,4-difluorocyclohexyl)methyl)-2H-indazole-3-carboxamide |
| **SC-126** | 2-((4,4-difluorocyclohexyl)methyl)-6-fluoro-N-(3-fluoro-5-(methylsulfonyl)phenyl)-2H-indazole-3 -carboxamide |
| **SC-127** | N-(3-sulfamoylphenyl)-2-((tetrahydro-2H-pyran-2-yl)methyl)-2H-indazole-3-carboxamide |

(continued)

| Ex. | Name |
|---|---|
| **SC-128** | 2-((1-cyanocyclohexyl)methyl)-N-(3-(methylsulfonyl)phenyl)-2H-indazole-3-carboxamide |
| **SC-129** | 2-((4,4-difluorocyclohexyl)methyl)-7-fluoro-N-(3-sulfamoylphenyl)-2H-indazole-3-carboxamide |
| **SC-130** | N-(4-fluoro-3-sulfamoylphenyl)-2-((tetrahydro-2H-pyran-4-yl)methyl)-2H-indazole-3-carboxamide |
| **SC-131** | N-[3-(methylsulfamoyl)phenyl]-2-(oxan-4-ylmethyl)indazole-3-carboxamide |
| **SC-132** | 2-(cyclobutylmethyl)-6-fluoro-N-(3-sulfamoylphenyl)indazole-3-carboxamide |
| **SC-133** | 2-(cyclobutylmethyl)-6-fluoro-N-(3-methylsulfonylphenyl)indazole-3-carboxamide |
| **SC-134** | N-(2-carbamoylpyridin-4-yl)-2-(cyclohexylmethyl)-6-fluoroindazole-3-carboxamide |
| **SC-135** | 2-[(4,4-difluorocyclohexyl)methyl]-N-(2-sulfamoylpyridin-4-yl)indazole-3-carboxamide |
| **SC-136** | N-(2-carbamoylpyridin-4-yl)-2-[(4,4-difluorocyclohexyl)methyl]-6-fluoroindazole-3-carboxamide |
| **SC-137** | 2-[(4,4-difluorocyclohexyl)methyl]-6-fluoro-N-(4-fluoro-3-hydroxyphenyl)indazole-3-carboxamide |
| **SC-138** | 2-[(4,4-difluorocyclohexyl)methyl]-7-fluoro-N-(4-fluoro-3-methylsulfonylphenyl)indazole-3-carboxamide |
| **SC-139** | 2-[(4,4-difluorocyclohexyl)methyl]-7-fluoro-N-(2-sulfamoylpyridin-4-yl)indazole-3-carboxamide |
| **SC-140** | 4-fluoro-2-(oxan-4-ylmethyl)-N-(3-sulfamoylphenyl)indazole-3-carboxamide |
| **SC-141** | 2-(cyclobutylmethyl)-4-fluoro-N-(3-sulfamoylphenyl)indazole-3-carboxamide |
| **SC-142** | 2-[(3,3-difluorocyclopentyl)methyl]-6-methyl-N-(3-sulfamoylphenyl)indazole-3-carboxamide |
| **SC-143** | 2-(oxan-2-ylmethyl)-N-(3-sulfamoylphenyl)-6-(trifluoromethyl)indazole-3-carboxamide |
| **SC-144** | 2-(cyclohexylmethyl)-N-(2-hydroxypyridin-4-yl)-5-(trifluoromethyl)indazole-3-carboxamide |
| **SC-145** | 2-[(3,3-difluorocyclopentyl)methyl]-7-methyl-N-(3-sulfamoylphenyl)indazole-3-carboxamide |
| **SC-146** | N-(3-carbamoylphenyl)-2-[(1-fluorocyclohexyl)methyl]indazole-3-carboxamide |
| **SC-147** | 2-[(1-fluorocyclohexyl)methyl]-N-(3-methylsulfonylphenyl)indazole-3-carboxamide |
| **SC-148** | N-(3-methylsulfonylphenyl)-2-[[1-(trifluoromethyl)cyclobutyl]methyl]indazole-3-carboxamide |
| **SC-149** | N-(4-carbamoylpyrimidin-2-yl)-2-(cyclohexylmethyl)-6-fluoroindazole-3-carboxamide |
| **SC-150** | 2-[(3,3-difluorocyclopentyl)methyl]-6-fluoro-N-(3-sulfamoylphenyl)indazole-3-carboxamide |
| **SC-151** | 2-[(3,3-difluorocyclopentyl)methyl]-6-fluoro-N-(2-sulfamoylpyridin-4-yl)indazole-3-carboxamide |
| **SC-152** | 2-[(3,3-difluorocyclopentyl)methyl]-6-fluoro-N-(4-fluoro-3-sulfamoylphenyl)indazole-3-carboxamide |
| **SC-153** | N-(2-carbamoylpyridin-4-yl)-2-[(4,4-difluorocyclohexyl)methyl]indazole-3-carboxamide |
| **SC-154** | N-(3-methylsulfonylphenyl)-2-(oxepan-4-ylmethyl)indazole-3-carboxamide |
| **SC-155** | 2-(cyclohexylmethyl)-4-methyl-N-(3-methylsulfonylphenyl)indazole-3-carboxamide |
| **SC-156** | 4-methyl-2-(oxan-4-ylmethyl)-N-(3-sulfamoylphenyl)indazole-3-carboxamide |
| **SC-157** | 4-methoxy-2-(oxan-4-ylmethyl)-N-(3-sulfamoylphenyl)indazole-3-carboxamide |
| **SC-158** | N-(3-carbamoyl-4-fluorophenyl)-4-methyl-2-(oxan-4-ylmethyl)indazole-3-carboxamide |
| **SC-159** | N-(3 -carbamoyl-4-fluorophenyl)-4-methoxy-2-(oxan-4-ylmethyl)indazole-3-carboxamide |
| **SC-160** | N-(2-fluoro-3 -sulfamoylphenyl)-2-(oxan-4-ylmethyl)indazole-3 -carboxamide |
| **SC-161** | N-(3-carbamoyl-4-fluorophenyl)-4-cyclopropyl-2-(oxan-4-ylmethyl)indazole-3-carboxamide |
| **SC-162** | 7-methyl-2-(oxan-4-ylmethyl)-N-(3-sulfamoylphenyl)indazole-3-carboxamide |
| **SC-163** | N-(3-carbamoyl-4-fluorophenyl)-7-methyl-2-(oxan-4-ylmethyl)indazole-3-carboxamide |
| **SC-164** | 2-[(2-methyloxan-4-yl)methyl]-N-(3-sulfamoylphenyl)indazole-3-carboxamide |
| **SC-165** | 2-[(3,3-difluorocyclopentyl)methyl]-N-(3-sulfamoylphenyl)-6-(trifluoromethyl)indazole-3-carboxamide |
| **SC-166** | N-(3-carbamoylphenyl)-2-[(4,4-difluorocyclohexyl)methyl]-7-fluoroindazole-3-carboxamide |
| **SC-167** | 7-methyl-N-(2-methylsulfonylpyridin-4-yl)-2-(oxan-2-ylmethyl)indazole-3-carboxamide |
| **SC-168** | 6-fluoro-N-(3-sulfamoylphenyl)-2-[[3-(trifluoromethyl)cyclobutyl]methyl]indazole-3-carboxamide |
| **SC-169** | 2-[(3,3-difluorocyclohexyl)methyl]-6-fluoro-N-(3-methylsulfonylphenyl)indazole-3-carboxamide |
| **SC-170** | N-(3-methylsulfonylphenyl)-2-[[3-(trifluoromethoxy)cyclobutyl]methyl]indazole-3-carboxamide |
| **SC-171** | 2-[(3,3-difluorocyclopentyl)methyl]-7-fluoro-N-[3-(methylsulfamoyl)phenyl]indazole-3-carboxamide |
| **SC-172** | N-[3-(cyclopropylsulfamoyl)phenyl]-2-[(3,3-difluorocyclopentyl)methyl]-7-fluoroindazole-3-carboxamide |
| **SC-173** | 2-[(4,4-difluorocyclohexyl)methyl]-7-fluoro-N-(3-methylsulfinylphenyl)indazole-3-carboxamide |
| **SC-174** | 2-[(4,4-difluorocyclohexyl)methyl]-7-fluoro-N-(3-methylsulfonylphenyl)indazole-3-carboxamide |
| **SC-175** | 2-(oxan-4-ylmethyl)-N-(3-sulfamoylphenyl)-4-(trifluoromethyl)indazole-3-carboxamide |

(continued)

| Ex. | Name |
|---|---|
| **SC-176** | N-(3-carbamoyl-4-fluorophenyl)-2-(oxan-4-ylmethyl)-4-(trifluoromethyl)indazole-3-carboxamide |
| **SC-177** | 2-(cyclohexylmethyl)-N-(3-methylsulfonylphenyl)-4-(trifluoromethyl)indazole-3-carboxamide |
| **SC-178** | 2-[(3,3-dimethylcyclobutyl)methyl]-N-(3-methylsulfonylphenyl)indazole-3-carboxamide |
| **SC-179** | N-(3-methylsulfonylphenyl)-2-[[1-(trifluoromethyl)cyclopentyl]methyl]indazole-3-carboxamide |
| **SC-180** | 2-[(4,4-difluorocyclohexyl)methyl]-7-fluoro-N-(4-fluoro-3-sulfamoylphenyl)indazole-3-carboxamide |
| **SC-181** | 2-[(2,2-difluorocyclopentyl)methyl]-6-fluoro-N-(3-sulfamoylphenyl)indazole-3-carboxamide |
| **SC-182** | 2-[(2,2-difluorocyclopentyl)methyl]-6-fluoro-N-(4-fluoro-3-sulfamoylphenyl)indazole-3-carboxamide |
| **SC-183** | 6-fluoro-N-(3-methylsulfonylphenyl)-2-(spiro[2.3]hexan-5-ylmethyl)indazole-3-carboxamide |
| **SC-184** | N-(2-carbamoylpyridin-4-yl)-2-(cyclopentylmethyl)-6-fluoroindazole-3-carboxamide |
| **SC-185** | 2-[(1-fluorocyclopentyl)methyl]-N-(3-methylsulfonylphenyl)indazole-3-carboxamide |
| **SC-186** | 7-methyl-2-(oxan-2-ylmethyl)-N-(2-sulfamoylpyridin-4-yl)indazole-3-carboxamide |
| **SC-187** | 2-[(2,2-difluorocyclopentyl)methyl]-6-fluoro-N-(2-sulfamoylpyridin-4-yl)indazole-3-carboxamide |
| **SC-188** | 2-[(2,2-difluorocyclopentyl)methyl] -6-fluoro-N-(2-sulfamoylpyridin-4-yl)indazole-3- carboxamide |
| **SC-189** | N-(2-carbamoylpyridin-4 yl)-7-fluoro-2-(oxan-2-ylmethyl)indazole-3-carboxamide |
| **SC-190** | 2-(cyclohexylmethyl)-N-(2-hydroxypyridin-4-yl)-4-(trifluoromethyl)indazole-3-carboxamide |
| **SC-191** | 7-chloro-2-[(4,4-difluorocyclohexyl)methyl]-N-(2-sulfamoylpyridin-4-yl)indazole-3-carboxamide |
| **SC-192** | 7-fluoro-N-(2-methylsulfonylpyridin-4-yl)-2-(oxan-2-ylmethyl)indazole-3-carboxamide |
| **SC-193** | 7-fluoro-2-(oxan-2-ylmethyl)-N-(2-sulfamoylpyridin-4-yl)indazole-3-carboxamide |
| **SC-194** | 2-(oxan-4-ylmethyl)-N-(2-sulfamoylpyridin-4-yl)indazole-3-carboxamide |
| **SC-195** | 2- [(3,3-difluorocyclopentyl)methyl] -N-(3 -sulfamoylphenyl)-7-(trifluoromethyl)indazole-3-carboxamide |
| **SC-196** | 7-(difluoromethyl)-N-(2-methylsulfonylpyridin-4-yl)-2-(oxan-2-ylmethyl)indazole-3-carboxamide |
| **SC-197** | 2-[(5,5-difluorooxan-2-yl)methyl]-7-methyl-N-(3-sulfamoylphenyl)indazole-3-carboxamide |
| **SC-198** | N-(2-carbamoylpyridin-4-yl)-2-[(4,4-difluorocyclohexyl)methyl]-7-fluoroindazole-3-carboxamide |
| **SC-199** | N-(2-carbamoylpyridin-4 yl)-7-methyl-2-(oxan-2-ylmethyl)indazole-3-carboxamide |
| **SC-200** | N-(2-carbamoylpyridin-4-yl)-2-(oxan-2-ylmethyl)-7-(trifluoromethyl)indazole-3-carboxamide |
| **SC-201** | N-(2-methylsulfonylpyridin-4-yl)-2-(oxan-2-ylmethyl)-7-(trifluoromethyl)indazole-3-carboxamide |
| **SC-202** | 7-fluoro-N-(1-methylsulfonylpyrazol-4-yl)-2-(oxan-2-ylmethyl)indazole-3 -carboxamide |
| **SC-203** | 2-(3-bicyclo[3.1.0]hexanylmethyl)-N-(3-methylsulfonylphenyl)indazole-3-carboxamide |
| **SC-204** | 2-[(3,3-difluorocyclopentyl)methyl]-4-methyl-N-(3-sulfamoylphenyl)indazole-3-carboxamide |
| **SC-205** | 4-(difluoromethyl)-2-(oxan-4-ylmethyl)-N-(2-sulfamoylpyridin-4-yl)indazole-3-carboxamide |
| **SC-206** | N-(1-methylsulfonylpyrazol-4-yl)-2-(oxan-2-ylmethyl)-7-(trifluoromethyl)indazole-3-carboxamide |
| **SC-207** | 7-methyl-N-(1-methylsulfonylpyrazol-4-yl)-2-(oxan-2-ylmethyl)indazole-3-carboxamide |
| **SC-208** | 6-fluoro-2-[(4-hydroxy-4-methylcyclohexyl)methyl]-N-(3-methylsulfonylphenyl)indazole-3-carboxamide |
| **SC-209** | 2-(oxan-2-ylmethyl)-N-(2-sulfamoylpyridin-4-yl)-7-(trifluoromethyl)indazole-3-carboxamide |
| **SC-210** | 6-chloro-2-(oxan-4-ylmethyl)-N-(2-sulfamoylpyridin-4-yl)indazole-3-carboxamide |
| **SC-211** | 2-[(1-hydroxycyclohexyl)methyl]-N-(3-methylsulfonylphenyl)indazole-3-carboxamide |
| **SC-212** | 7-(difluoromethyl)-2-(oxan-2-ylmethyl)-N-(2-sulfamoylpyridin-4-yl)indazole-3-carboxamide N-(2-carbamoylpyridin-4-yl)-2-[[(2S)-5,5-difluorooxan-2-yl]methyl]-7-methylindazole-3- |
| **SC-213** | carboxamide |
| **SC-214** | 7-(difluoromethyl)-N-(1-methylsulfonylpyrazol-4-yl)-2-(oxan-2-ylmethyl)indazole-3-carboxamide |
| **SC-215** | N-(2-carbamoylpyridin-4-yl)-7-(difluoromethyl)-2-(oxan-2 -ylmethyl)indazole-3-carboxamide |
| **SC-216** | N-(2-carbamoylpyridin-4-yl)-2-[(5,5-difluorooxan-2-yl)methyl]-7-methylindazole-3-carboxamide |
| **SC-217** | N-(2-carbamoylpyridin-4-yl)-2-[(6,6-dimethyloxan-2-yl)methyl]-7-methy lindazole-3-carboxamide |
| **SC-218** | N-(2-carbamoylpyridin-4 -yl)-7-cyclopropyl-2-(oxan-4-ylmethyl)indazole-3-carboxamide |
| **SC-219** | 7-cyclopropyl-N-(2-methylsulfonylpyridin-4-yl)-2-(oxan-4-ylmethyl)indazole-3-carboxamide |
| **SC-220** | 4-chloro-7-methyl-2-(oxan-2-ylmethyl)-N-(3-sulfamoylphenyl)indazole-3-carboxamide |
| **SC-221** | 7-cyclopropyl-2-(oxan-4-ylmethyl)-N-(3-sulfamoylphenyl)indazole-3-carboxamide |
| **SC-222** | N-(2-carbamoylpyridin-4 yl)-4-fluoro-7-methyl-2-(oxan-2-ylmethyl)indazole-3-carboxamide |

(continued)

| Ex. | Name |
|---|---|
| **SC-223** | N-(2-carbamoylpyridin-4-yl)-2-[(3,3 -difluorocyclobutyl)methyl] -7-methy lindazole-3 - carboxamide |
| **SC-224** | N-(2-carbamoylpyridin-4-yl)-7-chloro-2-[(3,3-difluorocyclobutyl)methyl]-6-methylindazole-3-carboxamide |
| **SC-225** | N-(2-carbamoylpyridin-4-yl)-7-cyano-2-[(3,3-difluorocyclobutyl)methyl]indazole-3-carboxamide |
| **SC-226** | N-(2-carbamoylpyridin-4-yl)-4-chloro-2-[(3,3-difluorocyclopentyl)methyl]-7-methylindazole-3-carboxamide |
| **SC-227** | N-(2-carbamoylpyridin-4-yl)-2-[(3,3-difluorocyclopentyl)methyl]-4-fluoro-7-methylindazole-3-carboxamide |
| **SC-228** | N-(2-carbamoylpyridin-4-yl)-7-chloro-2-[(3,3-difluorocyclopentyl)methyl]-4-methylindazole-3-carboxamide |
| **SC-229** | 2-((3,3-difluoro-1-methylcyclobutyl)methyl)-7-methyl-N-(2-(S-methylsulfonimidoyl)pyridin-4-yl)-2H-indazole-3-carboxamide |
| **SC-230** | 2-((3,3-difluoro-1-methylcyclobutyl)methyl)-7-fluoro-N-(2-(S-methylsulfonimidoyl)pyridin-4-yl)-2H-indazole-3 -carboxamide |
| **SC-231** | 7-cyclopropyl-2-((3,3-difluoro-1-methylcyclobutyl)methyl)-N-(2-(S-methylsulfonimidoyl)pyridin-4-yl)-2H-indazole-3-carboxamide |
| **SC-232** | 4-chloro-2-((3,3-difluoro-1-methylcyclobutyl)methyl)-7-methyl-N-(2-(S-methylsulfonimidoyl)pyridin-4-yl)-2H-indazole-3-carboxamide |
| **SC-233** | 2-((3,3-difluoro-1-methylcyclobutyl)methyl)-7-(difluoromethyl)-N-(2-(S-methylsulfonimidoyl)pyridin-4-yl)-2H-indazole-3-carboxamide |
| **SC-234** | 2-((3,3-difluoro-1-methylcyclobutyl)methyl)-4-fluoro-7-methyl-N-(2-(S-methylsulfonimidoyl)pyridin-4-yl)-2H-indazole-3-carboxamide |
| **SC-235** | 2-((3,3-difluoro-1-methylcyclobutyl)methyl)-4-methyl-N-(2-(S-methylsulfonimidoyl)pyridin-4-yl)-2H-indazole-3-carboxamide |
| **SC-236** | 2-((3,3-difluoro-1-methylcyclobutyl)methyl)-6-fluoro-N-(2-(S-methylsulfonimidoyl)pyridin-4-yl)-2H-indazole-3-carboxamide |
| **SC-237** | 2-((3,3-difluoro-1-methylcyclobutyl)methyl)-N-(2-(N,S-dimethylsulfonimidoyl)pyridin-4-yl)-7-methyl-2H-indazole-3 -carboxamide |
| **SC-238** | 2-((3,3-difluoro-1-methylcyclobutyl)methyl)-N-(2-(N,S-dimethylsulfonimidoyl)pyridin-4-yl)-7-fluoro-2H-indazole-3-carboxamide |
| **SC-239** | 7-cyclopropyl-2-((3,3-difluoro-1-methylcyclobutyl)methyl)-N-(2-(N,S-dimethylsulfonimidoyl)pyridin-4-yl)-2H-indazole-3-carboxamide |
| **SC-240** | 4-chloro-2-((3,3-difluoro-1-methylcyclobutyl)methyl)-N-(2-(N,S-dimethylsulfonimidoyl)pyridin-4-yl)-7-methyl-2H-indazole-3-carboxamide |
| **SC-241** | 2-((3,3-difluoro-1-methylcyclobutyl)methyl)-7-(difluoromethyl)-N-(2-(N,S-dimethylsulfonimidoyl) pyridin-4-yl)-2H-indazole-3-carboxamide |
| **SC-242** | 2-((3,3-difluoro-1-methylcyclobutyl)methyl)-N-(2-(N,S-dimethylsulfonimidoyl)pyridin-4-yl)-4-fluoro-7-methyl-2H-indazole-3-carboxamide |
| **SC-243** | 2-((3,3-difluoro-1-methylcyclobutyl)methyl)-N-(2-(N,S-dimethylsulfonimidoyl)pyridin-4-yl)-4-methyl-2H-indazole-3-carboxamide |
| **SC-244** | 2-((3,3-difluoro-1-methylcyclobutyl)methyl)-N-(2-(N,S-dimethylsulfonimidoyl)pyridin-4-yl)-6-fluoro-2H-indazole-3-carboxamide |
| **SC-245** | 4-chloro-2-((3,3-difluoro-1-methylcyclobutyl)methyl)-7-methyl-N-(3-(S-methylsulfonimidoyl)phenyl)-2H-indazole-3-carboxamide |
| **SC-246** | 4-chloro-2-((3,3-difluorobicyclo[3.1.0]hexan-1-yl)methyl)-7-methyl-N-(2-(S-methylsulfonimidoyl) pyridin-4-yl)-2H-indazole-3-carboxamide |

in the form of the free compound or a physiologically acceptable salt thereof.

In a preferred embodiment, the compound accorsding to the invention is an inhibitor of $Na_V1.8$. In the sense of the invention, the term "inhibitor of $Na_V1.8$" preferably means that the respective compound exhibits in a patch clamp assay an IC50 value on $Na_V1.8$ of at most 10 μM ($10 \cdot 10^{-6}$ mol/L); more preferably at most 1 μM; still more preferably at most 500 nM ($10^{-9}$ mol/L); even more preferably at most 100 nM; and most preferably at most 10 nM.

A preferred assay for testing compounds for their potency and method for determining an IC50 on $Na_V1.8$ is described in the experimental part down below.

In a preferred embodiment, the compound according to the invention is a selective inhibitor of $Na_V1.8$. In the sense of the invention, the term "selective inhibitor of $Na_V1.8$" preferably means that the respective compound preferably does not exhibit any inhibitory activity on $Na_V1.1$, $Na_V1.2$, $Na_V1.4$, $Na_V1.5$ and $Na_V1.6$. The skilled artisan knows suitable ways to determine whether a compound exhibits inhibitory effects on any of $Na_V1.1$, $Na_V1.2$, $Na_V1.4$, $Na_V1.5$ and $Na_V1.6$.

The invention therefore relates to a compound according to the invention for use in the inhibition of $Na_V1.8$.

Therefore, another aspect of the invention relates to a compound according to the invention for use in the treatment of pain. Still another aspect of the invention relates to a method of treatment of pain; comprising the administration of a therapeutically effective amount of a compound according to the invention to a subject in need thereof, preferably a human.

A further aspect of the invention relates to a compound according to the invention as medicament.

Another aspect of the invention relates to a pharmaceutical dosage form comprising a compound according to the invention. Preferably, the pharmaceutical dosage form comprises a compound according to the invention and one or more pharmaceutical excipients such as physiologically acceptable carriers, additives and/or auxiliary substances; and optionally one or more further pharmacologically active ingredient. Examples of suitable physiologically acceptable carriers, additives and/or auxiliary substances are fillers, solvents, diluents, colorings and/or binders. These substances are known to the person skilled in the art (see H. P. Fiedler, Lexikon der Hilfsstoffe fur Pharmazie, Kosmetik und angrenzende Gebiete, Editio Cantor Aulendoff).

The pharmaceutical dosage form according to the invention is preferably for systemic, topical or local administration, preferably for oral administration. Therefore, the pharmaceutical dosage form can be in form of a liquid, semisolid or solid, e.g. in the form of injection solutions, drops, juices, syrups, sprays, suspensions, tablets, patches, films, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and can also be administered as such.

The pharmaceutical dosage form according to the invention is preferably prepared with the aid of conventional means, devices, methods and processes known in the art. The amount of the compound according to the invention to be administered to the patient may vary and is e.g. dependent on the patient's weight or age and also on the type of administration, the indication and the severity of the disorder. Preferably 0.001 to 100 mg/kg, more preferably 0.05 to 75 mg/kg, most preferably 0.05 to 50 mg of a compound according to the invention are administered per kg of the patient's body weight.

Therefore, another aspect of the invention relates to the pharmaceutical dosage form according to the invention for use in the treatment of pain. Still another aspect of the invention relates to a method of treatment of pain; comprising the administration of a pharmaceutical dosage form according to the invention to a subject in need thereof, preferably a human.

## EXAMPLES

The following abbreviations are used in the descriptions of the experiments: ABPR = automatic back pressure regulator ACN = acetonitrile; aq. = aqueous; Bn = benzyl; Boc = tert-butyloxycarbonyl; dba = dibenzylideneacetone, mCPBA = meta-chloroperoxybenzoic acid; DCE = dichloroethane, DCM = dichloromethane; DIAD = diisopropylazodicarboxylate; DIPEA = N,N-diisopropylethylamine; DMF = *N,N*-dimethylformamide; DMSO = dimethylsulfoxide; EDC·HCl = 1-ethyl-3-(3-dimethylaminopropyl)carbodiimide hydrochloride; EtOAc or EA = EtOAc; EtOH = ethanol; HATU = 1-[bis(dimethylamino)methylene]-1*H*-1,2,3-triazolo[4,5-b]pyridinium 3-oxide hexafluorophosphate; h = hour; HPLC = high-performance liquid chromatography; Int = intermediate; KHMDS = potassium bis(trimethylsilyl)amide; LCMS = liquid chromatography-mass spectrometry; LiHMDS = lithium bis(trimethylsilyl)amide; MeOH = methanol; min = minute; Ms = methanesulfonyl; MTBE = methyl tert-butyl ether; MW = molecular weight; NMR = nuclear magnetic resonance; prep. = preparative; PTSA = *para*-toluenesulfonylchloride; RP = reversed phase; ambient temperature = room temperature; $R_t$ = retention time; sat. = saturated; SFC = supercritical fluid chromatography; SM = starting material; TEA = triethylamine; TFA = trifluoroacetic acid; TMSCl = trimethylsilylchloride; Tf = trifluoromethylsulfonyl; THF = tetrahydrofuran; TLC = thin-layer chromatography; Ts = para-toluene sulfonyl; Xantphos = 4,5-bis(diphenylphosphino)-9,9-dimethylxanthene

### General synthesis schemes

As illustrated in Scheme 1, compounds of the invention can be prepared by N-alkylation of Alkyl 1*H*-indazole-5-carboxylates or alkyl 1*H*-(aza)-indazole-5-carboxylates of the general formula (A) with appropriately functionalized alkylation reagents of the general formula (B) (where Y is leaving group, such as Br, Cl, OTs, OMs, OTf) under basic conditions to afford compounds of general formula (C). Alternatively, intermediates of the general formula (C) can be

prepared using a Mitsunobu reaction between Alkyl 1*H*-indazole-5-carboxylates or alkyl 1*H*-(aza)-indazole-5-carboxylates of type (A) and alcohols of the general formula (B) (where Y is OH) (Chem. Rev. 2009, 109, 6, 2551-2651). Intermediates of the general formula (C) can be further converted into heteroarylcarboxamides of the general formula (E) upon nucleophilic addition of (hetero)arylamines of the general formula (D) in the presence of a strong anionic base, preferably LiHMDS or KHMDS.

**Scheme 1:** L, X, R1, R2, R3, R4, R5, A1, A2, A3 are as defined in claim 1.

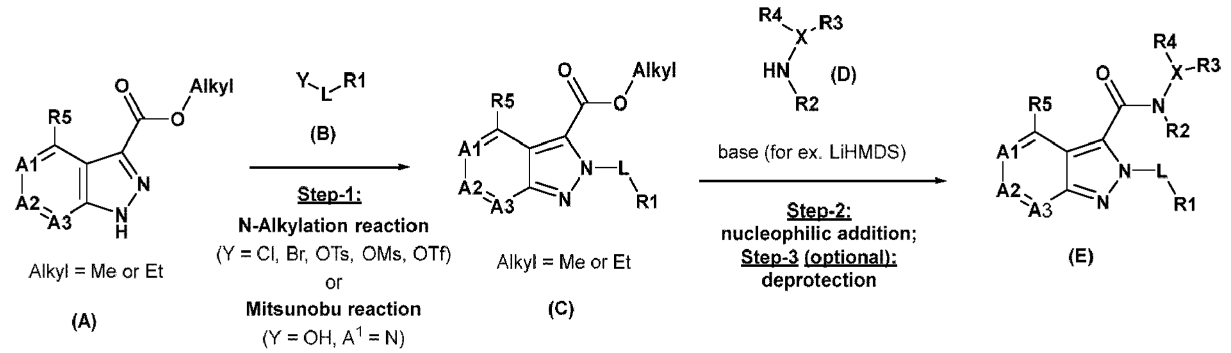


Alternatively and as illustrated in Scheme 2, intermediates of the general formula (C) can be hydrolized to carboxylic acids of the general formula (F) and converted to compounds of the general formula (E) using amide coupling with (hetero)arylamines of the general formula (D) in the presence of a carboxylic acid activating reagent, preferably EDC·HCl, HATU, $POCl_3$ or $SOCl_2$, and a base, preferably pyridine or DIPEA (March's Advanced Organic Chemistry, 2007, 6th Edition, page 1427-1474) to provide compounds of the general formula (E).

**Scheme 2:** L, X, R1, R2, R3, R4, R5, A1, A2, A3 are as defined in claim 1.

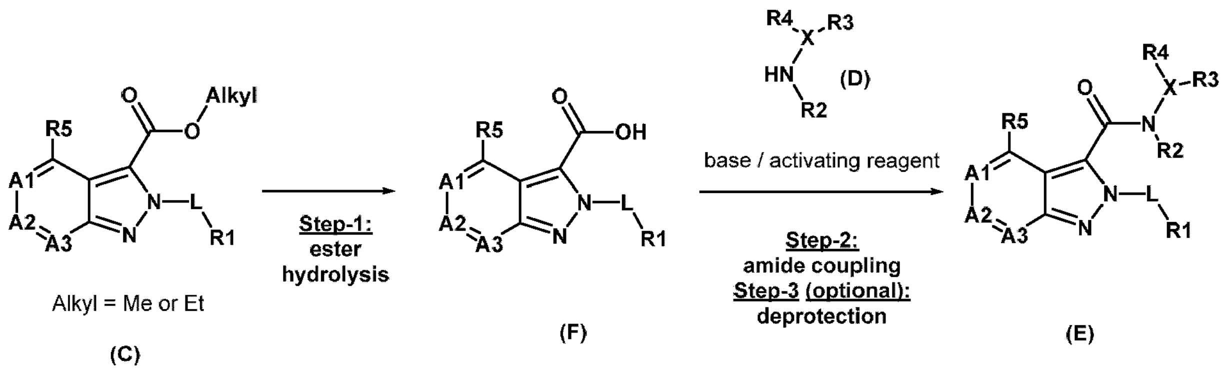


Alternatively and as illustrated in Scheme 3, carboxylic acids of the general formula (F) can be converted into carboxamides of the general formula (H) upon amide coupling with amines of the general formula (G) under coupling conditions well precedented in the literature (March's Advanced Organic Chemistry, 2007, 6th Edition, page 1427-1474). Intermediates of the general formula (H) can be further used in metal-catalyzed C-N coupling reactions with the corresponding aryl halides or heteroaryl halides of the general formula (I), preferably with corresponding aryl bromides, aryl iodides, heteroaryl bromides or heteroaryl iodides, to provide compounds of the general formula (E). Metal catalyzed C-N coupling reactions are generally known in the art (Current Organic Synthesis, 2011, 8, 53). Favorable C-N coupling reactions are palladium and copper catalyzed cross-coupling reactions (Chem. Rev. 2016, 116, 12564; Chem. Soc. Rev. 2014, 43, 3525; Chem. Sci. 2010, 1, 13).

**Scheme 3:** L, X, R1, R2, R3, R4, R5, A1, A2, A3 are as defined in claim 1. Z = halogen.

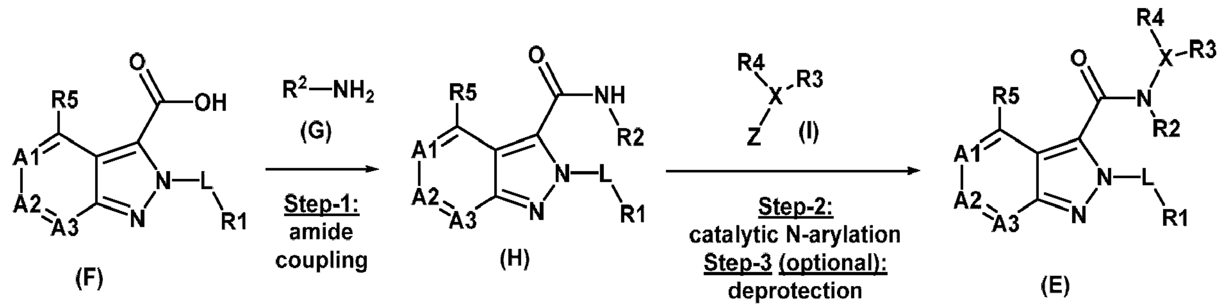


In some embodiments, (hetero)arylamines of the general formula (D) or (hetero)arylhalides of the general formula (I) need to bear one or several protecting groups, such as benzyl (Bn), 4-methoxybenzyl (PMB), 2,4-dimethoxybenzyl (DMB), tert-butyloxycarbonyl (Boc) or methyl (Me), which are deprotected after amide bond formation (optional step-3, Schemes 1-3) using standard deprotection protocols known in the art (T. W. Green, P. G. M. Wuts, Protective Groups in Organic Synthesis, Wiley-Interscience, New York, 1999).

Alkyl 1*H*-indazole-5-carboxylates or alkyl 1*H*-(aza)-indazole-5-carboxylates of the general formula (A), alkylation reagents of the general formula (B), (hetero)arylamines of the general formula (D) and (hetero)arylhalides of the general formula (I) can be either commercially available or prepared as described in the present invention or synthesized according to the standard procedures known to the person skilled in the art.

In some embodiments, carboxamides of the general formula (E) need to be converted into target compounds using further protocols known to the person skilled in the art, for example oxidation (SC-19), Grignard addition (SC-26), nitrile hydrolysis (SC-86) and mesylation (SC-36).

**Synthesis of intermediates**

Svnthesis of *N,N*-di-Boc 4-bromopyridine-2-sulfonamide **(Int-A1)**:

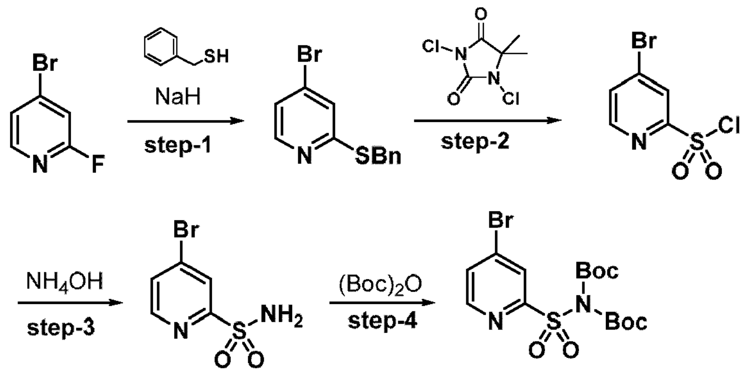


Int-A1

Step-1: To a stirred solution of NaH (60% in mineral oil, 0.142 mmol, 1.0 eq.) in THF was added phenylmethanethiol (17.6 g, 0.142 mmol) at 0 °C and the reaction mixture was stirred at 0 °C for 2 h. 4-bromo-2-fluoropyridine (35.0 g, 0.142 mmol) was added and the reaction mixture was stirred for further 2 h at ambient temperature. The reaction mixture was diluted with cold water (500 mL) and extracted with EtOAc (2 × 250 mL). The combined organic layers were dried over anhydrous $Na_2SO_4$ and concentrated under reduced pressure to afford 2-(benzylthio)-4-bromopyridine (35.0 g, 96%) which was used in the next step without further purification.

Step-2: To a stirred solution of 2-(benzylthio)-4-bromopyridine (30 g, 108 mmol, 1.0 eq.) in DCM (30 mL) was added portion wise acetic acid (60 mL), water (120 mL) and 1,3-dichloro-5,5-dimethyl imidazolidine-2,4-dione (63 g, 324 mmol) at 0 °C. The reaction mixture was stirred at ambient temperature under an argon atmosphere for 16 h. The reaction mixture was diluted with water (100 mL) and extracted with EtOAc (3 × 200 mL). The combined organic layers were washed with water (200 mL) and brine (200 mL), dried over anhydrous $Na_2SO_4$ and concentrated under reduced pressure to afford 4-bromopyridine-2-sulfonyl chloride (60 g, crude). The crude product was used in the next step without further

purification.

Step-3: To a stirred solution of 4-bromopyridine-2-sulfonyl chloride (60 g, 24 mmol, 1.0 eq.) in acetonitrile (100 mL) was added sat. aq. $NH_4OH$ (100 mL) at 0 °C and the reaction mixture was stirred at ambient temperature for 3 h. The reaction progress was monitored by TLC and LCMS. The reaction mixture was diluted with water (80 mL) and extracted with EtOAc (3 × 300 mL). The combined organic layers were washed with water (200 mL) and brine (200 mL), dried over anhydrous $Na_2SO_4$ and concentrated under reduced pressure to yield the crude product. This crude compound was purified by flash column chromatography (silica gel) using 40% EtOAc in petroleum ether as an eluent to afford 4-bromopyridine-2-sulfonamide (8.0 g) as a brown solid.

Step-4: To a stirred solution of 4-bromopyridine-2-sulfonamide (8.0 g, 34 mmol, 1.0 eq.) in 2-methyl THF (160 mL) were added 4-DMAP (4.1 g, 34 mmol) and $(Boc)_2O$ (38 mL, 169 mmol) dropwise at 0 °C and the reaction mixture was stirred at 80 °C for 3 h. The reaction mixture was diluted with water (100 mL) and extracted with EtOAc (3 × 200 mL). The combined organic layers were washed with cold water (200 mL) and brine (200 mL), dried over anhydrous $Na_2SO_4$ and concentrated under reduced pressure to obtain the crude product which was purified by flash column chromatography on silica gel (230 mesh size) using 40% EtOAc in petroleum ether as eluent to afford N,N-di-Boc 4-bromopyridine-2-sulfonamide **(Int-A1)** (7.0 g). LCMS m/z $[M+H]^+$= 393.1 (calc. = 393.0).

Svnthesis of 4-amino-N,N-bis(4-methoxybenzyl)pyridine-2-sulfonamide **(Int-A2):**

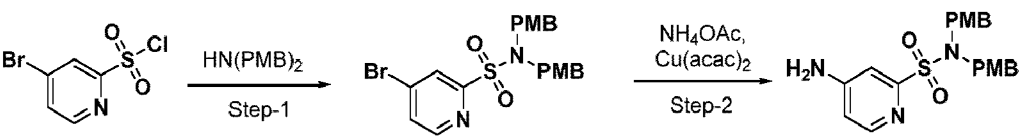


Step-1: To a solution of 4-bromopyridine-2-sulfonyl chloride ( 2.3 g, 9.01mmol, 1.0 equiv.) in DCM (40 mL) were added DIPEA (4.47 mL, 27.03 mmol, 3.0 equiv.) and bis[(4-methoxyphenyl)methyl]amine (1.3 g, 5.40 mmol, 0.6 equiv.) at 0 °C. The reaction mixture was stirred at ambient temperature for 2 h. The reaction mixture was diluted with ice water and extracted with DCM (3x100 mL). The combined organic layer was washed with brine, dried over anhydrous $Na_2SO_4$, filtered and evaporated under reduced pressure to get crude product, which was purified by column chromatography (silica gel; 0-20% ethylacetate in hexane as eluent) to yield 4-bromo-N-[(3-methoxyphenyl)methyl]-N-[(4-methoxyphenyl)methyl]pyridine-2-sulfonamide. Yield: 32% (0.8 g, 2.89 mmol)

Step-2: A solution of 4-bromo-N-[(3-methoxyphenyl)methyl]-N-[(4-methoxyphenyl)methyl]pyridine-2-sulfonamide (0.75 g, 1.58 mmol, 1 equiv.) in NMP (20 mL) was degassed with argon for 20 minutes. Cesium carbonate (1.5 g, 4.75 mmol, 3 equiv.), ammonium acetate (1.2 g, 15.82 mmol, 10 equiv.), copper acetylacetone (0.125 g, 0.47 mmol, 0.3 equiv.) and 2-acetylcyclohexane (0.13 g, 0.95 mmol, 0.6 equiv.) were then added at ambient temperature. The reaction mixture was heated to 90 °C for 16 h in a sealed tube. The reaction mixture was cooled to ambient temperature and diluted with water. The aqueous part was extracted with ethyl acetate (3x40 mL). The combined organic layers were washed with brine, dried over anhydrous $Na_2SO_4$, filtered and evaporated under reduced pressure to get the crude product, which was purified by column chromatography (silica gel; 0-50% ethylacetate in hexane as eluent) to yield 4-amino-N,N-bis[(4-methoxyphenyl)methyl]pyridine-2-sulfonamide (**Int-A2**). Yield: 77% (0.50 g, 1.21 mmol).

Synthesis of 5-(methylsulfonyl)thiophen-2-amine **(Int-A4):**

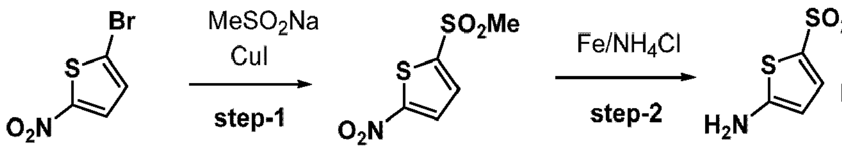


**Int-A4**

Step-1: To a mixture of 2-bromo-5-nitrothiophene (500 mg, 2.40 mmol, 1.00 eq.) and $Me_SO_2Na$ (367 mg, 3.60 mmol, 1.50 eq.) in DMF (6 mL) was added CuI (317 mg, 4.80 mmol, 2.00 eq.) at ambient temperature and the reaction mixture was heated to 110 °C for 8h. The reaction mixture was filtered through celite and the filtrate was diluted with water (50 mL) and extracted with EtOAc (100 mL). The organic layer was washed with cold brine (50 mL), dried over $Na_2SO_4$ and concentrated under reduced pressure to get the crude product which was purified by flash column chromatography (silica gel; 40% EtOAc/hexanes) to yield 2-(methylsulfonyl)-5-nitrothiophene (110 mg, 0.531 mmol, 22%).

Step-2: To a solution of 2-(methylsulfonyl)-5-nitrothiophene (110 mg, 0.53 mmol, 1.00 eq.) in MeOH (3 mL) were added sat. aq. $NH_4Cl$ (3 mL) and Fe powder (148 mg, 2.65 mmol, 5.00 eq.) and the resulting mixture stirred at ambient temperature for 16 h. The reaction mixture was filtered through celite and the filtrate was diluted with water (25 mL) and extracted with DCM (50 mL). The organic layer was washed with brine (25 mL), dried over anhydrous $Na_2SO_4$ and concentrated under reduced pressure to get crude 5-(methylsulfonyl)thiophen-2-amine **(Int-A4)** (70 mg, 0.395 mmol, 74%) which was directly used in the next step. $^1H$ NMR (400 MHz, DMSO-*d6*): δ (ppm) = 7.22 (d, 1H), 6.71 (s, 2H), 5.90 (d, 1H), 3.11 (s, 3H).

Synthesis of ethyl 7-(difluoromethyl)-2H-indazole-3-carboxylate (**Int-A5):**

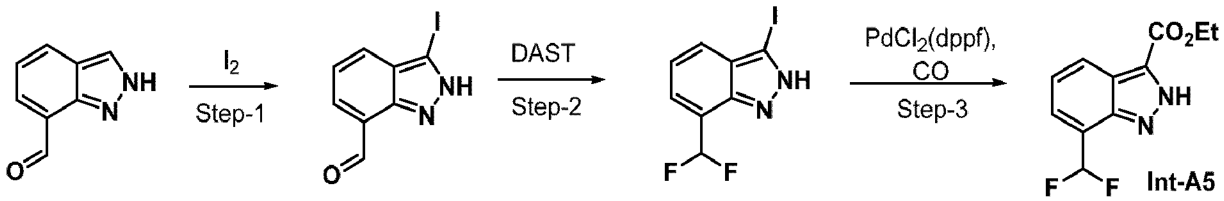


Int-A5

Step-1: To a solution of 2H-indazole-7-carbaldehyde (0.2 g, 1.36 mmol, 1 equiv.) in dry DMF (3.5 mL) was added $K_2CO_3$ (0.56 g, 4.0 mmol, 3 equiv.) and iodine (0.69 g, 2.72 mmol, 2 equiv.) at 0 °C. The reaction mixture was stirred at ambient temperature for 16 h. The reaction mixture was diluted with ice water and extracted with ethyl acetate (3 × 50 mL). The combined organic layers were washed with sat. $Na_2S_2O_3$ solution, dried over anhydrous $Na_2SO_4$ and concentrated to get the crude product which was purified by column chromatography (silica gel gel 100-200 mesh, 10-20% ethyl acetate in hexane as eluent) to yield 3-iodo-2H-indazole-7-carbaldehyd. Yield: 70% (0.26 g, 0.26 mmol).

Step-2: To a solution 3-iodo-2H-indazole-7-carbaldehyde (2.0 g, 7.38 mmol, 1 equiv.) in DCM (60 mL) was added DAST (2.2 mL, 18.44 mmol, 2.5 equiv.) dropwise at 0 °C. The reaction mixture was stirred at ambient temperature for 16 h. Reaction mixture was quenched with sat. $NaHCO_3$ solution at 0 °C and the mixture was stirred at ambient temperature for 10 minutes. The reaction mixture was extracted with DCM (3×250 mL). The combined organic layers were dried over anhyd. $Na_2SO_4$ and concentrated to get the crude product which was purified by column chromatography (silica gel gel 100-200 mesh, 0-20% ethyl acetate in hexane as eluent) to yield 7-(difluoromethyl)-3-iodo-2H-indazole. Yield: 30% (0.65 g, 2.2 mmol).

Step-3: To a solution of 7-(difluoromethyl)-3-iodo-2H-indazole (0.3 g, 1.02 mmol, 1 equiv.) in ethanol (20 mL) was added NaOAc (0.25 g, 3.06 mmol, 3 equiv.) at ambient temperature. The reaction mixture was degassed with nitrogen for 15 min followed by the addition of $PdCl_2$(dppf) (0.075 g, 0.1 mmol, 0.1 equiv.) at ambient temperature. The reaction mixture was heated in a Parr autoclave vessel at 80 °C under 5.5 bar pressure of CO gas for 16 h. The reaction mixture was cooled to ambient temperature, filtered through celite and the filtrate was concentrated to get the crude product which was purified by column chromatography (silica gel gel 100-200 mesh, 0-30% EA in hexane as eluent) to yield ethyl 7-(difluoromethyl)-2H-indazole-3-carboxylate. Yield: 82% (0.20 g, 0.83 mmol), LCMS m/z $[M+H]^+$= 241.0 (calc. = 241.1).

Synthesis of methyl 2-(cyclobutylmethyl)-2*H*-indazole-3-carboxylate **(Int-B1):**

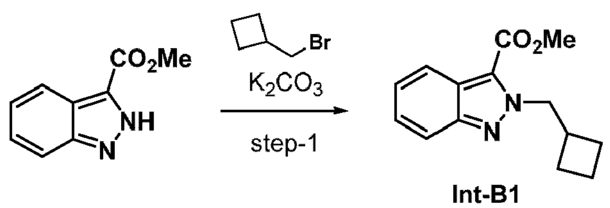


Int-B1

Step-1: To a stirred solution of methyl 2*H*-indazole-3-carboxylate (1.50 g, 8.52 mmol, 1.00 eq.) in acetonitrile (20 mL) was added $K_2CO_3$ (3.50 g, 25.6 mmol) and (bromomethyl)cyclobutane (1.80 g, 12.8 mmol) at ambient temperature and the reaction mixture was stirred at 90 °C under argon atmosphere for 3h. The reaction progress was monitored by

TLC. The reaction mixture was diluted with water (50 mL) and extracted with EtOAc (2 × 100 mL). The combined organic layers were washed with water (100 mL), brine (100 mL), dried over anhydrous $Na_2SO_4$ and concentrated under reduced pressure to get methyl 2-(cyclobutylmethyl)-2*H*-indazole-3-carboxylate (**Int-B1,** 400 mg, 70%, crude). The resulting crude product was used in the next steps without further purification. LCMS m/z $[M+H]^+$= 245.1 (calc. = 245.1).

Svnthesis of 2-(cyclohexylmethyl)-6-fluoro-2*H*-indazole-3-carboxylic acid **(Int-B2):**

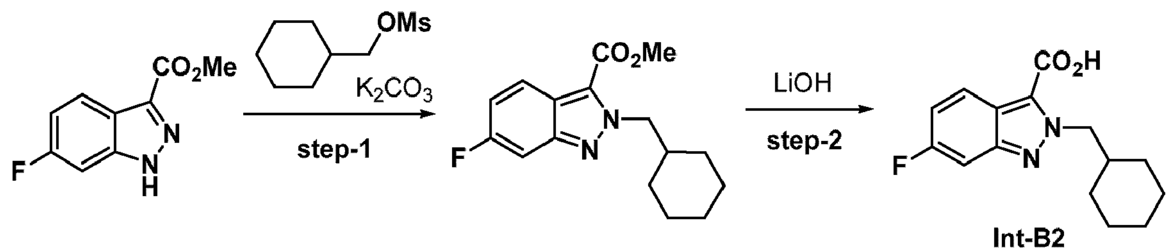


**Int-B2**

Step-1: To a stirred solution of methyl 6-fluoro-1*H*-indazole-3-carboxylate (0.40 g, 2.06 mmol, 1.00 eq.) in DMF (15 mL) were added $K_2CO_3$ (0.42 g, 3.09 mmol) and cyclohexylmethyl methanesulfonate (0.59 g, 3.09 mmol) at ambient tmperature and the reaction mixture was stirred at 90 °C for 16 h. The reaction mixture was cooled to ambient temerature, quenched with crushed ice and the aqueous phase was extracted with EtOAc (3 × 100 mL). The combined organic layers were washed with brine, dried over $Na_2SO_4$ and concentrated under reduced pressure to get the crude product which was purified by flash column chromatography (silica gel, 0-10% EtOAc in hexanes). Methyl 2-(cyclohexylmethyl)-6-fluoro-2*H*-indazole-3-carboxylate was isolated in 33% yield (0.2 g, 0.688 mmol) yield.

Step-2: To a stirred solution of methyl 2-(cyclohexylmethyl)-6-fluoro-2*H*-indazole-3-carboxylate (0.30 g, 1.03 mmol, 1.00 eq.) in THF (8 mL) and water (2.5 mL) was added LiOH monohydrate (0.07 g, 1.55mmol) portionwise at 0 °C. The reaction mixture was stirred at ambient temperature for 16 and was then concentrated to furnish a solid residue which was diluted with water and washed with EtOAc (20 mL). The aqueous phase was cooled with ice and acidified with sat. aq. $NaHSO_4$ to pH ~ 3-4 followed by extraction with EtOAc (3 × 100 mL). The combined organic layers were washed with brine, dried over $Na_2SO_4$ and concentrated under reduced pressure to yield 2-(cyclohexylmethyl)-6-fluoro-2*H*-indazole-3-carboxylic acid (**Int-B2**). Yield: 98% (0.28 g, 1.01 mmol). LCMS: m/z $[M-H]^-$ = 274.9 (calc. 275.1).

Svnthesis of 6-fluoro-2-((tetrahydro-,2*H*-pyran-2-yl)methyl)-2*H*-indazole-3-carboxylic acid (**Int-B4**):

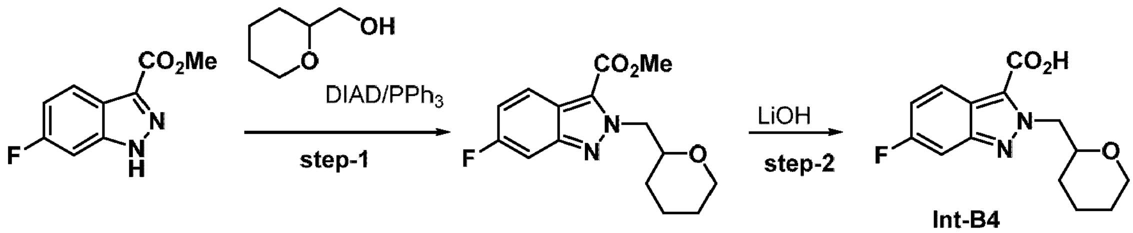


**Int-B4**

Step-1: To a solution of methyl 6-fluoro-1*H*-indazole-3-carboxylate (500 mg, 2.83 mmol, 1.00 eq.) and (tetrahydro-2H-pyran-2-yl)methanol (0.6 mL, 5.67 mmol, 2.00 eq.) in THF (20 mL) was added $PPh_3$ (1.5 g, 5.67 mmol, 2.00 eq.) at ambient temperature and the reaction mixture was stirred for 15 min followed by addition of DIAD (1.1 mL, 5.67 mmol, 2.00 eq.) at 0 °C. The resulting mixture was stirred at ambient temperature for 16 h and was then concentrated to dryness to get the crude product which was purified by flash column chromatography (silica gel; 5% EtOAc/hexanes) to afford methyl 6-fluoro-2-((tetrahydro-2H-pyran-2-yl)methyl)-2H-indazole-3-carboxylate (420 mg, 1.43 mmol, 50%).

Step-2: To a solution of methyl 6-fluoro-2-((tetrahydro-2H-pyran-2-yl)methyl)-2H-indazole-3-carboxylate (420 mg, 1.43 mmol, 1.00 eq.) in THF/$H_2O$ (10 mL, 4:1) was added LiOH monohydrate (181 mg, 4.31 mmol, 3 eq.) at 0 °C. The reaction mixture was stirred at ambient temperature for 16 h and was then concentrated to dryness, diluted with water (12 mL), acidified with aq. $NaHSO_4$ to pH = 4 and extracted with EtOAc (50 mL). The organic layer was dried over $Na_2SO_4$ and concentrated under reduced pressure to give 6-fluoro-2-((tetrahydro-2H-pyran-2-yl)methyl)-2H-indazole-3-carboxylic acid (**Int-B4**) (270 mg, 0.97 mmol, 67%). LCMS m/z $[M+H]^+$ = 278.5 (calc. 279.1).

Svnthesis of methyl 2-(cycloheptylmethyl)-2*H*-indazole-3-carboxylate **(Int-B8):**

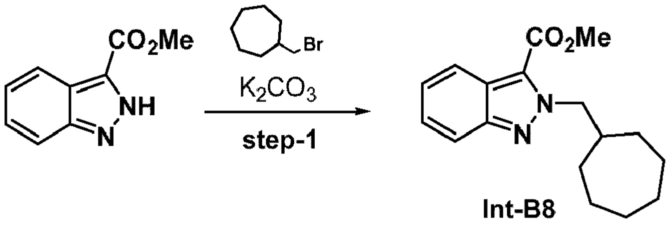


**Int-B8**

Step-1: To a stirred solution of methyl 2*H*-indazole-3-carboxylate (300 mg, 1.70 mmol, 1.00 eq.) in acetonitrile (10 mL) was added $K_2CO_3$ (706 mg, 5.11mmol) and (bromomethyl)cycloheptane (485 mg, 2.56 mmol) at ambient temperature and the reaction mixture was stirred at 90 °C under an inert atmosphere for 3 h. The reaction mixture was diluted with water (50 mL) and extracted with EtOAc (2x100 mL). The combined organic layers were washed with water (50 mL) and brine (50 mL), dried over anhydrous $Na_2SO_4$ and concentrated under reduced pressure to give methyl-2-(cycloheptylylmethyl)-2*H*-indazole-3-carboxylate (**Int-B8**) (100 mg) which was used in subsequent transformations without further purification. LCMS: m/z $[M+H]^+$ = 287.2 (calc. 287.2).

Svnthesis of 2-((tetrahydrofuran-2-yl)methyl)-2*H*-indazole-3-carboxylic acid **(Int-B11):**

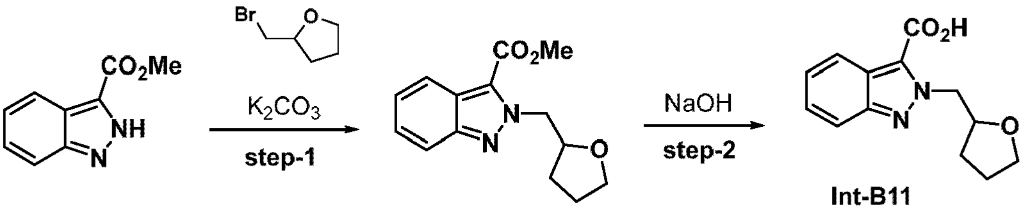


**Int-B11**

Step-1: To a stirred solution of methyl 2*H*-indazole-3-carboxylate (4.00 g, 22.7 mmol, 1.00 eq.) in acetonitrile (80 mL) were added $K_2CO_3$ (9.40 g, 68.2 mmol) and racemic 2-(bromomethyl)tetrahydrofuran (4.50 g, 27.3 mmol) at ambient temperature. The reaction mixture was heated to 80 °C for 16 h. The mixture was then filtered and the filter cake was washed with EtOAc (2 × 100 mL). The combined filtrate was washed with water (100 mL) and brine (100 mL), dried over anhydrous $Na_2SO_4$ and concentrated under reduced pressure to yield the crude product (~4.5 g). Another batch of 4.0 g of methyl 2*H*-indazole-3-carboxylate was converted to 4.5 g of crude product. The combined crude product was purified by flash column chromatography over silica gel (100 - 200 mesh) using 10% EtOAc in petroleum ether as an eluent to afford methyl 2-((tetrahydrofuran-2-yl)methyl)-2*H*-indazole-3-carboxylate (1.5 g, 13%).

Step-2: To a stirred solution of methyl 2-((tetrahydrofuran-2-yl)methyl)-2*H*-indazole-3-carboxylate (200 mg, 0.769 mmol, 1.00 eq.) in THF/MeOH/$H_2O$ (1:1:0.5, 25 mL) was added 1N aq. NaOH (123 mg, 3.076 mmol) at ambient temperature. The reaction mixture was stirred at 60 °C for 3 h. The reaction mixture was concentrated under reduced pressure to give a crude residue which was diluted with water (10 mL), acidified to pH ~2 with 1N aq. HCl solution and extracted with 5% MeOH in DCM (2 × 50 mL). The combined organic layers were washed with water (50 mL) and brine (50 mL), dried over anhydrous $Na_2SO_4$ and concentrated under reduced pressure to afford 2-((tetrahydrofuran-2-yl)methyl)-2*H*-indazole-3-carboxylic acid (**Int-B11**) (180 mg, 95%). Another batch of methyl 2-((tetrahydrofuran-2-yl)methyl)-2*H*-indazole-3-carboxylate (500 mg) was converted to **Int-B11** (350 mg). LCMS: m/z $[M+H]^+$ = 247.2 (calc. 247.1).

The following intermediates were prepared by analogy to the procedures described above:

| Intermediate structure/code | Procedure | Analytics |
|---|---|---|
| 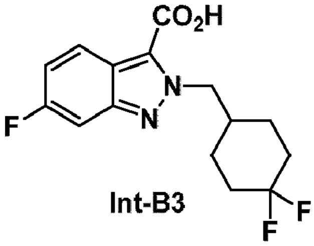 Int-B3 | in analogy to the synthesis of **Int-B2** using 5 methyl 6-fluoro-1*H*-indazole-3-carboxylate (4.63 mmol) and (4,4-difluorocyclohexyl)methyl methanesulfonate in the first step | $^1H$ NMR (400 Hz, DMSO-*d6*): 13.87 (br s, 1H), 8.04 (dd, 1H), 7.20 (dt, 1H), 4.78 (d, 2H), 2.15 (m, 1H), 1.98 (m, 2H), 1.84 - 1.67 (m, 2H), 1.62 - 1.55 (m, 2H), 1.376 - 1.29 (m, 2H). |
| 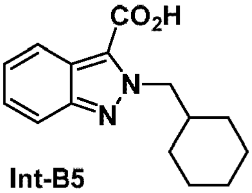 Int-B5 | in analogy to the synthesis of **Int-B4** using methyl 1H-indazole-3-carboxylate (28.40 mmol) and cyclohexylmethanol in the first step | LCMS: m/z $[M+H]^+$ = 258.7 (calc. = 259.1) |
| 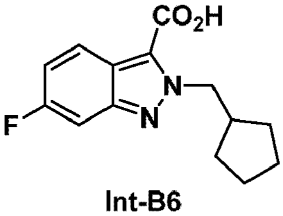 Int-B6 | in analogy to the synthesis of **Int-B4** using methyl 6-fluoro-1H-indazole-3-carboxylate (2.57 mmol) and cyclopentylmethanol in the first step | LCMS: m/z $[M+H]^+$ = 263.2 (calc. 263.1) |
| 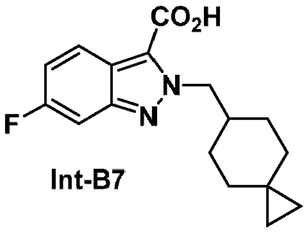 Int-B7 | in analogy to the synthesis of **Int-B4** using methyl 6-fluoro-1H-indazole-3-carboxylate (1.3 mmol), spiro[2.5]octan-6-ylmethanol in the first step | LCMS: m/z $[M+H]^+$ = 303.1 (calc. 303.1) |
| 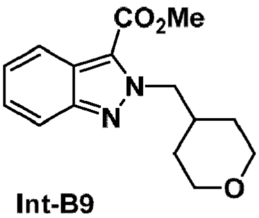 Int-B9 | in analogy to the synthesis of **Int-B8** using methyl 2*H*-indazole-3-carboxylate and 4-(bromomethyl)tetrahydro-2*H*-pyran | LCMS: m/z $[M+H]^+$ = 275.1 (calc. 275.1) |
| 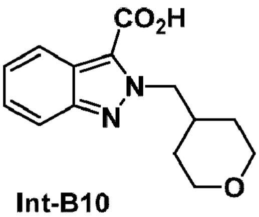 Int-B10 | starting from **Int-B9** and in analogy to step-2 of the synthesis of **Int-B2** | LCMS: m/z $[M+H]^+$ = 261.2 (calc. 261.1) |
| 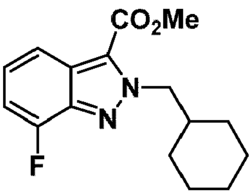 Int-B12 | in analogy to the synthesis of **Int-B8** using methyl 7-fluoro-2*H*-indazole-3-carboxylate and (bromomethyl)cyclohexane | LCMS: m/z $[M+H]^+$ = 291.1 (calc. 291.1) |

(continued)

| Intermediate structure/code | Procedure | Analytics |
|---|---|---|
| 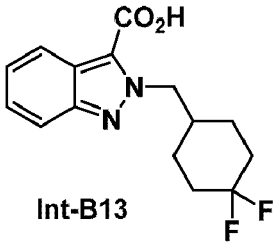 **Int-B13** | in analogy to the synthesis of **Int-B11** using methyl 2*H*-indazole-3-carboxylate and 4-(bromomethyl)-1,1-difluorocyclohexane in the first step | no analytics |
| 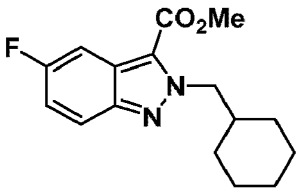 **Int-B14** | in analogy to the synthesis of **Int-B8** using methyl 5-fluoro-2*H*-indazole-3-carboxylate and (bromomethyl)cyclohexane | LCMS: m/z $[M+H]^+$ = 291.1 (calc. 291.1) |
| 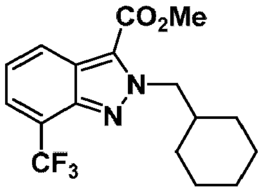 **Int-B15** | in analogy to the synthesis of **Int-B8** using methyl 7-(trifluoromethyl)-1*H*-indazole-3-carboaylate and (bromomethyl) cyclohexane | LCMS: m/z $[M+H]^+$ = 341.2 (calc. 341.1) |
| 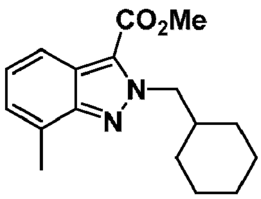 **Int-B16** | in analogy to the synthesis of **Int-B8** using methyl 7-methyl-1*H*-indazole-3-carboxylate and (bromomethyl) cyclohexane | LCMS: m/z $[M+H]^+$ = 287.2 (calc. 287.2) |
| 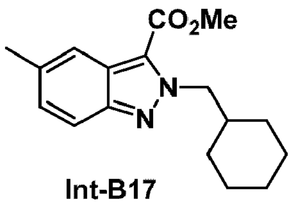 **Int-B17** | in analogy to the synthesis of Int-B8 using methyl 5-methyl-1*H*-indazole-3-carboxylate and (bromomethyl) cyclohexane | LCMS: m/z $[M+H]^+$ = 287.2 (calc. 287.2 |
| 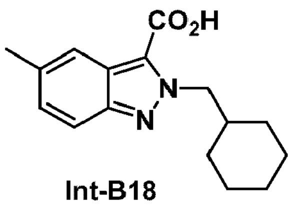 **Int-B18** | starting from **Int-B17** and in analogy to step-2 of the synthesis of **Int-B11** | LCMS: m/z $[M+H]^+$ = 273.2 (calc. 273.2) |
| 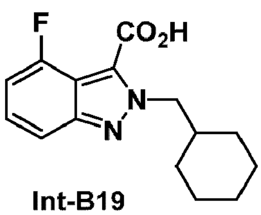 **Int-B19** | in analogy to the synthesis of **Int-B11** using methyl 4-fluoro-2//- indazole-3-carboxylate and (bromomethyl)cyclohexane in the first step | LCMS: m/z $[M+H]^+$ = 277.1 (calc. 277.1) |

(continued)

| Intermediate structure/code | Procedure | Analytics |
|---|---|---|
| 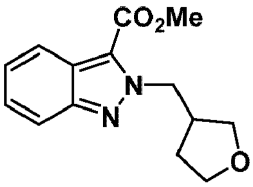 **Int-B20** | in analogy to step-1 of the synthesis of **Int-B11** using methyl 2H-indazole-3-carboxylate and racemic 3-(bromomethyl) tetrahydrofurane | LCMS: m/z $[M+H]^+$ = 261.3 (calc. 261.1) |
| 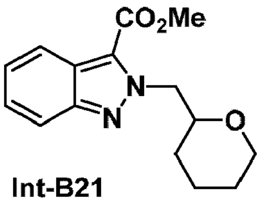 **Int-B21** | in analogy to step-1 of the synthesis of **Int-B11** using methyl 2*H*-indazole-3-carboxylate and racemic tetrahydro-2*H*-pyran-2-yl)methyl methanesulfonate | LCMS: m/z $[M+H]^+$ = 275.1 (calc. 275.1) |
| 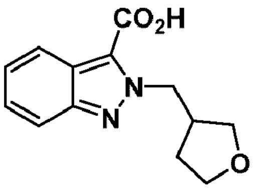 **Int-B22** | starting from **Int-B20** and in analogy to step-2 of the synthesis of **Int-B11** | LCMS: m/z $[M+H]^+$ = 247.1 (calc. 247.1) |
| 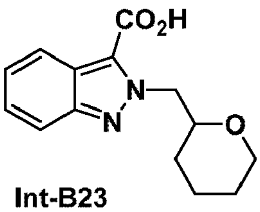 **Int-B23** | starting from **Int-B21** and in analogy to step-2 of the synthesis of **Int-B11** | LCMS: m/z $[M+H]^+$ = 261.1 (calc. 261.1) |
| 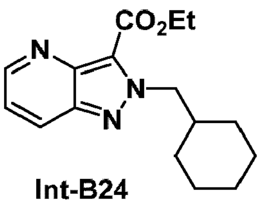 **Int-B24** | in analogy to the synthesis of **Int-B8** using methyl 2*H*-pyrazolo[4,3-b]pyridine-3-carboxylate and (bromomethyl) cyclohexane | LCMS: m/z $[M+H]^+$ = 288.4 (calc. 288.2) |
| 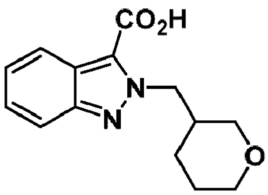 **Int-B25** | in analogy to the synthesis of **Int-B11** using methyl 2//-indazolc-3-carboxylate and 3-(Bromomethyl)tetrahydro-2*H*-pyran in the first step | LCMS: m/z $[M+H]^+$ = 261.1 (calc. 261.1) |
| 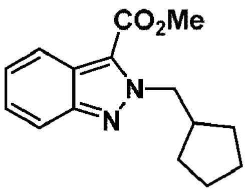 **Int-B26** | in analogy to the synthesis of **Int-B8** using methyl 2*H*-indazole-3-carboxylate and (bromomethyl)cyclopentane | LCMS: m/z $[M+H]^+$ = 259.1 (calc. 259.1) |

(continued)

| Intermediate structure/code | Procedure | Analytics |
|---|---|---|
| 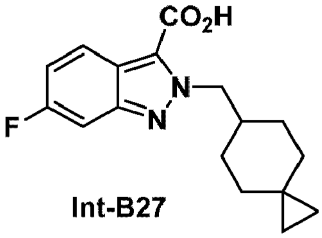 Int-B27 | in analogy to the synthesis of **Int-B4** using methyl 6-fluoro-1*H*-indazole-3-carboxylate and spiro[2.5]octan-6-ylmethanol in the first step | LCMS: m/z $[M+H]^+$ = 303.1 (calc. 303.1) |
| 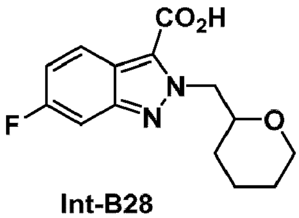 Int-B28 | in analogy to the synthesis of **Int-B4** using methyl 6-fluoro-1*H*-indazole-3-carboxylate and racemic (tetrahydro-2*H*-pyran-2yl) methanol in the first step | LCMS: m/z $[M+H]^+$ = 278.5 (calc. 279.1) |
| 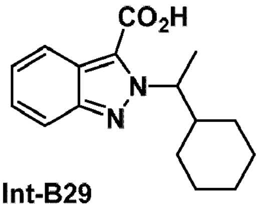 Int-B29 | in analogy to the synthesis of **Int-BII-rac** using methyl 2*H*-indazole-3-carboxylate and racemic (1-bromoethyl)cyclohexane in the first step | LCMS: m/z $[M+H]^+$ = 273.4 (calc. 273.2) |
| 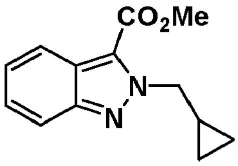 Int-B30 | in analogy to the synthesis of **Int-B8** using methyl 5-methyl-1*H*-indazole-3-carboxylate and (bromomethyl) cyclopropane | LCMS: m/z $[M+H]^+$ = 231.2 (calc 231.1) |
| 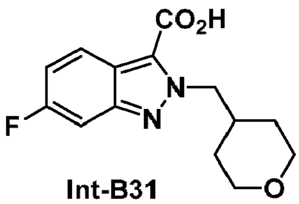 Int-B31 | in analogy to the synthesis of **Int-B4** using methyl 6-fluoro-1*H*-indazole-3-carboxylate and (tetrahydro-2*H*-pyran-4-yl)methanol in the first step | LCMS: m/z $[M+H]^+$ = 279.4 (calc. 279.1) |
| 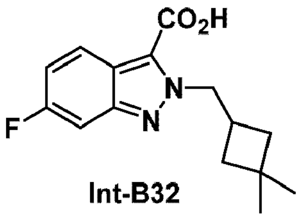 Int-B32 | in analogy to the synthesis of **Int-B4** using methyl 6-fluoro-1*H*-indazole-3-carboxylate and (3,3-dimethylcyclobutyl)methanol in the first step | LCMS: m/z $[M+H]^+$ = 277.2 (calc. 277.1) |
| 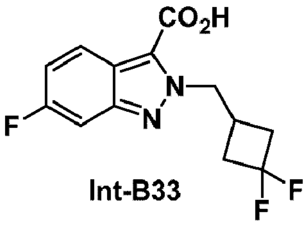 Int-B33 | in analogy to the synthesis of **Int-B4** using methyl 6-fluoro-1*H*-indazole-3-carboxylate and (3,3-difluorocyclobutyl)methanol in the first step | LCMS: m/z $[M+H]^+$ = 285.3 (calc. 285.1) |

(continued)

| Intermediate structure/code | Procedure | Analytics |
|---|---|---|
| 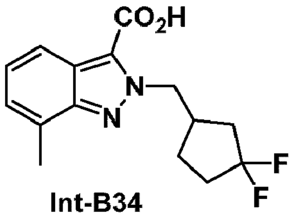 Int-B34 | in analogy to the synthesis of **Int-B11** procedure using (3,3-difluorocyclopentyl) methyl methanesulfonate and methyl 7-methyl-2H-indazole-3-carboxylate in the first step | LCMS: m/z $[M+H]^+$ = 295.3 (calc. 295.1) |
| 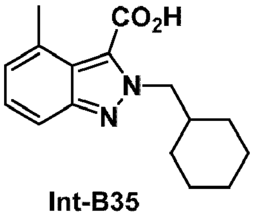 Int-B35 | in analogy to the synthesis of **Int-B2** procedure using cyclohexylmethyl methanesulfonate and methyl 4-methyl-2H-indazole-3 -carboxylate in the first step | LCMS: m/z $[M+H]^+$ = 273.4 (calc. 273.1) |

Synthesis of 4-chloro-2-((3,3-difluorocyclopentyl)methyl)-7-methyl-2H-indazole-3-carboxylic acid (**Int-B36**):

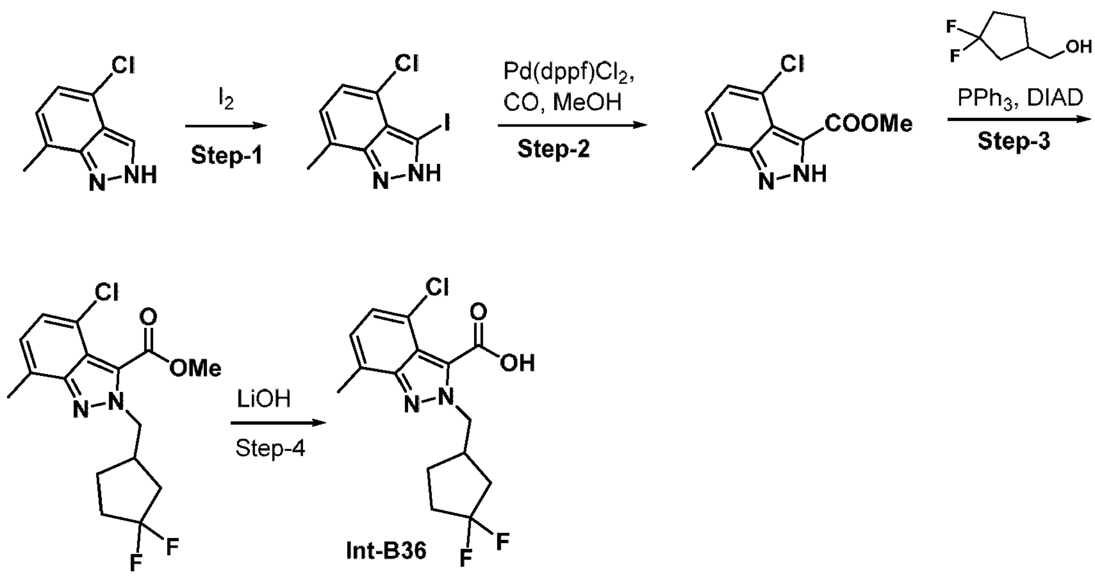

Int-B36

Step-1: To a stirred solution of 4-chloro-7-methyl-2H-indazole (2.1 g, 12.65 mmol) in DMF (10 mL) was added NIS (3.4 g, 15.18 mmol) followed by KOH (1.41 g, 25.3 mmol) portion wise at ambient temperature. The reaction mixture was stirred at 80 °C for 4 h. The reaction mixture was quenched with ice cold water (40 mL). The precipitated solid was filtered off and dried under reduced pressure to afford 4-chloro-3-iodo-7-methyl-2H-indazole (3.1 g).

Step 2: To a stirred solution of 4-chloro-3-iodo-7-methyl-2H-indazole (3.0 g, 10.27 mmol) in MeOH/toluene (1:1, 20 mL) was added $Et_3N$ (2.9 mL, 20.54 mmol) and the mixture was degassed using argon for 20 min. Then, $PdCl_2(dppf)\cdot DCM$ (1.5 g, 20.54 mmol) was added and the mixture was heated to 100 °C for 12 h. The reaction mixture was filtered through a celite bed, and the celite bed was washed with EtOAc (20 mL). The filtrate was concentrated under reduced pressure to get a residue which was diluted with water (50 mL) and extracted with EtOAc (2×50 mL). The combined organic layers were washed with brine (20 mL), dried over anhydrous $Na_2SO_4$ and concentrated under reduced pressure to get the crude product which was purified by column chromatography (silica gel 230-400 mesh, 30% ethyl acetate in pet ether as an eluent) to afford methyl 4-chloro-7-methyl-2H-indazole-3-carboxylate (3.0 g).

Step 3: To a stirred solution of TPP (5.8 g, 22.3 mmol) in THF (1.0 mL) was added DIAD (4.5 g, 22.3 mmol) dropwise at 0 °C for 5 min and then the mixture was stirred for 20 min. Then methyl 4-chloro-7-methyl-2H-indazole-3-carboxylate (2.0 g, 8.92 mmol) and (3,3-difluorocyclopentyl)methanol (1.4 g, 10.71 mmol) were added and the reacton mixture was stirred for 12 h at ambient temperature. The reaction mixture was quenched with water (50 mL) and extracted with EtOAc (3×50 mL). The combined organic layers were washed with brine (20 mL), dried over anhydrous $Na_2SO_4$, filtered and concentrated under reduced pressure to get crude product which was purified by column chromatography (silica gel 230-400 mesh, 20% EA in pet-ether as eluent) to afford methyl 4-chloro-2-((3,3-difluorocyclopentyl)methyl)-7-methyl-2H-indazole-3-carboxylate (1.6 g).

Step 4: To a stirred solution of methyl 4-chloro-2-((3,3-difluorocyclopentyl)methyl)-7-methyl-2H-indazole-3-carboxylate (1.5 g, 4.545 mmol) in THF:$H_2O$ (1:1, 10.0 mL) was added LiOH (381 mg, 9.09 mmol) at ambient temperature and the mixture was stirred at ambient temperature for 24 h. The reaction mixture was acidified to pH ~3 using 1N aq. HCl solution and extracted with EtOAc (3×20 mL). The combined organic layers were washed with brine (20 mL), dried over anhydrous $Na_2SO_4$, and concentrated under reduced pressure to afford 4-chloro-2-((3,3-difluorocyclopentyl)methyl)-7-methyl-2H-indazole-3-carboxylic acid (1.64 g). LCMS: m/z $[M+H]^+$ = 329.1 (calc. 329.1).

Synthesis of 4-chloro-7-methyl-2-((tetrahydro-2H-pyran-2-yl)methyl)-2H-indazole-3-carboxylic acid (**Int-B37**):

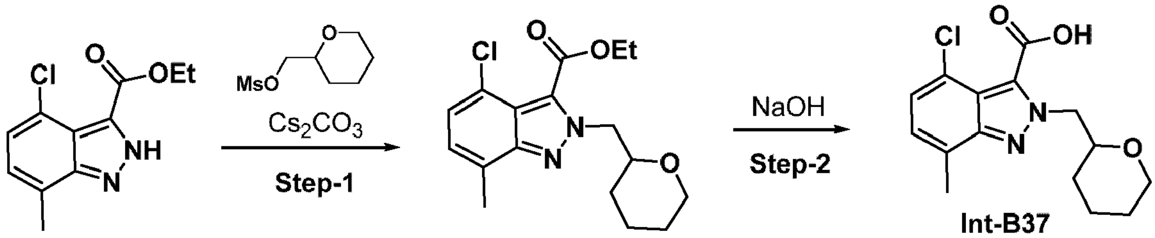


**Int-B37**

Step-1: To a solution of ethyl 4-chloro-7-methyl-2H-indazole-3-carboxylate (2.5 g, 10.5 mmol, 1.0 equiv.) and (oxan-2-yl)methyl methanesulfonate (2.8 g, 15.75 mmol , 1.5 equiv.) in DMF (20 mL) was added $Cs_2CO_3$ (10.2 g, 31.5 mmol, 3.0 equiv.) at ambient temperature and the reaction was stirred at 80 °C for 16 h. The reaction mixture was quenched with cold water (100 mL) and extracted with ethyl acetate (3 × 100 mL). The combined organic layers were washed with cold brine (100 mL), dried over sodium sulfate and concentrated under reduced pressure to get the crude material which was purified by column chromatography (silica gel, 0-20% ethyl acetate in hexane as eluent) to yield ethyl 4-chloro-7-methyl-2-[(oxan-2-yl)methyl]-2H-indazole-3-carboxylate. Yield: 38%

Step 2: To a solution of ethyl 4-chloro-7-methyl-2-[(oxan-2-yl)methyl]-2H-indazole-3-carboxylate (900 mg, 2.67 mmol, 1.0 equiv.) in MeOH (25 mL) was added 2N NaOH (5 mL) at ambient temperature and the reaction mixture was heated to 70 °C for 3 h. The reaction mixture was concentrated under reduced pressure, diluted with water (30 mL), acidified with sat. $KHSO_4$ solution up to pH-3-4, extracted with DCM (3 × 100 mL). The combined organic layers were dried over sodium sulfate and concentrated to get 4-chloro-7-methyl-2-[(oxan-2-yl)methyl]-2H-indazole-3-carboxylic acid. Yield: 91% (750 g, 2.43 mmol). LCMS: m/z $[M+H]^+$ = 309.2 (calc. 309.1).

Svnthesis of 7-cyclopropyl-2-((tetrahydro-2H-pyran-4-yl)methyl)-2H-indazole-3-carboxylic acid (**Int-B38**):

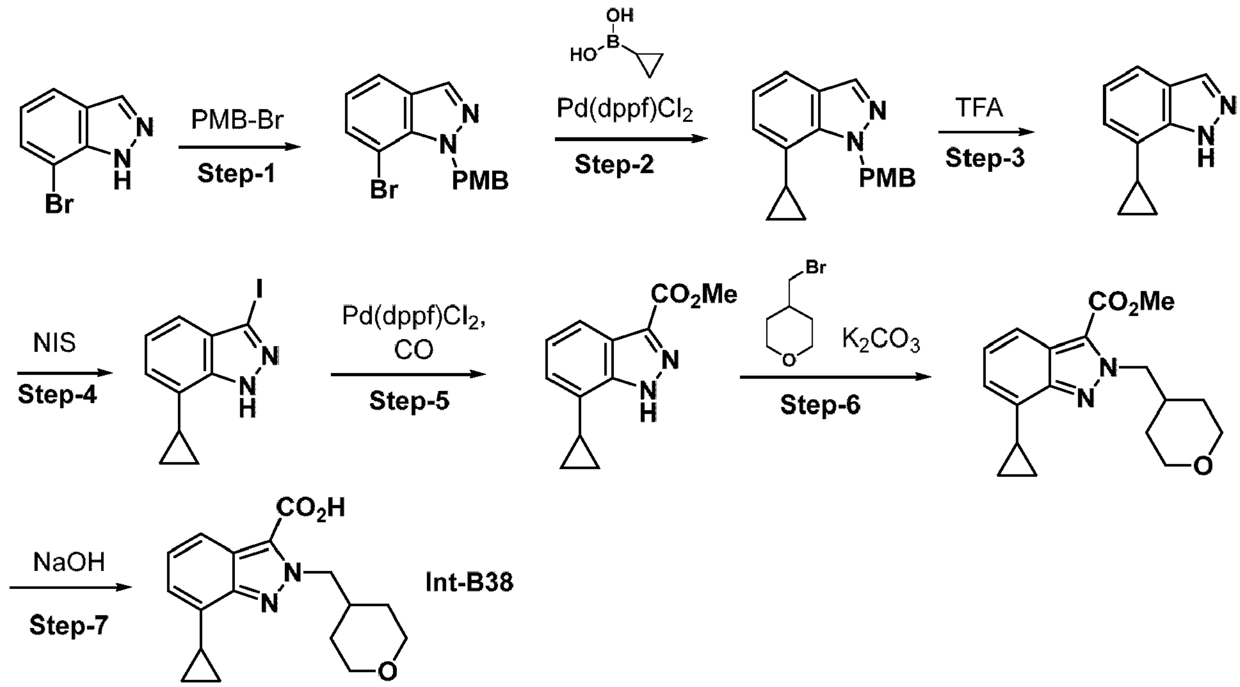


Int-B38

Step-1: A solution of 7-bromo-1H-indazole (2.5 g, 12.6 mmol), para methoxybenzyl chloride (2.9 g, 18.9 mmol) and $K_2CO_3$ (5.2 g, 37.8 mmol) in $CH_3CN$ (50 mL) was heated to 80 °C for 16 h. The reaction mixture was allowed to cool to ambient temperature and was filtered through a celite pad. The filtrate was concentrated under reduced pressure and the obtained residue was diluted with ethyl acetate (500 mL). The organic layer was washed with water (2 × 150 mL), dried over anhyd. $Na_2SO_4$ and concentrated under reduced pressure to afford 7-bromo-1-(4-methoxybenzyl)-1H-indazole (4.53 g, crude).

Step-2: In a sealed tube a mixture of 7-bromo-1-(4-methoxybenzyl)-1H-indazole (4.5 g, 14.19 mmol), cyclopropylboronic acid (1.8 g, 21.29 mmol), $K_3PO_4$ (9.0 g, 42.5 mmol) in 1,4-dioxane (20 mL) and water (20 mL) was degassed and purged with nitrogen gas for 15 min. The mixture was then heated to 100 °C for 16 hours under a nitrogen atmosphere. The reaction mixture was filtered, the filtrate was diluted with water (50 mL) and extracted with ethyl acetate (2 × 100 mL). The combined organic layers were dried over anhydrous $Na_2SO_4$ and concentrated under reduced pressure to afford 7-cyclopropyl-1-(4-methoxybenzyl)-1H-indazole (4.8 g, crude).

Step-3: In a sealed tube, 7-cyclopropyl-1-(4-methoxybenzyl)-1H-indazole (4.8 mmol, 17.26 mmol) was dissolved in trifluoroacetic acid (24 mL) at 0 °C under a nitrogen atmosphere. The reaction mixture was heated to 90 °C for 16 h. The reaction mixture was then adjusted with saturated $NaHCO_3$ solution at 0 °C to pH ~8 and extracted with ethyl acetate (2 × 200 mL). The combined organic layers were dried over anhydrous $Na_2SO_4$ and concentrated under reduced pressure to afford 7-cyclopropyl-1H-indazole (3.5 g, crude).

Step-4: A solution of 7-cyclopropyl-1H-indazole (3.5 g, 22 mmol), N-iodosuccinamide (7.4 g, 33 mmol) and sodium hydroxide (2.46 g, 44 mmol) in DMF (15 mL) was stirred for 2 h at ambient temperature. The reaction was quenched with a saturated solution of sodium bisulfite (150 mL). A precipitate was formed which was filtered off and washed with water (3 × 30 mL). The solid was left to dry at 40 °C under vacuum to afford 7-cyclopropyl-3-iodo-1H-indazole (2.7 g, crude).

Step-5: In a steel pressure vessel, a mixture of 7-cyclopropyl-3-iodo-1H-indazole (2.5 g, 8.80 mmol), Pd(dppf)$Cl_2$ (720 mg, 0.88 mmol), dppf (970 mg, 1.77 mmol) and $Et_3N$ (2.5 mL, 17.6 mmol) in toluene/methanol (25 mL/ 25 mL) was stirred at 100 °C for 16 h under an atmosphere of carbon monoxide (100 psi). The resulting reaction mixture was cooled to ambient temperature and was filtered through a pad of celite and concentrated under reduced pressure to get the crude material, which was purified by column chromatoghraphy (100 - 200 mesh silica gel) and eluted with 12-16% of ethyl acetate in pet ether to afford methyl 7-cyclopropyl-1H-indazole-3-carboxylate (1.2 g, 63%).

Step-6: A solution of methyl 7-cyclopropyl-1H-indazole-3-carboxylate (1.0 g, 4.629 mmol), 4-(bromomethyl)tetrahydro-2H-pyran (1.24 g, 6.944 mmol) and $K_2CO_3$ (1.91 g, 13.88 mmol) in $CH_3CN$ (20 mL) was heated to 80 °C and was stirred for 16 h. The reaction mixture was allowed to cool to ambient temperature and was filtered through a celite pad. The filtrate was concentrated under reduced pressure, and the obtained residue was diluted with ethyl acetate (50 mL). The organic layer was washed with water (2 × 50 mL), dried over anhyd. $Na_2SO_4$ and concentrated under reduced pressure to get the crude material which was purified by reverse phase column chromatography eluting with 60% acetonitrile in 0.1% formic acid in water to afford methyl 7-cyclopropyl-2-((tetrahydro-2H-pyran-4-yl)methyl)-2H-indazole-3-carboxylate (508 mg, 35%).

Step-7: To a solution of methyl 7-cyclopropyl-2-((tetrahydro-2H-pyran-4-yl)methyl)-2H-indazole-3-carboxylate (380 mg, 1.210 mmol) in MeOH:THF (1:2, 5 mL) was added a solution of NaOH (242 mg, 6.050 mmol) in water (5 mL). The resulting suspension was stirred at 70 °C for 2 h. The reaction mixture was diluted with water (15 mL), acidified to pH ~2 with 1N aq. HCl solution and extracted with ethyl acetate (2 × 30 mL). The combined organic layers were dried over anhyd. $Na_2SO_4$ and concentrated under reduced pressure to afford 7-cyclopropyl-2-((tetrahydro-2H-pyran-4-yl)methyl)-2H-indazole-3-carboxylic acid **(Int-B38,** 350 mg, yield: 96%). LCMS: m/z $[M+H]^+$ = 301.2 (calc. 301.2).

Synthesis of 7-methyl-2-((tetrahydro-2H-pyran-2-yl)methyl)-2H-indazole-3-carboxylic acid **(Int-B39),** synthesized in form of enantiomer:

Enantiomer 2 of 7-methyl-2-((tetrahydro-2H-pyran-2-yl)methyl)-2H-indazole-3-carboxylic acid (**Int-B39b**):

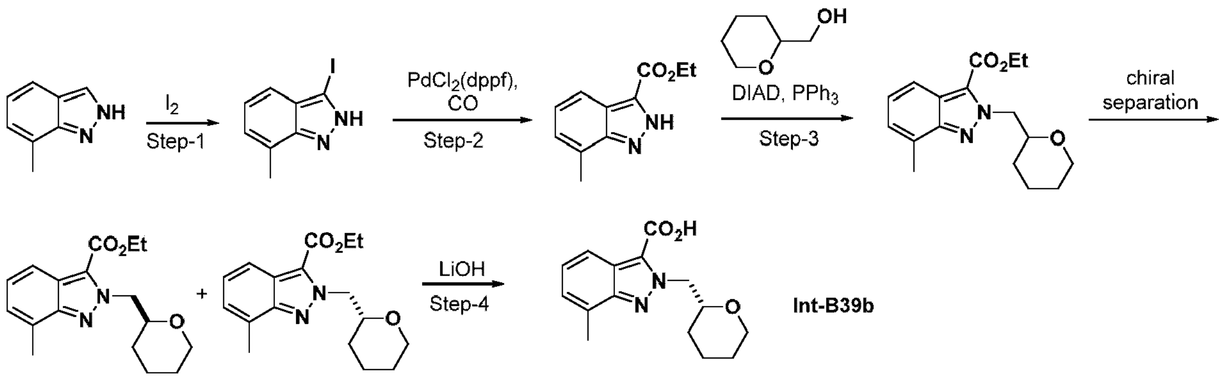


**Int-B39b**

Step-1: To a stirred solution of 7-methyl-2H-indazole (5 g, 37.88 mmol, 1.0 equiv.) in DMF (50 mL) were added KOH (6.37 g, 113.63 mmol, 3.0 equiv.) and iodine (19.2 g, 75.76 mmol, 2.0 equiv.) at 0 °C. The resulting mixture was stirred at ambient temperature for 6 h. The reaction mixture was quenched with saturated sodium thiosulfate solution and extracted with EtOAc (3 × 500 mL). The combined organic layers were washed with brine, dried over $Na_2SO_4$ and concentrated under reduced pressure to get the crude product which was purified by column chromatography (silica gel; 0-50% EtOAc in Hexane as eluent) to yield 3-iodo-7-methyl-2H-indazole. Yield: 80% (7.7 g, 29.84 mmol).

Step-2: A solution of 3-iodo-7-methyl-2H-indazole (11 g, 42.63 mmol, 1.0 equiv.) in ethanol (300 mL) was degassed with nitrogen for 15 min, followed by the addition of sodium acetate (10.5 g, 127.91 mmol, 3.0 equiv.) and $PdCl_2$(dppf) (1.6 g, 2.13 mmol, 0.05 equiv.) at ambient temperature. The reaction mixture was heated to 80 °C for 16 h under 5.52 bar pressure of carbon monoxide. The reaction mixture was cooled to ambient temperature and filtered through celite. The filtrate was concentrated under reduced pressure to get the crude product which was purified by column chromatography (silica gel; 0-50 % EtOAc in hexane as eluent) to yield ethyl 7-methyl-2H-indazole-3-carboxylate. Yield: 80% (7 g, 34.27 mmol)

Step-3: To a stirred solution of ethyl 7-methyl-2H-indazole-3-carboxylate (1.0 g, 4.9 mmol, 1.0 equiv.) in THF (16 mL), were added oxan-2-ylmethanol (1.14 g, 9.8 mmol, 2.0 equiv.) and $PPh_3$ (2.57 g, 9.8 mmol, 2.0 equiv.) at 0 °C. A solution of DIAD (1.94 mL, 9.8 mmol, 2.0 equiv.) in toluene (4 mL) was added to the reaction mixture at 0 °C and the reaction was heated to 80 °C for 16 h. The reaction mixture was concentrated under reduced pressure to get the crude product which was initially purified by column chromatography (silica gel; 0-30% EtOAc in Hexane as eluent) followed by chiral HPLC purification to afford ethyl 7-methyl-2-[oxan-2-ylmethyl]-2H-indazole-3-carboxylate (first eluting, chiral HPLC (column: Chiralpak IC (4.6 x150 mm) 5 μm, flow rate: 1.0 mL/min, 80% hexane, 20% isopropanol, $R_t$ = 5.73 min.) and ethyl 7-methyl-2-[(oxan-2-ylmethyl]-2H-indazole-3-carboxylate (second eluting, chiral HPLC (column: Chiralpak IC (4.6 x150 mm) 5 μm, flow rate: 1.0 mL/min, 80% hexane, 20% isopropanol, $R_t$ = 8.13 min). Yield (second eluting enantiomer): 26% (0.31 g, 1.02 mmol).

Step-3: To a stirred solution of ethyl 7-methyl-2-[oxan-2-ylmethyl]-2H-indazole-3-carboxylate (second eluting) (0.31 g, 1.02 mmol, 1.0 equiv.) in a mixture of THF (10 mL), EtOH (10 mL) and $H_2O$ (5 mL) was added $LiOH{\cdot}H_2O$ (0.086 g, 2.05 mmol, 2.0 equiv.) at ambient temperature. The reaction mixture was stirred at ambient temperature for 16 h. The reaction mixture was concentrated under reduced pressure and the aqueous part was acidified with saturated $KHSO_4$ solution to pH~2. The precipitated solid was filtered off and co-evaporated with toluene to yield 7-methyl-2-[oxan-2-ylmethyl]-2H-indazole-3-carboxylic acid (Int-B39b). Yield: Quantative (0.28 g, 1.02 mmol), LCMS: m/z $[M+H]^+$ = 275.4 (calc. 275.1).

The following examples were synthesized by analogy to the procedure described above using appropriate reactants and adjusted purification protocols:

| Intermediate structure/code | Procedure | Analytics |
| --- | --- | --- |
| 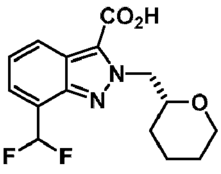 **Int-B40b** | As above, using second eluting ester enantiomer in step 3. | LCMS: m/z $[M+H]^+$ = 311.3 (calc. 311.1) |

Svnthesis of 7-cyclopropyl-2-((tetrahydro-2H-pyran-4-yl)methyl)-2H-indazole-3-carboxylic acid (**Int-B41**):

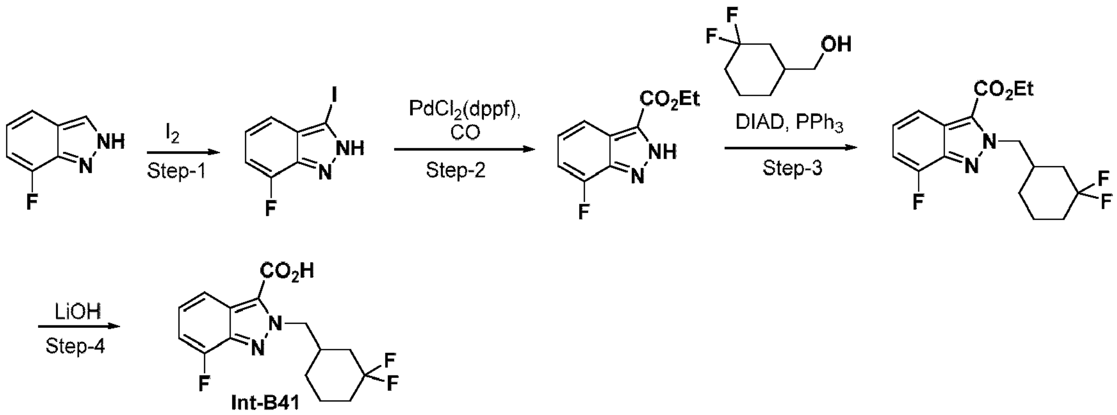

**Int-B41**

Step-1: To a stirred solution of 7-fluoro-2H-indazole (1 g, 7.35 mmol, 1 equiv.) in DMF (10 mL) were added KOH dust (1.24 g, 22.05 mmol, 3 equiv.) and $I_2$ (3.7 g, 14.70 mmol, 2 equiv.) at 0 °C. The reaction mixture was stirred at ambient temperature for 16 h. The reaction mixture was quenched with sat. $Na_2S_2O_3$ solution and extracted with EtOAc (3×100 mL). The combined organic part was washed with cold brine and sat $Na_2S_2O_3$ solution. The organic layer was dried over $Na_2SO_4$ and concentrated under reduced pressure to get 7-fluoro-3-iodo-2H-indazole. Yield: 96% (1.86 g, 7.09 mmol)

Step-2: To a stirred solution of 7-fluoro-3-iodo-2H-indazole (1 g, 3.82 mmol, 1.0 equiv.) in EtOH (10 mL) was added NaOAc (1.31 g, 15.96 mmol, 4.2 equiv.) at ambient temperature and the mixture was degassed with nitrogen for 15 minutes. $PdCl_2$(dppf) (0.15 g, 0.20 mmol, 0.05 equiv.) was added to the reaction mixture and mixture was heated to 80 °C in an autoclave using 5.51 bar CO gas pressure for 16 h. The reaction mixture was cooled to ambient temperature and was filtered through celite. The filtrate was concentrated under reduced pressure to get the crude product, which was purified by column chromatography (silica gel, 0-50% EtOAc in hexane as eluent) to yield ethyl 7-fluoro-2H-indazole-3-carboxylate. Yield: 82% (0.65 g, 3.12 mmol)

Step-3: To a stirred solution of ethyl 7-fluoro-2H-indazole-3-carboxylate (0.8 g, 3.84 mmol, 1 equiv.) in THF-toluene (5:1) (24 mL) were added (3,3-difluorocyclohexyl)methanol (0.69 g, 4.61 mmol, 1.2 equiv.), $PPh_3$ (2 g, 7.68 mmol, 2.0 equiv.) and DIAD (1.5 mL, 7.68 mmol, 2.0 equiv.) at 0 °C. The reaction mixture was stirred at ambient temperature for 16 h. The reaction mixture was concentrated under reduced pressure to get the crude product which was purified by column chromatography (0-20% ethyl acetate/hexane as eluent) to yield ethyl 2-[(3,3-difluorocyclohexyl)methyl]-7-fluoro-2H-indazole-3-carboxylate. Yield: 46 % (0.6 g, 1.76 mmol)

Step-4: To a stirred solution of ethyl 2-[(3,3-difluorocyclohexyl)methyl]-7-fluoro-2H-indazole-3-carboxylate (0.55 g, 1.61 mmol, 1 equiv.) in a mixture of THF:EtOH:$H_2O$ (2:2:1) (15 mL) was added LiOH·$H_2O$ (0.1 g, 1.21 mmol, 1.5 equiv.) at ambient temperature. The reaction mixture was stirred at ambient temperature for 16 h. The reaction mixture was concentrated under reduced pressure, diluted with water and acidified with saturated $KHSO_4$ solution to maintain pH-2. The precipitated solid was filtered off and co-evaporated with toluene to yield 2-[(3,3-difluorocyclohexyl)methyl]-7-fluoro-2H-indazole-3-carboxylic acid **(Int-B41).** Yield: 89% (0.45 g, 1.44 mmol), LCMS: m/z $[M+H]^+$ = 313.2 (calc. 313.1).

**Selected examples**

Svnthesis of 2-(cyclohexylmethyl)-6-fluoro-*N*-(2-hydroxypyridin-4-yl)indazole-3-carboxamide (**SC-05**):

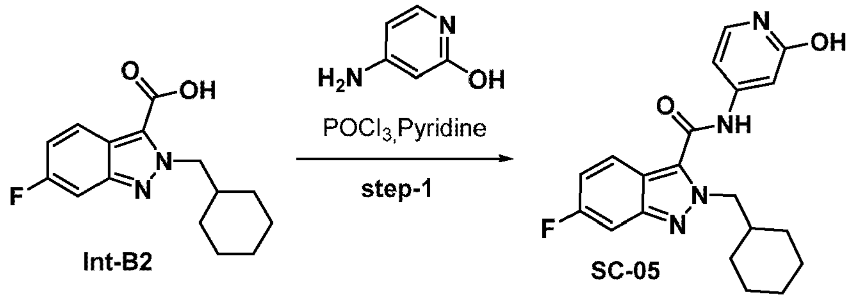


Int-B2

SC-05

Step-1: To a stirred solution of 2-(cyclohexylmethyl)-6-fluoro-1*H*-indazole-3-carboxylic acid **(Int-B2)** (0.20 g, 0.72 mmol, 1.00 eq.) and 4-amino-1,2-dihydropyridin-2-one (0.096 g, 0.86 mmol) in pyridine (6 mL) was added $POCl_3$ (0.02 mL, 2.17 mmol) dropwise at -10 °C. The reaction mixture was stirred at ambient temperture for 16 h and subsequently concentrated to dryness. The residue was dissolved in water (20 mL) and extracted with EtOAc (3 x 100 mL). The combined organic layers were washed with brine, dried over $Na_2SO_4$ and concentrated under reduced pressure to get the crude product which was purified by flash column chromatography (silica gel; 0-10% methanol in DCM) and washed with acetonitrile to yield 2-(cyclohexylmethyl)-6-fluoro-*N*-(2-oxo-1,2-dihydropyridin-4-yl)-2H-indazole-3-carboxamide **(SC-05).** Yield: 18% (0.05 g, 0.135 mmol). $^1$H NMR (400 MHz, DMSO-*d6*): δ (ppm) = 11.32 (s, 1H), 10.82 (s, 1H), 7.82-7.79 (m, 1H), 7.52 (d, 1H), 7.35 (d, 1H), 7.20-7.16 (m, 1H), 6.80 (s, 1H), 6.52 (d, 1H), 4.52 (d, 2H), 2.05-1.94 (m, 1H), 1.75-1.57 (m, 3H), 1.49-1.47 (m, 2H), 1.01- 0.98 (m, 5H). LCMS: m/z $[M+H]^+$ = 369.3 (calc. = 369.2).

Svnthesis of 2-((4,4-difluorocyclohexyl)methyl)-6-fluoro-N-(2-hydroxypyridin-4-yl)-2H-indazole-3-carboxamide **SC-06**):

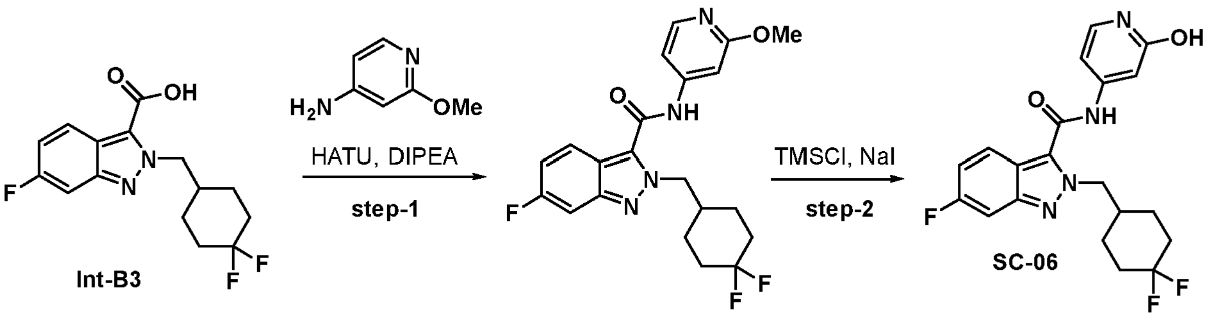


Int-B3

SC-06

Step-1: To a stirred solution of 2-[(4,4-difluorocyclohexyl)methyl]-6-fluoro-2*H*-indazole-3-carboxylic acid (**Int-B3**) (0.20 g, 0.64 mmol, 1.00 eq.) in DMF (2.0 mL) were added2-methoxypyridin-4-amine (0.119 g, 0.961 mmol), HATU (0.487 g, 1.28 mmol) and DIPEA (0.44 mL, 2.56 mmol) at 0 °C and the reaction mixture was stirred at ambient temperature for 16 h. The reaction mixture was diluted with water (30 mL) and extracted with EtOAc (3 × 50 mL). The combined organic layers were washed with sat. aq. $NH_4Cl$ (50 mL), sat. aq. NaHCOs, brine (50 mL), dried over $Na_2SO_4$ and concentrated under reduced pressure to give the crude material which was purified by flash column chromatography (silica gel; 0 - 30% EtOAc in hexanes) to yield 2-[(4,4-difluorocyclohexyl)methyl]-6-fluoro-*N*-(2-methoxypyridin-4-yl)-2*H*-indazole-3-carboxamide. Yield: 74% (0.21 g, 0.477 mmol).

Step-2: To a stirred solution of 2-[(4,4-difluorocyclohexyl)methyl]-6-fluoro-*N*-(2-methoxypyridin-4-yl)-2*H*-indazole-3-carboxamide (0.2 g, 0.48 mmol, 1.00 eq.) in acetonitrile (12 mL) were added NaI (1.4 g, 9.56 mmol) and TMSCl (1.2 mL, 9.56 mmol) at ambient temperature and the mixture was stirred at 90 °C for 3 h. The reaction mixture was cooled, quenched with sat. aq. $NaHSO_4$ (20 mL) and extracted with EtOAc (3 × 50 mL). The combined organic layers were washed with brine (20 mL), dried over $Na_2SO_4$ and concentrated under reduced pressure to get the crude material which was purified by flash column chromatography (silica; 0 - 10% methanol in DCM) and washed with acetonitrile to

yield 2-[(4,4-difluorocyclohexyl)methyl]-6-fluoro-*N*-(2-oxo-1,2-dihydropylidin-4-yl)-2H-indazole-3-carboxamide **(SC-06).** Yield: 77% (0.15 g, 0.370 mmol). $^1$H NMR (400 MHz, DMSO-*d6*): δ (ppm) = 11.33 (bs, 1H), 10.84 (s, 1H), 7.85-7.81(m, 1H), 7.55-7.52 (m, 1H), 7.36 (d, 1H), 7.22-7.17 (m, 1H), 6.80 (d, 1H), 6.54-6.52 (m, 1H), 4.60 (d, 2H), 2.29-1.98 (m, 1H), 1.971.89 (m, 2H), 1.88-1.81 (m, 2H), 1.76-1.58 (m, 2H), 1.33-1.19 (m, 2H). LCMS: m/z $[M+H]^+$ = 405.2 (calc. 405.2).

Svnthesis of 2-(cyclohexylmethyl)-N-(2-hydroxypyridin-4-yl)-2H-indazole-3-carboxamide (**SC-10**):

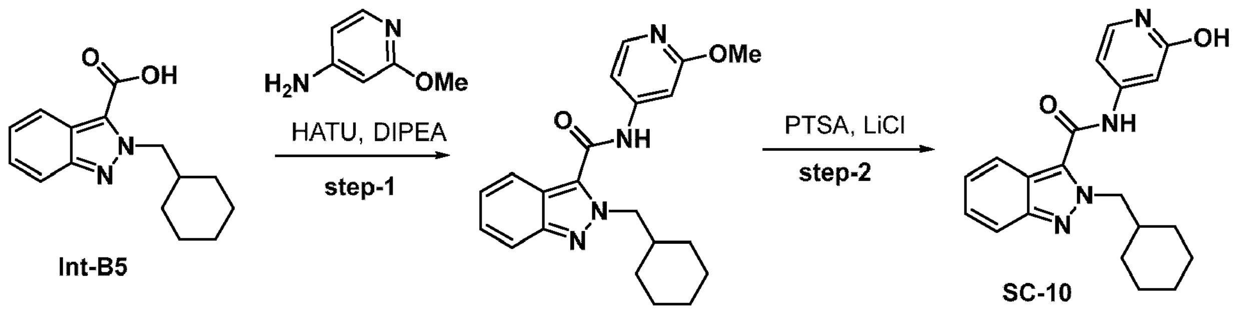

Int-B5

SC-10

Step-1: To a solution of 2-(cyclohexylmethyl)-2*H*-indazole-3-carboxylic acid (Int-B5) (245 mg, 0.94 mmol, 1.00 eq.) and 2-methoxypyridin-4-amine (353 mg, 2.84 mmol, 3.00 eq.) in DMF (5 mL) were added HATU (720 mg, 1.89 mmol, 2.00 eq.) and DIPEA (0.5 mL, 2.84 mmol, 3.00 eq.) at ambient temperature. The reaction mixture was stirred at ambient temperature for 16 h, then diluted with water (50 mL) and extracted with EtOAc (2 × 50 mL). The combined organic layers were dried over $Na_2SO_4$ and concentrated under reduced pressure to get the crude product which was purified by flash column chromatography (silica gel; 40-30% EtOAc/hexanes) to yield 2-(cyclohexylmethyl)-*N*-(2-methoxypyridin-4-yl)-2*H*-indazole-3-carboxamide (130 mg, 0.35 mmol, 37%).

Step-2: To a solution of 2-(cyclohexylmethyl)-*N*-(2-methoxypyridin-4-yl)-2*H*-indazole-3-carboxamide (100 mg, 0.27 mmol, 1.00 eq.) in DMF (5 mL) were added p-toluenesulfonic acid (236 mg, 1.37 mmol, 5 eq.) and LiCl (58 mg, 1.37 mmol, 5 eq.) and the reaction mixture was heated at 110 °C for 3 h. The reaction mixture was concentrated to dryness, water (20 mL) was added and the resulting mixture was extracted with 10%MeOH/DCM (2 × 50 mL). The combined organic layers were dried over $Na_2SO_4$ and concentrated under reduced pressure to get the crude product which was purified by prep. HPLC to yield 2-(cyclohexylmethyl)-*N*-(2-oxo-1,2-dihydropyridin-4-yl)-2H-indazole-3-carboxamide **(SC-10)** (43 mg, 0.12 mmol, 45%). Prep. HPLC conditions: column YMC Triart C18 (250 × 20 mm, 5μ) operating at ambient temperature and flow rate of 16 mL/min; mobile phase: A = 20mM $NaHCO_3$ in water, B = acetonitrile; gradient: initial 80% A and 20% B then to 60% A and 40% B in 3 min, then to 30% A and 70% B in 22 min, then to 100% B in 23 min. $^1$H NMR (400 MHz, DMSO-*d6*): δ (ppm) = 11.30 (bs, 1H), 10.76 (s, 1H), 7.76- 7.74 (m, 2H),7.38 -7.34 (m, 2H), 7.27 (t, 1H), 6.81 (s, 1H), 6.55-6.53 (m, 1H), 4.56 (d, 2H), 1.97-1.92 (m, 1H), 1.63-1.47 (m, 5H), 1.16-1.12 (m, 3H), 1.04-0.99 (m, 2H). LCMS: m/z $[M+H]^+$ = 351.3 (calc. 351.2).

Svnthesis of -(cyclobutylmethyl)-N-(2-hydroxypyridin-4-yl)-2H-indazole-3-carboxamide (**SC-12**):

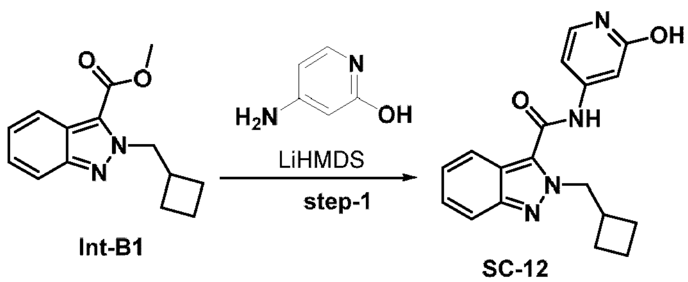

Int-B1

SC-12

Step-1: To a stirred solution of methyl 2-(cyclobutylmethyl)-2*H*-indazole-3-carboxylate (**Int-B1**) (300 mg, 1.23 mmol, 1.00 eq.) in THF (20 mL) were added 4-aminopyridin-2(1*H*)-one (162 mg, 1.475 mmol) and 1M LiHMDS in THF (5 mL) dropwise at ambient temperature. The reaction mixture was stirred at ambient temperature for 5 h, then concentrated under reduced pressure to get the crude product which was purified by prep. HPLC to afford 2-(cyclobutylmethyl)-*N*-(2-oxo-1,2-dihydropyridin-4-yl)-2*H*-indazole-3-carboxamide (SC-12) (22 mg, 5%). Prep. HPLC conditions: column X-SELECT-C18 (250x19, 5 μM), mobile phase: 10 mM $NaHCO_3$ in $H_2O$/MeOH, gradient: (time(min)/%MeOH): 0/20, 8/70, 10/60, 11/80, 11.1/98, 14/98, 14.1/20, 16/20; flow rate: 17 mL/min. $^1H$ NMR (400 MHz, DMSO-*d6*): δ (ppm) = 11.38 (brs, 1H), 11.20 (brs, 1H), 7.79 (d, 1H), 7.74 (d, 1H), 7.36 - 7.32 (m, 2H), 7.24 (t, 1H), 6.80 (s, 1H), 6.53 (d, 1H), 4.75 (d, 2H), 2.91 - 2.85 (t, 1H), 1.95 - 1.92 (m, 2H), 1.86 - 1.81 (m, 4H). LCMS: m/z $[M+H]^+$= 323.1 (calc. 323.1).

Synthesis of 2-(cyclohexylmethyl)-6-fluoro-*N*-(3-(methylsulfinyl)phenyl)-2*H*-indazole-3-carboxamide (**SC-19**):

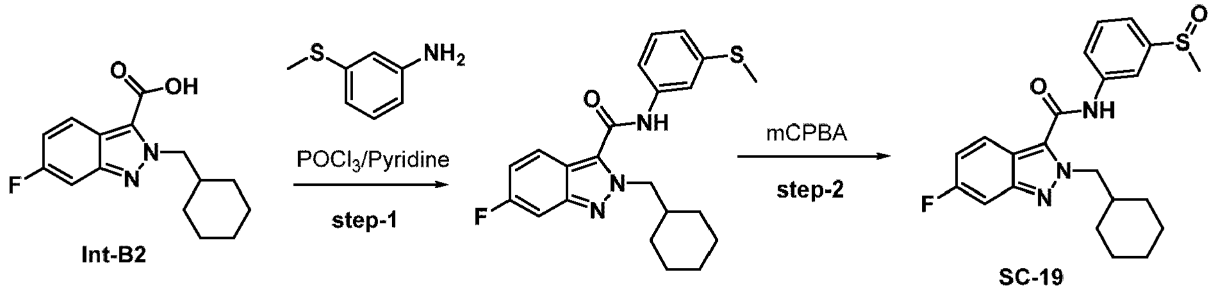

Int-B2
SC-19

Step-1: To a mixture of 2-(cyclohexylmethyl)-6-fluoro-2*H*-indazole-3-carboxylic acid (Int-B2) (250 mg, 0.92 mmol, 1.00 eq.) and 3-(methylthio)aniline (0.2 mL, 1.85 mmol, 2.00 eq.) in pyridine (5 mL) was added $POCl_3$ (0.25 mL, 2.77 mmol, 3.00 eq.) dropwise at 0 °C. The reaction mixture was stirred at ambient temperature for 3 h, then poured into ice-water (25 mL) and extracted with EtOAc (2 × 50 mL). The combined organic layers were washed with water (25 mL), sat. aq. $NaHCO_3$ (25 mL) and brine (25 mL), dried over $Na_2SO_4$ and concentrated under reduced pressure to get the crude product which was purified by flash column chromatography (silica gel; 20% EtOAc/hexanes) to yield 2-(cyclohexylmethyl)-6-fluoro-*N*-(3-(methylthio)phenyl)-2*H*-indazole-3-carboxamide (218 mg, 0.54 mmol, 59%).

Step-2: To a solution of ethyl 2-(cyclohexylmethyl)-6-fluoro-*N*-(3-(methylthio)phenyl)-2*H*-indazole-3-carboxamide (210 mg, 0.52 mmol, 1.00 eq.) in DCM (20 mL) was added a solution of mCPBA (77% purity, 83 mg, 0.56 mmol, 0.70 eq.) in DCM (2 mL) dropwise at 0 °C. The reaction mixture was stirred at ambient temperature for 16 h, then diluted with DCM (100 mL) and washed with sat. aq. $NaHCO_3$ (50 mL) and brine (50 mL), dried over $Na_2SO_4$ and the solvent was evaporated under reduced pressure to get the crude product which was purified by flash column chromatography (silica gel; 2-5% EtOAc/hexanes) to yield 2-(cyclohexylmethyl)-6-fluoro-*N*-(3-(methylsulfinyl)phenyl)-2*H*-indazole-3-carboxamide (SC-19) (106 mg, 0.25 mmol, 48%). $^1H$ NMR (400 MHz, DMSO-*d6* ): δ (ppm) = 11.02 (s, 1H), 8.18 (s, 1H), 7.90- 7.84 (m, 2H), 7.59 (t, 1H), 7.53-7.50 (m, 1H), 7.43 (d, 1H), 7.19-7.14 (m, 1H), 4.55 (d, 2H), 2.77 (s, 3H), 2.00-1.95 (m, 1H), 1.62-1.48 (m, 5H), 1.16-1.02 (m, 5H). LCMS: m/z $[M+H]^+$ = 414.2 (calc. 414.2).

Synthesis of N-(3-sulfamoylphenyl)-2-((tetrahydro-2*H*-pyran-4-yl)methyl)-2*H*-indazole-3-carboxamide (**SC-15**):

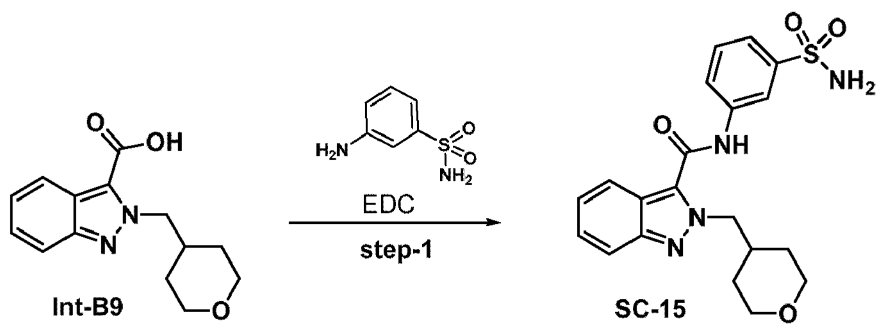

Int-B9
SC-15

Step-1: To a stirred solution of methyl 2-((tetrahydro-2*H*-pyran-4-yl)methyl)-2*H*-indazole-3-carboxylate (Int-B9) (100 mg, 0.384 mmol, 1.00 eq.) in pyridine (4 mL) was added 3-aminobenzenesulfonamide (99.2 mg, 0.576 mmol) and EDC HCl (80.8 mg, 1.10 eq) at ambient temperature. The reaction mixture was stirred at ambient temperature for 16 h, then quenched with 1N aq. HCl (5 mL) and extracted with EtOAc (2x50 mL). The combined organic layers were dried

over anhydrous $Na_2SO_4$ and concentrated under reduced pressure to get the crude product which was purified by prep. HPLC to afford *N*-(3-Sulfamoylphenyl)-2-((tetrahydro-2H-pyran-4-yl)mcthyl)-2//-indazolc-3-carboxamide **(SC-15)** (90 mg, 56%). Prep. HPLC conditions: column: HICHROM 5 C18(250x21.2), 5 μm; mobile phase: 10 mM aq. $NaHCO_3$/acetonitrile; gradient: (T(min)/%B): 0/15, 8/70, 11/70, 11.1/98, 14/98, 14.1/15, 17/15; flow rate: 17 mL/min. $^1H$ NMR (400 MHz, DMSO-*d6*): δ (ppm) = 10.98 (s, 1H), 8.39 (s, 1H), 7.91 - 7.86 (m, 1H), 7.83 (d, 1H), 7.76 (d, 1H), 7.63 - 7.55 (m, 2H), 7.43 - 7.34 (m, 3H), 7.26 (t, 1H), 4.64 (d, 2H), 3.82 - 3.77 (m, 2H), 3.27 - 3.20 (m, 2H), 2.29 - 2.22 (m, 1H), 1.43 - 1.25 (m, 4H). LCMS: m/z $[M+H]^+$ = 415.1 (calc. 415.1).

Synthesis of *N*-(3-sulfamoylphenyl)-2-((tetrahydrofuran-2-yl)methyl)-2*H*-indazole-3-carboxamide (**SC-16**), synthesized in form of enantiomer:

Enantiomer 1 of *N*-(3-sulfamoylphenyl)-2-((tetrahydrofuran-2-yl)methyl)-2*H*-indazole-3-carboxamide **(SC-16a):**

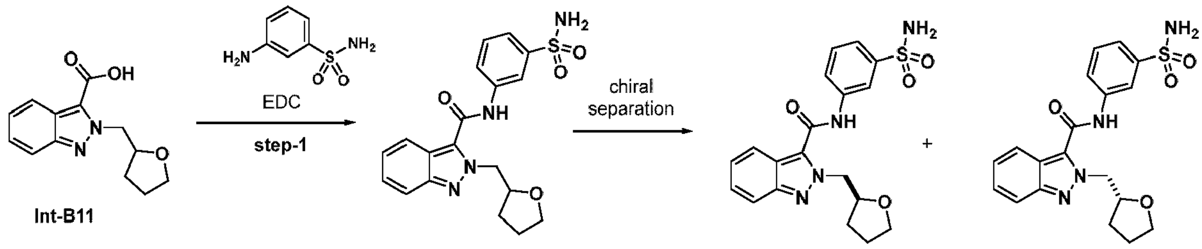


Int-B11 SC-16 SC-16a SC-16b

Step-1: To a solution of racemic 2-((tetrahydrofuran-2-yl)methyl)-2*H*-indazole-3-carboxylic acid (**Int-B11**) (200 mg, 0.913 mmol, 1.00eq.) and 3-aminobenzenesulfonamide (167.8 mg, 0.976 mmol) in pyridine (10 mL) was added EDC hydrochloride (186.6 mg, 0.976 mmol) at ambient temperature. The reaction mixture was stirred at ambient temperature for 16 h, then diluted with water (30 mL) and extracted with EtOAc (2 × 50 mL). The combined organic layers were dried over anhydrous $Na_2SO_4$ and concentrated under reduced pressure to get the crude product which was purified by flash chromatography (column: BUCHI FlashPure C18 40μm reverse phase) using 0.1% aq. formic acid and acetonitrile as an eluent to afford racemic N-(3-sulfamoylphenyl)-2-((tetrahydrofuran-2-yl)methyl)-2*H*-indazole-3-carboxamide **(SC-16)** (160 mg, 49%). 160 mg of the above racemic mixture were subjected to preparative chiral SFC separation using following conditions: column Chiralpak IC (30 × 250 mm), 5 μm; mobile phase 50% $CO_2$ / 50.0% isopropanol; flow 70.0 g/min; back pressure 140.0 bar; load/injection: 10.17 mg. Fractions obtained from SFC were concentrated under reduced pressure to afford 65 mg of target enantiomer 1 of N-(3-sulfamoylphenyl)-2-((tetrahydrofuran-2-yl)methyl)-2H-indazole-3-carboxamide **(SC-16a,** first eluting enantiomer) and 66 mg of enantiomer 2 of N-(3-sulfamoylphenyl)-2-((tetrahydrofuran-2-yl)methyl)-2*H*-indazole-3-carboxamide **(SC-16b,** second eluting enantiomer). Analytical data for **SC-16a:** $^1H$ NMR (400 MHz, DMSO-*d6*): δ (ppm) = 10.98 (brs, 1H), 8.39 (s, 1H), 7.90 (d, 1H), 7.81 (d, 1H), 7.74 (d, 1H), 7.59 - 7.55 (m, 2H), 7.41 - 7.33 (m, 3H), 7.24 (t, 1H), 4.87 - 4.72 (m, 2H), 4.30 (s, 1H), 3.68 - 3.63 (m, 1H), 3.57 - 3.52 (m, 1H), 1.96 - 1.92 (m, 1H), 1.76 - 1.68 (m, 3H). LCMS: m/z $[M+H]^+$ = 401.1 (calc. 401.1).

Synthesis of N-(3-Carbamoyl-4-fluorophenyl)-2-((tetrahydro-2*H*-pyran-4-yl)methyl)-2*H*-indazole-3-carboxamide (**SC-21**):

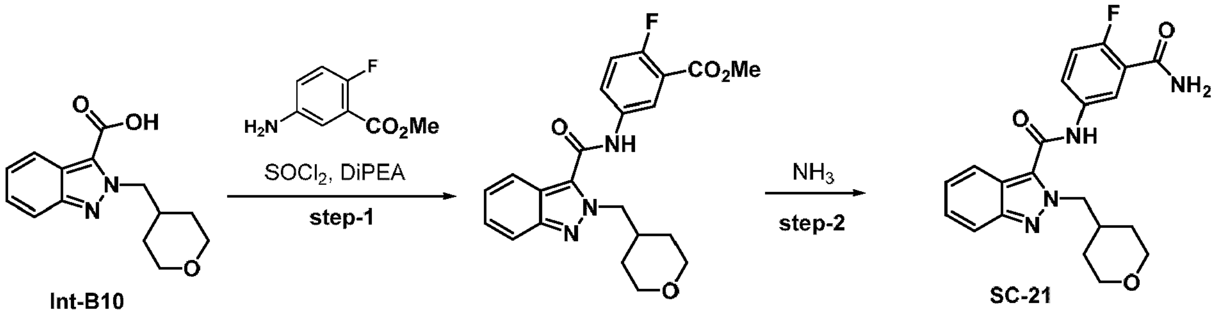


Int-B10 SC-21

Step-1: To a stirred solution of 2-((tetrahydro-2*H*-pyran-4-yl)methyl)-2*H*-indazole-3-carboxylic acid **(Int-B10)** (200 mg, 0.769 mmol) in DCM (10 mL) was added thionylchloride (1 mL). The reaction mixture was heated to 70 °C for 2 h, then concentrated under reduced pressure to get crude compound, which was directly added to a solution of methyl 5-amino-2-fluorobenzoate (156 mg, 0.923 mmol) and DIPEA (2.0 mL, 11.538 mmol) in DCM (5 mL) at 0 °C. The reaction mixture was stirred at ambient temperature for 16 h. The reaction mixture was quenched with water (50 mL) and extracted with DCM (2x50 mL). The combined organic layers were washed with water (50 mL), brine (50 mL), dried over anhydrous $Na_2SO_4$ and concentrated under reduced pressure to get the crude product which was purified by flash chromatography (column: BUCHI FlashPure C18 40μm reverse phase) using 0.1% aq. formic acid and acetonitrile as an eluent to afford methyl 2-fluoro-5-(2-((tetrahydro-2*H*-pyran-4-yl)methyl)-2*H*-indazole-3-carboxamido)benzoate (150 mg, 41%).

Step-2: A stirred solution of methyl 2-fluoro-5-(2-((tetrahydro-2*H*-pyran-4-yl)methyl)-2*H*-indazole-3-carboxamido)benzoate (130 mg, 0.316 mmol, 1.00 eq.) in methanolic $NH_3$ (7M, 2 mL) was stirred in a sealed tube at 80 °C for 5 h. The reaction progress was monitored by TLC and LCMS. The reaction mixture was concentrated under reduced pressure to give the crude product which was purified by flash chromatography (column: BUCHI FlashPure C18 40 μm reverse phase) using 0.1% aq. formic acid and acetonitrile as an eluent to afford N-(3-carbamol-4-fluorophenyl)-2-((tetrahydro-2*H*-pyran-4-yl)methyl)-2*H*-indazole-3-carboxamide (**SC-21**) (75 mg, 60%). $^1H$ NMR (400 MHz, DMSO-*d6*): δ (ppm) = 10.81 (s, 1H), 8.11 - 8.07 (dd, 1H), 7.90 - 7.81 (m, 2H), 7.77 - 7.68 (m, 3H), 7.39 - 7.23 (m, 3H), 4.63 (d, 2H), 3.82 - 3.77 (m, 2H), 3.26 - 3.19 (m, 2H), 2.27 - 2.22 (m, 1H), 1.43 - 1.28 (m, 4H). LCMS: m/z $[M+H]^+$ = 397.1 (calc. 397.2).

Synthesis of 2-(cyclohexylmethyl)-*N*-(3-(2-hydroxypropan-2-yl)phenyl)-2*H*-indazole-3-carboxamide (**SC-26**):

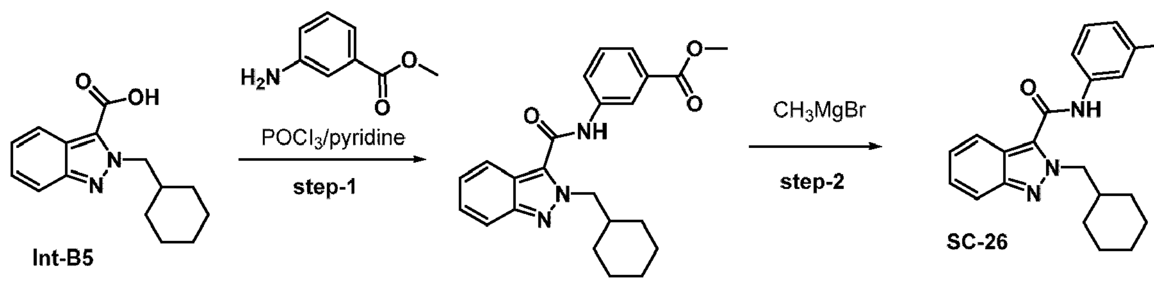


Int-B5

SC-26

Step-1: To a mixture of 2-(cyclohexylmethyl)-2*H*-indazole-3-carboxylic acid **(Int-B5)** (300 mg, 1.15 mmol, 1.00 eq.) and methyl 3-aminobenzoate (262 mg, 1.73 mmol, 1.5 eq.) in pyridine (5 mL) was added $POCl_3$ (1 mL, 3.45 mmol, 3.0 eq.) at 0 °C and the resulting mixture was stirred at ambient temperature for 3 h. The reaction mixture was diluted with ice-cold water (25 mL) and extracted with EtOAc (70 mL). The organic layer was washed with brine (50 mL), dried over anhydrous $Na_3SO_4$ and concentrated under reduced pressure to get the crude product which was purified by flash column chromatography (silica gel; 40% EtOAc/hexanes) to yield methyl 3-(2-(cyclohexylmethyl)-2*H*-indazole-3-carboxamido)benzoate (160 mg, 0.409 mmol, 35%).

Step-2: To a solution of methyl 3-(2-(cyclohexylmethyl)-2*H*-indazole-3-carboxamido)benzoate (130 mg, 0.332 mmol, 1.0 eq.) in THF (5 mL) was added 3M solution of $CH_3MgBr$ in ether (2 mL) at 0 °C dropwise and the reaction mixture was stirred at ambient temperature for 16 h. The reaction mixture was quenched with sat. aq. $NH_4Cl$ (25 mL) and extracted with EtOAc (100 mL). The organic layer was washed with brine (50 mL) and dried over anhydrous $Na_2SO_4$. The solvent was evaporated under reduced pressure to get the crude product which was purified by flash column chromatography (silica gel; 0-50% EtOAc/hexanes) to yield 2-(cyclohexylmethyl)-N-(3-(2-hydroxypropan-2-yl)phenyl)-2*H*-indazole-3-carboxamide (SC-26) (70 mg, 0.179 mmol, 53%). $^1H$ NMR (400 MHz, DMSO-*d6*): δ (ppm) = 10.64 (s, 1H), 7.89 (s, 1H), 7.79 (d, 1H), 7.74 (d, 1H), 7.62 (d, 1H), 7.36-7.28 (m, 2H), 7.25-7.21 (m, 2H), 5.04 (s, 1H), 4.58 (d, 2H), 1.97 (bs, 1H), 1.62-1.49 (m, 5H), 1.43 (s, 6H), 1.14-1.00 (m, 5H). LCMS: m/z $[M+H]^+$ =391.8 (calc. =392.2).

Synthesis of 2-(cyclohexylmethyl)-*N*-(1-(methulsulfonul)-1*H*-pyrazol-4-yl)-2*H*-indazole-3-carboxamide (SC-36):

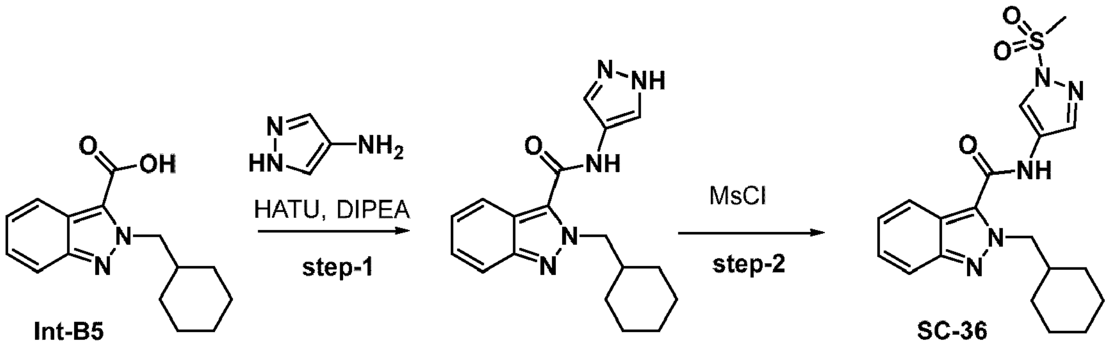

Int-B5 SC-36

Step-1: To a mixture of 2-(cyclohexylmethyl)-2*H*-indazole-3-carboxylic acid **(Int-B5)** (150 mg, 0.579 mmol, 1.00 eq.) in DMF (3 mL) were added DIPEA (0.2 mL, 1.737 mmol, 3.0 eq.) and HATU (437 mg,1.15 mmol, 1.5 eq.) and the resulting mixture was stirred at ambient temperature for 15min. 1*H*-pyrazol-4-amine (242 mg, 1.15 mmol, 1.5 eq.) was added and the reaction mixture was stirred at ambient temperature for 16 h. The reaction mixture was quenched with ice-cooled water (25 mL) and extracted with EtOAc (100 mL). The organic layer was washed with brine (50 mL), dried over anhydrous $Na_2SO_4$ and concentrated under reduced pressure to get the crude product which was purified by flash chromatography (silica gel, 10-40% EtOAc/hexane) to yield 2-(cyclohexylmethyl)-*N*-(4,5-dihydro-1*H*-pyrazol-4-yl)-2*H*-indazole-3-carboxamide (220 mg, 0.676 mmol, 87%).

Step-2: To a solution of 2-(cyclohexylmethyl)-*N*-(4,5-dihydro-1*H*-pyrazol-4-yl)-2*H*-indazole-3-carboxamide (200 mg, 0.619 mmol, 1.00 eq.) and DIPEA (0.4 mL, 1.85 mmol, 3.00 eq.) in DCM (8 mL) was added $MeSO_2Cl$ (0.2 mL, 0.928 mmol, 1.50 eq.) at 0 °C and the reaction mixture was stirred at ambient temperature for 16 h. The reaction mixture was diluted with ice cold water (25 mL) and extracted with DCM (70 mL). The organic layer was washed with brine (50 mL), dried over anhydrous $Na_2SO_4$ and concentrated under reduced pressure to get the crude product which was purified by prep-HPLC to yield 2-(cyclohexylmethyl)-*N*-(1-(methylsulfonyl)-1H-pyrazol-4-yl)-2*H*-indazole-3-carboxamide **(SC-36)** (15 mg, 0.03mmol, 6%). Prep. HPLC conditions: column Sunfire C18 (150 × 19 mm, 10μ) operating at ambient temperature and flow rate of 16 mL/min. Mobile phase: A = 0.1% formic acid in water, B = acetonitrile; gradient initial 80% A and 20% B, then to 30% A and 70% B in 18 min, then to 5% A and 95% B in 19 min. $^1H$ NMR at 100 °C (400 MHz, DMSO-*d6*): δ (ppm) = 10.65 (s, 1H), 8.49 (s, 1H), 8.15 (s, 1H), 7.88 (d, 1H), 7.74 (d, 1H), 7.37-7.33 (m, 1H, ), 7.27-7.23 (m, 1H), 4.63 (d, 2H), 3.51 (s, 3H), 2.06-2.02 (m, 1H), 1.66-1.53 (m, 5H), 1.27-1.19 (m, 5H). LCMS: m/z $[M+H]^+$ = 402.2 (calc. =402.2).

Synthesis of 2-(cyclohexylmethyl)-*N*-(1-methyl-2-oxo-1,2-dihydropyridin-4-yl)-2*H*-indazole-3-carboxamide (**SC-39**):

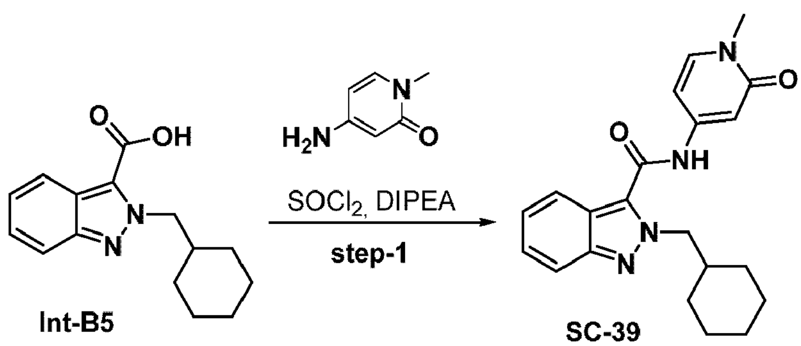

Int-B5 SC-39

Step-1: To a solution of 2-(cyclohexylmethyl)-2*H*-indazole-3-carboxylic acid **(Int-B5)** (120 mg, 0.463 mmol, 1.00 eq.) in DCM (10 mL) was added $SOCl_2$ (1 mL) at ambient temperature. The reaction mixture was stirred at 60 °C for 2 h, then concentrated under reduced pressure to get the crude acid chloride which was dissolved in DCM (5 mL) and added to a solution of 4-amino-1-methylpyridin-2(1*H*)-one (68.8 mg, 0.555 mmol) and DIPEA (0.4 mL, 2.315 mmol) in DCM (10 mL) at ambient temperature. The resulting reaction mixture was stirred at ambient temperature for 16 h. The reaction mixture was concentrated under reduced pressure to get the crude product which was purified by flash chromatography (column: BUCHI FlashPure C18 40μm reverse phase) using 0.1% aq. formic acid and acetonitrile as an eluent to afford 2-(cyclohexylmethyl)-*N*-(1-methyl-2-oxo-1,2-dihydropyridin-4-yl)-2*H*-indazole-3-carboxamide (**SC-39,**

30 mg, 17%). $^1H$ NMR (400 MHz, DMSO-*d6*): δ (ppm) = 10.78 (s, 1H), 7.76 (d, 2H), 7.66 (d, 1H), 7.36 (t, 1H), 7.26 (t, 1H), 6.87 (d, 1H), 6.58 (dd, 1H), 4.56 (d, 2H), 3.39 (s, 3H), 2.00 - 1.90 (m, 1H), 1.69 - 1.52 (m, 3H), 1.51 - 1.46 (m, 2H), 1.28 - 1.10 (m, 3H), 1.08 - 0.95 (m, 2H). LCMS: m/z $[M+H]^+$ = 365.2 (calc. =365.2).

Svnthesis of -(cyclohexylmethyl)-N-(2-hydroxypyridin-4-yl)-7-methyl-2H-indazole-3-carboxamide (**SC-43**):

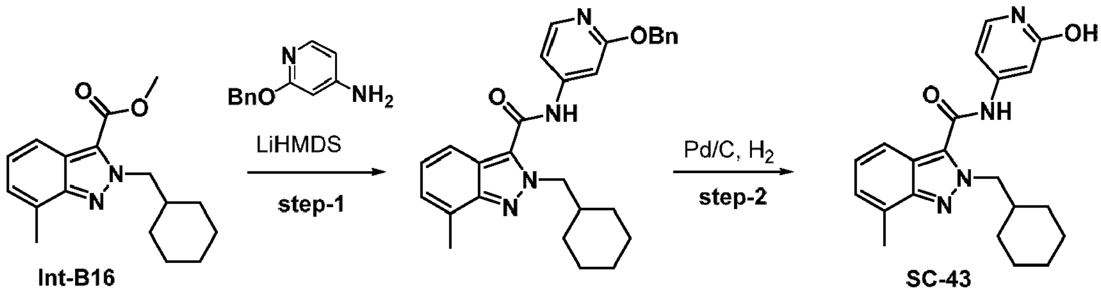


Step-1: To a stirred solution of methyl 2-(cyclohexylmethyl)-7-methyl-2*H*-indazole-3-carboxylate **(Int-B16,** 200 mg, 1.05 mmol, 1.00 eq.) in THF (10 mL) were added 4-aminopyridin-2(1*H*)-one (252 mg, 1.263 mmol) and 1M LiHMDS (3.0 mL) at ambient temperature. The reaction mixture was stirred at ambient temperature for 5 h. The reaction mixture was diluted with water (50 mL) and extracted with EtOAc (2 X 50 mL). The combined organic layers were washed with water (50 mL) and brine (50 mL), dried over anhydrous $Na_2SO_4$ and concentrated under reduced pressure to get *N*-(2-(benzyloxy)-1,2-dihydropyridin-4-yl)-2-(cyclohexylmethyl)-7-methyl-2*H*-indazole-3-carboxamide (300 mg; 94%).

Step-2: To a stirred solution of *N*-(2-(benzyloxy)-1,2-dihydropyridin-4-yl)-2-(cyclohexylmethyl)-7-methyl-2*H*-indazole-3-carboxamide (200 mg, 1.052 mmol, 1.00 eq.) in MeOH (10 mL) was added 10% Pd/C (100 mg) at ambient temperature. The reaction mixture was stirred at ambient temperature under hydrogen gas pressure (80 psi) for 1 h. The reaction mixture was filtered a through celite bed and the filter cake was washed with methanol (10 mL). The combined filtrate was concentrated under reduced pressure to get the crude product which was purified by prep. HPLC to get 2-(cyclohexylmethyl)-7-methyl-*N*-(2-oxo-1,2-dihydropyridin-4-yl)-2*H*-indazole-3-carboxamide (SC-43) (60 mg; 37%). Prep. HPLC conditions: columnX-SELECT-C18 (150x19mm, 5μm), mobile phase: component A: 10 mM $NaHCO_3$ in $H_2O$; component B: acetonitrile; gradient: (T min/%B): 0/40, 8/70, 8.4/70, 8.5/98, 12/98, 12.1/40, 15/40; flow rate: 17 mL/min. $^1H$ NMR (400 MHz, DMSO-*d6*): δ (ppm) = 11.29 (s, 1H), 10.72 (s, 1H), 7.56 (d, 1H), 7.35 (d, 1H), 7.16 - 7.14 (m, 2H), 6.80 (s, 1H, 6.53 (d, 1H), 4.57 (d, 2H), 2.56 (s, 3H), 1.93 (brs, 1H), 1.63 - 1.48 (m, 5H), 1.14 - 1.00 (m, 5H). LCMS: m/z $[M+H]^+$ = 365.2 (calc. =365.2).

Svnthesis of -(cyclohexylmethyl)-N-(2-hydroxypyridin-4-yl)-5-methyl-2H-indazole-3-carboxamide (**SC-48**):

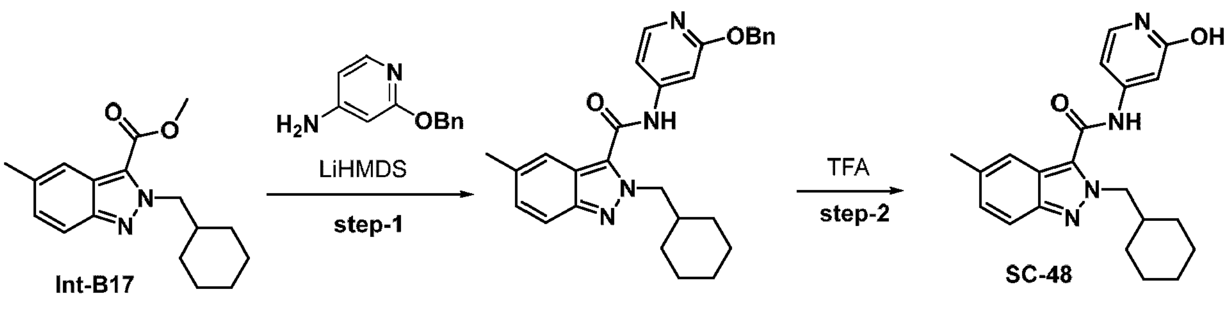


Step-1: To a solution of methyl 2-(cyclohexylmethyl)-5-methyl-2*H*-indazole-3-carboxylate **(Int-B17)** (120 mg, 0.419 mmol, 1.00 eq.) and 2-(benzyloxy)pyridin-4-amine (101 mg, 0.503 mmol) in THF (10 mL) was added 1M LiHMDS (1.25 mL, 1.257 mmol) at ambient temperature. The reaction mixture was stirred at ambient temperature for 16 h. The reaction mixture was diluted with water (20 mL) and extracted with EtOAc (2 × 20 mL). The combined organic layers were dried over anhydrous $Na_2SO_4$ and concentrated under reduced pressure to afford N-(2-(benzyloxy)pyridin-4-yl)-2-(cyclohexylmethyl)-5-methyl-2*H*-indazole-3-carboxamide (135 mg, crude).

Step-2: To a stirred solution of *N*-(2-(benzyloxy)pyridin-4-yl)-2-(cyclohexylmethyl)-5-methyl-2*H*-indazole-3-carboxamide (120 mg, 0.421 mmol) in DCM (10 mL) was added TFA (1 mL) at ambient temperature. The reaction mixture was heated to 80 °C and stirred at 80 °C for 16 h. The reaction mixture was concentrated under reduced pressure to get the crude product which was purified by prep. HPLC to afford 2-(cyclohexylmethyl)-5-methyl-*N*-(2-oxo-1,2-dihydropyridin-4-yl)-2H-indazole-3-carboxamide **(SC-48)** (77 mg, 50% over 2 steps). Prep HPLC conditions: column: KROMOSIL, C-18 (150x25 mM, 7 μm); mobile phase: component A: 10 mM $NaHCO_3$ in $H_2O$, component B: acetonitrile; gradient: (T, min / %B): 0/5, 8/60, 11/70, 11.1/98, 16/98, 16.1/5, 18/5; flow rate: 22 mL/min; $^1H$ NMR (400 MHz, DMSO-*d6*): δ (ppm) = 11.29 (s, 1H), 10.69 (s, 1H), 7.66 (d, 1H), 7.50 (s, 1H), 7.35 (d, 1H), 7.20 (dd, 1H), 6.80 (d, 1H), 6.55 (dd, 1H), 4.52 (d, 2H), 2.42 (s, 3H), 1.99 - 1.90 (m, 1H), 1.67 - 1.52 (m, 3H), 1.50 - 1.44 (m, 2H), 1.27 - 1.10 (m, 3H), 1.08 - 0.96 (m, 2H). LCMS: m/z $[M+H]^+$ = 365.1 (calc. =365.2).

Svnthesis of N-(3-Carbamoylphenyl)-2-[(4,4-difluorocyclohexyl)methyl]-6-fluoro-2*H*-indazole-3-carboxamide (**SC-76**):

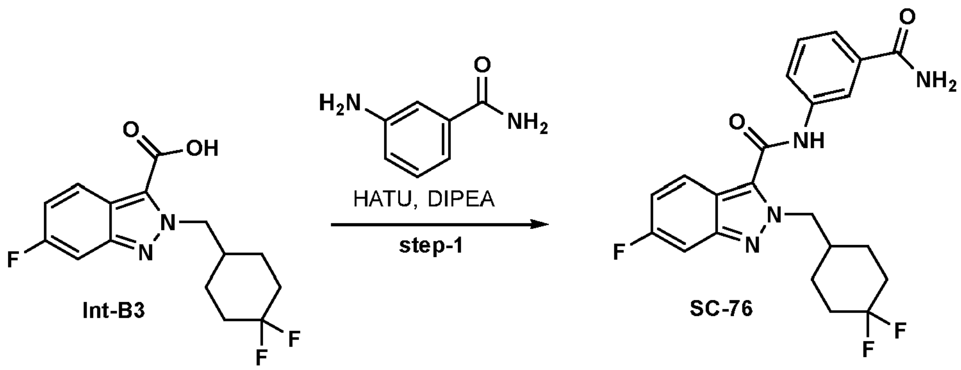


Int-B3

SC-76

Step-1: To a stirred solution of 2-[(4,4-difluorocyclohexyl)methyl]-6-fluoro-2*H*-indazole-3-carboxylic acid (**Int-B3**) (0.1 g, 0.32 mmol, 1.00 eq.), in DMF (3 mL) were added 3-aminobenzamide (0.065 g, 0.48 mmol), HATU (0.24 g, 0.64 mmol) and DIPEA (0.2 mL, 1.28 mmol) at 0 °C. The reaction mixture was stirred at ambient temperature for 16 h, then filtered and the filtrate was purified by prep. HPLC to yield N-(3-carbamoylphenyl)-2-[(4,4-difluorocyclohexyl)methyl]-6-fluoro-2*H*-indazole-3-carboxamide (SC-76). Yield: 58% (0.08 g, 0.19 mmol). $^1H$ NMR (400 MHz, DMSO-*d6*): δ (ppm) = 10.87 (s, 1H), 8.26 (d, 1H), 7.99 (s, 1H), 7.91-7.87 (m, 2H), 7.66 (d, 1H), 7.54-7.40 (m, 3H), 7.18-7.16 (m, 1H), 4.63 (d, 2H), 2.19 (m, 1H), 1.99-1.97 (m, 2H), 1.85-1.60 (m, 4H), 1.35-1.29 (m, 2H). LCMS: m/z $[M+H]^+$ = 431.3 (calc. = 431.2). Prep HPLC conditions: column: YMC-Actus Triart C18 (250 × 20 mm, 5μ) operating at ambient temperature and flow rate of 16.0 mL/min. Mobile phase: A = 20mM $NH_4HCO_3$ in water, B = acetonitrile; gradient profile: initial composition of 70% A and 30% B, then to 55% A and 45% B in 3 min, then to 25% A and 75% B in 22 min, then to 5% A and 95% B in 23 min, held this composition up to 25 min.

The following examples were synthesized by analogy to the procedures described above using appropriate reactants and adjusted purification protocols:

| Ex. No. | Structure | Procedure | Reactants | Analytical data |
|---|---|---|---|---|
| **SC-11** | 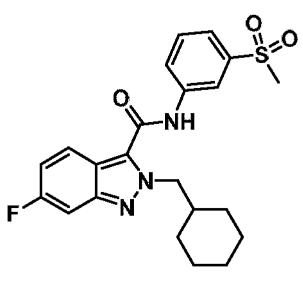 | **SC-05** | **Int-B2,** 3-(methylsulfonyl)aniline | $^1H$ NMR (400 MHz, DMSO-*d6*): δ (ppm) = 11.10 (bs, 1H), 8.43 (s, 1H), 8.03 (d, 1H),7.92-7.89 (m, 1H), 7.73-7.66 (m, 2H), 7.52 (d, 1H), 7.19 (t, 1H), 4.55 (d, 2H), 3.24 (s, 3H), 1.98 (bs, 1H), 1.63-1.1.57 (m, 3H), 1.51-1.48 (m, 2H), 1.14-1.12 (m, 3H), 1.05-1.01 (m, 2H). LCMS: m/z $[M+H]^+$ = 430.3 (calc. 430.2). |

(continued)

| Ex. No. | Structure | Procedure | Reactants | Analytical data |
|---|---|---|---|---|
| **SC-13** | 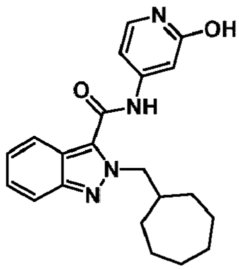 | **SC-12** | Int-B8, 4-amino-pyridin-2(1H)-one | $^1$H NMR (400 MHz, DMSO-*d6*): δ (ppm) = 11.25 (brs, 1H, 10.92 (brs, 1H), 7.81 (brs, 1H), 7.73 (d, 1H), 7.34 - 7.24 (m, 3H), 6.77 (s, 1H), 6.51 (d, 1H), 4.58 (d, 2H), 2.18 (m,1H), 1.60 - 1.45 (m, 8H), 1.43 - 1.41 (m, 2H), 1.35 - 1.33 (m, 2H). LCMS: m/z [M+H]$^+$ = 365.2 (calc. 365.2). |
| **SC-14** | 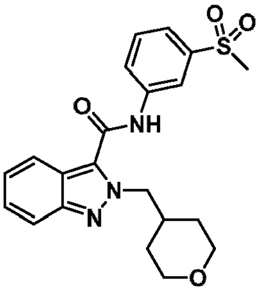 | **SC-12** | **Int-B5,** 3-(methylsulfo nyl)aniline | $^1$H NMR (400 MHz, DMSO-*d6*): δ (ppm) = 11.04 (s, 1H), 8.43 (s, 1H), 8.02 (d, 1H), 7.90 (d, 1H), 7.75 (d, 1H), 7.68 - 7.62 (m, 2H), 7.36 (t, 1H), 7.24 (d, 1H), 4.67 (d, 2H), 3.82 - 3.78 (m, 2H), 3.27 - 3.20 (m, 5H), 2.31 - 2.23 (m, 1H), 1.43 - 1.26 (m, 4H).<br>LCMS: m/z [M+H]$^+$ = 414.1 (calc. 414.1). |
| **SC-17** | 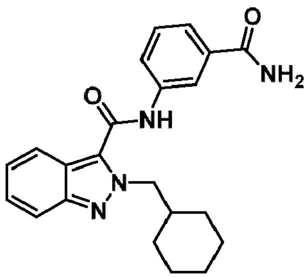 | **SC-15** | **Int-B5,** 3-aminobenzamide | $^1$H NMR (400 MHz, DMSO-*d6*): δ (ppm) = 10.80 (s, 1H), 8.27 (s, 1H), 7.99 (bs, 1H,), 7.89 (d, 1H), 7.83 (d, 1H), 7.75 (d, 1H), 7.65 (s, 1H), 7.45 (t, 1H), 7.40-7.34 (m, 2H), 7.25 (t, 1H), 4.59 (d, 2H), 1.98 (bs,1H), 1.62-1.49 (m, 5H), 1.14-1.00 (m, 5H). LCMS: m/z [M+H]$^+$ = 377.2 (calc. 377.2). |
| **SC-20** | 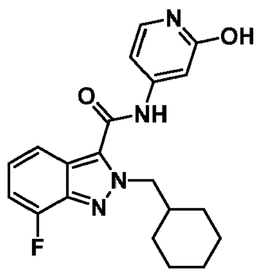 | **SC-12** | **Int-B12,** 4-amino-pyridin-2(1*H*)-one | $^1$H NMR (400 MHz, DMSO-*d6*): δ (ppm) = 11.23 (brs, 1H), 10.86 (s, 1H), 7.65 (s, 1H), 7.30 (s, 1H), 7.16 - 7.13 (m, 2H), 6.75 (s, 1H), 6.48 (d, 3H), 4.62 (s, 2H), 1.98 - 1.94 (m, 1H), 1.63 - 1.58 (d, 3H), 1.50 - 1.47 (d, 2H), 1.17 - 1.12 (m, 3H), 1.06 - 1.00 (m, 2H). LCMS: m/z [M+H]$^+$ = 369.1 (calc. 369.2). |
| **SC-22** | 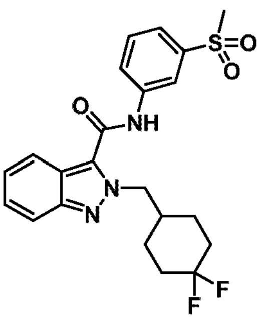 | **SC-15** | **Int-B13,** 3-(methylsulfo nyl) aniline | $^1$H NMR (500 MHz, DMSO-*d6*): δ (ppm) = 11.06 (s, 1H), 8.44 (s, 1H), 8.04 (d, 1H), 7.86 (d, 1H), 7.77 (d, 1H), 7.72 - 7.67 (m, 2H), 7.38 (t, 1H), 7.28 (t, 1H), 4.67 (d, 2H), 3.24 (s, 3H), 2.20 - 2.18 (m, 1H), 1.99 - 1.97 (m, 2H), 1.84 - 1.72 (m, 2H), 1.63 - 1.60 (m, 2H), 1.35 - 1.29 (m, 2H); LCMS: m/z [M+H]$^+$ = 448.1 (calc. 448.1). |

(continued)

| Ex. No. | Structure | Procedure | Reactants | Analytical data |
|---|---|---|---|---|
| SC-24 | 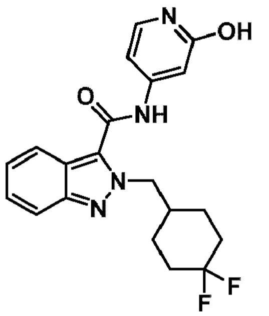 | SC-12 | **Int-B13,** 4-aminopyridi n-2 (1*H*)-one | $^1$H NMR (500 MHz, DMSO-*d6*): δ (ppm) = 11.31 (s,1H), 10.77 (s,1H), 7.56 (d, 2H), 7.40 - 7.34 (m, 2H), 7.30 - 7.26 (m, 1H), 6.81 (d, 1H), 6.55 - 6.53 (dd, 1H), 4.63 (d, 2H), 2.17 (brs, 1H), 1.99 - 1.97 (m, 2H), 1.82 - 1.72 (m, 2H), 1.62 - 1.58 (m, 2H), 1.35 - 1.29 (m, 2H); LCMS: m/z [M+H]$^+$ = 387.1 (calc. 387.2). |
| SC-25 | 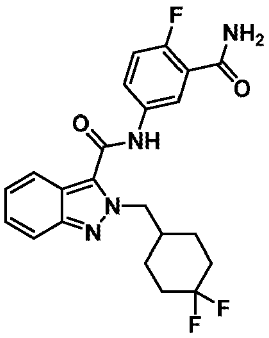 | SC-21 | **Int-B13,** methyl 5-amino-2-fluorobenzoa te (step-1) | $^1$H NMR (400 MHz, DMSO-*d6*): δ (ppm) = 10.82 (s,1H), 8.10 - 8.08 (dd, 1H), 7.89 - 7.82 (m, 2H), 7.76 - 7.69 (m, 3H), 7.39 - 7.25 (m, 3H), 4.65 (d, 2H), 2.19 (brs,1H), 1.99 - 1.97 (m, 2H), 1.85 - 1.70 (m, 2H), 1.63 - 1.60 (m, 2H), 1.35 - 1.26 (m, 2H); LCMS: m/z [M+H]$^+$ = 431.1 (calc. 431.2). |
| SC-28 | 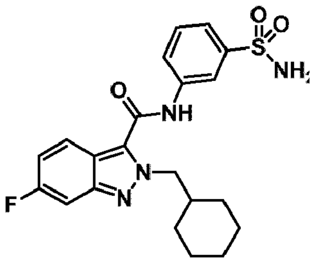 | SC-15 | **Int-B2,** 3-aminobenze nesulfonami de | $^1$H NMR (400 MHz, DMSO-*d6*): δ (ppm) = 11.04 (s, 1H), 8.38 (s, 1H), 7.89-7.85 (m, 2H), 7.63-7.57 (m, 2H), 7.53-7.50 (m, 1H), 7.42 (bs, 2H), 7.20-7.15 (m, 1H), 4.55 (d, 2H), 1.99-1.95 (m, 1H), 1.62-1.47 (m, 5H), 1.16-1.12 (m, 3H), 1.05-0.99 (m, 2H). LCMS: m/z [M+H]$^+$ = 431.3 (calc. 431.2). |
| SC-32 | 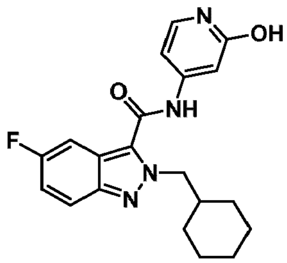 | SC-12 | Int-B14 | $^1$H NMR (400 MHz, DMSO-*d6*): δ (ppm) = 11.31 (s, 1H), 10.70 (s, 1H), 7.86 - 7.83 (m, 1H), 7.46 (d, 1H), 7.35 (d, 1H), 7.32 - 7.27 (m, 1H), 6.81 (s, 1H), 6.53 (d, 1H), 4.55 (d, 2H), 1.95 - 1.92 (m, 1H), 1.63 - 1.58 (m, 3H), 1.48 (d, 2H), 1.14 - 1.12 (d, 3H), 1.04 - 1.01 (m, 2H); LCMS: m/z [M+H]$^+$ = 369.1 (calc. 369.2). |
| SC-37 | 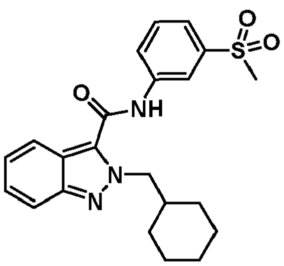 | SC-05 | **Int-B5,** 3-(methylsulfo nyl)aniline | $^1$H NMR (400 MHz, DMSO-*d6*): δ (ppm) = 11.05 (s, 1H), 8.44 (s, 1H), 8.04 (d, 1H), 7.85 (d, 1H), 7.77-7.66 (m, 3H), 7.37 (t, 1H), 7.26 (t, 1H), 4.60 (d, 2H), 3.24 (s, 3H), 1.98 (bs, 1H), 1.63-1.48 (m, 5H), 1.18-1.00 (m, 5H); LCMS: m/z [M+H]$^+$ = 412.2 (calc. = 412.2). |

(continued)

| Ex. No. | Structure | Procedure | Reactants | Analytical data |
|---|---|---|---|---|
| SC-38 | 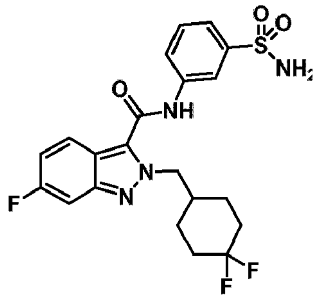 | SC-15 | **Int-B3,** 3-aminobenze nesulfonami de | $^1$H NMR (400 MHz, DMSO-*d6*): δ (ppm) = 11.05 (s, 1H), 8.38 (s, 1H), 7.91-7.88 (m, 2H), 7.61-7.53 (m, 3H), 7.43 (s, 2H), 7.19 (s, 1H), 4.63 (d, 2H), 2.19 (bs, 1H), 1.99 (bs, 2H), 1.82-1.74 (m, 2H), 1.63-1.60 (m, 2H), 1.35-1.30 (m, 2H); LCMS: m/z [M+H]$^+$ = 467.5 (calc. =467.1). |
| SC-42 | 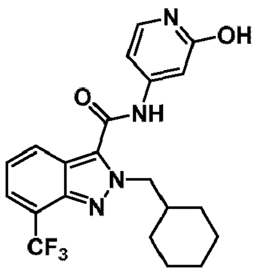 | SC-12 | **Int-B15,** 4-aminopyridi n-2 (1H)-one | $^1$H NMR (400 MHz, DMSO-*d6*): δ (ppm) = 11.30 (brs, 1H), 10.91 (s, 1H), 8.12 (d, 1H), 7.78 (d, 1H), 7.39 - 7.34 (m, 2H), 6.79 (s, 1H), 6.51 (d, 1H), 4.63 (d, 2H,) 1.97 - 1.92 (m, 1H), 1.63 - 1.58 (m, 3H), 1.52 - 1.49 (m, 2H), 1.14 - 1.01 (m, 5H); LCMS: m/z [M+H]$^+$ = 419.1 (calc. 419.2). |
| SC-44 | 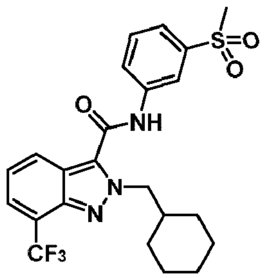 | SC-12 | **Int-B15,** 3-(methylsulfo nyl)aniline | $^1$H NMR (400 MHz, DMSO-*d6*): δ (ppm) = 11.21 (s, 1H), 8.43 (s, 1H), 8.20 (m, 1H), 8.03 (d, 1H), 7.81 - 7.69 (m, 3H), 7.38 (t, 1H), 4.65 (d, 2H), 3.25 (s, 3H), 1.98 (m, 1H), 1.63 - 1.50 (m, 5H), 1.23 - 1.02 (m, 5H); LCMS: m/z [M+H]$^+$ = 480.1 (calc. 480.2). |
| SC-45 | 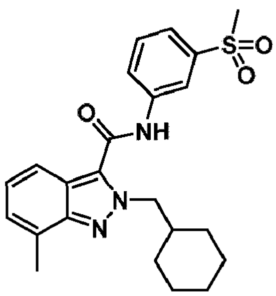 | SC-12 | **Int-B16,** 3-(methylsulfo nyl)aniline | $^1$H NMR (400 MHz, DMSO-*d6*): δ (ppm) = 11.02 (s, 1H), 8.44 (s, 1H), 8.04 (t, 1H), 7.72 - 7.64 (m, 3H), 7.18 - 7.13 (m, 2H), 4.60 (d, 2H), 3.24 (s, 3H), 2.57 (s, 3H), 1.99 - 1.94 (m, 1H), 1.63 - 1.50 (m, 5H),1.14 - 1.01 (m, 5H); LCMS: m/z [M+H]$^+$ = 426.1 (calc. 426.2). |
| SC-46 | 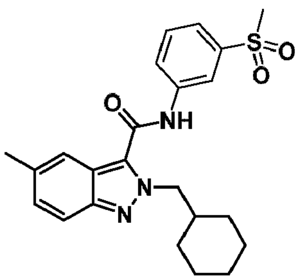 | SC-39 | **Int-B18,** 3-(methylsulfo nyl)aniline | $^1$H NMR (400 MHz, DMSO-*d6*): δ (ppm) = 10.98 (s, 1H), 8.43 (s, 1H), 8.04 - 8.00 (m, 1H), 7.73 - 7.64 (m, 3H), 7.59 (s, 1H), 7.21 (dd, 1H), 4.55 (d, 2H), 3.24 (s, 3H), 2.42 (s, 3H), 2.01 - 1.91 (m, 1H), 1.67 - 1.53 (m, 3H), 1.52 - 1.45 (m, 2H), 1.27 - 1.10 (m, 3H), 1.09 - 0.98 (m, 2H); LCMS: m/z [M+H]$^+$ = 426.1 (calc. 426.2). |

(continued)

| Ex. No. | Structure | Procedure | Reactants | Analytical data |
|---|---|---|---|---|
| **SC-47** | 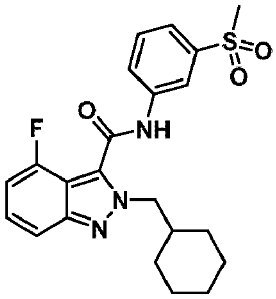 | **SC-39** | **Int-B19,** 3-(methylsulfonyl)aniline | $^1$H NMR (400 MHz, DMSO-*d6*): δ (ppm) = 11.24 (s, 1H), 8.43 (s, 1H), 7.96 (d, 1H), 7.74 - 7.65 (m, 2H), 7.59 (d, 1H), 7.37 - 7.30 (m, 1H), 7.03 - 6.96 (m, 1H), 4.53 (d, 2H), 3.25 (s, 3H), 2.05 - 1.97 (m, 1H), 1.68 - 1.50 (m, 5H), 1.20 - 1.00 (m, 5H); LCMS: m/z $[M+H]^+$ = 430.1 (calc. 430.2). |
| **SC-49** | 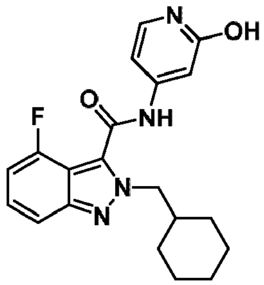 | **SC-48** | **Int-B19,** 2-(benzyloxy) pyridin-4-amine (step-1) | $^1$H NMR (400 MHz, DMSO-*d6*): δ (ppm) = 11.35 (s, 1H), 10.90 (s, 1H), 7.59 (d, 1H), 7.38 - 7.30 (m, 2H), 7.03 - 7.96 (m, 1H), 6.81 (s, 1H), 6.44 (dd, 1H), 4.49 (d, 2H), 1.99 - 1.90 (m, 1H), 1.68 - 1.55 (m, 3H), 1.54 - 1.48 (m, 2H), 1.27 - 0.97 (m, 5H); LCMS: m/z $[M+H]^+$ = 369.1 (calc. 369.2). |
| **SC-50a** | 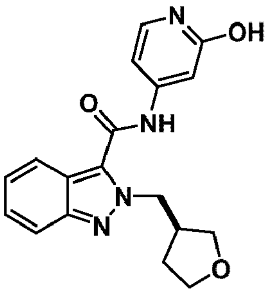 | **SC-48** | **Int-B20,** 2-(benzyloxy) pyridin-4-amine (step-1) | $^1$H NMR (400 MHz, DMSO-*d6*): δ (ppm) = 11.32 (bs, 1H), 10.80 (s, 1H), 7.77 (d, 2H), 7.41 - 7.26 (m, 3H), 6.82 (d, 1H), 6.54 (dd, 1H), 4.76 - 4.62 (m, 2H), 3.82 - 3.74 (m, 1H), 3.67 - 3.60 (m, 2H), 3.56 - 3.52 (m, 1H), 2.94 - 2.86 (m, 1H), 1.96 - 1.88 (m, 1H), 1.71 - 1.63 (m, 1H); LCMS: m/z $[M+H]^+$ = 339.1 (calc. 339.1); chiral SFC: column : CHIRALCEL OJ-3 (4.6x150mm) 3 μm, co-solvent: MeOH; flow : 3g/min; % of co-solvent: 30%, ABPR : 1500 psi, T: 30 °C, $R_t$ = 1.93 min (first eluting). |
| **SC-53b** | 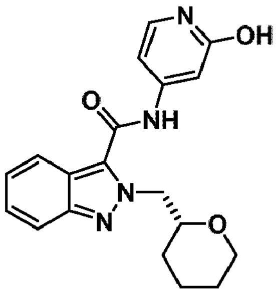 | **SC-48** | **Int-B21,** 2-(benzyloxy) pyridin-4-amine (step-1) | $^1$H NMR (400 MHz, DMSO-*d6*): δ (ppm) = 11.31 (s, 1H), 10.77 (s, 1H), 7.75 - 7.73 (m, 2H), 7.38 - 7.34 (m, 2H), 7.27 -7.23 (m, 1H), 6.81 (d, 1H), 6.54 - 6.52 (dd,, 1H), 4.83 - 4.77 (m, 1H), 4.68 - 4.64 (m, 1H), 3.80 - 3.73 (m, 2H), 3.24 - 3.18 (m, 1H), 1.75 (m, 1H), 1.62 (d, 1H), 1.49 - 1.41 (m, 3H), 1.28 - 1.22 (m, 1H). LCMS: m/z $[M+H]^+$ = 353.1 (calc. 353.2); chiral SFC: column: CHIRALPAK AD-3 (4.6x150 mm) 3 μm; co-solvent: 0.5% $iPrNH_2$ in iPrOH, flow : 3g/min; % of co-solvent : 35%; ABPR : 1500 psi; T: 30 °C; $R_t$ = 4.48 min (2nd eluting). |

(continued)

| Ex. No. | Structure | Procedure | Reactants | Analytical data |
|---|---|---|---|---|
| **SC-56a** | 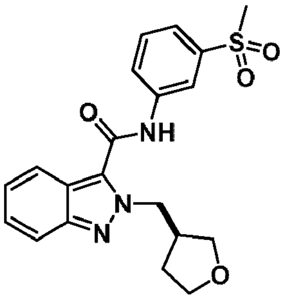 | **SC-39** | **Int-B22,** 3-(methylsulfonyl)aniline | $^1H$ NMR (400 MHz, DMSO-*d6*): δ (ppm) = 11.06 (bs, 1H), 8.43 (s, 1H), 8.05 - 7.90 (m, 2H), 7.78 - 7.60 (m, 3H), 7.36 (t, 1H), 7.24 (t, 1H), 4.86 - 4.70 (m, 2H), 3.82 - 3.77 (m, 1H), 3.68 - 3.54 (m, 3H), 3.22 (s, 3H), 2.98 - 2.90 (m, 1H), 1.96 - 1.86 (m, 1H), 1.74 - 1.64 (m, 1H); LCMS: m/z $[M+H]^+$ = 400.1 (calc. 400.1); chiral SFC: column: CHIRALCEL OJ-3 (4.6x150mm) 3 μm, co-solvent: 0.5% $Et_2NH$ in MeOH; flow : 3g/min; % of co-Solvent : 20%, ABPR : 1500 psi, T: 30 °C, $R_t$ = 4.12 min (first eluting). |
| **SC-58a** | 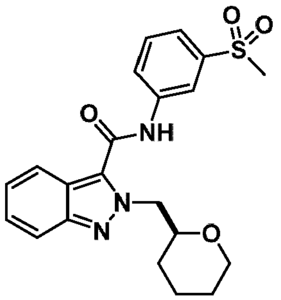 | **SC-39** | **Int-B23,** 3-(methylsulfonyl)aniline | $^1H$ NMR (400 MHz, DMSO-*d6*): δ (ppm) = 11.03 (s, 1H), 8.43 (s, 1H), 7.99 (d, 1H), 7.84 (d, 1H), 7.73 - 7.63 (m, 3H), 7.35 (t, 1H), 7.22 (t, 1H), 4.87 - 4.82 (m, 1H), 4.70 - 4.67 (m, 1H), 3.83 - 3.73 (m, 2H), 3.22 (s, 4H), 1.74 (m, 1H), 1.60 (d, 1H), 1.43 - 1.39 (m, 3H), 1.28 - 1.23 (m, 1H); LCMS: m/z $[M+H]^+$ = 414.1 (calc. 414.1); Chiral SFC: column: CHIRALCEL OD-3 (4.6x150mm) 3 μm, co-solvent: MeOH; flow : 3g/min; % of co-solvent : 30%, ABPR : 1500 psi, T: 30 °C, $R_t$ = 2.21 min (first eluting). |
| **SC-60** | 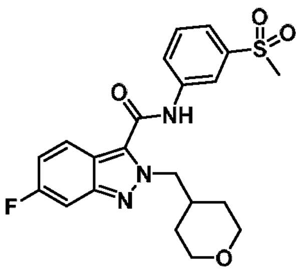 | **SC-05** | **Int-B31,** 3-(methylsulfonyl)aniline | $^1H$ NMR (400 MHz, DMSO-*d6*): δ (ppm) = 11.11 (s, 1H), 8.43 (s, 1H), 8.03 (d, 1H), 7.94-7.92 (m, 1H), 7.72-7.67 (m, 2H), 7.53 (d, 1H), 7.21-7.17 (m, 1H), 4.61 (d, 2H), 3.80 (d, 2H), 3.34-3.21 (m, 5H), 2.26 (bs, 1H), 1.42-1.30 (m, 4H). LCMS: m/z $[M+H]^+$ = 432.4 (calc. 432.1). |
| **SC-62** | 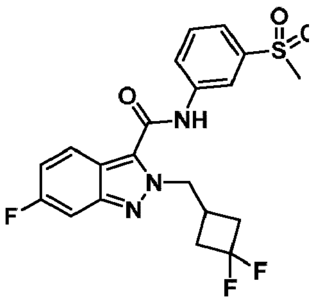 | **SC-05** | **Int-B33,** 3-(methylsulfonyl)aniline | $^1H$ NMR (400 MHz, DMSO-*d6*): δ (ppm) = 11.12 (s, 1H), 8.44 (s, 1H), 8.04 (d, 1H), 7.96-7.92 (m, 1H,), 7.74-7.67(m, 2H), 7.55(d, 1H), 7.23-7.15 (m, 1H), 4.81 (d, 2H), 3.24 (s, 3H), 2.95-2.84 (m, 1H), 2.68-2.64 (m, 2H), 2.61-2.57 (s, 2H); LCMS: m/z $[M+H]^+$ = 437.4 (calc. 438.1). |

(continued)

| Ex. No. | Structure | Procedure | Reactants | Analytical data |
|---|---|---|---|---|
| **SC-67** | 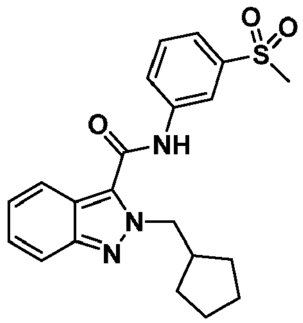 | **SC-15** | **Int-B6,** 3-(methylsulfonyl)aniline | $^1$H NMR (400 MHz, DMSO-*d6*): δ (ppm) = 11.05 (s, 1H), 8.44 (s, 1H), 8.04 (d, 1H), 7.86 (d, 1H), 7.78 - 7.64 (m, 3H), 7.39 - 7.34 (m, 1H), 7.29 - 7.23 (m, 1H), 4.67 (d, 2H), 3.24 (s, 3H), 2.57 - 2.53 (m, 1H), 1.61 - 1.47 (m, 6H), 1.35 - 1.22 (m, 2H); LCMS: m/z $[M+H]^+$ = 398.4 (calc. 398.2). |
| **SC-68** | 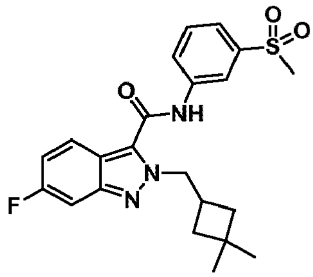 | **SC-05** | **Int-B32,** 3-(methylsulfonyl)aniline | $^1$H NMR (400 MHz, DMSO-*d6*): δ (ppm) = 11.10 (s, 1H), 8.43 (s, 1H), 8.04 (d, 1H), 7.92-7.89 (m, 1H,), 7.74-7.67(m, 2H), 7.53(d, 1H), 7.20-7.15 (m, 1H), 4.71 (d, 2H), 3.24 (s, 3H), 2.86-2.80 (m, 1H), 1.77-1.67 (m, 4H), 1.23-0.91 (m, 6H). LCMS: m/z $[M+H]^+$ = 430.2 (calc. 430.2). |
| **SC-70** | 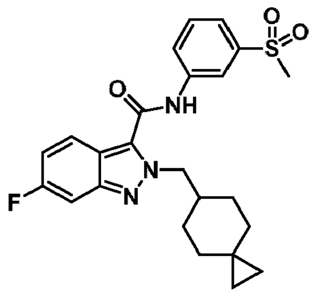 | **SC-05** | **Int-B27,** 3-(methylsulfonyl)aniline | $^1$H NMR (400 MHz, DMSO-*d6*): δ (ppm) = 11.11 (s, 1H), 8.43 (s, 1H), 8.04 (d, 1H), 7.93-7.90 (m, 1H), 7.74-7.67 (m, 2H), 7.55-7.52 (m, 1H), 7.21-7.16 (m, 1H), 4.61 (d, 2H), 3.25 (s, 3H), 2.18 (bs, 1H), 1.62-1.48 (m, 2H), 1.23-1.17 (m, 2H), 0.89-0.85 (m, 2H), 0.24-0.23 (m, 2H), 0.16-0.13 (m, 2H); LCMS: m/z $[M+H]^+$ = 456.3 (calc. 456.2). |
| **SC-71** | 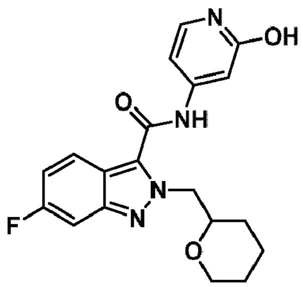 | **SC-05** (for step-1), **SC-06** (step-2) | **Int-B28,** 2-methoxypyri din-4-amine in step-1 | $^1$H NMR (400 MHz, DMSO-*d6*): δ (ppm) = 11.31 (bs, 1H), 10.81 (s, 1H), 7.81-7.77 (m, 1H), 7.52 -7.49 (m, 1H), 7.35 (d, 1H), 7.19-7.14 (m, 1H), 6.80 (s, 1H), 6.53-6.50 (m, 1H), 4.79-4.74 (m, 1H), 4.66-4.61 (m, 1H), 3.76-3.73 (m, 2H), 3.21-3.19 (m ,1H), 1.75-1.61 (m, 2H), 1.44-1.40 (m, 3H), 1.24-1.22 (m, 1H); LCMS: m/z $[M+H]^+$ = 371.2 (calc. 371.1). |
| **SC-73** | 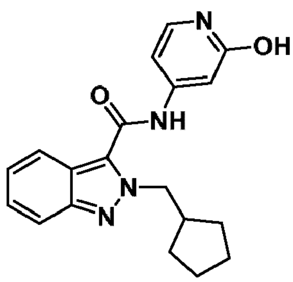 | **SC-43** | **Int-B26,** 2-(benzloxy)pyridine-4-amine in step-1 | $^1$H NMR (400 MHz, DMSO-*d6*): δ (ppm) = 11.30 (s, 1H), 10.76 (s, 1H), 7.78 - 7.73 (m, 2H), 7.40 - 7.33 (m, 2H), 7.30 - 7.24 (m, 1H), 6.82 (d, 1H), 6.54 (dd, 1H), 4.63 (d, 2H), 2.53 - 2.50 (m, 1H), 1.62 - 1.46 (m, 6H), 1.35 - 1.24 (m, 2H); LCMS: m/z $[M+H]^+$ = 337.1 (calc. 337.2). |

(continued)

| Ex. No. | Structure | Procedure | Reactants | Analytical data |
|---|---|---|---|---|
| **SC-74a** | 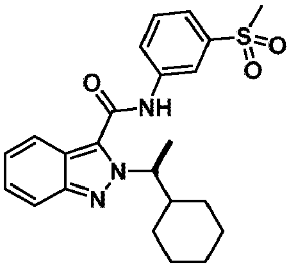 | **SC-39** | **Int-B29,** 3-(methylsulfo nyl)aniline | $^1$H NMR (400 MHz, DMSO-*d6*): δ (ppm) = 11.11 (s, 1H), 8.48 (s, 1H), 8.01 (d, 1H), 7.86 - 7.64 (m, 4H), 7.36 (t, 1H), 7.25 (t, 1H), 5.00 (bs, 1H), 3.24 (s, 3H), 2.00 - 1.88 (m, 2H), 1.72 (d, 1H), 1.60 - 1.49 (m, 5H), 1.30 - 1.18 (m, 1H), 1.13 - 0.97 (m, 3H), 0.90 - 0.85 (m, 2H); LCMS: m/z $[M+H]^+$ = 426.1 (calc. 426.2); chiral SFC: column : CHIRALCEL IE-3 (4.6x150mm) 3 μm, co-solvent: MeOH; flow : 3g/min; % of co-solvent : 30%, ABPR: 1500 psi, T: 30 °C, $R_t$ = 2.25 min (first eluting). |
| **SC-74b** | 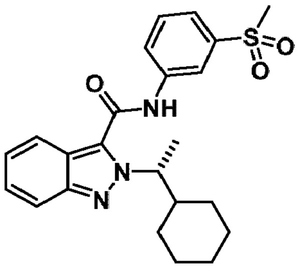 | **SC-39** | **Int-B29,** 3-(methylsulfo nyl)aniline | $^1$H NMR (400 MHz, DMSO-*d6*): δ (ppm) = 11.11 (s, 1H), 8.48 (s, 1H), 8.01 (d, 1H), 7.86 - 7.64 (m, 4H), 7.36 (t, 1H), 7.25 (t, 1H), 5.00 (bs, 1H), 3.24 (s, 3H), 2.00 - 1.88 (m, 2H), 1.72 (d, 1H), 1.60 - 1.49 (m, 5H), 1.30 - 1.18 (m, 1H), 1.13 - 0.97 (m, 3H), 0.90 - 0.85 (m, 2H). ); LCMS: m/z $[M+H]^+$ = 426.1 (calc. 426.2); chiral SFC: column : CHIRALCEL IE-3 (4.6x150 mm) 3 μm, co-solvent: MeOH; flow : 3g/min; % of co-solvent : 30%, ABPR: 1500 psi, T: 30 °C, $R_t$ = 2.96 min (second eluting). |
| **SC-77** | 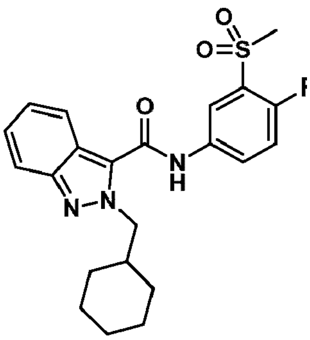 | **SC-05** | **Int-B5,** 4-fluoro-3-methanesulf onylaniline | $^1$H NMR (400 MHz, DMSO-*d6*): δ (ppm) = 11.04 (s, 1H), 8.42-8.40 (m, 1H), 8.11-8.08 (m, 1H), 7.84 (d, 1H), 7.77 (d, 1H), 7.59-7.55 (m, 1H), 7.38-7.35 (m, 1H), 7.28-7.24 (m, 1H), 4.59 (d, 2H), 3.37 (s, 3H), 2.00-1.95 (m, 1H), 1.62-1.48 (m, 5H), 1.23-1.00 (m, 5H). LCMS: m/z $[M+H]^+$ = 430.3 (calc. 430.2). |
| **SC-80** | 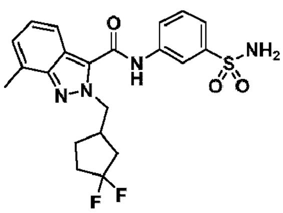 | **SC-15** | **Int-B34,** 3-aminobenze nesulfonami de | $^1$H NMR (400 MHz, DMSO-*d6*): δ (ppm) = 11.96 (s, 1H), 8.39 (s, 1H), 7.91-7.86 (m, 1H), 7.67-7.57 (m, 3H), 7.42 (s, 2H), 7.20-7.15 (m, 2H), 4.80-4.71 (m, 2H), 2.87-2.77 (m, 1H), 2.57 (s, 3H), 2.21-1.95 (m, 4H), 1.86-1.78 (m, 1H), 1.68-1.10 (m, 1H); LCMS: m/z $[M-H]^-$ = 447.1 (calc. 447.1). |

(continued)

| Ex. No. | Structure | Procedure | Reactants | Analytical data |
|---|---|---|---|---|
| SC-81 | 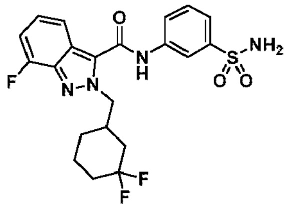 | SC-15 | **Int-B41,** 3-aminobenze nesulfonami de | $^1$H NMR (400 MHz, DMSO-*d6*): δ (ppm) = 11.06 (s, 1H), 8.38 (s, 1H), 7.88 (d, 1H), 7.67-7.58 (m, 3H), 7.43 (s, 2H), 7.26-7.17 (m, 2H), 4.75-4.64 (m, 2H), 2.32 (s, 1H), 1.95-1.87 (m, 2H), 1.77-1.63 (m, 3H), 1.57-1.54 (m, 1H), 1.39-1.35 (m, 1H), 1.20-1.15 (m, 1H); LCMS: m/z [M+H]$^+$ = 467.3 (calc. 467.1). |

Svnthesis of N-(2-carbamoylpyridin-4-yl)-4-chloro-2-((3,3-difluorocyclopentyl)methyl)-7-methyl-2H-indazole-3-carboxamide **(SC-82),** synthesized in form of enantiomers:

Enantiomer 1 of N-(2-carbamoylpyridin-4-yl)-4-chloro-2-((3,3-difluorocyclopentyl)methyl)-7-methyl-2H-indazole-3-carboxamide **(SC-82a)** and

Enantiomer 2 of N-(2-carbamoylpyridin-4-yl)-4-chloro-2-((3,3-difluorocyclopentyl)methyl)-7-methyl-2H-indazole-3-carboxamide **(SC-82b)**

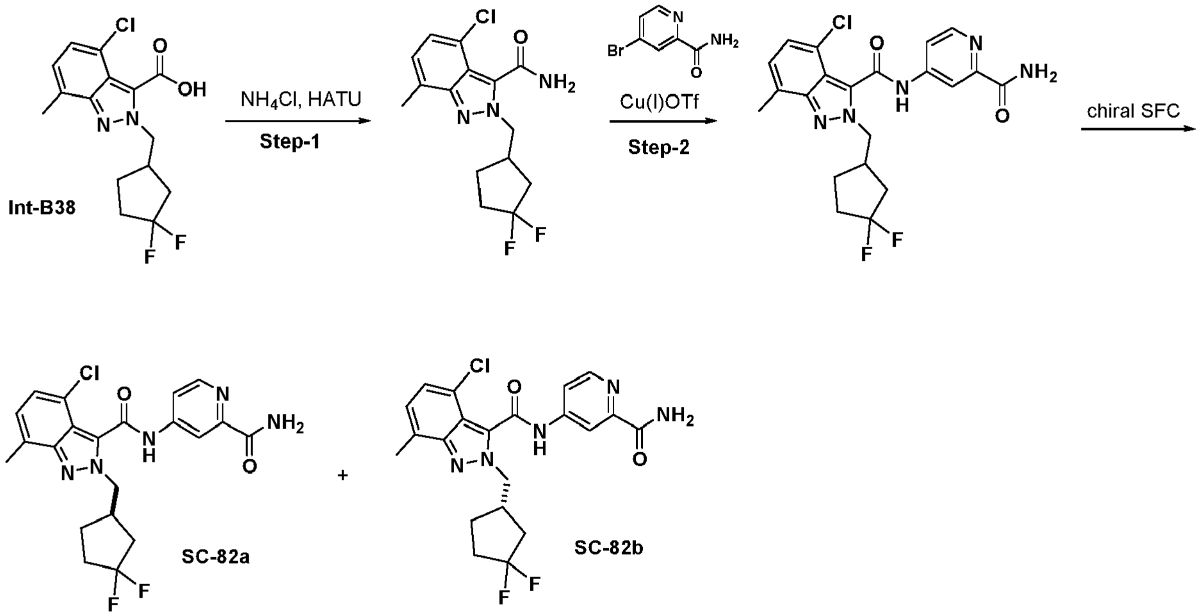

Int-B38

SC-82a + SC-82b

Step 1: To a stirred solution of 4-chloro-2-((3,3-difluorocyclopentyl)methyl)-7-methyl-2H-indazole-3-carboxylic acid **(Int-B38,** 1.0 g, 3.030 mmol) and $NH_4Cl$ (800 mg, 15.15 mmol) in DMF (10.0 mL) were added HATU (1.5 g, 45.45 mmol) followed by DIPEA (1.3 mL, 9.09 mmol) at 0 °C. The reaction mixture was stirred at ambient temperature for 12 h. The reaction mixture was diluted with water (30 mL), the resulting precipitate was filtered off, washed with water (10 mL), and dried under reduced pressure to afford 4-chloro-2-((3,3-difluorocyclopentyl)methyl)-7-methyl-2H-indazole-3-carboxamide (1.4 g).

Step-2: To a stirred solution of 4-chloro-2-((3,3-difluorocyclopentyl)methyl)-7-methyl-2H-indazole-3-carboxamide (826 mg, 2.512 mmol) and 4-bromopicolinamide (600 mg, 3.015 mmol) in 1,4-dioxane (5.0 mL), was added $Cs_2CO_3$ (2.04 g, 6.281 mmol) and the mixture was degassed using argon for 20 min. To the reaction mixture were added Cu(I)trifluoromethane sulfonate benzene complex (315 mg, 0.628 mmol) and trans-N,N-dimethyl cyclohexane diamine (107 mg, 0.7531 mmol) and the reaction mixture was stirred at 100 °C for 16 h. The reaction mixture was cooled to

ambient temperature, filtered through a pad of celite and the celite pad was washed with ethyl acetate (20 mL). The filtrate was concentrated under reduced pressure to get the crude product which was purified by prep-HPLC to afford N-(2-carbamoylpyridin-4-yl)-4-chloro-2-((3,3-difluorocyclopentyl)methyl)-7-methyl-2H-indazole-3-carboxamide (200 mg). The racemate was separated by chiral SFC to obtain 70 mg of **SC-82a** and 53 mg of **SC-82b.**

$^1H$ NMR (400 MHz, DMSO-*d6*): δ (ppm) = 11.52 (s, 1H), 8.59 (d, 1H), 8.40 (s, 1H), 8.11 (s, 1H), 7.86 (d, 1H), 7.67 (s, 1H), 7.20-7.11 (m, 2H), 4.57-4.55 (m, 2H), 2.87-2.83 (m, 1H), 2.54 (s, 3H), 2.21-2.00 (m, 4H), 1.87-1.82 (m, 1H), 1.66-1.60 (m, 1H); LCMS: m/z $[M+H]^+$ = 448.3 (calc. = 448.1).

**SC-82a:** chiral SFC: column : CHIRALCEL OJ-3 (4.6x150 mm) 3 μm, co-solvent: 0.5% diethylamine in MeOH; flow : 3 mL/min; % of co-solvent: 15%, ABPR: 1500 psi, T: 30 °C, $R_t$ = 2.30 min (first eluting).

**SC-82b:** chiral SFC: column : CHIRALCEL OJ-3 (4.6x150 mm) 3 μm, co-solvent: 0.5% diethylamine in MeOH; flow : 3 mL/min; % of co-solvent: 15%, ABPR: 1500 psi, T: 30 °C, $R_t$ = 2.57 min (second eluting).

Svnthesis of 2-(cyclohexylmethyl)-4-methyl-N-(3-(methylsulfonyl)phenyl)-2H-indazole-3-carboxamide (**SC-83**):

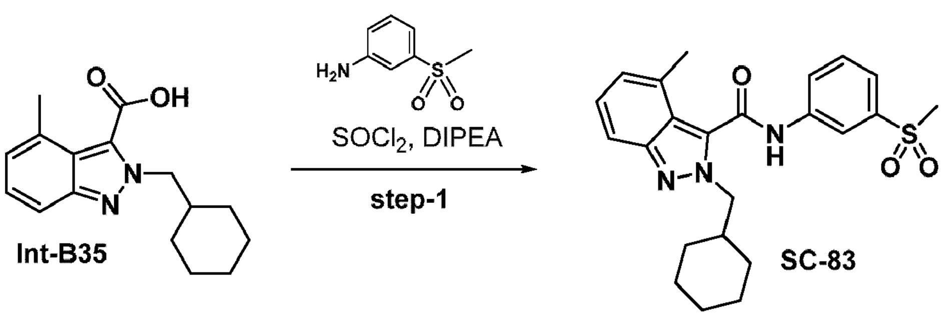


Step 1: To a stirred solution of 2-(cyclohexylmethyl)-4-methyl-2H-indazole-3-carboxylic acid **(Int-B35)** (100 mg, 0.367 mmol) in dichloromethane (10 mL) was added $SOCl_2$ (1.0 mL) and the reaction was stirred at 50 °C for 1 h. The reaction mixture was concentrated under reduced pressure to give a residue. The residue was dissolved in THF (5 mL) and was added to a stirred solution of 3-(methylsulfonyl)aniline (100 mg, 0.484 mmol) and DIPEA (0.2 mL, 1.212 mmol) in THF (5 mL) dropwise at ambient temperature. The reaction mixture was stirred at ambient temperature under an argon atmosphere for 16 h. The reaction mixture was diluted with water (50 mL), the resulting precipitate was filtered off, washed with water (10 mL), and dried under reduced pressure to get the crude material which was purified by prep-HPLC to get 2-(cyclohexylmethyl)-4-methyl-N-(3-(methylsulfonyl)phenyl)-2H-indazole-3-carboxamide (15 mg, 9%). $^1H$ NMR (400 MHz, DMSO-*d6*): δ (ppm) = 11.35 (s, 1H), 8.46 (s, 1H), 7.95 (d, 1H), 7.73-7.67 (m, 2H), 7.54 (d, 1H), 7.22 (t, 2H), 6.93 (d, 1H), 4.34 (d, 2H), 3.32-3.26 (m, 3H), 2.45 (s, 3H), 2.03-1.99 (m, 1H), 1.64-1.51 (m, 5H), 1.19-0.98 (m, 5H). LCMS: m/z $[M+H]^+$ = 426.1 (calc. = 426.2).

Synthesis of 4-chloro-7-methyl-N-(3-sulfamoylphenyl)-2-((tetrahydro-2H-pyran-2-yl)methyl)-2H-indazole-3-carboxamide **(SC-84),** synthesized in form of enantiomers:

Enantiomer 1 of 4-chloro-7-methyl-N-(3-sulfamoylphenyl)-2-((tetrahydro-2H-pyran-2-yl)methyl)-2H-indazole-3-carboxamide **(SC-84a)** and

Enantiomer 2 of 4-chloro-7-methyl-N-(3-sulfamoylphenyl)-2-((tetrahvdro-2H-pyran-2-yl)methyl)-2H-indazole-3-carboxamide **(SC-84b)**

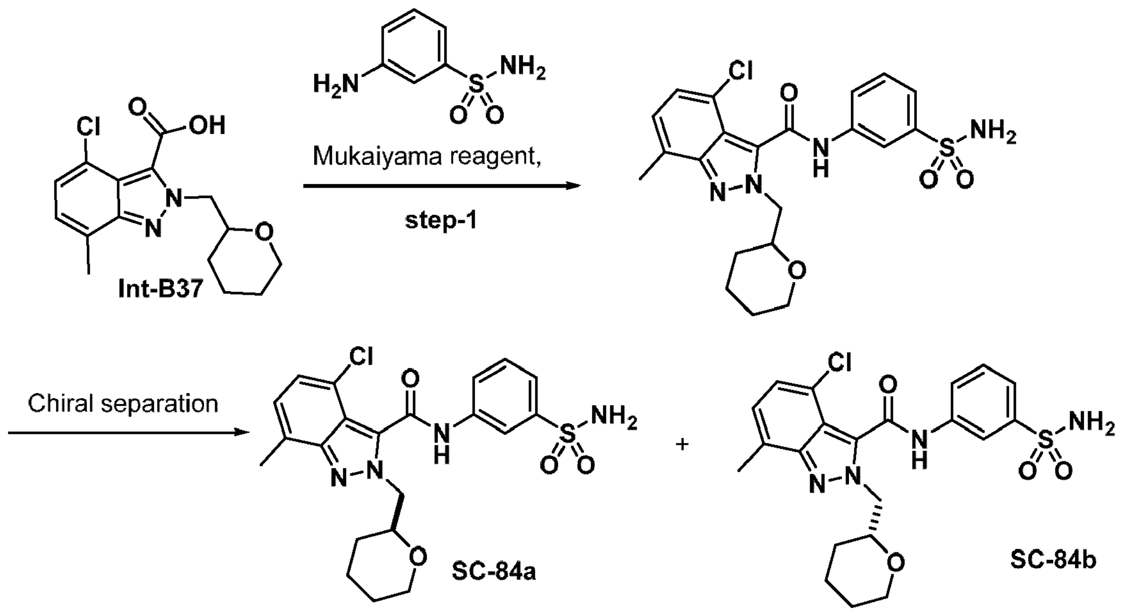


Int-B37

SC-84a

SC-84b

Step 1: To a solution of 4-chloro-7-methyl-2-[(oxan-2-yl)methyl]-2H-indazole-3-carboxylic acid (**Int-B37,** 300 mg, 0.974 mmol, 1.0 equiv.) in DMF (5 ml) were added Mukaiyama reagent (747 mg, 2.92 mmol, 2.0 equiv.) and DIPEA (0.5 ml, 2.92 mmol, 3.0 equiv.) followed by addition of 3-aminobenzene-1-sulfonamide (201 mg, 1.16 mmol, 1.2 equiv.). The reaction mixture was stirred at ambient temperature for 16 h and was then quenched with water (25 mL) and extracted with ethyl acetate (3 × 50 mL). The combined organic layers were washed with cold brine (30 mL), dried over sodium sulfate and concentrated under reduced pressure to get the crude material which was purified by reverse phase chromatography to yield racemic 4-chloro-7-methyl-2-[(oxan-2-yl)methyl]-N-(3-sulfamoylphenyl)-2H-indazole-3-carboxamide. Yield: 51% (230 mg, 0.497 mmol). The racemic mixture was then separated using chiral HPLC. Data for **SC-84a:** $^{1}H$ NMR (400 MHz, DMSO-*d6*): δ (ppm) = 11.14 (s, 1H), 8.40 (s, 1H), 7.78 (d, 1H), 7.62-7.55 (s, 2H,), 7.43 (s, 2H), 7.16-7.08 (m, 2H), 4.62-4.52 (m, 2H) 3.83-3.72 (m, 2H), 3.26-3.22 (m, 1H), 2.50 (s, 3H), 1.73-1.78 (m, 1H), 1.65-161 (m, 1H), 1.44-1.40 (m, 3H), 1.28-1.19 (m, 1H). LCMS: m/z $[M+H]^+$ = 463.2 (calc. = 463.1); chiral HPLC: column: Chiralpak IG (4.6x250 mm) 5 μm, mobile phase: hexane/ethyl acetate/ethanol/isopropylamne 50/25/25/0.1, flow rate: 1.0 mL/min $R_t$ = 4.43 min (first eluting).

Synthesis of 7-cyclopropyl-N-(3-sulfamoylphenyl)-2-((tetrahydro-2H-pyran-4-yl)methyl)-2H-indazole-3-carboxamide (**SC-85**):

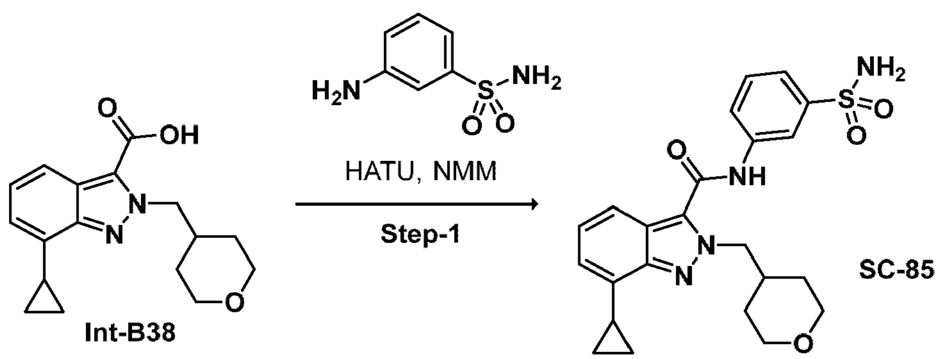


Int-B38

SC-85

Step 1: To a stirred solution of 7-cyclopropyl-2-((tetrahydro-2H-pyran-4-yl)methyl)-2H-indazole-3-carboxylic acid **(Int-B38)** (80 mg, 0.266 mmol) and 3-aminobenzenesulfonamide (55 mg, 0.319 mmol) in dry DMF (1 mL) was added N-methyl morpholine (80 mg, 0.798 mmol) followed by HATU (151 mg, 0.399 mmol) at ambient temperature. The resulting reaction mixture was stirred at ambient temperature for 2 h. The reaction mixture was quenched with ice cold water (5 mL) and the resulting precipitate was filtered off, washed with n-pentane and dried under reduced pressure to

get the crude product which was purified by prep-HPLC to afford 7-cyclopropyl-N-(3-sulfamoylphenyl)-2-((tetrahydro-2H-pyran-4-yl)methyl)-2H-indazole-3-carboxamide (SC-85, 18 mg, yield: 15%). $^1$H NMR (400 MHz, DMSO-*d6*): δ (ppm) = 10.91 (s, 1H), 8.38 (s, 1H), 7.89-7.87 (m, 1H), 7.61-7.57 (m, 3H), 7.41 (s, 2H), 7.15-7.13 (m, 1H), 6.92 (d, 1H), 4.66 (d, 2H), 3.82-3.79 (m, 2H), 3.26-3.22 (m, 2H), 2.49-2.43 (m, 1H), 2.26-2.22 (m, 1H), 1.43-1.31 (m, 4H), 1.07-1.02 (m, 4H). LCMS: m/z $[M+H]^+$ = 455.3 (calc. = 455.2).

Svnthesis of N-(2-carbamoylpyridin-4-yl)-2-(cyclohexylmethyl)-6-fluoro-2H-indazole-3-carboxamide (**SC-86**):

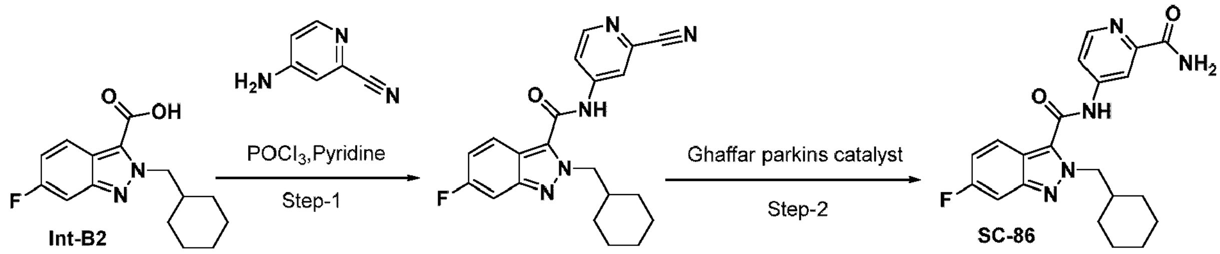


Int-B2

SC-86

Step 1: To a stirred solution of 2-(cyclohexylmethyl)-6-fluoro-2H-indazole-3-carboxylic acid **(Int-B2,** 0.2 g, 0.72 mmol, 1 equiv.) in pyridine (2 mL) were added $POCl_3$ (0.11 mL, 1.09 mmol, 1.5 equiv.) and 4-aminopyridine-2-carboxamide (0.12 g, 0.86 mmol, 1.2 equiv.) at 0 °C. The reaction mixture was stirred at ambient temperature for 1 h. The reaction mixture was concentrated under reduced pressure and was ten quenched with crushed ice. The qqueous part was extracted with ethyl acetate (3×40 mL). The combined organic layers were washed with brine and dried over $Na_2SO_4$ and concentrated under reduced pressure to get crude N-(2-cyanopyridin-4-yl)-2-(cyclohexylmethyl)-6-fluoro-2H-indazole-3-carboxamide. This crude material was directly used in the next step without further purification. Yield: 29% (0.08 g, 0.21 mmol)

Step-2: To a stirred solution of N-(2-cyanopyridin-4-yl)-2-(cyclohexylmethyl)-6-fluoro-2H-indazole-3-carboxamide (0.08 g, 0.21 mmol, 1 equiv.) in EtOH (5 mL) and $H_2O$ (2 mL) was added Ghaffar Parkins catalyst (9 mg, 0.02 mmol, 0.1 equiv.) at ambient temperature. The reaction mixture was heated to80 °C for 16 h. The reaction mixture was concentrated under reduced pressure to get the crude product which was purified by column chromatography (silica; 0-30% acetone in hexane as eluent) to yield N-(2-carbamoylpyridin-4-yl)-2-(cyclohexylmethyl)-6-fluoro-2H-indazole-3-carboxamide (SC-86) Yield: 48% (0.04 g, 0.1 mmol). $^1$H NMR (400 MHz, DMSO-*d6*): δ (ppm) = 11.26 (s, 1H), 8.58 (d, 1H), 8.44 (m, 1H), 8.12 (s, 1H), 7.92-7.89 (m, 2H), 7.66 (s, 1H), 7.55-7.53 (m, 1H), 7.22-7.17 (m, 1H), 4.56 (d, 2H), 1.97 (s, 1H), 1.62-1.48 (m, 5H), 1.23-1.12 (m, 3H), 1.05-1.00 (m, 2H). LCMS: m/z $[M+H]^+$ = 396.3 (calc. = 396.2).

Svnthesis of 2-(cyclohexylmethyl)-6-fluoro-N-(2-sulfamoylpyridin-4-yl)-2H-indazole-3-carboxamide (**SC-87**):

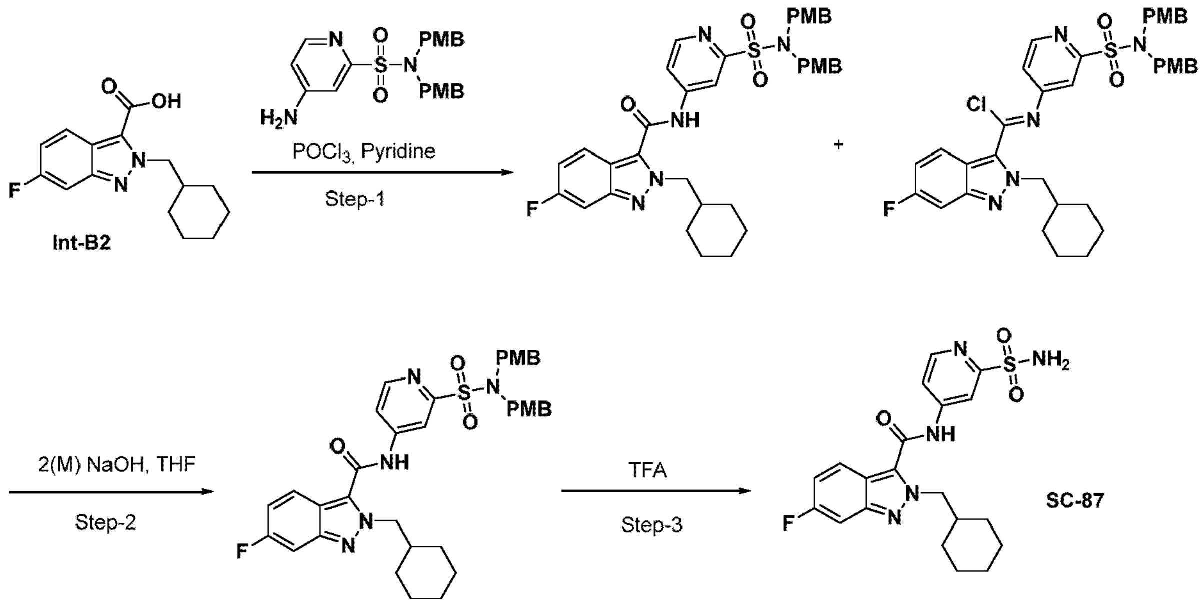


Int-B2

SC-87

Step 1: To a stirred solution of 2-(cyclohexylmethyl)-6-fluoro-2H-indazole-3-carboxylic acid **(Int-B2,** 0.15 g, 0.36 mmol, 1 equiv.) in pyridine (3 mL) were added $POCl_3$ (0.06 mL, 0.54 mmol, 1.5 equiv.) and 4-amino-N,N-bis[(4-methoxyphenyl)methyl]pyridine-2-sulfonamide (0.12 g, 0.43 mmol, 1.2 equiv.) at 0 °C. The reaction mixture was stirred at ambient temperature for 2 h. The reaction mixture was concentrated and diluted with ice cold water. The aqueous part was extracted with EtOAc (3×50 mL). The combined organic layers were washed with brine, dried over $Na_2SO_4$ and concentrated under reduced pressure to get a crude mixture of N-(2-{bis[(4-methoxyphenyl)methyl]sulfamoyl}pyridin-4-yl)-2-(cyclohexylmethyl)-6-fluoro-2H-indazole-3-carboxamide and (Z)-N-(2-{bis[(4-methoxyphenyl)methyl]sulfamoyl} pyridin-4-yl)-2-(cyclohexylmethyl)-6-fluoro-2H-indazole-3-carbonimidoyl chloride. This crude mixture was directly used in the next step without further purification. Yield: Quantitative (0.25 g, crude)

Step-2: To a stirred solution of N-(2-{bis[(4-methoxyphenyl)methyl]sulfamoyl}pyridin-4-yl)-2-(cyclohexylmethyl)-6-fluoro-2H-indazole-3-carboxamide and (Z)-N-(2-{bis[(4-methoxyphenyl)methyl]sulfamoyl}pyridin-4-yl)-2-(cyclohexylmethyl)-6-fluoro-2H-indazole-3-carbonimidoyl chloride (0.25 g, crude, 1 equiv.) in THF (8 mL) was added 2(M) NaOH (4 mL) at ambient temperature. The reaction mixture was heated to 70 °C for 16 h. The reaction mixture was evaporated to get the crude material which was acidified with saturated $NaHSO_4$ solution to maintain pH-2. The resulting mixture was extracted with EtOAc (2×60 mL). The combined organic layers were dried over $Na_2SO_4$ and concentrated to get crude N-(2-{bis[(4-methoxyphenyl)methyl]sulfamoyl}pyridin-4-yl)-2-(cyclohexylmethyl)-6-fluoro-2H-indazole-3-carboxamide. This crude material was directly used in the next step without further purification. Yield: Quantitative (0.25 g, 0.37 mmol).

Step-3: To a stirred solution of N-(2-{bis[(4-methoxyphenyl)methyl]sulfamoyl}pyridin-4-yl)-2-(cyclohexylmethyl)-6-fluoro-2H-indazole-3-carboxamide (0.25 g, 0.37 mmol, 1 equiv.) in THF (3 mL) was added TFA (4 mL) at ambient temperature. The resulting mixture was heated to 80 °C for 2 h. The reaction mixture was evaporated to get the crude material which was basified with saturated $NaHCO_3$ solution to maintain pH~10 and was then extracted with DCM (2×80 mL). The combined organic layers were washed with brine, dried over $Na_2SO_4$ and concentrated to get the crude material which was purified by reverse phase preparative HPLC to yield 2-[(4,4-difluorocyclohexyl)methyl]-N-(2-sulfamoylpyridin-4-yl)-2H-indazole-3-carboxamide. Yield: 25% over 3 steps, (0.04 g, 0.09 mmol). $^1H$ NMR (400 MHz, DMSO-*d6*): δ (ppm) = 11.41 (s, 1H), 8.68 (d, 1H), 8.39 (s, 1H), 7.93-7.90 (m, 2H), 7.57-7.54 (m, 1H), 7.49 (s, 2H), 7.24-7.18 (m, 1H), 4.57 (d, 2H), 1.98-1.96 (m, 1H), 1.64-1.48 (m, 5H), 1.16-1.12 (m, 3H), 1.06-0.99 (m, 2H). LCMS: m/z $[M+H]^+$ = 432.3 (calc. = 432.1).

The following examples were synthesized by analogy to the procedures described above using appropriate reactants and adjusted purification protocols:

| Ex. No. | Structure | Reactants | Analytical data |
|---|---|---|---|
| SC-88b | 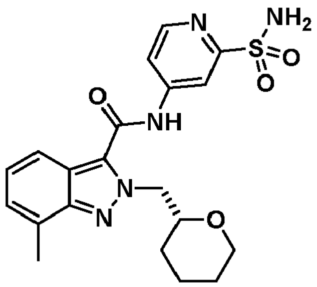 | Int-B39b | $^1$H NMR (400 MHz, DMSO-*d6*): δ (ppm) = 11.31 (s, 1H), 8.64-8.63 (m, 1H), 8.40 (s, 1H), 7.90-7.88 (m, 1H), 7.63-7.61 (m, 1H), 7.47 (s, 2H), 7.18-7.15 (m, 2H), 4.89-4.83 (m, 1H), 4.73-4.69 (m, 1H), 3.79-3.71 (m, 2H), 3.24-3.17 (m,1H), 2.57 (s, 3H), 1.77-1.75 (m, 1H), 1.66-1.63 (m,1H), 1.47-1.40 (m, 3H), 1.28-1.23 (m, 1H); LCMS: m/z $[M+H]^+$ = 430.3 (calc. = 430.2); chiral HPLC: column: Chiralpak IC (4.6x250 mm) 5 μm, mobile phase: hexane/ ethyl acetate/ethanol/isopropylamne 50/25/25/0.1, flow rate: 1.0 mL/min $R_t$ = 8.19 min (second eluting). |
| SC-89b | 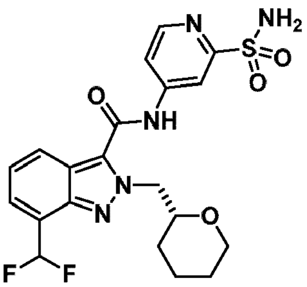 | Int-B40b | $^1$H NMR (400 MHz, DMSO-*d6*): δ (ppm) = 11.42 (s, 1H), 8.66 (d, 1H), 8.40 (d, 1H), 8.01 (d, 1H), 7.90-7.88 (m, 1H), 7.64 (d, 1H), 7.55-7.27 (m, 4H), 4.91-4.86 (m, 1H), 4.77-4.73 (m, 1H), 3.78-3.70 (m, 2H), 3.23-3.19 (m, 1H), 1.78-1.66 (m, 2H), 1.45-1.23 (m, 4H); LCMS: m/z $[M+H]^+$ = 466.3 (calc. = 466.1); chiral HPLC: column: Chiralpak IC (4.6x250 mm) 5 μm, mobile phase: hexane/ethyl acetate/ ethanol/isopropylamne 50/25/25/0.1, flow rate: 1.0 mL/min $R_t$ = 6.32 min (second eluting). |

The following prophetic examples could be synthesized according to the general schemes and by analogy to the synthetic procedures described above:

| Structure | Prophetic example number | Chemical name |
|---|---|---|
| 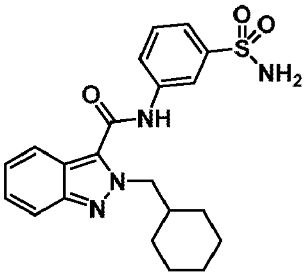 | **SC-18** | 2-(cyclohexylmethyl)-N-(3-sulfamoylphenyl)-2H-indazole-3- carboxamide |
| 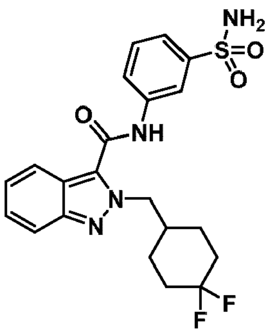 | **SC-23** | 2-((4,4-difluorocyclohexyl)methyl)-N-(3-sulfamoylphenyl)-2H-indazole-3- carboxamide |

(continued)

| Structure | Prophetic example number | Chemical name |
|---|---|---|
| 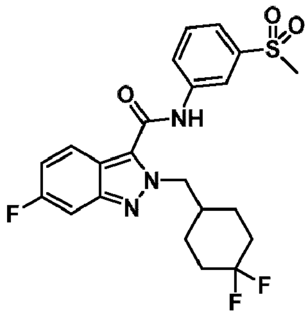 | **SC-27** | 2-((4,4-difluorocyclohexyl)methyl)-6-fluoro-N-(3-(methylsulfonyl)phenyl)-2H-indazole-3-carboxamide |
| 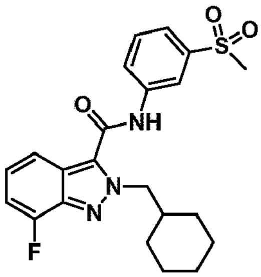 | **SC-29** | 2-(cyclohexylmethyl)-7-fluoro-N-(3-(methylsulfonyl)phenyl)-2H-indazole-3- carboxamide |
| 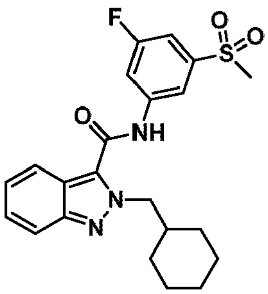 | **SC-34** | 2-(cyclohexylmethyl)-N-(3-fluoro-5-(methylsulfonyl)phenyl)-2H-indazole-3- carboxamide |
| 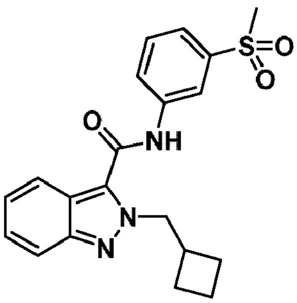 | **SC-64** | 2-(cyclobutylmethyl)-N-(3-(methylsulfonyl)phenyl)-2H-indazole-3- carboxamide |
| 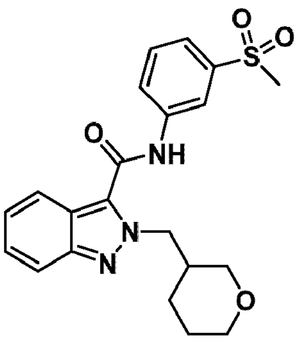 | **SC-65** | N-(3-(methylsulfonyl)phenyl)-2-((tetrahydro-2H-pyran-3-yl)methyl)-2H-indazole-3-carboxamide |

(continued)

| Structure | Prophetic example number | Chemical name |
|---|---|---|
| 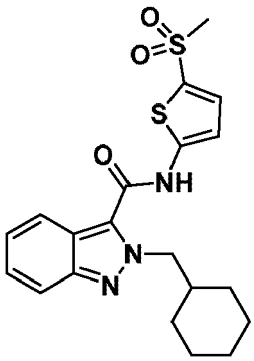 | **SC-69** | 2-(cyclohexylmethyl)-N-(5-(methylsulfonyl)thiophen-2-yl)-2H-indazole-3-carboxamide |
| 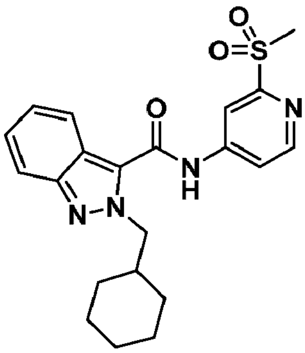 | **SC-78** | 2-(cyclohexylmethyl)-N-(2-(methylsulfonyl)pyridin-4-yl)-2H-indazole-3-carboxamide |
| 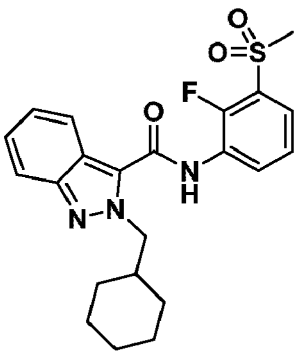 | **SC-79** | 2-(cyclohexylmethyl)-N-(2-fluoro-3-(methylsulfonyl) phenyl)-2H-indazole-3-carboxamide |
| 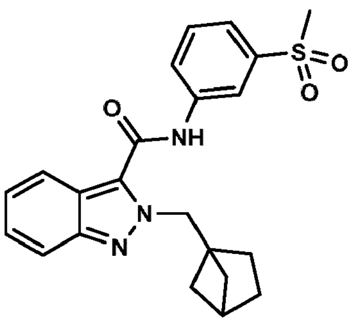 | **SC-90** | 2-(1-bicyclo[2.1.1]hexanylmethyl)-*N*-(3-methylsulfonylphenyl)indazole-3-carboxamide |

(continued)

| Structure | Prophetic example number | Chemical name |
|---|---|---|
| 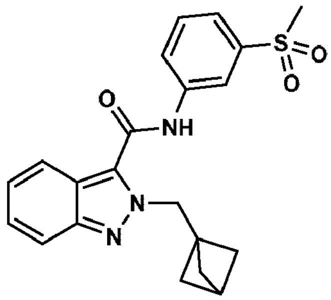 | **SC-91** | 2-(1-bicyclo[1.1.1]pentanylmethyl)-N-(3-methylsulfonylphenyl)indazole-3-carboxamide |
| 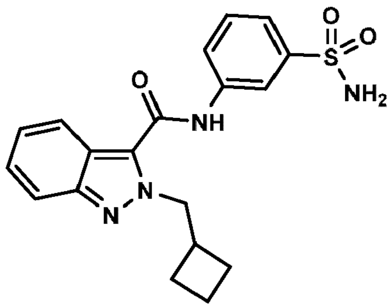 | **SC-92** | 2-(cyclobutylmethyl)-N-(3-sulfamoylphenyl)indazole-3-carboxamide |
| 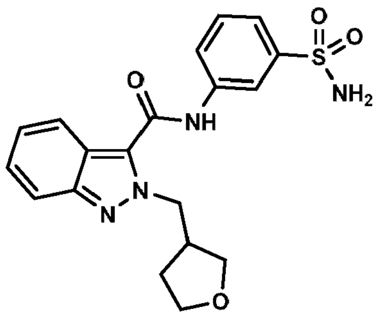 | **SC-93** | 2-(oxolan-3-ylmethyl)-*N*-(3-sulfamoylphenyl) indazole-3-carboxamide |
| 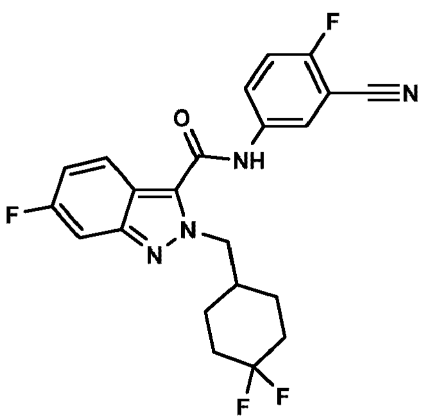 | **SC-94** | *N*-(3-cyano-4-fluorophenyl)-2-[(4,4-difluorocyclohexyl)methyl]-6-fluoroindazole-3-carboxamide |

(continued)

| Structure | Prophetic example number | Chemical name |
|---|---|---|
| 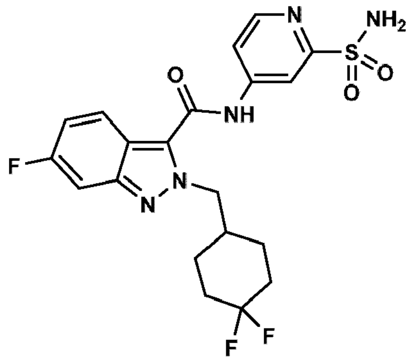 | **SC-95** | 2-[(4,4-difluorocyclohexyl)methyl]-6-fluoro-*N*-(2-sulfamoylpyridin-4-yl)indazole-3-carboxamide |
| 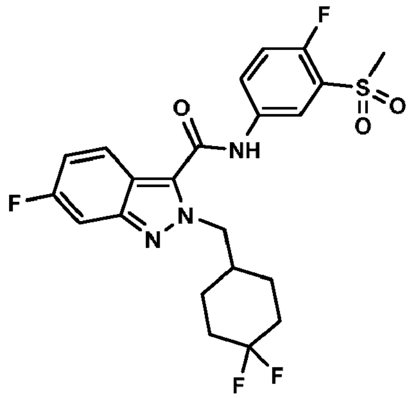 | **SC-96** | 2-[(4,4-difluorocyclohexyl)methyl]-6-fluoro-*N*-(4-fluoro-3- methylsulfonylphenyl)indazole-3-carboxamide |
| 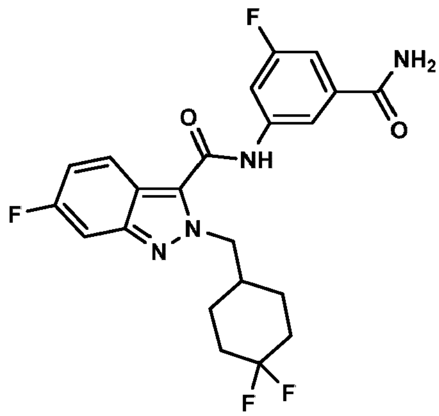 | **SC-97** | *N*-(3-carbamoyl-5-fluorophenyl)-2-[(4,4-difluorocyclohexyl)methyl]-6-fluoroindazole-3-carboxamide |

(continued)

| Structure | Prophetic example number | Chemical name |
|---|---|---|
| 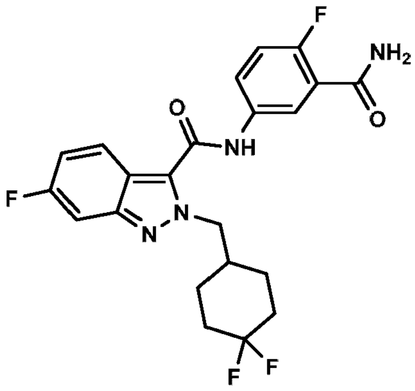 | **SC-98** | *N*-(3-carbamoyl-4-fluorophenyl)-2-[(4,4-difluorocyclohexyl)methyl]-6-fluoroindazole-3-carboxamide |
| 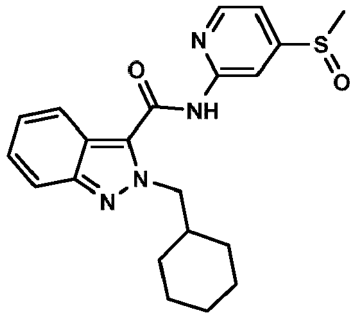 | **SC-99** | 2-(cyclohexylmethyl)-*N*-[4-[*(R)*-methylsulfinyl]pyridin-2-yl]indazole-3-carboxamide |
| 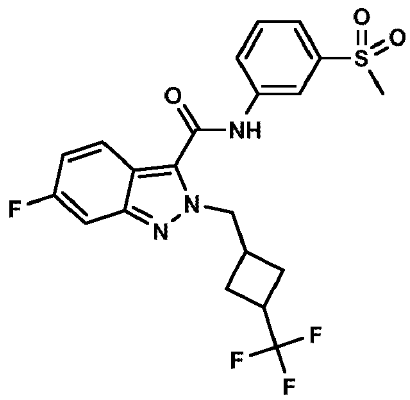 | **SC-100** | 6-fluoro-*N*-(3-methylsulfonylphenyl)-2-[[3-(trifluoromethyl)cyclobutyl]methyl]indaz ole-3-carboxamide |
| 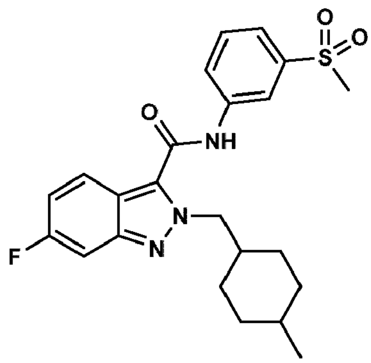 | **SC-101** | 6-fluoro-2-[(4-methylcyclohexyl)methyl]-*N*-(3-methylsulfonylphenyl)indazole-3-carboxamide |

(continued)

| Structure | Prophetic example number | Chemical name |
|---|---|---|
| 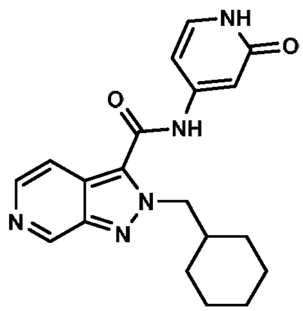 | **SC-102** | 2-(cyclohexylmethyl)-*N*-(2-hydroxypyridin-4-yl) pyrazolo[3,4-c]pyridine-3-carboxamide |
| 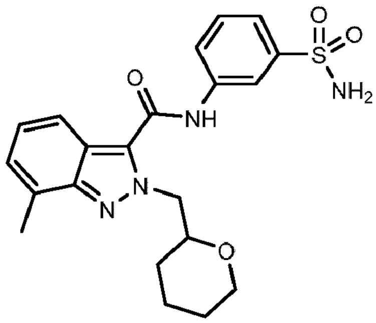 | **SC-103** | 7-methyl-2-(oxan-2-ylmethyl)-N-(3-sulfamoylphenyl) indazole-3-carboxamide |
| 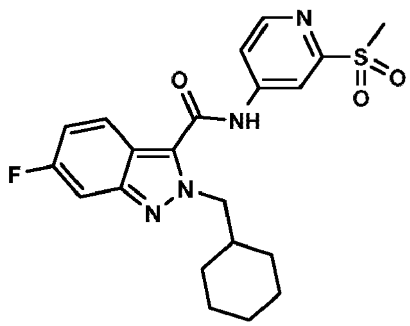 | **SC-104** | 2-(cyclohexylmethyl)-6-fluoro-*N*-(2-methylsulfonylpyridin-4-yl)indazole-3-carboxamide |
| 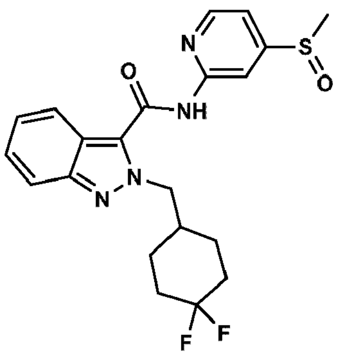 | **SC-105** | 2-[(4,4-difluorocyclohexyl)methyl]-*N*-[4-[ *(R)*-methylsulfinyl]pyridin-2-yl]indazole-3 -carboxamide |

(continued)

| Structure | Prophetic example number | Chemical name |
|---|---|---|
| 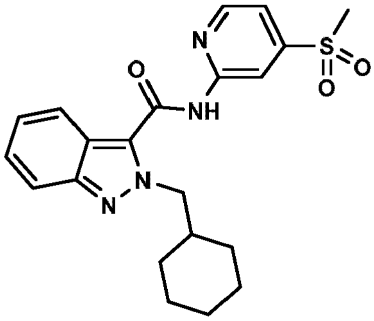 | **SC-106** | 2-(cyclohexylmethyl)-*N*-(4-methylsulfonylpyridin-2-yl) indazole-3-carboxamide |
| 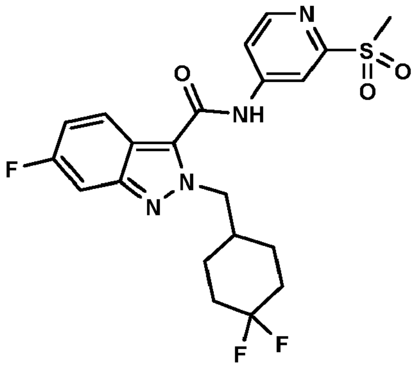 | **SC-107** | 2-[(4,4-difluorocyclohexyl)methyl]-6-fluoro-N-(2-methylsulfonylpyridin-4-yl)indazole-3-carboxamide |
| 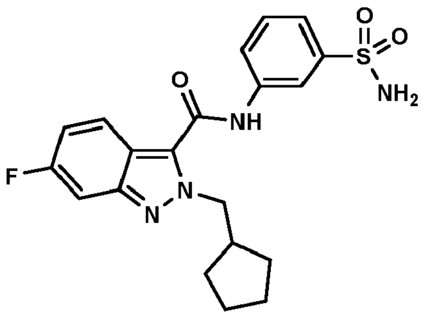 | **SC-108** | 2-(cyclopentylmethyl)-6-fluoro-*N*-(3-sulfamoylphenyl) indazole-3-carboxamide |
| 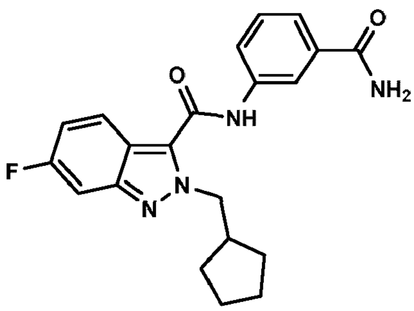 | **SC-109** | *N*-(3-carbamoylphenyl)-2-(cyclopentylmethyl)-6-fluoroindazole-3- carboxamide |

(continued)

| Structure | Prophetic example number | Chemical name |
|---|---|---|
| 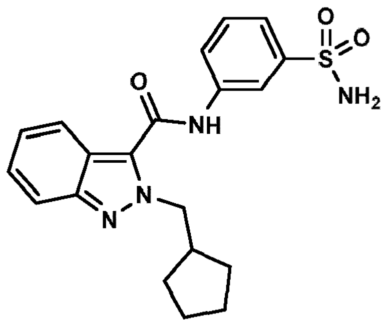 | **SC-110** | 2-(cyclopentylmethyl)-*N*-(3-sulfamoylphenyl) indazole-3-carboxamide |
| 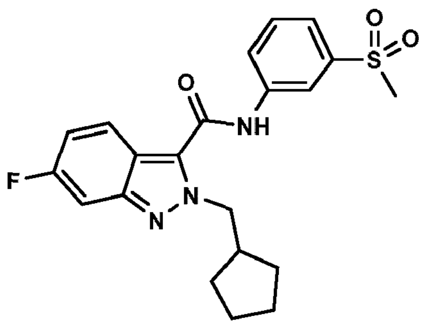 | **SC-110** | 2-(cyclopentylmethyl)-6-fluoro-*N*-(3-methylsulfonylphenyl)indazole-3-carboxamide |
| 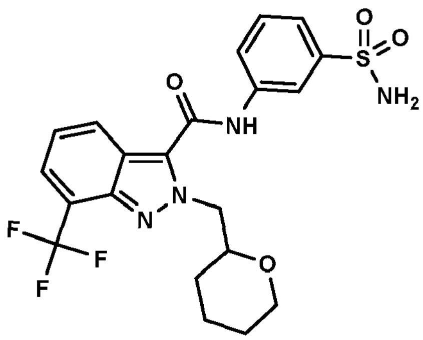 | **SC-111** | 2-(oxan-2-ylmethyl)-N-(3-sulfamoylphenyl)-7-(trifluoromethyl)indazole-3-carboxamide |
| 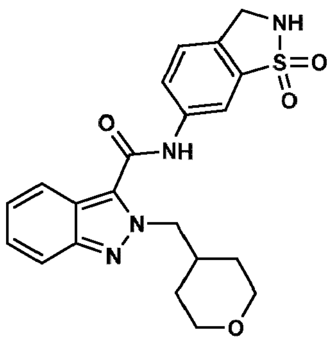 | **SC-112** | *N*-(1,1-dioxo-2,3-dihydro-1,2-benzothiazol-6-yl)-2-(oxan-4-ylmethyl)indazole-3-carboxamide |

(continued)

| Structure | Prophetic example number | Chemical name |
|---|---|---|
| 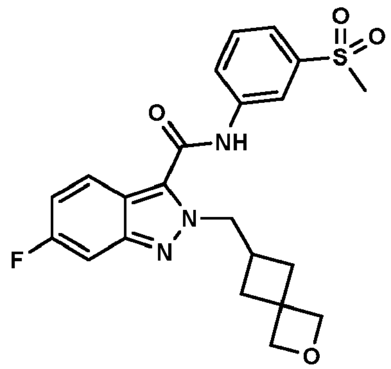 | **SC-113** | 6-fluoro-*N*-(3-methylsulfonylphenyl)-2-(2-oxaspiro [3.3]heptan-6-ylmethyl)indazole-3-carboxamide |
| 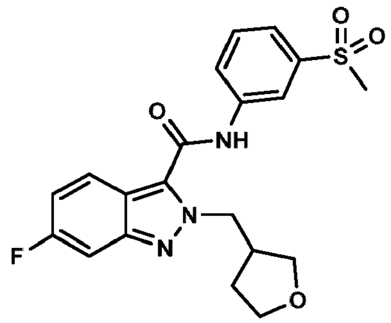 | **SC-114** | 6-fluoro-*N*-(3-methylsulfonylphenyl)-2-[[rac-(3R)-oxolan-3-yl]methyl]indazole-3-carboxamide |
| 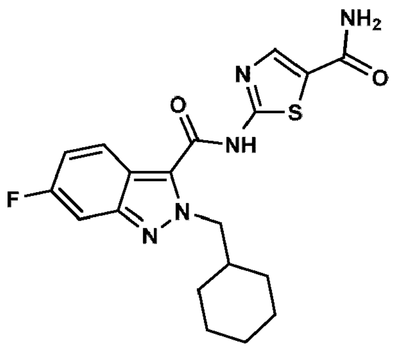 | **SC-115** | 2-[[2-(cyclohexylmethyl)-6-fluoroindazole-3-carbonyl] amino]-1,3-thiazole-5-carboxamide |
| 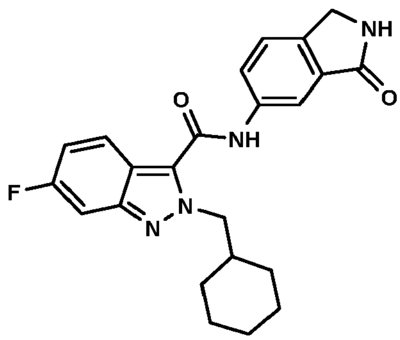 | **SC-116** | 2-(cyclohexylmethyl)-6-fluoro-*N*-(3-oxo-1,2-dihydroisoindol-5-yl)indazole-3-carboxamide |

(continued)

| Structure | Prophetic example number | Chemical name |
|---|---|---|
| 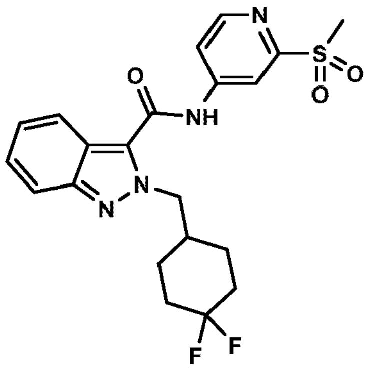 | **SC-117** | 2-[(4,4-difluorocyclohexyl)methyl]-*N*-(2-methylsulfonylpyridin-4-yl)indazole-3-carboxamide |
| 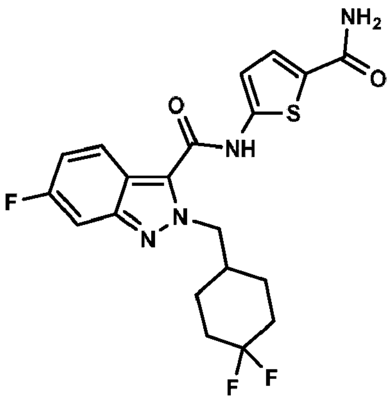 | **SC-118** | 7V-(5-carbamoylthiophen-2-yl)-2-[(4,4-difluorocyclohexyl)methyl]-6-fluoroindazole-3-carboxamide |
| 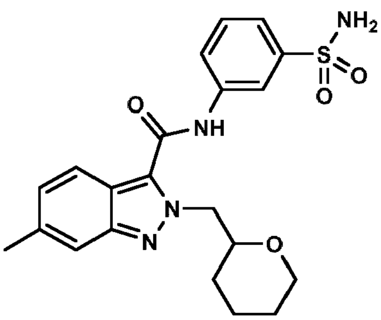 | **SC-119** | 6-methyl-2-(oxan-2-ylmethyl)-N (3-sulfamoylphenyl) indazole-3-carboxamide |
| 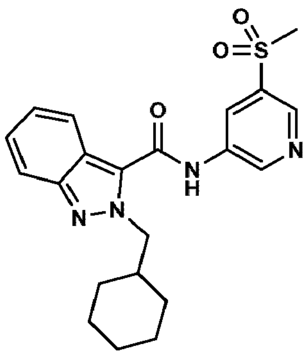 | **SC-120** | 2-(cyclohexylmethyl)-*N*-(5-methylsulfonylpyridin-3-yl) indazole-3- carboxamide |

(continued)

| Structure | Prophetic example number | Chemical name |
|---|---|---|
| 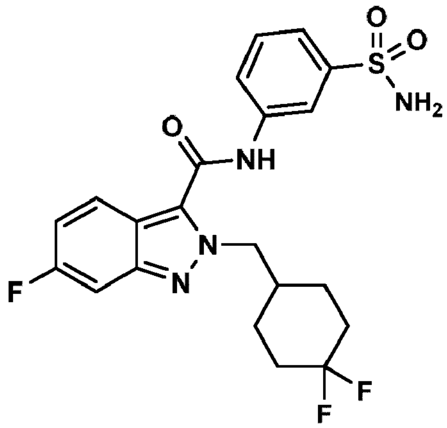 | **SC-121** | 2-(cyclohexylmethyl)-N-(5-methylsulfonylpyridin-3-yl) indazole-3-carboxamide |
| 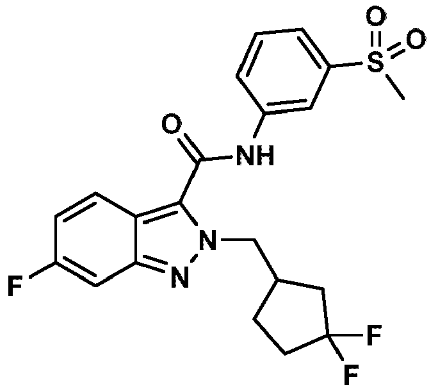 | **SC-122** | 2-[(4,4-difluorocyclohexyl)methyl]-6-fluoro-N-(3-sulfamoylphenyl)indazole-3-carboxamide |
| 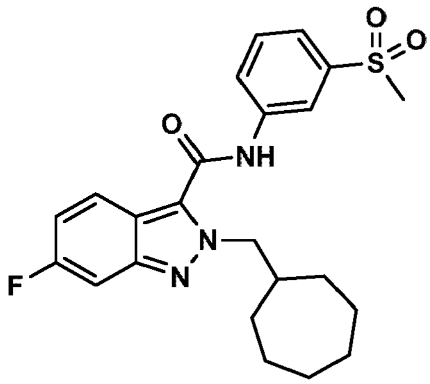 | **SC-123** | 2-(cycloheptylmethyl)-6-fluoro-N-(3-methylsulfonylphenyl)indazole-3 - carboxamide |

(continued)

| Structure | Prophetic example number | Chemical name |
|---|---|---|
| 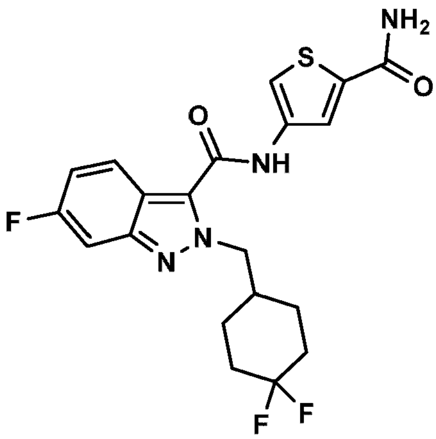 | **SC-124** | N-(5-carbamoylthiophen-3-yl)-2-((4,4-difluorocyclohexyl)methyl)-6-fluoro-2H-indazole-3 -carboxamide |
| 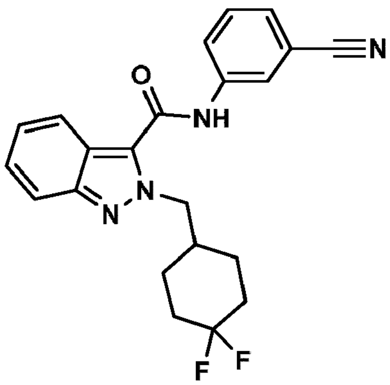 | **SC-125** | N-(3-cyanophenyl)-2-((4,4-difluorocyclohexyl) methyl)-2H-indazole-3 -carboxamide |
| 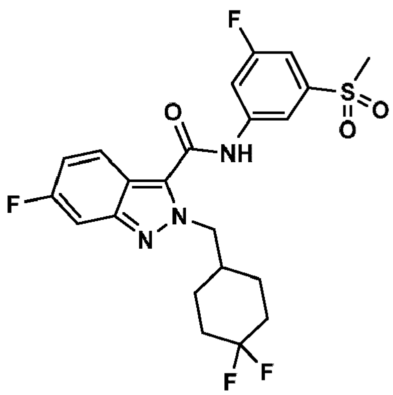 | **SC-126** | 2-((4,4-difluorocyclohexyl)methyl)-6-fluoro-N-(3-fluoro-5-(methylsulfonyl)phenyl)-2H-indazole-3-carboxamide |
| 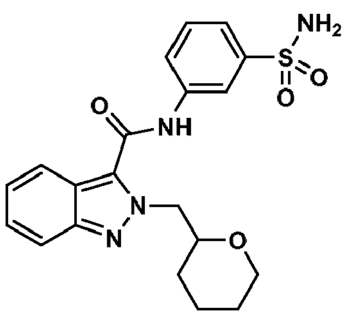 | **SC-127** | N-(3-sulfamoylphenyl)-2-((tetrahydro-2H-pyran-2-yl) methyl)-2H-indazole-3-carboxamide |

(continued)

| Structure | Prophetic example number | Chemical name |
|---|---|---|
| 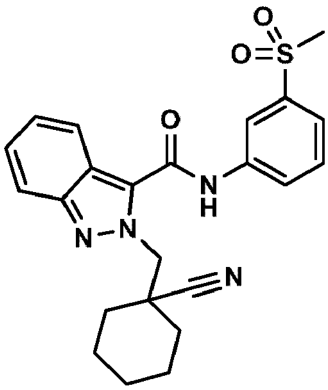 | **SC-128** | 2-((1-cyanocyclohexyl)methyl)-N-(3-(methylsulfonyl) phenyl)-2H-indazole-3-carboxamide |
| 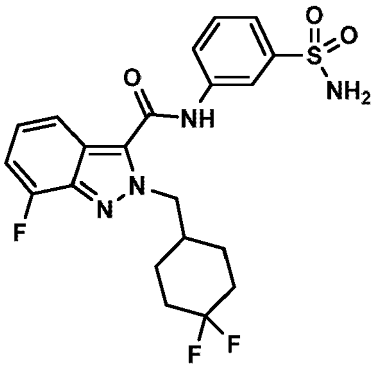 | **SC-129** | 2-((4,4-difluorocyclohexyl)methyl)-7-fluoro-N-(3-sulfamoylphenyl)-2H-indazole-3-carboxamide |
| 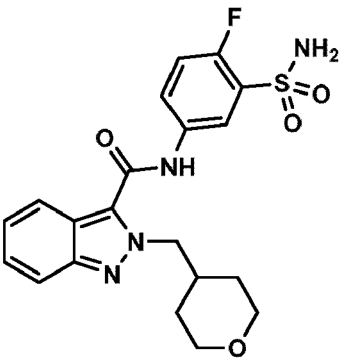 | **SC-130** | N-(4-fluoro-3-sulfamoylphenyl)-2-((tetrahydro-2H-pyran-4-yl)methyl)-2H-indazole-3 -carboxamide |
| 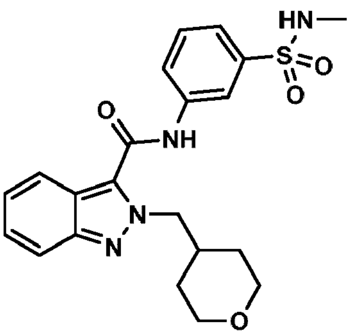 | **SC-131** | N-[3-(methylsulfamoyl)phenyl]-2-(oxan-4-ylmethyl) indazole-3 -carboxamide |

(continued)

| Structure | Prophetic example number | Chemical name |
|---|---|---|
| 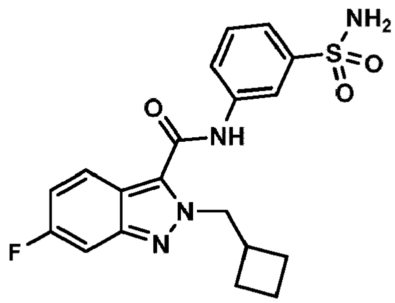 | **SC-132** | 2-(cyclobutylmethyl)-6-fluoro-N-(3-sulfamoylphenyl) indazole-3-carboxamide |
| 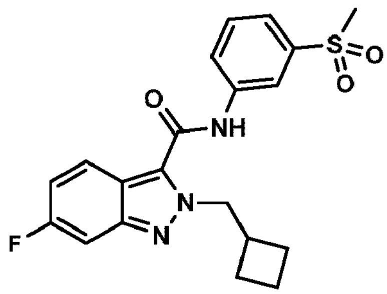 | **SC-133** | 2-(cyclobutylmethyl)-6-fluoro-N-(3-methylsulfonylphenyl)indazole-3-carboxamide |
| 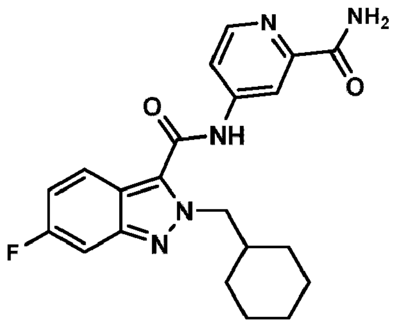 | **SC-134** | N-(2-carbamoylpyridin-4-yl)-2-(cyclohexylmethyl)-6-fluoroindazole-3 - carboxamide |
| 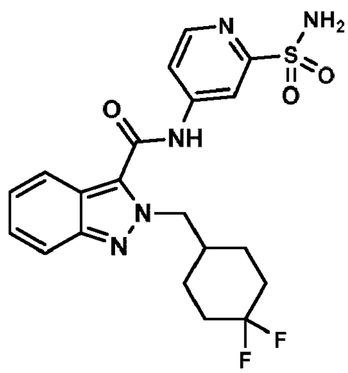 | **SC-135** | 2-[(4,4-difluorocyclohexyl)methyl]-N-(2-sulfamoylpyridin-4-yl)indazole-3-carboxamide |

(continued)

| Structure | Prophetic example number | Chemical name |
|---|---|---|
| 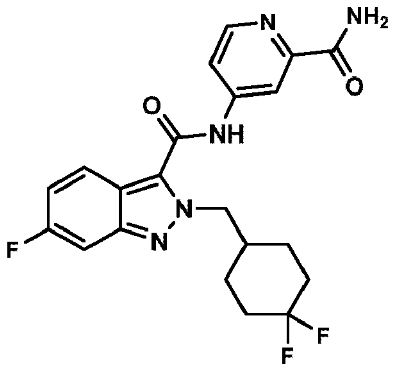 | **SC-136** | N-(2-carbamoylpyridin-4-yl)-2-[(4,4-difluorocyclohexyl)methyl]-6-fluoroindazole-3-carboxamide |
| 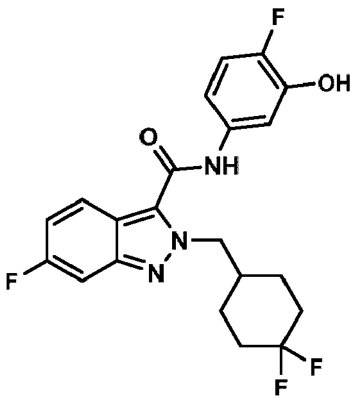 | **SC-137** | 2-[(4,4-difluorocyclohexyl)methyl]-6-fluoro-N-(4-fluoro-3-hydroxyphenyl)indazole-3-carboxamide |
| 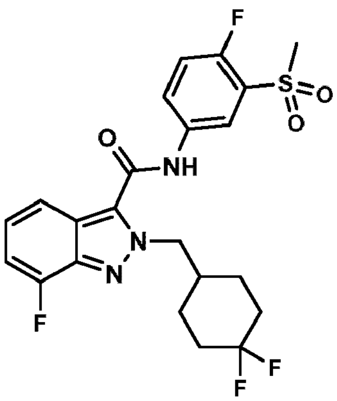 | **SC-138** | 2-[(4,4-difluorocyclohexyl)methyl]-7-fluoro-N-(4-fluoro-3-methylsulfonylphenyl)indazole-3-carboxamide |
| 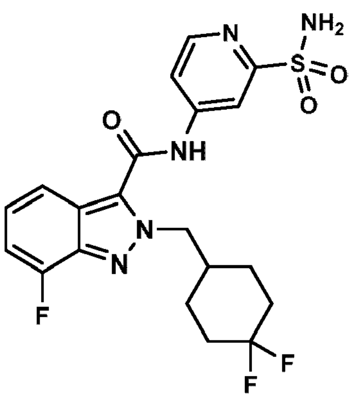 | **SC-139** | 2-[(4,4-difluorocyclohexyl)methyl]-7-fluoro-N-(2-sulfamoylpyridin-4-yl)indazole-3-carboxamide |

(continued)

| Structure | Prophetic example number | Chemical name |
|---|---|---|
| 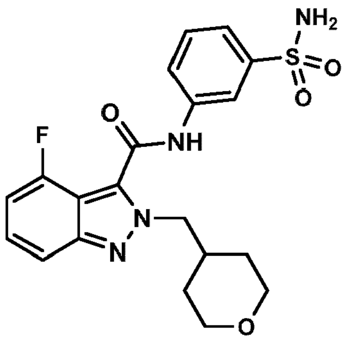 | **SC-140** | 4-fluoro-2-(oxan-4-ylmethyl)-N-(3 - sulfamoylphenyl) indazole-3-carboxamide |
| 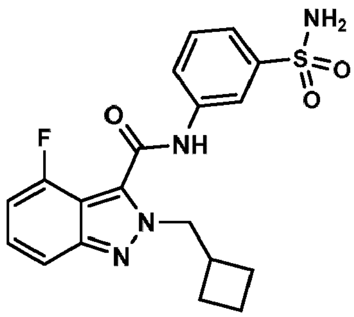 | **SC-141** | 2-(cyclobutylmethyl)-4-fluoro-N-(3-sulfamoylphenyl) indazole-3-carboxamide |
| 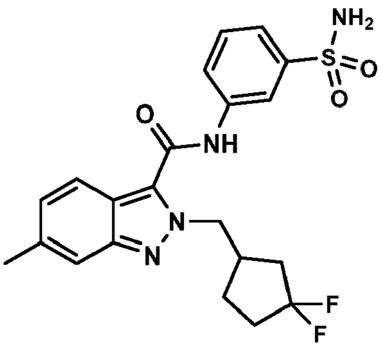 | **SC-142** | 2-[(3,3-difluorocyclopentyl)methyl]-6-methyl-N-(3-sulfamoylphenyl)indazole-3-carboxamide |
| 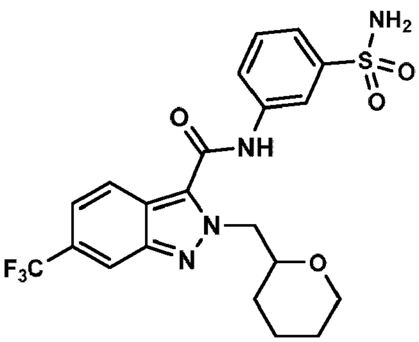 | **SC-143** | 2-(oxan-2-ylmethyl)-N-(3-sulfamoylphenyl)-6-(trifluoromethyl)indazole-3-carboxamide |

(continued)

| Structure | Prophetic example number | Chemical name |
|---|---|---|
| 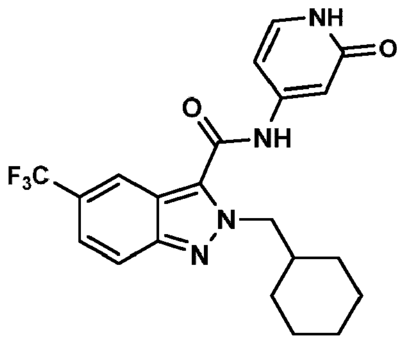 | **SC-144** | 2-(cyclohexylmethyl)-N-(2-hydroxypyridin-4-yl)-5-(trifluoromethyl)indazole-3-carboxamide |
| 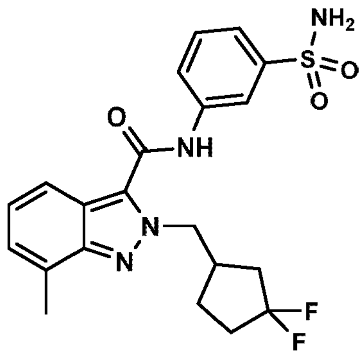 | **SC-145** | 2-[(3,3-difluorocyclopentyl)methyl]-7-methyl-N-(3-sulfamoylphenyl)indazole-3-carboxamide |
| 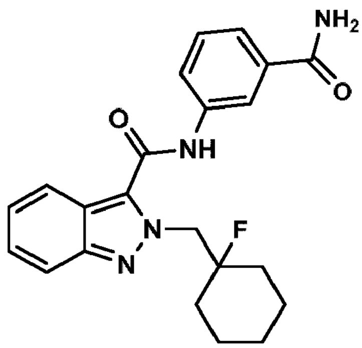 | **SC-146** | N-(3-carbamoylphenyl)-2-[(1-fluorocyclohexyl)methyl]indazole-3-carboxamide |
| 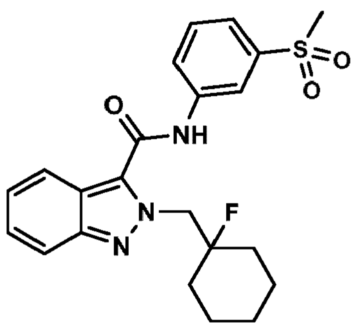 | **SC-147** | 2-[(1-fluorocyclohexyl)methyl]-N-(3-methylsulfonylphenyl)indazole-3-carboxamide |

(continued)

| Structure | Prophetic example number | Chemical name |
|---|---|---|
| 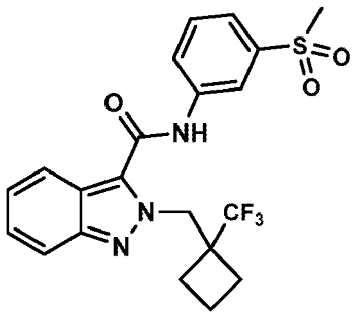 | **SC-148** | N-(3-methylsulfonylphenyl)-2-[[1-(trifluoromethyl) cyclobutyl]methyl]indaz ole-3-carboxamide |
| 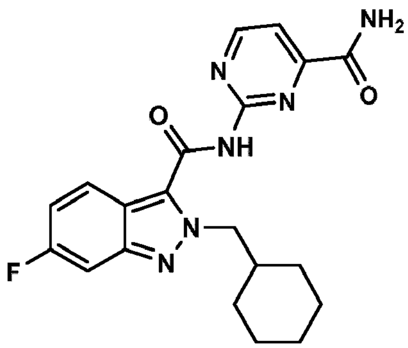 | **SC-149** | N-(4-carbamoylpyrimidin-2-yl)-2-(cyclohexylmethyl)-6-fluoroindazole-3 - carboxamide |
| 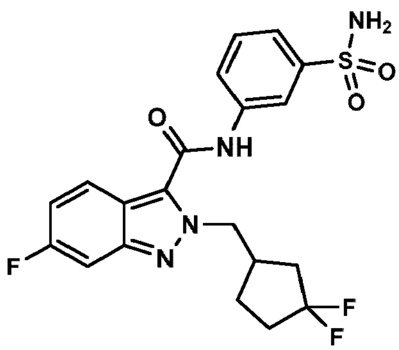 | **SC-150** | 2-[(3,3-difluorocyclopentyl)methyl]-6-fluoro-N-(3-sulfamoylphenyl)indazole-3-carboxamide |
| 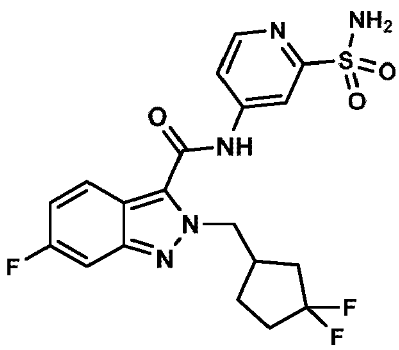 | **SC-151** | 2-[(3,3-difluorocyclopentyl)methyl]-6-fluoro-N-(2-sulfamoylpyridin-4-yl)indazole-3-carboxamide |

(continued)

| Structure | Prophetic example number | Chemical name |
|---|---|---|
| 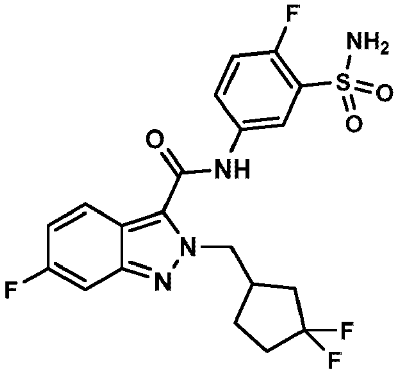 | **SC-152** | 2-[(3,3-difluorocyclopentyl)methyl]-6-fluoro-N-(4-fluoro-3-sulfamoylphenyl)indazole-3-carboxamide |
| 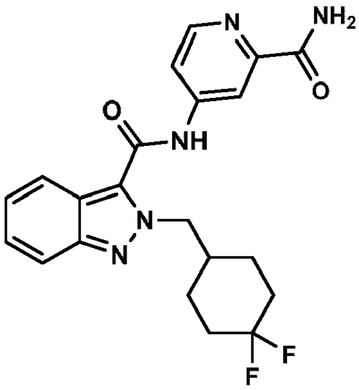 | **SC-153** | N-(2-carbamoylpyridin-4-yl)-2-[(4,4-difluorocyclohexyl)methyl]indazole-3-carboxamide |
| 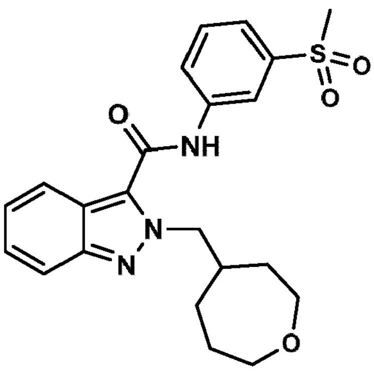 | **SC-154** | N-(3-methylsulfonylphenyl)-2-(oxepan-4-ylmethyl) indazole-3-carboxamide |
| 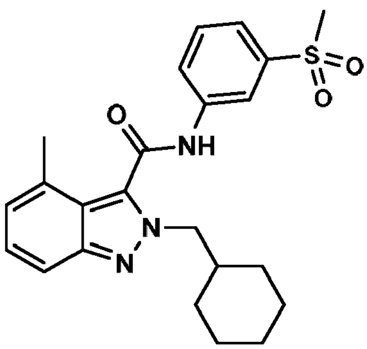 | **SC-155** | 2-(cyclohexylmethyl)-4-methyl-N-(3-methylsulfonylphenyl)indazole-3-carboxamide |

(continued)

| Structure | Prophetic example number | Chemical name |
|---|---|---|
| 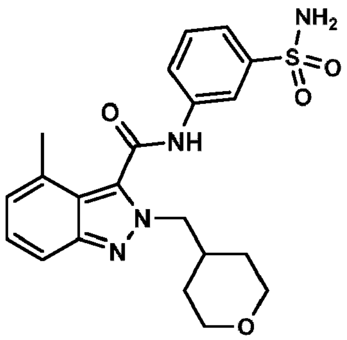 | **SC-156** | 4-methyl-2-(oxan-4-ylmethyl)-N-(3-sulfamoylphenyl) indazole-3-carboxamide |
| 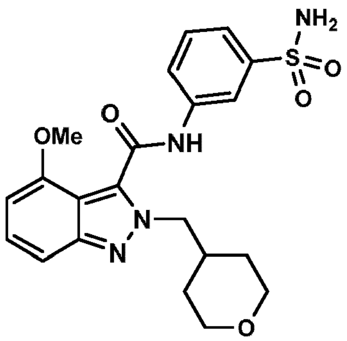 | **SC-157** | 4-methoxy-2-(oxan-4-ylmethyl)-N-(3-sulfamoylphenyl)indazole-3-carboxamide |
| 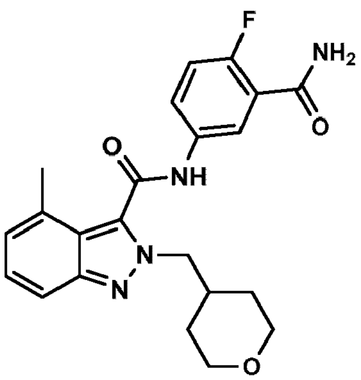 | **SC-158** | N-(3-carbamoyl-4-fluorophenyl)-4-methyl-2-(oxan-4-ylmethyl)indazole-3-carboxamide |
| 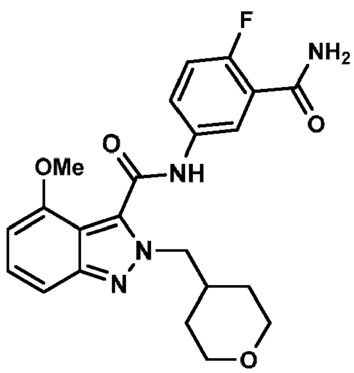 | **SC-159** | N-(3-carbamoyl-4-fluorophenyl)-4-methoxy-2-(oxan-4-ylmethyl)indazole-3-carboxamide |

(continued)

| Structure | Prophetic example number | Chemical name |
|---|---|---|
| 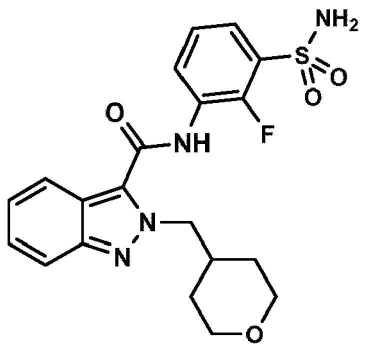 | **SC-160** | N-(2-fluoro-3-sulfamoylphenyl)-2-(oxan-4-ylmethyl) indazole-3 - carboxamide |
| 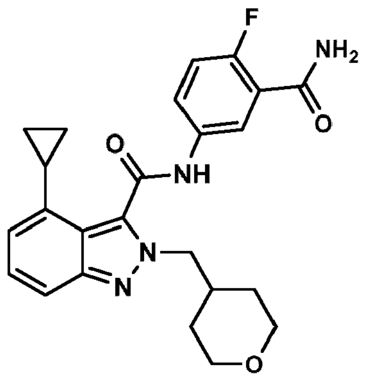 | **SC-161** | N-(3-carbamoyl-4-fluorophenyl)-4-cyclopropyl-2-(oxan-4-ylmethyl)indazole-3-carboxamide |
| 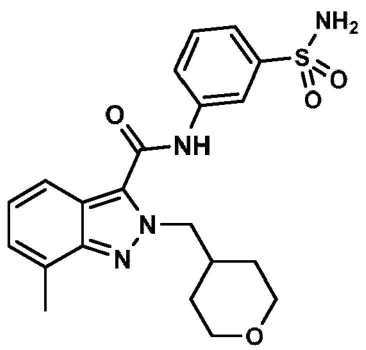 | **SC-162** | 7-methyl-2-(oxan-4-ylmethyl)-N-(3-sulfamoylphenyl) indazole-3-carboxamide |
| 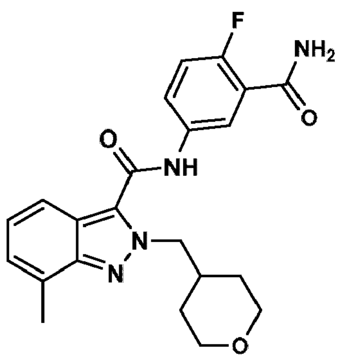 | **SC-163** | N-(3-carbamoyl-4-fluorophenyl)-7-methyl-2-(oxan-4-ylmethyl)indazole-3-carboxamide |

(continued)

| Structure | Prophetic example number | Chemical name |
|---|---|---|
| 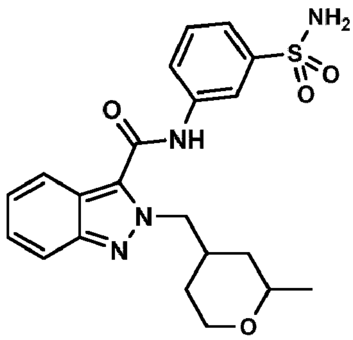 | **SC-164** | 2-[(2-methyloxan-4-yl)methyl]-N-(3-sulfamoylphenyl) indazole-3-carboxamide |
| 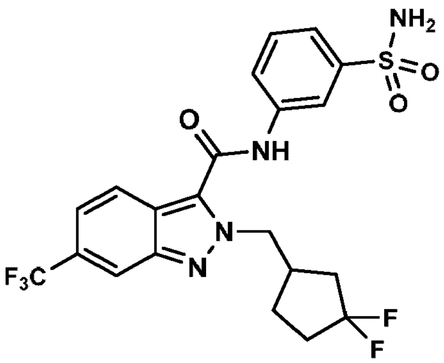 | **SC-165** | 2-[(3,3-difluorocyclopentyl)methyl]-N-(3-sulfamoylphenyl)-6-(trifluoromethyl)indazole-3-carboxamide |
| 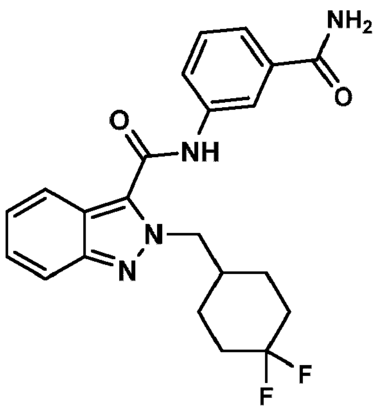 | **SC-166** | N-(3-carbamoylphenyl)-2-[(4,4-difluorocyclohexyl) methyl]-7-fluoroindazole-3 -carboxamide |
| 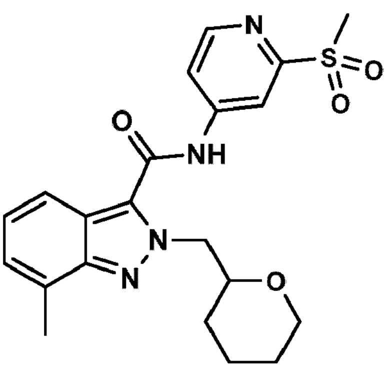 | **SC-167** | 7-methyl-N-(2-methylsulfonylpyridin-4-yl)-2-(oxan-2-ylmethyl)indazole-3-carboxamide |

(continued)

| Structure | Prophetic example number | Chemical name |
|---|---|---|
| 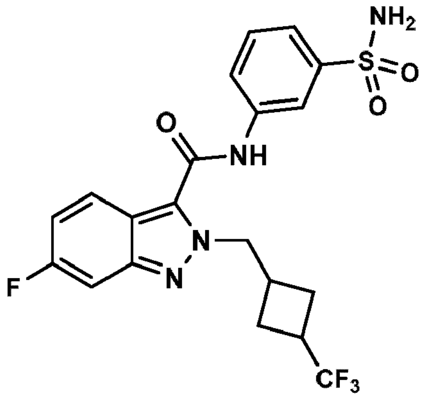 | **SC-168** | 6-fluoro-N-(3-sulfamoylphenyl)-2-[[3-(trifluoromethyl) cyclobutyl]methyl]indaz ole-3-carboxamide |
| 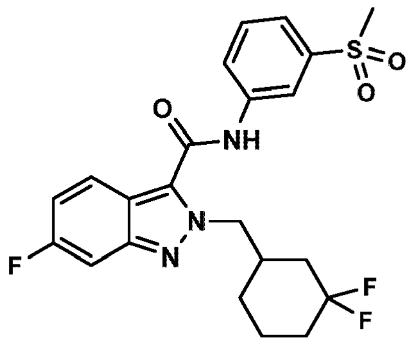 | **SC-169** | 2-[(3,3-difluorocyclohexyl)methyl]-6-fluoro-N-(3-methylsulfonylphenyl)indazole-3-carboxamide |
| 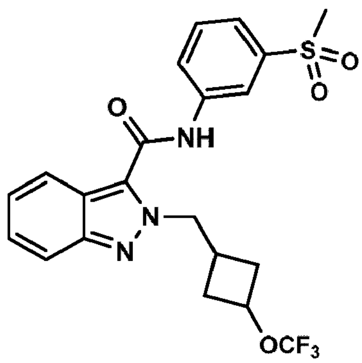 | **SC-170** | N-(3-methylsulfonylphenyl)-2-[[3-(trifluoromethoxy) cyclobutyl]methyl]ind azole-3-carboxamide |
| 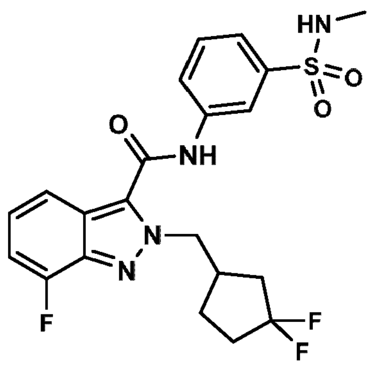 | **SC-171** | 2-[(3,3-difluorocyclopentyl)methyl]-7-fluoro-N-[3-(methylsulfamoyl)phenyl]indazole-3-carboxamide |

(continued)

| Structure | Prophetic example number | Chemical name |
|---|---|---|
| 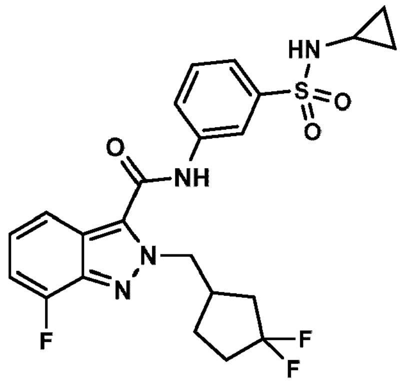 | **SC-172** | N-[3-(cyclopropylsulfamoyl)phenyl]-2-[(3,3-difluorocyclopentyl)methyl]-7-fluoroindazole-3-carboxamide |
| 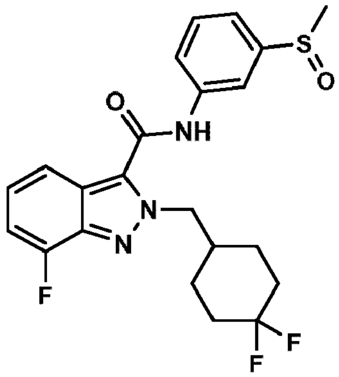 | **SC-173** | 2-[(4,4-difluorocyclohexyl)methyl]-7-fluoro-N-(3-methylsulfinylphenyl)indazole-3-carboxamide |
| 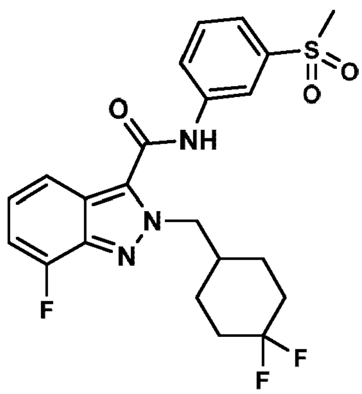 | **SC-174** | 2-[(4,4-difluorocyclohexyl)methyl]-7-fluoro-N-(3-methylsulfonylphenyl)indazole-3-carboxamide |
| 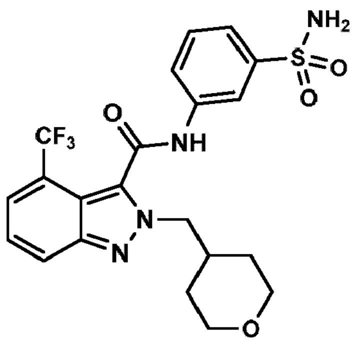 | **SC-175** | 2-(oxan-4-ylmethyl)-N-(3-sulfamoylphenyl)-4-(trifluoromethyl)indazole-3-carboxamide |

(continued)

| Structure | Prophetic example number | Chemical name |
|---|---|---|
| 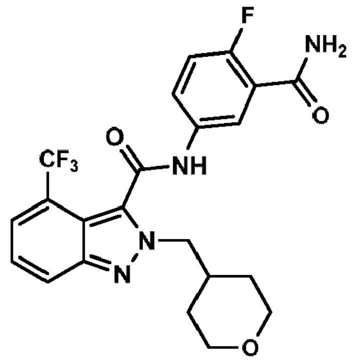 | **SC-176** | N-(3-carbamoyl-4-fluorophenyl)-2-(oxan-4-ylmethyl)-4-(trifluoromethyl)indazole-3-carboxamide |
| 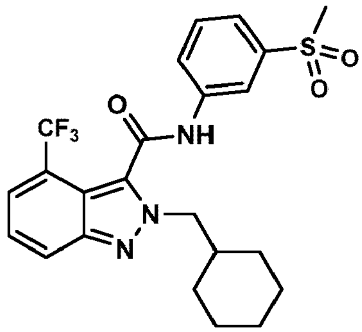 | **SC-177** | 2-(cyclohexylmethyl)-N-(3-methylsulfonylphenyl)-4-(trifluoromethyl)indazole-3 -carboxamide |
| 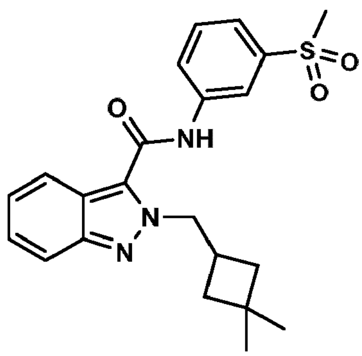 | **SC-178** | 2-[(3,3-dimethylcyclobutyl)methyl]-N-(3-methylsulfonylphenyl)indazole-3-carboxamide |
| 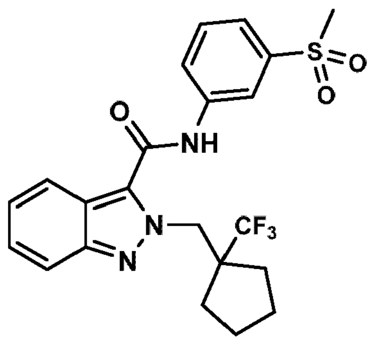 | **SC-179** | N-(3-methylsulfonylphenyl)-2-[[1-(trifluoromethyl) cyclopentyl]methyl]inda zole-3-carboxamide |

(continued)

| Structure | Prophetic example number | Chemical name |
|---|---|---|
| 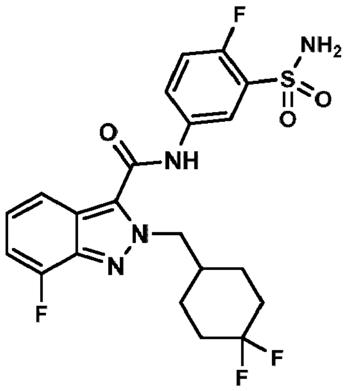 | **SC-180** | 2-[(4,4-difluorocyclohexyl)methyl]-7-fluoro-N-(4-fluoro-3-sulfamoylphenyl)indazole-3-carboxamide |
| 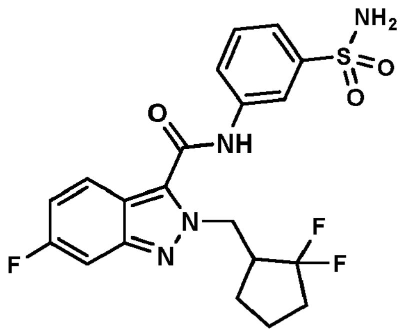 | **SC-181** | 2-[(2,2-difluorocyclopentyl)methyl]-6-fluoro-N-(3-sulfamoylphenyl)indazole-3-carboxamide |
| 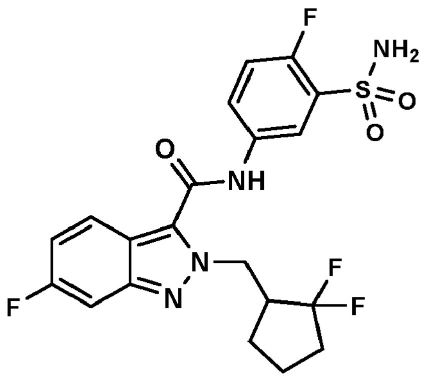 | **SC-182** | 2-[(2,2-difluorocyclopentyl)methyl]-6-fluoro-N-(4-fluoro-3-sulfamoylphenyl)indazole-3-carboxamide |
| 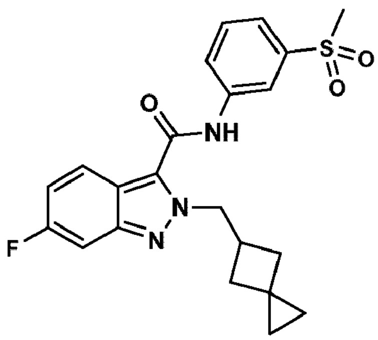 | **SC-183** | 6-fluoro-N-(3-methylsulfonylphenyl)-2-(spiro[2.3]hexan-5-ylmethyl)indazole-3-carboxamide |

(continued)

| Structure | Prophetic example number | Chemical name |
|---|---|---|
| 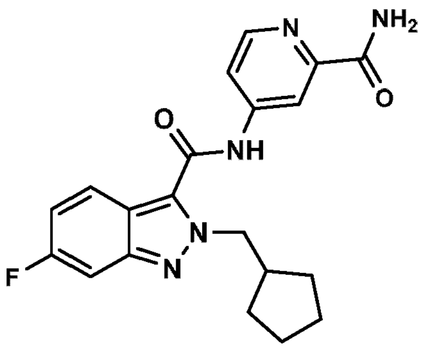 | **SC-184** | N-(2-carbamoylpyridin-4-yl)-2-(cyclopentylmethyl)-6-fluoroindazole-3 - carboxamide |
| 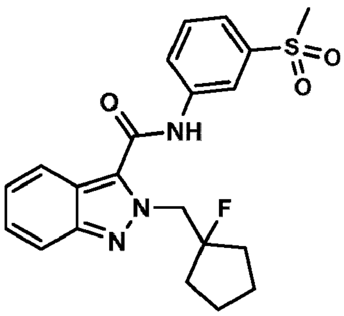 | **SC-185** | 2-[(1-fluorocyclopentyl)methyl]-N-(3-methylsulfonylphenyl)indazole-3-carboxamide |
| 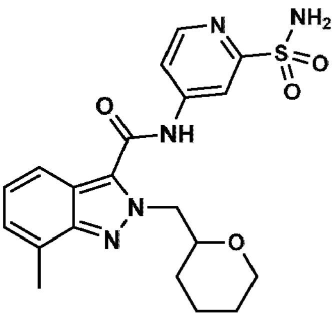 | **SC-186** | 7-methyl-2-(oxan-2-ylmethyl)-N-(2-sulfamoylpyridin-4-yl)indazole-3-carboxamide |
| 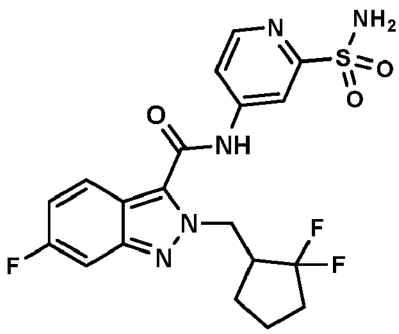 | **SC-187** | 2-[(2,2-difluorocyclopentyl)methyl]-6-fluoro-N-(2-sulfamoylpyridin-4-yl)indazole-3-carboxamide |

(continued)

| Structure | Prophetic example number | Chemical name |
|---|---|---|
| 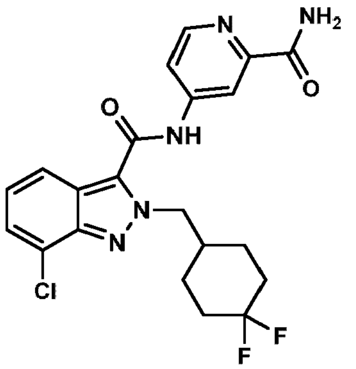 | **SC-188** | 2-[(2,2-difluorocyclopentyl)methyl]-6-fluoro-N-(2-sulfamoylpyridin-4-yl)indazole-3-carboxamide |
| 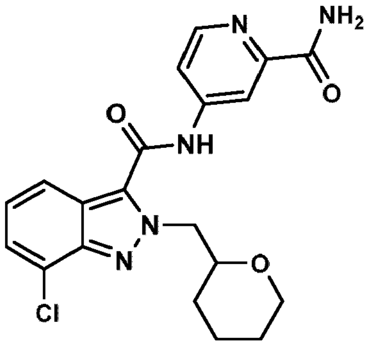 | **SC-189** | N-(2-carbamoylpyridin-4-yl)-7-fluoro-2-(oxan-2-ylmethyl)indazole-3 - carboxamide |
| 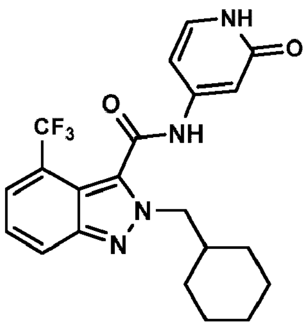 | **SC-190** | 2-(cyclohexylmethyl)-N-(2-hydroxypyridin-4-yl)-4-(trifluoromethyl)indazole-3 -carboxamide |
| 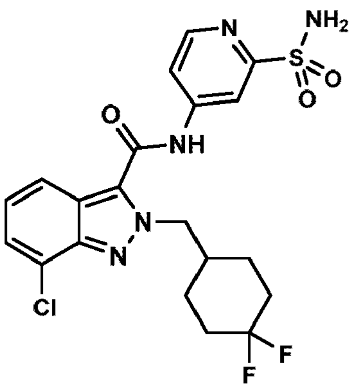 | **SC-191** | 7-chloro-2-[(4,4-difluorocyclohexyl)methyl]-N-(2-sulfamoylpyridin-4-yl)indazole-3-carboxamide |

(continued)

| Structure | Prophetic example number | Chemical name |
|---|---|---|
| 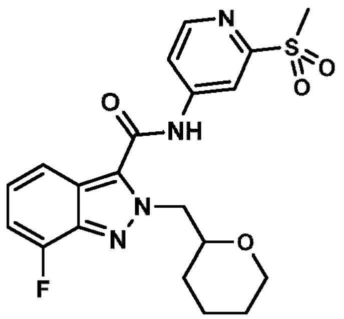 | **SC-192** | 7-fluoro-N-(2-methylsulfonylpyridin-4-yl)-2-(oxan-2-ylmethyl)indazole-3-carboxamide |
| 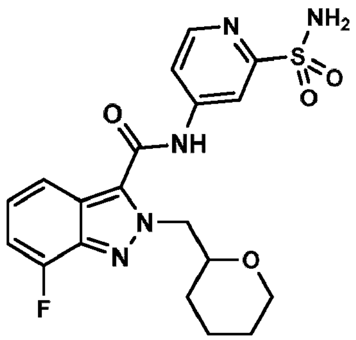 | **SC-193** | 7-fluoro-2-(oxan-2-ylmethyl)-N-(2-sulfamoylpyridin-4-yl)indazole-3-carboxamide |
| 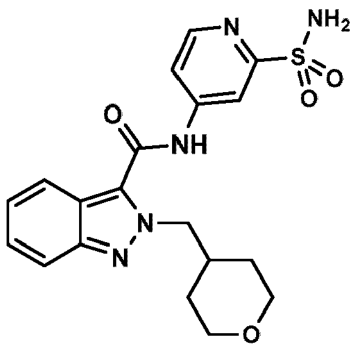 | **SC-194** | 2-(oxan-4-ylmethyl)-N-(2-sulfamoylpyridin-4-yl) indazole-3-carboxamide |
| 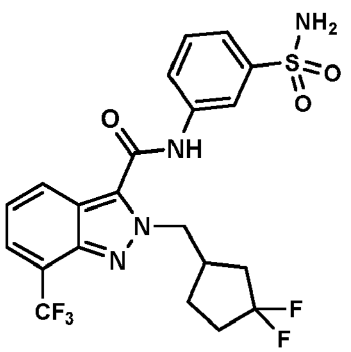 | **SC-195** | 2-[(3,3-difluorocyclopentyl)methyl]-N-(3-sulfamoylphenyl)-7-(trifluoromethyl)indazole-3-carboxamide |

(continued)

| Structure | Prophetic example number | Chemical name |
|---|---|---|
| 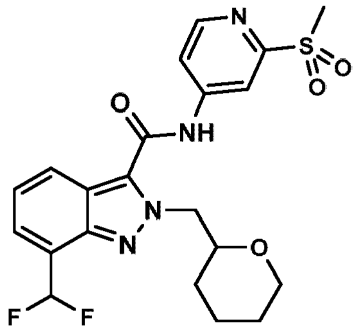 | **SC-196** | 7-(difluoromethyl)-N-(2-methylsulfonylpyridin-4-yl)-2-(oxan-2-ylmethyl)indazole-3-carboxamide |
| 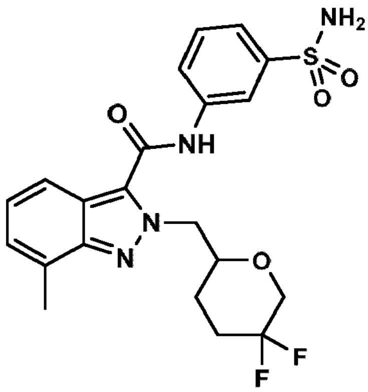 | **SC-197** | 2-[(5,5-difluorooxan-2-yl)methyl]-7-methyl-N-(3-sulfamoylphenyl)indazole-3-carboxamide |
| 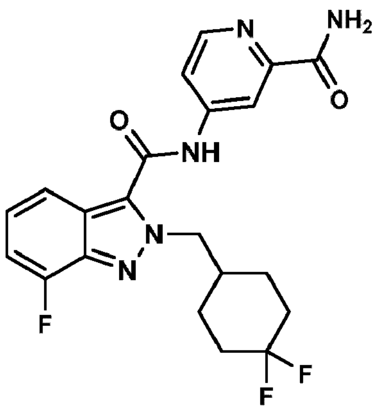 | **SC-198** | N-(2-carbamoylpyridin-4-yl)-2-[(4,4-difluorocyclohexyl)methyl]-7-fluoroindazole-3-carboxamide |
| 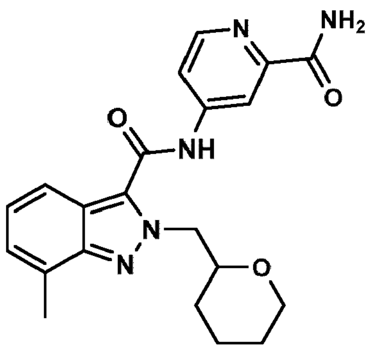 | **SC-199** | N-(2-carbamoylpyridin-4-yl)-7-methyl-2-(oxan-2-ylmethyl)indazole-3-carboxamide |

(continued)

| Structure | Prophetic example number | Chemical name |
|---|---|---|
| 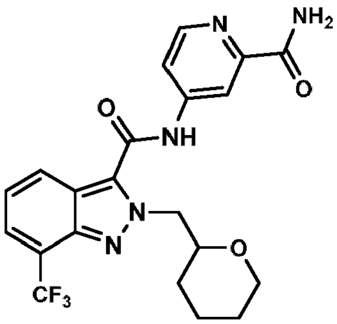 | **SC-200** | N-(2-carbamoylpyridin-4-yl)-2-(oxan-2-ylmethyl)-7-(trifluoromethyl)indazole-3- carboxamide |
| 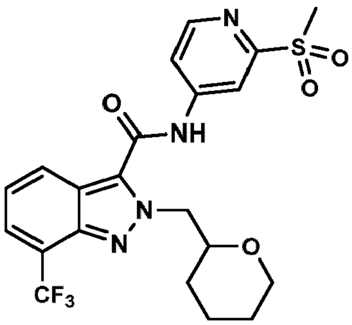 | **SC-201** | N-(2-methylsulfonylpyridin-4-yl)-2-(oxan-2-ylmethyl)-7-(trifluoromethyl)indazole-3-carboxamide |
| 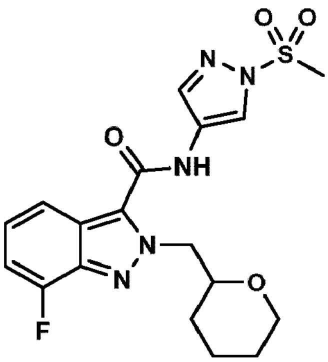 | **SC-202** | 7-fluoro-N-(1-methylsulfonylpyrazol-4-yl)-2-(oxan-2-ylmethyl)indazole-3-carboxamide |
| 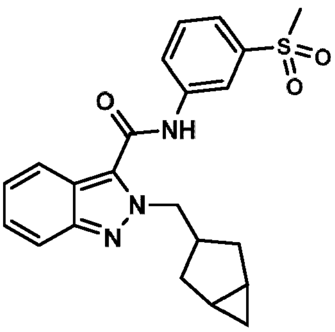 | **SC-203** | 2-(3-bicyclo[3.1.0]hexanylmethyl)-N-(3-methylsulfonylphenyl)indazole-3-carboxamide |

(continued)

| Structure | Prophetic example number | Chemical name |
|---|---|---|
| 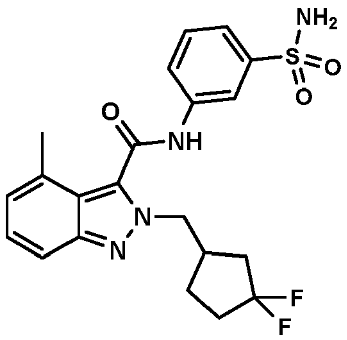 | **SC-204** | 2-[(3,3-difluorocyclopentyl)methyl]-4-methyl-N-(3-sulfamoylphenyl)indazole-3-carboxamide |
| 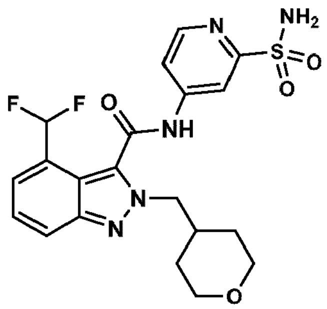 | **SC-205** | 4-(difluoromethyl)-2-(oxan-4-ylmethyl)-N-(2-sulfamoylpyridin-4-yl)indazole-3 - carboxamide |
| 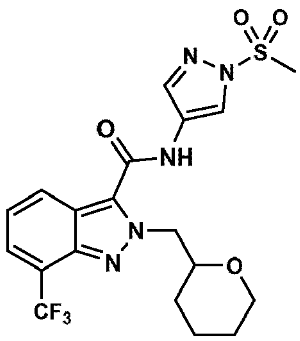 | **SC-206** | N-(1-methylsulfonylpyrazol-4-yl)-2-(oxan-2-ylmethyl)-7-(trifluoromethyl)indazole-3-carboxamide |
| 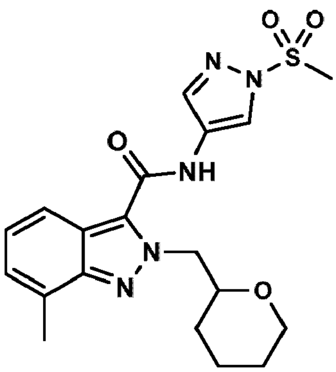 | **SC-207** | 7-methyl-N-(1-methylsulfonylpyrazol-4-yl)-2-(oxan-2-ylmethyl)indazole-3-carboxamide |

(continued)

| Structure | Prophetic example number | Chemical name |
|---|---|---|
| 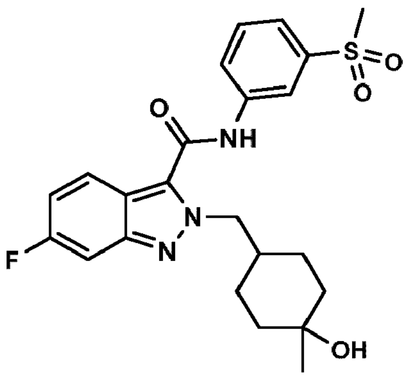 | **SC-208** | 6-fluoro-2-[(4-hydroxy-4-methylcyclohexyl) methyl]-N-(3-methylsulfonylphenyl)indazole-3-carboxamide |
| 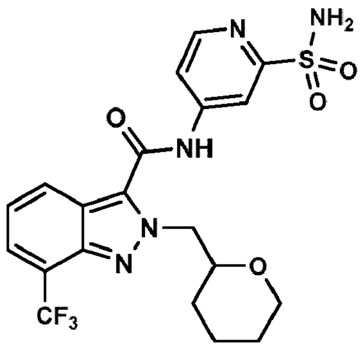 | **SC-209** | 2-(oxan-2-ylmethyl)-N-(2-sulfamoylpyridin-4-yl)-7-(trifluoromethyl)indazole-3 -carboxamide |
| 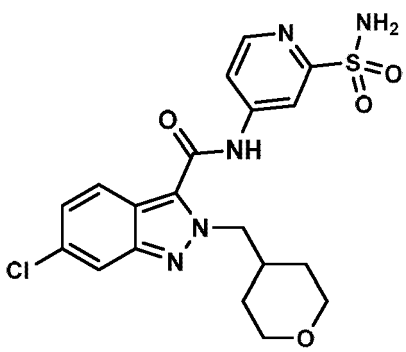 | **SC-210** | 6-chloro-2-(oxan-4-ylmethyl)-N-(2-sulfamoylpyridin-4-yl)indazole-3-carboxamide |
| 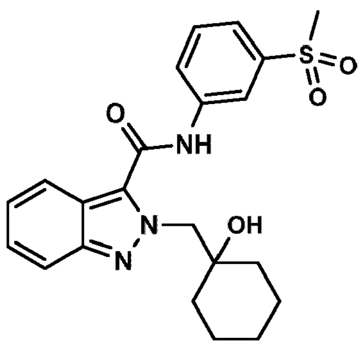 | **SC-211** | 2-[(l-hydroxycyclohexyl)methyl]-N-(3-methylsulfonylphenyl)indazole-3-carboxamide |

(continued)

| Structure | Prophetic example number | Chemical name |
|---|---|---|
| 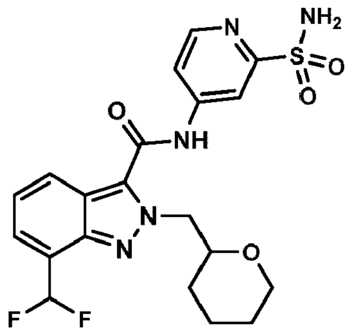 | **SC-212** | 7-(difluoromethyl)-2-(oxan-2-ylmethyl)-N-(2-sulfamoylpyridin-4-yl)indazole-3-carboxamide |
| 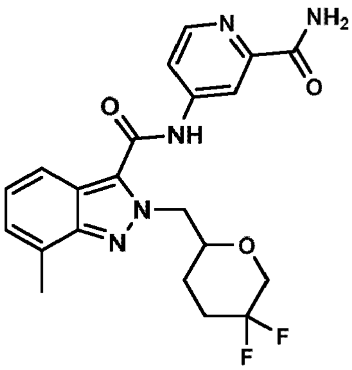 | **SC-213** | N-(2-carbamoylpyridin-4-yl)-2-[[(2S)-5,5-difluorooxan-2-yl]methyl]-7-methylindazole-3-carboxamide |
| 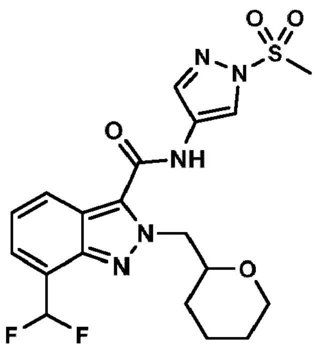 | **SC-214** | 7-(difluoromethyl)-N-(1-methylsulfonylpyrazol-4-yl)-2-(oxan-2-ylmethyl)indazole-3-carboxamide |
| 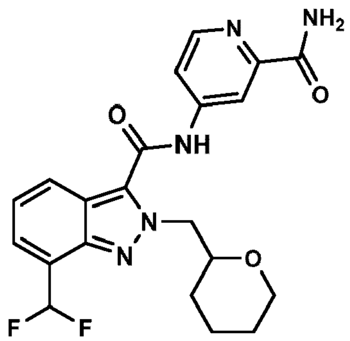 | **SC-215** | N-(2-carbamoylpyridin-4-yl)-7-(difluoromethyl)-2-(oxan-2-ylmethyl)indazole-3-carboxamide |

(continued)

| Structure | Prophetic example number | Chemical name |
|---|---|---|
| 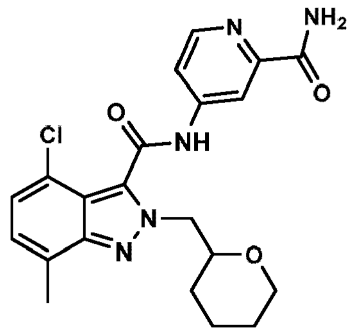 | **SC-216** | N-(2-carbamoylpyridin-4-yl)-2-[(5,5-difluorooxan-2-yl)methyl]-7-methylindazole-3-carboxamide |
| 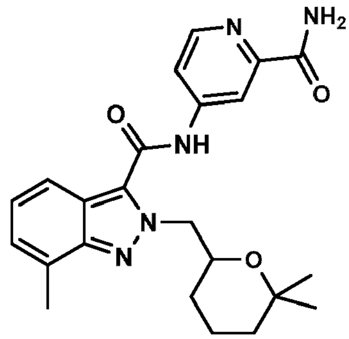 | **SC-217** | N-(2-carbamoylpyridin-4-yl)-2-[(6,6-dimethyloxan-2-yl)methyl]-7-methylindazole-3-carboxamide |
| 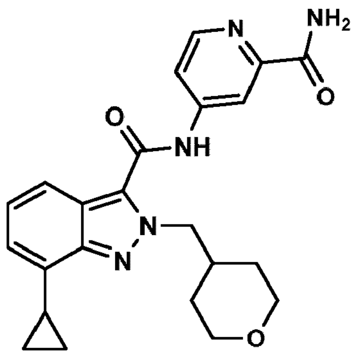 | **SC-218** | N-(2-carbamoylpyridin-4-yl)-7-cyclopropyl-2-(oxan-4-ylmethyl)indazole-3-carboxamide |
| 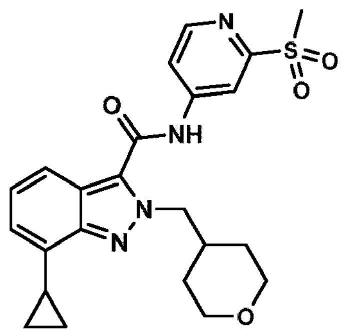 | **SC-219** | 7-cyclopropyl-N-(2-methylsulfonylpyridin-4-yl)-2-(oxan-4-ylmethyl)indazole-3-carboxamide |

(continued)

| Structure | Prophetic example number | Chemical name |
|---|---|---|
| 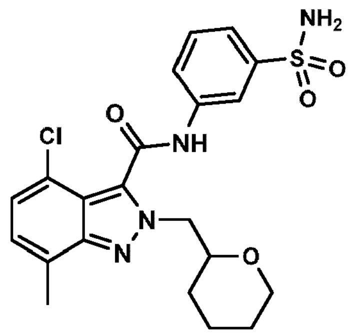 | **SC-220** | 4-chloro-7-methyl-2-(oxan-2-ylmethyl)-N-(3-sulfamoylphenyl)indazole-3-carboxamide |
| 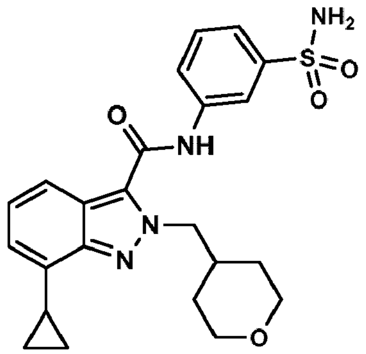 | **SC-221** | 7-cyclopropyl-2-(oxan-4-ylmethyl)-N-(3-sulfamoylphenyl)indazole-3- carboxamide |
| 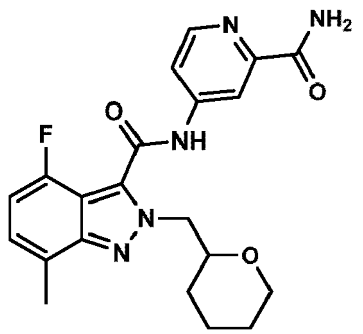 | **SC-222** | N-(2-carbamoylpyridin-4-yl)-4-fluoro-7-methyl-2-(oxan-2-ylmethyl)indazole-3- carboxamide |
| 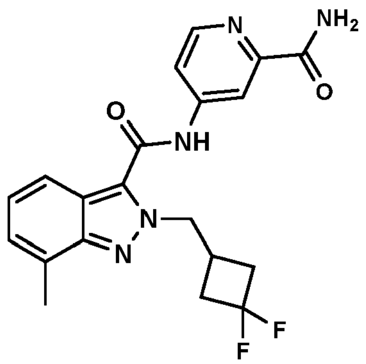 | **SC-223** | N-(2-carbamoylpyridin-4-yl)-2-[(3,3-difluorocyclobutyl)methyl]-7-methylindazole-3-carboxamide |

(continued)

| Structure | Prophetic example number | Chemical name |
|---|---|---|
| 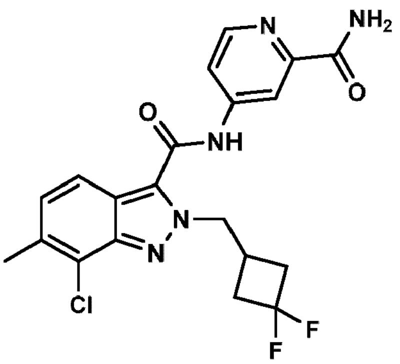 | **SC-224** | N-(2-carbamoylpyridin-4-yl)-7-chloro-2-[(3,3-difluorocyclobutyl)methyl]-6-methylindazole-3-carboxamide |
| 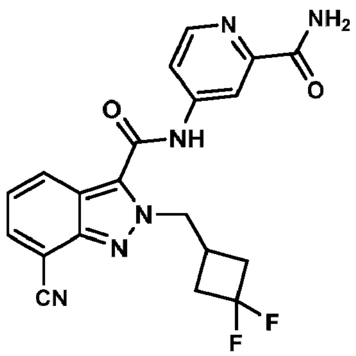 | **SC-225** | N-(2-carbamoylpyridin-4-yl)-7-cyano-2-[(3,3-difluorocyclobutyl)methyl]indazole-3-carboxamide |
| 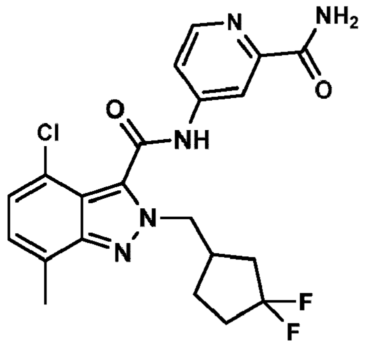 | **SC-226** | N-(2-carbamoylpyridin-4-yl)-4-chloro-2-[(3,3-difluorocyclopentyl)methyl]-7-methylindazole-3-carboxamide |
| 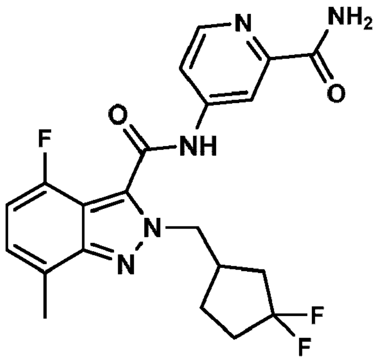 | **SC-227** | N-(2-carbamoylpyridin-4-yl)-2-[(3,3-difluorocyclopentyl)methyl]-4-fluoro-7-methylindazole-3-carboxamide |

(continued)

| Structure | Prophetic example number | Chemical name |
|---|---|---|
| 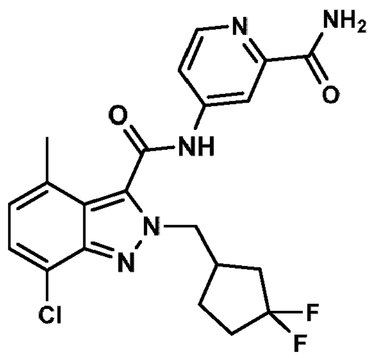 | **SC-228** | N-(2-carbamoylpyridin-4-yl)-7-chloro-2-[(3,3-difluorocyclopentyl)methyl]-4-methylindazole-3-carboxamide |
| 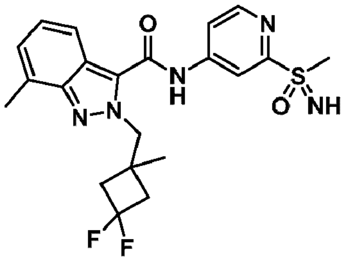 | **SC-229** | 2-((3,3-difluoro-1-methylcyclobutyl)methyl)-7-methyl-N-(2-(S-methylsulfonimidoyl)pyridin-4-yl)-2H-indazole-3-carboxamide |
| 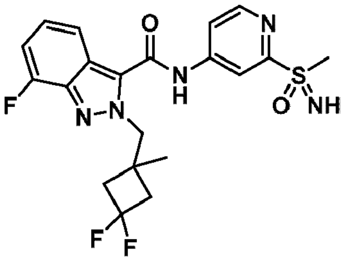 | **SC-230** | 2-((3,3-difluoro-1-methylcyclobutyl)methyl)-7-fluoro-N-(2-(S-methylsulfonimidoyl)pyridin-4-yl)-2H-indazole-3-carboxamide |
| 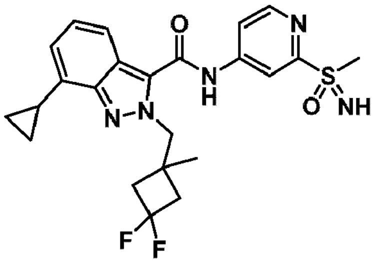 | **SC-231** | 7-cyclopropyl-2-((3,3-difluoro-1-methylcyclobutyl) methyl)-N-(2-(S-methylsulfonimidoyl)pyridin-4-yl)-2H-indazole-3 -carboxamide |
| 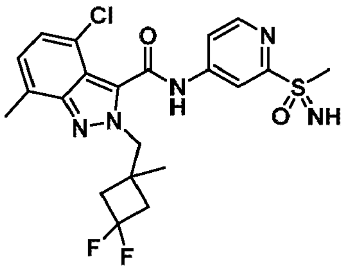 | **SC-232** | 4-chloro-2-((3,3-difluoro-1-methylcyclobutyl)methyl)-7-methyl-N-(2-(S-methylsulfonimidoyl)pyridin-4-yl)-2H-indazole-3 -carboxamide |

(continued)

| Structure | Prophetic example number | Chemical name |
|---|---|---|
| 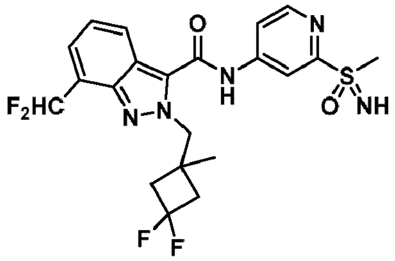 | **SC-233** | 2-((3,3-difluoro-1-methylcyclobutyl)methyl)-7-(difluoromethyl)-N-(2-(S-methylsulfonimidoyl)pyridin-4-yl)-2H-indazole-3 -carboxamide |
| 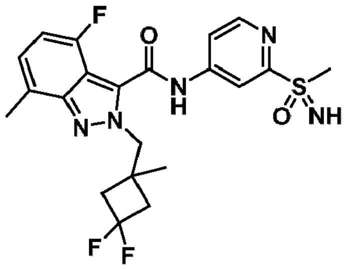 | **SC-234** | 2-((3,3-difluoro-1-methylcyclobutyl)methyl)-4-fluoro-7-methyl-N-(2-(S-methylsulfonimidoyl)pyridin-4-yl)-2H-indazole-3 -carboxamide |
| 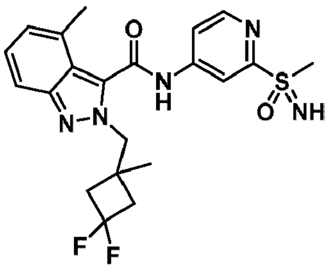 | **SC-235** | 2-((3,3-difluoro-1-methylcyclobutyl)methyl)-4-methyl-N-(2-(S-methylsulfonimidoyl)pyridin-4-yl)-2H-indazole-3 -carboxamide |
| 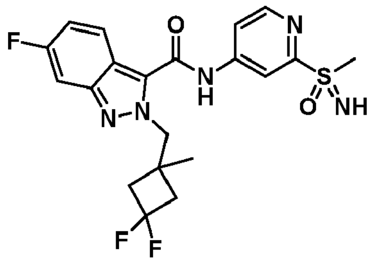 | **SC-236** | 2-((3,3-difluoro-1-methylcyclobutyl)methyl)-6-fluoro-N-(2-(S-methylsulfonimidoyl)pyridin-4-yl)-2H-indazole-3 -carboxamide |
| 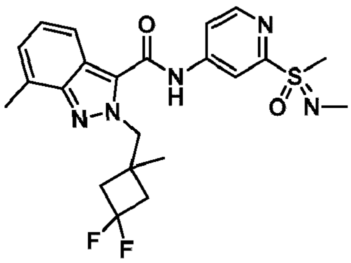 | **SC-237** | 2-((3,3-difluoro-1-methylcyclobutyl)methyl)-N-(2-(N, S-dimethylsulfonimidoyl)pyridin-4-yl)-7-methyl-2H-indazole-3-carboxamide |

(continued)

| Structure | Prophetic example number | Chemical name |
|---|---|---|
| 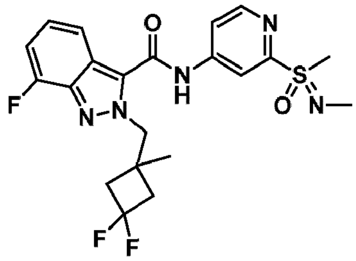 | **SC-238** | 2-((3,3-difluoro-1-methylcyclobutyl)methyl)-N-(2-(N, S-dimethylsulfommidoyl)pyridin-4-yl)-7-fluoro-2H-indazole-3-carboxamide |
| 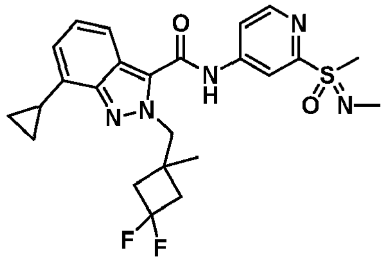 | **SC-239** | 7-cyclopropyl-2-((3,3-difluoro-1-methylcyclobutyl) methyl)-N-(2-(N,S-dimethylsulfonimidoyl)pyridin-4-yl)-2H-indazole-3 -carboxamide |
| 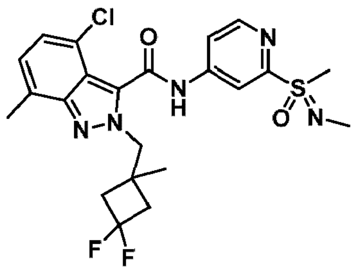 | **SC-240** | 4-chloro-2-((3,3-difluoro-1-methylcyclobutyl) methyl)-N-(2-(N,S-dimethylsulfommidoyl)pyridin-4-yl)-7-methyl-2H-indazole-3-carboxamide |
| 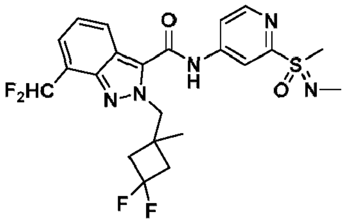 | **SC-241** | 2-((3,3-difluoro-1-methylcyclobutyl)methyl)-7-(difluoromethyl)-N-(2-(N,S-dimethylsulfonimidoyl) pyridin-4-yl)-2H-indazole-3-carboxamide |
| 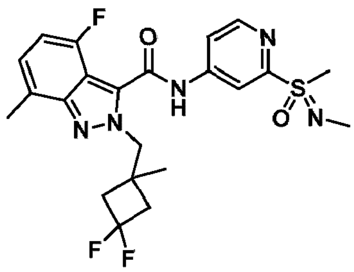 | **SC-242** | 2-((3,3-difluoro-1-methylcyclobutyl)methyl)-N-(2-(N, S-dimethylsulfonimidoyl)pyridin-4-yl)-4-fluoro-7-methyl-2H-indazole-3-carboxamide |

(continued)

| Structure | Prophetic example number | Chemical name |
|---|---|---|
| 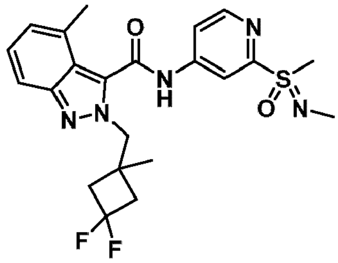 | SC-243 | 2-((3,3-difluoro-1-methylcyclobutyl)methyl)-N-(2-(N,S-dimethylsulfonimidoyl)pyridin-4-yl)-4-methyl-2H-indazole-3-carboxamide |
| 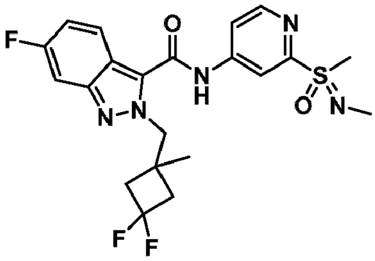 | SC-244 | 2-((3,3-difluoro-1-methylcyclobutyl)methyl)-N-(2-(N,S-dimethylsulfonimidoyl)pyridin-4-yl)-6-fluoro-2H-indazole-3 -carboxamide |
| 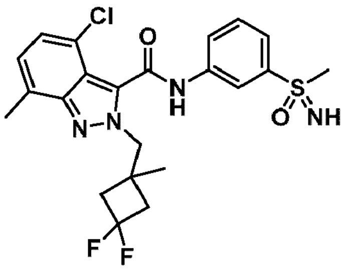 | SC-245 | 4-chloro-2-((3,3-difluoro-1-methylcyclobutyl)methyl)-7-methyl-N-(3-(S-methylsulfonimidoyl)phenyl)-2H-indazole-3-carboxamide |
| 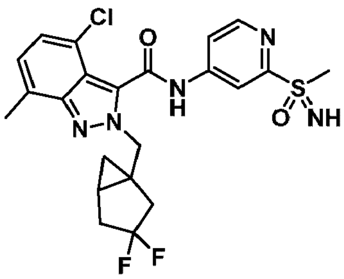 | SC-246 | 4-chloro-2-((3,3-difluorobicyclo[3.1.0]hexan-1-yl)methyl)-7-methyl-N-(2-(S-methylsulfonimidoyl)pyridin-4-yl)-2H-indazole-3-carboxamide |

## Electrophysiology: Voltage-Clamp recordings

The following $Na_V1.8$ recombinant cell line was used for recordings: HEK-$Na_V1.8$ (NM_006514.1) with b3 (NM_018400.3).

Sodium currents were measured using the patch clamp technique in the whole-cell configuration using the Qube384 (Sophion A/S, Copenhagen, Denmark) automated voltage clamp platform. "Multi hole" plates were used for the cell line expressing $Na_V1.8$ while "single hole" plates were used for the recombinant cell lines expressing the other subtypes. Appropriate filters (for minimum seal resistance and minimum current size) and series resistance compensation (for high quality sodium channel recordings) were applied. Data was collected at ambient room temperature.

The recording intracellular solution contained (in mM): NaCl 145 mM, KCl 4 mM, $CaCl_2$ 2 mM, $MgCl_2$ 1 mM, HEPES 10 mM, Glucose 10 mM, pH 7.4 (NaOH). The extracellular recording solution contained (in mM): CsF 120 mM, CsCl 20 mM, NaCl 10 mM, EGTA 10 mM, HEPES 10 mM, pH 7.2 (CsOH). Currents were recorded at 25 kHz sampling frequency and filtered at 5 kHz. Series resistance compensation was applied at 65%.

Vehicle (VEH) is the control condition where cells are exposed to 0.3 % DMSO without compound. All runs

include VEH controls being exposed to the same voltage protocols to assess non-compound related phenomena such as run down and then used to isolate compound dependent effects on currents.

To check for *state-dependence inhibition,* the following voltage-sequence was applied every 20 seconds: From a resting membrane potential of-120 mV, the first test pulse (P1; 20 ms to -10 mV) was applied to check for channels in the Resting State followed by a brief recovery (20 ms to -120 mV), then holding the membrane voltage to $V_{1/2}$ (4 seconds at voltage to obtain half the channel at Rest and half Inactivated) with a subsequent second test pulse (P2; 20 ms to -10 mV) to check for channels in the Inactivated State, followed by another brief recovery (20 ms to -120 mV) and a final third test pulse (P3; 20 ms to -10 mV) to check for recovered channels.

To check for *frequency-dependent inhibition,* a 10 Hz protocol and a 20 Hz protocol were applied; namely,

From a resting membrane potential of -120 mV, 40-pulses (10 ms to -10 mV) was applied at 10 Hz (at 100 ms between pulses) and then at 20 Hz (at 50 ms between pulses).

Each parameter was recorded (P1, P2, P3, P40 from 10Hz and P40 from 20Hz) during a control period (lasting ~ 5 minutes) when establishing the baseline and during compound period (lasting ~ 12 minutes) when test compound (or vehicle) was applied. For each parameter, the value at the end of the compound period was normalized to the vehicle baseline; as follows

$$Normalized\ Inhibition\ (Norm_{CPD}) = \frac{CPD\ Value_{end\ CPD\ period}}{VEH\ Value_{end\ Control\ Period}}$$

To adjust for any variance in the $Na^+$ current signal during the compound period (owing to cumulative inactivation independent of compound or shifts in biophysics over time), a dedicated segment of the recording wells in each 384 plate were dedicated to having only vehicle exposure. These vehicle-only recordings were used to correct for any apparent "run-up" or "run-down" in the experiment.

$$Normalized\ Inhibition\ (Norm_{VEH}) = \frac{VEH\ Value_{end\ CPD\ period}}{VEH\ Value_{end\ Control\ Period}}$$

The adjusted inhibition was calculated as follows:

$$\%\ Inhibition_{corrected} = 100 \times \frac{Norm_{CPD} - Norm_{VEH}}{100 - Norm_{VEH}}$$

Percent inhibition was determined and $IC_{50}$ values were calculated using a 4 parameter logistic model within XLFit Software (IDBS, Boston MA):

$$\%\ Inhibition_{corrected} = A + \frac{(B - A)}{(1 + \left(\frac{x}{C}\right)^{D}}$$

where A and B are the maximal and minimum inhibition respectively, C is the $IC_{50}$ concentration and D is the (Hill) slope.

The potency data of the example compounds are summarized in the table below (category A: human NaV1.8 $IC_{50} \leq 0.1$ μM; category B: 0.1 μM < human NaV1.8 $IC_{50}$ <_ 1 μM; category C: 1 μM < human NaV1.8 $IC_{50}$ <_ 10 μM; "n.d.": not determined):

| Example | Potency category | Example | Potency category | Example | Potency category |
|---|---|---|---|---|---|
| **SC-05** | A | **SC-37** | A | **SC-70** | C |
| **SC-06** | B | **SC-38** | A | **SC-71** | C |
| **SC-10** | B | **SC-39** | C | **SC-73** | B |
| **SC-11** | A | **SC-42** | A | **SC-74a** | B |
| **SC-12** | C | **SC-43** | A | **SC-74b** | C |
| **SC-13** | B | **SC-44** | A | **SC-76** | B |
| **SC-14** | C | **SC-45** | A | **SC-77** | A |
| **SC-15** | B | **SC-46** | B | **SC-80** | A |
| **SC-16** | C | **SC-47** | A | **SC-81** | A |
| **SC-17** | A | **SC-48** | C | **SC-82a** | A |
| **SC-19** | B | **SC-49** | B | **SC-82b** | A |
| **SC-20** | B | **SC-50** | C | **SC-83** | A |
| **SC-21** | C | **SC-53** | C | **SC-84a** | A |
| **SC-22** | B | **SC-56** | C | **SC-85** | A |
| **SC-24** | C | **SC-58** | B | **SC-86** | A |
| **SC-25** | B | **SC-60** | B | **SC-87** | A |
| **SC-26** | C | **SC-61** | B | **SC-88b** | A |
| **SC-28** | A | **SC-62** | B | **SC-89b** | A |
| **SC-32** | B | **SC-67** | B | | |
| **SC-36** | B | **SC-68** | A | | |

## Claims

**1.** A compound according to general formula (I)

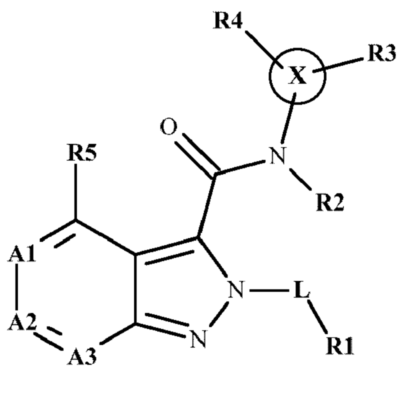

(I)

wherein

**L** represents $CH_2$, $CH(CH_3)$ or $CH(CH_2CH_3)$;
**R1** represents $C_{3-10}$-cycloalkyl, 4 to 10-membered heterocycloalkyl, 5 to 11-membered spiroalkyl, 5 to 11-membered heterospiroalkyl, 4 to 11-membered bicycloalkyl, or 5 to 11-membered heterobicycloalkyl;
**R2** represents H or $C_{1-6}$-alkyl;

**X** represents phenyl, or 5 to 9-membered heteroaryl;

**R3** represents S(O)-$C_{1-6}$-alkyl, S(O)-($C_{3-6}$-cycloalkyl), S(O)-(4 to 6-membered heterocycloalkyl), S(O)-phenyl, S(O)-(5 or 6-membered heteroaryl), $S(O)_2$-$C_{1-6}$-alkyl, $S(O)_2$-($C_{3-6}$-cycloalkyl), $S(O)_2$-(4 to 6-membered heterocycloalkyl), $S(O)_2$-phenyl, $S(O)_2$-(5 or 6-membered heteroaryl), S(O)-$NH_2$, S(O)-N(H)($C_{1-6}$-alkyl), S(O)N(H)($C_{3-6}$-cycloalkyl), S(O)N(H)(4 to 6-membered heterocycloalkyl), $S(O)_2$-$NH_2$, $S(O)_2$-N(H)($C_{1-6}$-alkyl), $S(O)_2$N(H)($C_{3-6}$-cycloalkyl), $S(O)_2$N(H)(4 to 6-membered heterocycloalkyl), S(O)-N($C_{1-6}$-alkyl)$_2$, $S(O)_2$-N($C_{1-6}$-alkyl)$_2$, C(O)-$C_{1-6}$-alkyl, C(O)-$NH_2$, C(O)-N(H)($C_{1-6}$-alkyl), C(O)-N(H)($C_{3-6}$-cycloalkyl), C(O)-N($C_{1-6}$-alkyl)$_2$, C(O)-N($C_{1-6}$-alkyl)($C_{3-6}$-cycloalkyl), CN, $C_{1-6}$-alkylene-OH, OH, =O, O-$C_{1-6}$-alkyl, $OCF_3$, $OCF_2H$, O-$C_{3-6}$-cycloalkyl, O-(4 to 6-membered heterocycloalkyl), $NH_2$, N(H)($C_{1-6}$-alkyl), N(H)(C(O)$C_{1-6}$-alkyl), N(H)(C(O)$C_{3-6}$-cycloalkyl), N(H)(C(O)$NH_2$), N(H)($S(O)_2$-$C_{1-6}$-alkyl), N(H)($S(O)_2$-(4 to 6-membered heterocycloalkyl), or S(O)(N**R3a**)**R3b**;

**R3a** represents H, $C_{1-6}$-alkyl, C(O)$C_{1-5}$-alkyl, C(O)$C_{1-5}$-alkyl-$NH_2$, C(O)$C_{1-5}$-alkyl-NH-$CH_3$, C(O)$C_{1-5}$-alkyl-N($CH_3$)$_2$, $C_{3-10}$-cycloalkyl, $C_{1-6}$-alkylene-$C_{3-10}$-cycloalkyl, 4 to 10-membered heterocycloalkyl, or $C_{1-6}$-alkylene-(4 to 10-membered heterocycloalkyl);

**R3b** represents $NH_2$, $C_{1-6}$-alkyl, $C_{3-10}$-cycloalkyl, $C_{1-6}$-alkylene-$C_{3-10}$-cycloalkyl, 4 to 10-membered heterocycloalkyl, or $C_{1-6}$-alkylene-(4 to 10-membered heterocycloalkyl);

or **R3a** and **R3b** mean $(CH_2)_{3-5}$ and together with the atoms to which they are attached form a ring;

**R4** represents H, F, Cl, Br, $C_{1-6}$-alkyl, $CF_3$, $CF_2H$, $CFH_2$ or $C_{3-6}$-cycloalkyl;

or **R3** and **R4** together with the atoms to which they are attached form a 5 to 6-membered heterocycloalkyl;

**R5** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl;

**A1** represents N or C**R6,** wherein **R6** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl;

**A2** represents N or C**R7,** wherein **R7** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl;

**A3** represents N or C**R8,** wherein **R8** represents H, F, Cl, CN, $C_{1-6}$-alkyl, $CHF_2$, $CH_2F$, $CF_3$, $C_{3-10}$-cycloalkyl, O-$C_{1-6}$-alkyl, or O-$C_{3-10}$-cycloalkyl;

wherein $C_{1-6}$-alkyl and $C_{1-6}$-alkylene in each case independently from one another is linear or branched, saturated or unsaturated;

wherein $C_{1-6}$-alkyl, $C_{1-6}$-alkylene, $C_{3-10}$-cycloalkyl, $C_{3-6}$-cycloalkyl, 4 to 10-membered heterocycloalkyl, 4 to 6-membered heterocycloalkyl, 4 to 11-membered bicycloalkyl, 5 to 11-membered heterobicycloalkyl, 5 to 11-membered spiroalkyl, and 5 to 11-membered heterospiroalkyl, in each case independently from one another are unsubstituted or substituted with one, two, three, four or more substituents independently from one another selected from the group consisting of F, Cl, CN, $C_{1-6}$-alkyl, $C_{1-6}$-alkylene-OH, $C_{1-6}$-alkylene-$OCH_3$, $CF_3$, $CF_2H$, $CFH_2$, C(O)-$C_{1-6}$-alkyl, OH, =O, $OCF_3$, $OCF_2H$, $OCFH_2$, O-$C_{1-6}$-alkyl, $C_{1-4}$-alkylene-O-$C_{1-4}$-alkylene-O-$CH_3$, $C_{0-4}$-alkylene-O-($C_{1-4}$-alkylene-O)$_{1-4}$-$CH_3$, $NH_2$, NH-$C_{1-6}$-alkyl, or N($C_{1-6}$-alkyl)$_2$;

wherein phenyl, 5 to 10-membered heteroaryl and 5 or 6-membered heteroaryl in each case independently from one another are unsubstituted or mono- or polysubstituted with one or more substituents selected from the group consisting of F, Cl, Br, CN, $C_{1-6}$-alkyl, $C_{3-6}$-cycloalkyl, $CF_3$, $CF_2H$, $CFH_2$, $C_{1-6}$-alkylene-$CF_3$, $C_{1-6}$-alkylene-$CF_2H$, $C_{1-6}$-alkylene-$CFH_2$, $C_{1-6}$-alkylene-OH, $C_{1-6}$-alkylene-$OCH_3$, C(O)-$C_{1-6}$-alkyl, $OCF_3$, $OCF_2H$, $OCFH_2$, and O-$C_{1-6}$-alkyl;

in the form of the free compound or a physiologically acceptable salt thereof.

2. The compound according to claim 1, wherein **L** represents $CH_2$ or CH($CH_3$).

3. The compound according to claim 1 or 2, wherein **R1** represents $C_{3-10}$-cycloalkyl, unsubstituted or substituted with one, two, three, four, or more substituents independently from one another selected from F, CN, $CH_3$, $CHF_2$, $CF_3$, OH, and $OCH_3$;

preferably cyclobutyl, cyclopentyl, cyclohexyl, or cycloheptyl, in each case optionally substituted with one, two or three substituents independently from one another selected from F, CN, $CH_3$, $CF_3$, OH, and $OCF_3$;

more preferably selected from cyclopropyl, fluoro cyclopropyl, cyano cyclopropyl, methyl cyclopropyl, trifluoromethyl cyclopropyl, trifluoromethoxy cyclopropyl, hydroxy cyclopropyl, difluoro cyclopropyl, dimethyl cyclopropyl, methyl hydroxy cyclopropyl, methyl difluoro cyclopropyl, cyclobutyl, fluoro cyclobutyl, cyano cyclobutyl, methyl cyclobutyl, trifluoromethyl cyclobutyl, trifluoromethoxy cyclobutyl, hydroxy cyclobutyl, difluoro cyclobutyl, dimethyl cyclobutyl, methyl hydroxy cyclobutyl, methyl difluoro cyclobutyl, cyclopentyl, fluoro cyclopentyl, cyano cyclopentyl, methyl cyclopentyl, trifluoromethyl cyclopentyl, trifluoromethoxy cyclopentyl, hydroxy cyclopentyl, difluoro cyclopentyl, dimethyl cyclopentyl, methyl hydroxy cyclopentyl, methyl difluoro cyclopentyl, cyclohexyl, fluoro cyclohexyl, cyano cyclohexyl, methyl cyclohexyl, trifluoromethyl cyclohexyl, trifluoromethoxy cyclohexyl, hydroxy cyclohexyl, difluoro cyclohexyl, dimethyl cyclohexyl, methyl hydroxy cyclohexyl, methyl difluoro cyclohexyl, cycloheptyl, fluoro cycloheptyl, cyano cycloheptyl, methyl cycloheptyl, trifluoromethyl cycloheptyl, trifluoromethoxy cycloheptyl, hydroxy cycloheptyl, difluoro cycloheptyl, dimethyl cycloheptyl, methyl hydroxy cycloheptyl, and methyl difluoro cycloheptyl; still more preferably cyclobutyl, dimethyl cyclobutyl, difluoro cyclobutyl, trifluoromethyl cyclobutyl, trifluoromethoxy cyclobutyl, methyl difluoro cyclobutyl, cyclopentyl, fluoro cyclopentyl, difluoro cyclopentyl, methyl difluoro cyclopentyl, trifluoromethyl cyclopentyl, cyclohexyl, methyl cyclohexyl, fluoro cyclohexyl, difluoro cyclohexyl, cycloheptyl.

4. The compound according to claim 1 or 2, wherein **R1** represents 4 to 10-membered heterocycloalkyl, unsubstituted or substituted with one, two, three, four, or more substituents independently from one another selected from F, CN, $CH_3$, $CHF_2$, $CF_3$, OH, and $OCH_3$;
preferably oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, azepanyl, dioxepanyl, oxazepanyl, diazepanyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, piperazinyl, N-methylpyridinonyl, pyrazolidinyl, pyranyl; dihydroquinolinyl, dihydroisoquinolinyl, dihydroindolinyl, dihydroisoindolyl, tetrahydroquinolinyl, tetrahydroisoquinolinyl or tetrahydroindolinyl, in each case unsubstituted or substituted with one, two, three, four, or more substituents independently from one another selected from F, and $CH_3$;
more preferably selected from tetrahydrofuranyl, methyl tetrahydrofuranyl, dimethyl tetrahydrofuranyl, fluoro tetrahydrofuranyl, difluoro tetrahydrofuranyl, tetrahydropyranyl, methyl tetrahydropyranyl, dimethyl tetrahydropyranyl, fluoro tetrahydropyranyl, difluoro tetrahydropyranyl, oxepanyl, methyl oxepanyl, dimethyl oxepanyl, fluoro oxepanyl, and difluoro oxepanyl;
still more preferably tetrahydrofuranyl, tetrahydropyranyl, methyl tetrahydropyranyl, dimethyl tetrahydropyranyl, difluoro tetrahydropyranyl, or oxepanyl.

5. The compound according to claim 1 or 2, wherein **R1** represents 5 to 11-membered spiroalkyl or dispiroalkyl, unsubstituted or substituted with one, two, three, four, or more substituents independently from one another selected from F, $CH_3$, $OCH_3$, CN, $CHF_2$ and $CF_3$;
preferably selected from spiro[2.2]pentyl, methyl spiro[2.2]pentyl, fluoro spiro[2.2]pentyl, difluoro spiro[2.2]pentyl, methyl difluoro spiro[2.2]pentyl, spiro[2.3]hexyl, methyl spiro[2.3]hexyl, fluoro spiro[2.3]hexyl, difluoro spiro[2.3]hexyl, methyl difluoro spiro[2.3]hexyl, spiro[3.3]heptyl, methyl spiro[3.3]heptyl, fluoro spiro[3.3]heptyl, difluoro spiro[3.3]heptyl, methyl difluoro spiro[3.3]heptyl, and dispiro[2.0.2.1]heptyl;
more preferably selected from spiro[2.2]pentyl, difluoro spiro[2.2]pentyl, spiro[2.3]hexyl, methyl spiro[2.3]hexyl, fluoro spiro[2.3]hexyl, methyl difluoro spiro[2.3]hexyl, spiro[3.3]heptyl, and dispiro[2.0.2.1]heptyl.

6. The compound according to claim 1 or 2, wherein **R1** represents 5 to 11-membered heterospiroalkyl, unsubstituted or substituted with one, two, three, four, or more substituents independently from one another selected from F, CN, $CH_3$, $CHF_2$, $CF_3$, OH, and $OCH_3$;
preferably selected from oxaspiro[3.2]hexyl, azaspiro[3.2]hexyl, oxaspiro[3.3]heptyl, azaspiro[3.3]heptyl, oxaspiro[4.2]heptyl, azaspiro[4.2]heptyl, oxaspiro[3.4]octyl, azaspiro[3.4]octyl, oxaspiro[4.3]octyl, azaspiro[4.3]octyl, oxaspiro[5.2]octyl, azaspiro[5.2]octyl, oxaspiro[3.5]nonyl, azaspiro[3.5]nonyl, oxaspiro[4.4]nonyl, azaspiro[4.4]nonyl, oxaspiro[5.3]nonyl, and azaspiro[5.3]nonyl;
more preferably oxaspiro[3.3]heptyl.

7. The compound according to claim 1 or 2, wherein **R1** represents 4 to 11-membered bicycloalkyl, unsubstituted or substituted with one, two, three, four, or more substituents independently from one another selected from F, $CH_3$, $OCH_3$, CN, $CHF_2$ and $CF_3$;
preferably selected from bicylo[1.1.1]pentyl, methyl bicylo[1.1.1]pentyl, methoxy bicylo[1.1.1]pentyl, difluoromethyl bicylo[1.1.1]pentyl, trifluoromethyl bicylo[1.1.1]pentyl, fluoro bicylo[1.1.1]pentyl, difluoro bicylo[1.1.1]pentyl, methyl difluoro bicylo[1.1.1]pentyl, trifluoromethyl difluoro bicylo[1.1.1]pentyl, bicyclo[2.1.0]pentyl, methyl bicyclo[2.1.0]pentyl, trifluoromethyl bicyclo[2.1.0]pentyl, fluoro bicyclo[2.1.0]pentyl, difluoro bicyclo [2.1.0]pentyl, methyl difluoro bicyclo[2.1.0]pentyl, trifluoromethyl difluoro bicyclo[2.1.0]pentyl, bicyclo[3.1.0]hexyl, methyl bicyclo[3.1.0]hexyl, trifluoromethyl bicyclo[3.1.0]hexyl, fluoro bicyclo[3.1.0]hexyl, difluoro bicyclo[3.1.0]hexyl, methyl difluoro bicyclo[3.1.0]hexyl, trifluoromethyl difluoro bicyclo[3.1.0]hexyl, bicyclo[2.2.0]hexyl, methyl bicyclo[2.2.0]hexyl, trifluoromethyl bicyclo[2.2.0]hexyl, fluoro bicyclo[2.2.0]hexyl, difluoro bicyclo[2.2.0]hexyl, methyl difluoro bicyclo[2.2.0]hexyl, trifluoromethyl difluoro bicyclo[2.2.0]hexyl, bicyclo[2.1.1]hexyl, methyl bicyclo[2.1.1]hexyl, trifluoromethyl bicyclo[2.1.1]hexyl, fluoro bicyclo[2.1.1]hexyl, difluoro bicyclo[2.1.1]hexyl, fluoro bicyclo[2.1.1]hexyl, difluoro bicyclo[2.1.1]hexyl, methyl difluoro bicyclo[2.1.1]hexyl, trifluoromethyl difluoro bicyclo[2.1.1]hexyl, bicyclo[4.1.0]heptyl, methyl bicyclo[4.1.0]heptyl, trifluoromethyl bicyclo[4.1.0]heptyl, fluoro bicyclo[4.1.0]heptyl, difluoro bicyclo[4.1.0]heptyl, methyl difluoro bicyclo[4.1.0]heptyl, trifluoromethyl difluoro bicyclo[4.1.0]heptyl, bicyclo[2.2.1]heptyl, methyl bicyclo[2.2.1]heptyl, trifluoromethyl bicyclo[2.2.1]heptyl, fluoro bicyclo[2.2.1]heptyl, difluoro bicyclo[2.2.1]heptyl, methyl difluoro bicyclo[2.2.1]heptyl, trifluoromethyl difluoro bicyclo[2.2.1]heptyl, bicyclo[3.3.0]octyl, methyl bicyclo[3.3.0]octyl, trifluoromethyl bicyclo[3.3.0]octyl, fluoro bicyclo [3.3.0]octyl, difluoro bicyclo [3.3.0]octyl, methyl difluoro bicyclo[3.3.0]octyl, and trifluoromethyl difluoro bicyclo[3.3.0]octyl;
more preferably selected from bicylo[1.1.1]pentyl, trifluoromethyl-bicylo[1.1.1]pentyl, bicyclo[2.1.0]pentyl, difluoro bicyclo[2.1.0]pentyl, bicyclo[3.1.0]hexyl, difluoro bicyclo[3.1.0]hexyl, methyl difluoro bicyclo[3.1.0]hexyl, trifluoromethyl difluoro bicyclo[3.1.0]hexyl, bicyclo[2.2.0]hexyl, methyl bicyclo[2.2.0]hexyl, bicyclo[2.1.1]hexyl, fluoro bicyclo[2.1.1]hexyl, difluoro bicyclo[2.1.1]hexyl, bicyclo[4.1.0]heptyl, difluoro bicyclo[4.1.0]heptyl, bicyclo[2.2.1]heptyl, fluoro bicyclo[2.2.1]heptyl, bicyclo[3.3.0]octyl, and fluoro bicyclo [3.3.0]octyl.

**8.** The compound according to claim 1 or 2, wherein **R1** represents 5 to 11-membered heterobicycloalkyl, unsubstituted or substituted with one, two, three, four, or more substituents independently from one another selected from F, $CH_3$, $OCH_3$, CN, $CHF_2$ and $CF_3$; preferably 2-oxa-bicyclo[2.1.1]hexyl or methyl 2-oxa-bicyclo[2.1.1]hexyl.

**9.** The compound according to any of the preceding claims, wherein **R2** represents H.

**10.** The compound according to any of the preceding claims, wherein **X** represents phenyl, fluorophenyl, pyridyl, pyrazolyl, pyrrolo[2,3-b]pyridyl, pyridonyl, thienyl, thiazolyl, 2,3-dihydrobenzo[d]isothiazolyl 1,1-dioxide, isoindolinonyl, pyrimidinyl, pyrazinyl, pyridazinyl, isoxazolyl, triazolyl, oxazolyl, oxadiazolyl, indazolyl, pyrazolopyridyl, pyrrolyl, imidazolyl, isothiazolyl, furanyl, thiadiazolyl, N-methylpyridinonyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indolizinyl, indolyl, isoquinolinyl, naphthyridinyl, phenazinyl, phenothiazinyl, phthalazinyl, purinyl, phenazinyl, tetrazolyl or triazinyl;
preferably phenyl, thienyl, thiazolyl, pyrazolyl, pyridyl, or pyrimidinyl.

**11.** The compound according to any of the preceding claims, wherein **R3** represents S(O)-$C_{1-6}$-alkyl, $S(O)_2$-$C_{1-6}$-alkyl, S(O)-$NH_2$, S(O)-N(H)($C_{1-6}$-alkyl), S(O)N(H)($C_{3-6}$-cycloalkyl), C(O)-$NH_2$, CN, $C_{1-6}$-alkylene-OH, OH, =O, O-$C_{1-6}$-alkyl, $NH_2$, or S(O)(N**R3a**)**R3b**, wherein **R3a** represents H or $C_{1-6}$-alkyl, and **R3b** represents $C_{1-6}$-alkyl; preferably $SOCH_3$, $SO_2CH_3$, $SO_2NH_2$, $SO_2NHCH_3$, $SO_2NH$-cyclopropyl, $S(O)(NH)CH_3$; $S(O)(NCH_3)CH_3$, CN, $CONH_2$, $C(CH_3)_2OH$, OH, $OCH_3$, =O, or $NH_2$.

**12.** The compound according to any of the preceding claims, wherein **R4** represents H, F, $C_{1-6}$-alkyl, $CFH_2$, $CF_2H$, $CF_3$, or $C_{3-6}$-cycloalkyl; preferably H, F, or $CH_3$.

**13.** The compound according to any of the preceding claims, wherein

**R5** represents H, F, Cl, CN, $C_{1-6}$-alkyl, $CHF_2$, $CH_2F$, $CF_3$, O-$C_{1-6}$-alkyl, or $C_{3-10}$-cycloalkyl; preferably H, F, Cl, $CH_3$, $CHF_2$, $CF_3$, $OCH_3$, or cyclopropyl;
**A1** represents N or C**R6;** wherein **R6** represents H, F, Cl, CN, $C_{1-6}$-alkyl, $CHF_2$, $CH_2F$, $CF_3$, O-$C_{1-6}$-alkyl, or $C_{3-10}$-cycloalkyl; preferably H, $CH_3$, or $CF_3$;
**A2** represents N or C**R7;** wherein **R7** represents H, F, Cl, CN, $C_{1-6}$-alkyl, $CHF_2$, $CH_2F$, $CF_3$, O-$C_{1-6}$-alkyl, or $C_{3-10}$-cycloalkyl; preferably H, F, Cl, $CH_3$, or $CF_3$; and
**A3** represents C**R8;** wherein **R8** represents H, F, Cl, CN, $C_{1-6}$-alkyl, $CHF_2$, $CH_2F$, $CF_3$, O-$C_{1-6}$-alkyl, or $C_{3-10}$-cycloalkyl; preferably H, F, Cl, CN, $CH_3$, $CHF_2$, $CF_3$, or cyclopropyl.

**14.** The compound according to any of the preceding claims which is selected from the group consisting of

- 2-(cyclohexylmethyl)-6-fluoro-*N*-(2-hydroxypyridin-4-yl)indazole-3-carboxamide;
- 2-[(4,4-difluorocyclohexyl)methyl]-6-fluoro-*N*-(2-hydroxypyridin-4-yl)indazole-3-carboxamide;
- 2-(cyclohexylmethyl)-*N*-(2-hydroxypyridin-4-yl)indazole-3-carboxamide;
- 2-(cyclohexylmethyl)-6-fluoro-*N*-(3-methylsulfonylphenyl)indazole-3-carboxamide;
- 2-(cyclobutylmethyl)-*N*-(2-hydroxypyridin-4-yl)indazole-3-carboxamide;
- 2-(cycloheptylmethyl)-*N*-(2-hydroxypyridin-4-yl)indazole-3-carboxamide;
- *N*-(3-methylsulfonylphenyl)-2-(oxan-4-ylmethyl)indazole-3-carboxamide;
- 2-(oxan-4-yhnethyl)-*N*-(3-sulfamoylphenyl)indazole-3-carboxamide;
- 2-(oxolan-2-yhnethyl)-*N*-(3-sulfamoylphenyl)indazole-3-carboxamide;
- *N*-(3-carbamoylphenyl)-2-(cyclohexylmethyl)indazole-3-carboxamide;

• 2-(cyclohexylmethyl)-*N*-(3-sulfamoylphenyl)indazole-3-carboxamide;
• 2-(cyclohexylmethyl)-6-fluoro-*N*-[3-[(methylsulfinyl]phenyl]indazole-3-carboxamide;
• 2-(cyclohexylmethyl)-7-fluoro-*N*-(2-hydroxypyridin-4-yl)indazole-3-carboxamide;
• *N*-(3-carbamoyl-4-fluorophenyl)-2-(oxan-4-ylmethyl)indazole-3-carboxamide;
• 2-[(4,4-difluorocyclohexyl)methyl]-*N*-(3-methylsulfonylphenyl)indazole-3-carboxamide;
• 2-[(4,4-difluorocyclohexyl)methyl]-*N*-(3-sulfamoylphenyl)indazole-3-carboxamide;
• 2-[(4,4-difluorocyclohexyl)methyl]-*N*-(2-hydroxypyridin-4-yl)indazole-3-carboxamide;
• *N*-(3-carbamoyl-4-fluorophenyl)-2-[(4,4-difluorocyclohexyl)methyl]indazole-3-carboxamide;
• 2-(cyclohexylmethyl)-*N*-[3-(2-hydroxypropan-2-yl)phenyl]indazole-3-carboxamide;
• 2-[(4,4-difluorocyclohexyl)methyl]-6-fluoro-*N*-(3-methylsulfonylphenyl)indazole-3-carboxamide;
• 2-(cyclohexylmethyl)-6-fluoro-*N*-(3-sulfamoylphenyl)indazole-3-carboxamide;
• 2-(cyclohexylmethyl)-7-fluoro-*N*-(3-methylsulfonylphenyl)indazole-3-carboxamide;
• 2-(cyclohexylmethyl)-5-fluoro-*N*-(2-hydroxypyiidin-4-yl)indazole-3-carboxamide;
• 2-(cyclohexylmethyl)-*N*-(3-fluoro-5-methylsulfonylphenyl)indazole-3-carboxamide;
• 2-(cyclohexylmethyl)-*N*-(1-methylsulfonylpyrazol-4-yl)indazole-3-carboxamide;
• 2-(cyclohexylmethyl)-*N*-(3-methylsulfonylphenyl)indazole-3-carboxamide;
• 2-[(4,4-difluorocyclohexyl)methyl]-6-fluoro-*N*-(3-sulfamoylphenyl)indazole-3-carboxamide;
• 2-(cyclohexylmethyl)-*N*-(1-methyl-2-oxopyridin-4-yl)indazole-3-carboxamide;
• 2-(cyclohexylmethyl)-*N*-(2-hydroxypyridin-4-yl)-7-(trifluoromethyl)indazole-3-carboxamide;
• 2-(cyclohexylmethyl)-*N*-(2-hydroxypyiidin-4-yl)-7-methylindazole-3-carboxamide;
• 2-(cyclohexylmethyl)-*N*-(3-methylsulfonylphenyl)-7-(trifluoromethyl)indazole-3-carboxamide;
• 2-(cyclohexylmethyl)-7-methyl-*N*-(3-methylsulfonylphenyl)indazole-3-carboxamide;
• 2-(cyclohexylmethyl)-5-methyl-*N*-(3-methylsulfonylphenyl)indazole-3-carboxamide;
• 2-(cyclohexylmethyl)-4-fluoro-*N*-(3 -methylsulfonylphenyl)indazole-3 -carboxamide;
• 2-(cyclohexylmethyl)-*N*-(2-hydroxypyridin-4-yl)-5-methylindazole-3-carboxamide;
• 2-(cyclohexylmethyl)-4-fluoro-*N*-(2-hydroxypyridin-4-yl)indazole-3-carboxamide;
• *N*-(2-hydroxypyridin-4-yl)-2-(oxolan-3-ylmethyl)indazole-3-carboxamide;
• *N*-(2-hydroxypyridin-4-yl)-2-(oxan-2-ylmethyl)indazole-3-carboxamide;
• *N*-(3-methylsulfonylphenyl)-2-(oxolan-3-ylmethyl)indazole-3-carboxamide;
• *N*-(3-methylsulfonylphenyl)-2-(oxan-2-ylmethyl)indazole-3-carboxamide;
• 6-fluoro-*N*-(3-methylsulfonylphenyl)-2-(oxan-4-ylmethyl)indazole-3-carboxamide;
• 2-(2-cyclopentylethyl)-6-fluoro-*N*-(3-methylsulfonylphenyl)indazole-3-carboxamide;
• 2-[(3,3-difluorocyclobutyl)methyl]-6-fluoro-*N*-(3-methylsulfonylphenyl)indazole-3-carboxamide;
• 2-(cyclobutylmethyl)-*N*-(3-methylsulfonylphenyl)indazole-3-carboxamide;
• *N*-(3-methylsulfonylphenyl)-2-(oxan-3-ylmethyl)indazole-3-carboxamide;
• 2-(cyclopentylmethyl)-*N*-(3-methylsulfonylphenyl)indazole-3-carboxamide;
• 2-[(3,3-dimethylcyclobutyl)methyl]-6-fluoro-*N*-(3-methylsulfonylphenyl)indazole-3-carboxamide;
• 2-(cyclohexylmethyl)-*N*-(5-methylsulfonylthiophen-2-yl)indazolc-3-carboxamide;
• 6-fluoro-*N*-(3-methylsulfonylphenyl)-2-(spiro[2.5]octan-6-ylmethyl)indazole-3-carboxamide;
• 6-fluoro-*N*-(2-hydroxypyridin-4-yl)-2-[[oxan-2-yl]methyl]indazole-3-carboxamide;
• 2-(cyclopentylmethyl)-*N*-(2-hydroxypyridin-4-yl)indazole-3-carboxamide;
• 2-(1-cyclohexylethyl)-*N*-(3-methylsulfonylphenyl)indazole-3-carboxamide;
• *N*-(3-carbamoylphenyl)-2-[(4,4-difluorocyclohexyl)methyl]-6-fluoroindazole-3-carboxamide;
• 2-(cyclohexylmethyl)-*N*-(4-fluoro-3-methylsulfonylphenyl)indazole-3-carboxamide;
• 2-(cyclohexylmethyl)-*N*-(2-methylsulfonylpyridin-4-yl)indazole-3-carboxamide;
• 2-(cyclohexylmethyl)-*N*-(2-fluoro-3-methylsulfonylphenyl)indazole-3-carboxamide;
• 2-((3,3-difluorocyclopentyl)methyl)-7-methyl-*N*-(3-sulfamoylphenyl)-2H-indazole-3-carboxamide;
• 2-((3,3-difluorocyclohexyl)methyl)-7-fluoro-*N*-(3-sulfamoylphenyl)-2H-indazole-3-carboxamide;
• N-(2-carbamoylpyridin-4-yl)-4-chloro-2-((3,3-difluorocyclopentyl)methyl)-7-methyl-2H-indazole-3-carboxamide;
• 2-(cyclohexylmethyl)-4-methyl-*N*-(3-(methylsulfonyl)phenyl)-2H-indazole-3-carboxamide;
• 4-chloro-7-methyl-N-(3-sulfamoylphenyl)-2-((tetrahydro-2H-pyran-2-yl)methyl)-2H-indazole-3-carboxamide;
• 7-cyclopropyl-*N*-(3-sulfamoylphenyl)-2-((tetrahydro-2H-pyran-4-yl)methyl)-2H-indazole-3-carboxamide;
• *N*-(2-carbamoylpyridin-4-yl)-2-(cyclohexylmethyl)-6-fluoro-2H-indazole-3-carboxamide;
• 2-(cyclohexylmethyl)-6-fluoro-*N*-(2-sulfamoylpyridin-4-yl)-2H-indazole-3-carboxamide;
• 7-methyl-*N*-(2-sulfamoylpyridin-4-yl)-2-((tetrahydro-2H-pyran-2-yl)methyl)-2H-indazole-3-carboxamide;
• 7-(difluoromethyl)-N-(2-sulfamoylpyridin-4-yl)-2-((tetrahydro-2H-pyran-2-yl)methyl)-2H-indazole-3-carboxamide;

- 2-(1-bicyclo[2.1.1]hexanylmethyl)-*N*-(3-methylsulfonylphenyl)indazole-3-carboxamide;
- 2-(1-bicyclo[1.1.1]pentanylmethyl)-*N*-(3-methylsulfonylphenyl)indazole-3-carboxamide;
- 2-(cyclobutylmethyl)-*N*-(3-sulfamoylphenyl)indazole-3-carboxamide;
- 2-(oxolan-3-ylmethyl)-*N*(3-sulfamoylphenyl)indazole-3-carboxamide;
- *N*-(3-cyano-4-fluorophenyl)-2-[(4,4-difluorocyclohexyl)methyl]-6-fluoroindazole-3-carboxamide;
- 2-[(4,4-difluorocyclohexyl)methyl]-6-fluoro-*N*-(2-sulfamoylpyridin-4-yl)indazole-3-carboxamide;
- 2-[(4,4-difluorocyclohexyl)methyl]-6-fluoro-*N*-(4-fluoro-3-methylsulfonylphenyl)indazole-3-carboxamide;
- *N*-(3-carbamoyl-5-fluorophenyl)-2-[(4,4-difluorocyclohexyl)methyl]-6-fluoroindazole-3-carboxamide;
- *N*-(3-carbamoyl-4-fluorophenyl)-2-[(4,4-difluorocyclohexyl)methyl]-6-fluoroindazole-3-carboxamide;
- 2-(cyclohexylmethyl)-*N*-4-[*(R)*-methylsulfinyl]pyridin-2-yl]indazole-3-carboxamide;
- 6-fluoro-*N*-(3-methylsulfonylphenyl)-2-[[3-(trifluoromethyl)cyclobutyl]methyl]indazole-3-carboxamide;
- 6-fluoro-2-[(4-methylcyclohexyl)methyl]-*N*-(3-methylsulfonylphenyl)indazole-3-carboxamide;
- 2-(cyclohexylmethyl)-*N*-(2-hydroxypyridin-4-yl)pyrazolo[3,4-c]pyridine-3-carboxamide;
- 7-methyl-2-(oxan-2-ylmethyl)-*N*-(3-sulfamoylphenyl)indazole-3-carboxamide;
- 2-(cyclohexylmethyl)-6-fluoro-*N*-(2-methylsulfonylpyridin-4-yl)indazole-3-carboxamide;
- 2-[(4,4-difluorocyclohexyl)methyl]-*N*-[4-[(*R*)-methylsulfinyl]pyridin-2-yl]indazole-3-carboxamide;
- 2-(cyclohexylmethyl-*N*-(4-methylsulfonylpyridin-2-yl)indazole-3-carboxamide;
- 2-[(4,4-difluorocyclohexyl)methyl]-6-fluoro-*N*-(2-methylsulfonylpyridin-4-yl)indazole-3-carboxamide;
- 2-(cyclopentylmethyl)-6-fluoro-*N*-(3-sulfamoylphenyl)indazole-3-carboxamide;
- *N*-(3-carbamoylphenyl)-2-(cyclopentylmethyl)-6-fluoroindazole-3-carboxamide;
- 2-(cyclopentylmethyl)-*N*-(3-sulfamoylphenyl)indazole-3-carboxamide;
- 2-(cyclopentylmethyl)-6-fluoro-*N*-(3-methylsulfonylphenyl)indazole-3-carboxamide;
- 2-(oxan-2-ylmethyl)-*N*-(3-sulfamoylphenyl)-7-(trifluoromethyl)indazole-3-carboxamide;
- *N*-(1,1-dioxo-2,3-dihydro-1,2-benzothiazol-6-yl)-2-(oxan-4-ylmethyl)indazole-3-carboxamide;
- 6-fluoro-*N*-(3-methylsulfonylphenyl)-2-(2-oxaspiro[3.3]heptan-6-ylmethyl)indazole-3-carboxamide;
- 6-fluoro-*N*-(3-methylsulfonylphenyl)-2-[[rac-(3R)-oxolan-3-yl]methyl]indazole-3-carboxamide;
- 2-[[2-(cyclohexylmethyl)-6-fluoroindazole-3-carbonyl]amino]-1,3-thiazole-5-carboxamide;
- 2-(cyclohexylmethyl)-6-fluoro-*N*-(3-oxo-1,2-dihydroisoindol-5-yl)indazole-3-carboxamide;
- 2-[(4,4-difluorocyclohexyl)methyl]-*N*-(2-methylsulfonylpyndin-4-yl)indazole-3-carboxamide;
- *N*-(5-carbamoylthiophen-2-yl)-2-[(4,4-difluorocyclohexyl)methyl]-6-fluoroindazole-3-carboxamide;
- 6-methyl-2-(oxan-2-ylmethyl)-*N*-(3-sulfamoylphenyl)indazole-3-carboxamide;
- 2-(cyclohexylmethyl)-*N*-(5-methylsulfonylpyridin-3-yl)indazole-3-carboxamide;
- 2-(cyclohexylmethyl)-*N*-(5-methylsulfonylpyridin-3-yl)indazole-3-carboxamide;
- 2-[(4,4-difluorocyclohexyl)methyl]-6-fluoro-*N*-(3-sulfamoylphenyl)indazole-3-carboxamide;
- 2-(cycloheptylmethyl)-6-fluoro-*N*-(3-methylsulfonylphenyl)indazole-3-carboxamide;
- *N*-(5-carbamoylthiophen-3-yl)-2-((4,4-difluorocyclohexyl)methyl)-6-fluoro-2H-indazole-3-carboxamide;
- *N*-(3-cyanophenyl)-2-((4,4-difluorocyclohexyl)methyl)-2H-indazole-3-carboxamide;
- 2-((4,4-difluorocyclohexyl)methyl)-6-fluoro-N-(3-fluoro-5-(methylsulfonyl)phenyl)-2H-indazole-3-carboxamide;
- N-(3-sulfamoylphenyl)-2-((tetrahydro-2H-pyran-2 yl)methyl)-2H-indazole-3-carboxamide;
- 2-((1-cyanocyclohexyl)methyl)-*N*-(3-(methylsulfonyl)phenyl)-2H-indazole-3-carboxamide;
- 2-((4,4-difluorocyclohexyl)methyl)-7-fluoro-*N*-(3-sulfamoylphenyl)-2H-indazole-3-carboxamide;
- *N*-(4-fluoro-3-sulfamoylphenyl)-2-((tetrahydro-2H-pyran-4-yl)methyl)-2H-indazole-3-carboxamide;
- *N*-[3-(methylsulfamoyl)phenyl]-2-(oxan-4-ylmethyl)indazole-3-carboxamide;
- 2-(cyclobutylmethyl)-6-fluoro-N-(3-sulfamoylphenyl)indazole-3-carboxamide;
- 2-(cyclobutylmethyl)-6-fluoro-N-(3-methylsulfonylphenyl)indazole-3-carboxamide;
- N-(2-carbamoylpyridin-4-yl)-2-(cyclohexylmethyl)-6-fluoroindazole-3-carboxamide;
- 2-[(4,4-difluorocyclohexyl)methyl]-N-(2-sulfamoylpyridin-4-yl)indazole-3-carboxamide;
- N-(2-carbamoylpyridin-4-yl)-2-[(4,4-difluorocyclohexyl)methyl]-6-fluoroindazole-3-carboxamide;
- 2-[(4,4-difluorocyclohexyl)methyl]-6-fluoro-N-(4-fluoro-3-hydroxyphenyl)indazole-3-carboxamide;
- 2-[(4,4-difluorocyclohexyl)methyl]-7-fluoro-N-(4-fluoro-3-methylsulfonylphenyl)indazole-3-carboxamide;
- 2-[(4,4-difluorocyclohexyl)methyl]-7-fluoro-N-(2-sulfamoylpyridin-4-yl)indazole-3-carboxamide;
- 4-fluoro-2-(oxan-4-ylmethyl)-N-(3-sulfamoylphenyl)indazole-3-carboxamide;
- 2-(cyclobutylmethyl)-4-fluoro-N-(3-sulfamoylphenyl)indazole-3-carboxamide;
- 2-[(3,3-difluorocyclopentyl)methyl]-6-methyl-N-(3-sulfamoylphenyl)indazole-3-carboxamide;
- 2-(oxan-2-ylmethyl)-N-(3-sulfamoylphenyl)-6-(trifluoromethyl)indazole-3-carboxamide;
- 2-(cyclohexylmethyl)-N-(2-hydroxypyridin-4-yl)-5-(trifluoromethyl)indazole-3-carboxamide;
- 2-[(3,3-difluorocyclopentyl)methyl]-7-methyl-N-(3-sulfamoylphenyl)indazole-3-carboxamide;

- N-(3-carbamoylphenyl)-2-[(l-fluorocyclohexyl)methyl]indazole-3-carboxamide;
- 2-[(l-fluorocyclohexyl)methyl]-N-(3-methylsulfonylphenyl)indazole-3-carboxamide;
- N-(3-methylsulfonylphenyl)-2-[[1-(trifluoromethyl)cyclobutyl]methyl]indazole-3-carboxamide;
- N-(4-carbamoylpyrimidin-2-yl)-2-(cyclohexylmethyl)-6-fluoroindazole-3-carboxamide;
- 2-[(3,3-difluorocyclopentyl)methyl]-6-fluoro-N-(3-sulfamoylphenyl)indazole-3-carboxamide;
- 2-[(3,3-difluorocyclopentyl)methyl]-6-fluoro-N-(2-sulfamoylpyridin-4 yl)indazole-3-carboxamide;
- 2-[(3,3-difluorocyclopentyl)methyl]-6-fluoro-N-(4-fluoro-3-sulfamoylphenyl)indazole-3-carboxamide;
- N-(2-carbamoylpyridin-4-yl)-2-[(4,4-difluorocyclohexyl)methyl]indazole-3-carboxamide;
- N-(3-methylsulfonylphenyl)-2-(oxepan-4-ylmethyl)indazole-3-carboxamide;
- 2-(cyclohexylmethyl)-4-methyl-N-(3-methylsulfonylphenyl)indazole-3-carboxamide;
- 4-methyl-2-(oxan-4-ylmethyl)-N-(3-sulfamoylphenyl)indazole-3-carboxamide;
- 4-methoxy-2-(oxan-4-ylmethyl)-N-(3-sulfamoylphenyl)indazole-3-carboxamide;
- N-(3-carbamoyl-4-fluorophenyl)-4-methyl-2-(oxan-4-ylmethyl)indazole-3-carboxamide;
- N-(3-carbamoyl-4-fluorophenyl)-4-methoxy-2-(oxan-4-ylmethyl)indazole-3-carboxamide;
- N-(2-fluoro-3-sulfamoylphenyl)-2-(oxan-4-ylmethyl)indazole-3-carboxamide;
- N-(3-carbamoyl-4-fluorophenyl)-4-cyclopropyl-2-(oxan-4-ylmethyl)indazole-3-carboxamide;
- 7-methyl-2-(oxan-4-ylmethyl)-N-(3-sulfamoylphenyl)indazole-3-carboxamide;
- N-(3-carbamoyl-4-fluorophenyl)-7-methyl-2-(oxan-4-ylmethyl)indazole-3-carboxamide;
- 2-[(2-methyloxan-4-yl)methyl]-N-(3-sulfamoylphenyl)indazole-3-carboxamide;
- 2-[(3,3-difluorocyclopentyl)methyl]-N-(3-sulfamoylphenyl)-6-(trifluoromethyl)indazole-3-carboxamide;
- N-(3-carbamoylphenyl)-2-[(4,4-difluorocyclohexyl)methyl]-7-fluoroindazole-3-carboxamide;
- 7-methyl-N-(2-methylsulfonylpyridin-4-yl)-2-(oxan-2-ylmethyl)indazole-3-carboxamide;
- 6-fluoro-N-(3-sulfamoylphenyl)-2-[[3-(trifluoromethyl)cyclobutyl]methyl]indazole-3-carboxamide;
- 2-[(3,3-difluorocyclohexyl)methyl]-6-fluoro-N-(3-methylsulfonylphenyl)indazole-3-carboxamide;
- N-(3-methylsulfonylphenyl)-2-[[3-(trifluoromethoxy)cyclobutyl]methyl]indazole-3-carboxamide;
- 2-[(3,3-difluorocyclopentyl)methyl]-7-fluoro-N-[3-(methylsulfamoyl)phenyl]indazole-3-carboxamide;
- N-[3-(cyclopropylsulfamoyl)phenyl]-2-[(3,3-difluorocyclopentyl)methyl]-7-fluoroindazole-3-carboxamide;
- 2-[(4,4-difluorocyclohexyl)methyl]-7-fluoro-N-(3-methylsulfinylphenyl)indazole-3-carboxamide;
- 2-[(4,4-difluorocyclohexyl)methyl]-7-fluoro-N-(3-methylsulfonylphenyl)indazole-3-carboxamide;
- 2-(oxan-4-ylmethyl)-N-(3-sulfamoylphenyl)-4-(trifluoromethyl)indazole-3-carboxamide;
- N-(3-carbamoyl-4-fluorophenyl)-2-(oxan-4-ylmethyl)-4-(trifluoromethyl)indazole-3-carboxamide;
- 2-(cyclohexylmethyl)-N-(3-methylsulfonylphenyl)-4-(trifluoromethyl)indazole-3-carboxamide;
- 2-[(3,3-dimethylcyclobutyl)methyl]-N-(3-methylsulfonylphenyl)indazole-3 -carboxamide;
- N-(3-methylsulfonylphenyl)-2-[[1-(trifluoromethyl)cyclopentyl]methyl]indazole-3-carboxamide;
- 2-[(4,4-difluorocyclohexyl)methyl]-7-fluoro-N-(4-fluoro-3-sulfamoylphenyl)indazole-3-carboxamide;
- 2-[(2,2-difluorocyclopentyl)methyl]-6-fluoro-N-(3-sulfamoylphenyl)indazole-3-carboxamide;
- 2-[(2,2-difluorocyclopentyl)methyl]-6-fluoro-N-(4-fluoro-3-sulfamoylphenyl)indazole-3-carboxamide;
- 6-fluoro-N-(3-methylsulfonylphenyl)-2-(spiro[2.3]hexan-5-ylmethyl)indazole-3-carboxamide;
- N-(2-carbamoylpyridin-4-yl)-2-(cyclopentylmethyl)-6-fluoroindazole-3-carboxamide;
- 2-[(1-fluorocyclopentyl)methyl]-N-(3-methylsulfonylphenyl)indazole-3-carboxamide;
- 7-methyl-2-(oxan-2ylmethyl)-N-(2-sulfamoylpyridin-4-yl)indazole-3-carboxamide;
- 2-[(2,2-difluorocyclopentyl)methyl]-6-fluoro-N-(2-sulfamoylpyridin-4-yl)indazole-3-carboxamide;
- 2-[(2,2-difluorocyclopentyl)methyl]-6-fluoro-N-(2-sulfamoylpyridin-4-yl)indazole-3-carboxamide;
- N-(2-carbamoylpyridin-4-yl)-7-fluoro-2-(oxan-2-ylmethyl)indazole-3-carboxamide;
- 2-(cyclohexylmethyl)-N-(2-hydroxypyridin-4-yl)-4-(trifluoromethyl)indazole-3-carboxamide;
- 7-chloro-2-[(4,4-difluorocyclohexyl)methyl]-N-(2-sulfamoylpyridin-4-yl)indazole-3-carboxamide;
- 7-fluoro-N-(2-methylsulfonylpyridin-4-yl)-2-(oxan-2-ylmethyl)indazole-3-carboxamide;
- 7-fluoro-2-(oxan-2ylmethyl)-N-(2-sulfamoylpyridin-4yl)indazole-3-carboxamide;
- 2-(oxan-4-ylmethyl)-N-(2-sulfamoylpyridin-4-yl)indazole-3-carboxamide;
- 2-[(3,3-difluorocyclopentyl)methyl]-N-(3-sulfamoylphenyl)-7-(trifluoromethyl)indazole-3 - carboxamide;
- 7-(difluoromethyl)-N-(2-methylsulfonylpyridin-4-yl)-2-(oxan-2-ylmethyl)indazole-3-carboxamide;
- 2-[(5,5-difluorooxan-2-yl)methyl]-7-methyl-N-(3-sulfamoylphenyl)indazole-3-carboxamide;
- N-(2-carbamoylpyridin-4-yl)-2-[(4,4-difluorocyclohexyl)methyl]-7-fluoroindazole-3-carboxamide;
- N-(2-carbamoylpyridin-4-yl)-7-methyl-2-(oxan-2-ylmethyl)indazole-3-carboxamide;
- N-(2-carbamoylpyridin-4-yl)-2-(oxan-2-ylmethyl)-7-(trifluoromethyl)indazole-3-carboxamide;
- N-(2-methylsulfonylpyridin-4-yl)-2-(oxan-2-ylmethyl)-7-(trifluoromethyl)indazole-3-carboxamide;
- 7-fluoro-N-(1-methylsulfonylpyrazol-4-yl)-2-(oxan-2-ylmethyl)indazole-3-carboxamide;
- 2-(3-bicyclo[3.1.0]hexanylmethyl)-N-(3-methylsulfonylphenyl)indazole-3-carboxamide;

• 2-[(3,3-difluorocyclopentyl)methyl]-4-methyl-N-(3-sulfamoylphenyl)indazole-3-carboxamide;
• 4-(difluoromethyl)-2-(oxan-4ylmethyl)-N-(2-sulfamoylpyridin-4 yl)indazole-3-carboxamide;
• N-(1-methylsulfonylpyrazol-4-yl)-2-(oxan-2-ylmethyl)-7-(trifluoromethyl)indazole-3-carboxamide;
• 7-methyl-N-(1-methylsulfonylpyrazol-4-yl)-2-(oxan-2-ylmethyl)indazole-3-carboxamide;
• 6-fluoro-2-[(4-hydroxy-4-methylcyclohexyl)methyl]-N-(3-methylsulfonylphenyl)indazole-3-carboxamide;
• 2-(oxan-2-ylmethyl)-N-(2-sulfamoylpyridin-4-yl)-7-(trifluoromethyl)indazole-3-carboxamide;
• 6-chloro-2-(oxan-4-ylmethyl)-N-(2-sulfamoylpyridin-4-yl)indazole-3-carboxamide;
• 2-[(1-hydroxycyclohexyl)methyl]-N-(3-methylsulfonylphenyl)indazole-3-carboxamide;
• 7-(difluoromethyl)-2-(oxan-2-ylmethyl)-N-(2-sulfamoylpyridin-4-yl)indazole-3-carboxamide;
• N-(2-carbamoylpyridin-4-yl)-2-[[(2S)-5,5-difluorooxan-2-yl]methyl]-7-methylindazole-3-carboxamide;
• 7-(difluoromethyl)-N-(1-methylsulfonylpyrazol-4 yl)-2-(oxan-2-ylmethyl)indazole-3-carboxamide;
• N-(2-carbamoylpyridin-4-yl)-7-(difluoromethyl)-2-(oxan-2-ylmethyl)indazole-3-carboxamide;
• N-(2-carbamoylpyridin-4yl)-2-[(5,5-difluorooxan-2-yl)methyl]-7-methylindazole-3-carboxamide;
• N-(2-carbamoylpyridin-4-yl)-2-[(6,6-dimethyloxan-2-yl)methyl]-7-methylindazole-3-carboxamide;
• N-(2-carbamoylpyridin-4-yl)-7-cyclopropyl-2-(oxan-4ylmethyl)indazole-3-carboxamide;
• 7-cyclopropyl-N-(2-methylsulfonylpyridin-4-yl)-2-(oxan-4-ylmethyl)indazole-3-carboxamide;
• 4-chloro-7-methyl-2-(oxan-2-ylmethyl)-N-(3-sulfamoylphenyl)indazole-3-carboxamide;
• 7-cyclopropyl-2-(oxan-4-ylmethyl)-N-(3-sulfamoylphenyl)indazole-3-carboxamide;
• N-(2-carbamoylpyridin-4-yl)-4-fluoro-7-methyl-2-(oxan-2-ylmethyl)indazole-3-carboxamide;
• N-(2-carbamoylpyridin-4-yl)-2-[(3,3-difluorocyclobutyl)methyl]-7-methylindazole-3-carboxamide;
• N-(2-carbamoylpyridin-4-yl)-7-chloro-2-[(3,3 -difluorocyclobutyl)methyl] -6-methylindazole-3 - carboxamide;
• N-(2-carbamoylpyridin-4-yl)-7-cyano-2-[(3,3-difluorocyclobutyl)methyl]indazole-3-carboxamide;
• N-(2-carbamoylpyridin-4-yl)-4-chloro-2-[(3,3-difluorocyclopentyl)methyl]-7-methylindazole-3-carboxamide;
• N-(2-carbamoylpyridin-4-yl)-2-[(3,3-difluorocyclopentyl)methyl]-4-fluoro-7-methylindazole-3-carboxamide;
• N-(2-carbamoylpyridin-4-yl)-7-chloro-2-[(3,3 -difluorocyclopentyl)methyl] -4-methylindazole-3 - carboxamide;
• 2-((3,3-difluoro-1-methylcyclobutyl)methyl)-7-methyl-N-(2-(S-methylsulfonimidoyl)pyridin-4-yl)-2H-indazole-3 -carboxamide;
• 2-((3,3-difluoro-1-methylcyclobutyl)methyl)-7-fluoro-N-(2-(S-methylsulfonimidoyl)pyridin-4-yl)-2H-indazole-3 -carboxamide;
• 7-cyclopropyl-2-((3,3-difluoro-1-methylcyclobutyl)methyl)-N-(2-(S-methylsulfonimidoyl)pyridin-4-yl)-2H-indazole-3-carboxamide;
• 4-chloro-2-((3,3-difluoro-1-methylcyclobutyl)methyl)-7-methyl-N-(2-(S-methylsulfonimidoyl)pyridin-4-yl)-2H-indazole-3-carboxamide;
• 2-((3,3-difluoro-1-methylcyclobutyl)methyl)-7-(difluoromethyl)-N-(2-(S-methylsulfonimidoyl)pyridin-4-yl)-2H-indazole-3-carboxamide;
• 2-((3,3-difluoro-1-methylcyclobutyl)methyl)-4-fluoro-7-methyl-N-(2-(S-methylsulfonimidoyl)pyridin-4-yl)-2H-indazole-3-carboxamide;
• 2-((3,3-difluoro-1-methylcyclobutyl)methyl)-4-methyl-N-(2-(S-methylsulfonimidoyl)pyridin-4-yl)-2H-indazole-3 -carboxamide;
• 2-((3,3-difluoro-1-methylcyclobutyl)methyl)-6-fluoro-N-(2-(S-methylsulfonimidoyl)pyridin-4-yl)-2H-indazole-3 -carboxamide;
• 2-((3,3-difluoro-1-methylcyclobutyl)methyl)-N-(2-(N,S-dimethylsulfonimidoyl)pyridin-4-yl)-7-methyl-2H-indazole-3-carboxamide;
• 2-((3,3-difluoro-1-methylcyclobutyl)methyl)-N-(2-(N,S-dimethylsulfonimidoyl)pyridin-4-yl)-7-fluoro-2H-indazole-3-carboxamide;
• 7-cyclopropyl-2-((3,3-difluoro-1-methylcyclobutyl)methyl)-N-(2-(N,S-dimethylsulfonimidoyl)pyndin-4-yl)-2H-indazole-3 -carboxamide;
• 4-chloro-2-((3,3-difluoro-1-methylcyclobutyl)methyl)-N-(2-(N,S-dimethylsulfonimidoyl)pyridin-4-yl)-7-methyl-2H-indazole-3-carboxamide;
• 2-((3,3-difluoro-1-methylcyclobutyl)methyl)-7-(difluoromethyl)-N-(2-(N,S-dimethylsulfonimidoyl)pyndin-4-yl)-2H-indazole-3 -carboxamide;
• 2-((3,3-difluoro-1-methylcyclobutyl)methyl)-N-(2-(N,S-dimethylsulfonimidoyl)pyridin-4-yl)-4-fluoro-7-methyl-2H-indazole-3-carboxamide;
• 2-((3,3-difluoro-1-methylcyclobutyl)methyl)-N-(2-(N,S-dimethylsulfonimidoyl)pyridin-4-yl)-4-methyl-2H-indazole-3-carboxamide;
• 2-((3,3-difluoro-1-methylcyclobutyl)methyl)-N-(2-(N,S-dimethylsulfonimidoyl)pyridin-4-yl)-6-fluoro-2H-indazole-3-carboxamide;
• 4-chloro-2-((3,3-difluoro-1-methylcyclobutyl)methyl)-7-methyl-N-(3-(S-methylsulfonimidoyl)phenyl)-2H-indazole-3 -carboxamide; and
- 4-chloro-2-((3,3-difluorobicyclo[3.1.0]hexan-1-yl)methyl)-7-methyl-N-(2-(S-methylsulfonimidoyl)pyridin-4-yl)-2H-indazole-3-carboxamide;

in the form of the free compound or a physiologically acceptable salt thereof.

**15.** A pharmaceutical dosage form comprising a compound according to any of claims 1 to 14.

Europäisches Patentamt
European Patent Office
Office européen des brevets

# EUROPEAN SEARCH REPORT

Application Number
EP 22 21 3327

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2009/049180 A2 (VERTEX PHARMA [US]; JOSHI PRAMOD [US] ET AL.) 16 April 2009 (2009-04-16) * compounds 6-12 * ----- | 1-15 | INV. C07D231/56 C07D401/12 C07D403/12 C07D405/06 C07D405/14 C07D409/12 C07D417/12 C07D417/14 A61P29/00 C07D471/04 A61K31/416 |
| A,D | WO 2020/092187 A1 (MERCK SHARP & DOHME [US]; BRESLIN MICHAEL J [US] ET AL.) 7 May 2020 (2020-05-07) * tables 1-13 * ----- | 1-15 | |
| A | WO 2020/092667 A1 (MERCK SHARP & DOHME [US]; ARASAPPAN ASHOK [US] ET AL.) 7 May 2020 (2020-05-07) * tables 1-9 * ----- | 1-15 | |
| | | | TECHNICAL FIELDS SEARCHED (IPC) |
| | | | C07D A61P |

The present search report has been drawn up for all claims

1

| Place of search | Date of completion of the search | Examiner |
|---|---|---|
| Munich | 24 April 2023 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 21 3327

This annex lists the patent family members relating to the patent documents cited in the above-mentioned European search report. The members are as contained in the European Patent Office EDP file on
The European Patent Office is in no way liable for these particulars which are merely given for the purpose of information.

24-04-2023

| Patent document cited in search report | Publication date | Patent family member(s) | Publication date |
|---|---|---|---|
| WO 2009049180 A2 | 16-04-2009 | AU 2008310660 A1 | 16-04-2009 |
| | | CA 2701946 A1 | 16-04-2009 |
| | | CN 101883760 A | 10-11-2010 |
| | | EP 2227453 A2 | 15-09-2010 |
| | | JP 5555169 B2 | 23-07-2014 |
| | | JP 2011500598 A | 06-01-2011 |
| | | NZ 584475 A | 27-07-2012 |
| | | US 2009099233 A1 | 16-04-2009 |
| | | WO 2009049180 A2 | 16-04-2009 |
| WO 2020092187 A1 | 07-05-2020 | EP 3873468 A1 | 08-09-2021 |
| | | US 2022119363 A1 | 21-04-2022 |
| | | WO 2020092187 A1 | 07-05-2020 |
| WO 2020092667 A1 | 07-05-2020 | AR 116939 A1 | 30-06-2021 |
| | | AU 2019372057 A1 | 27-05-2021 |
| | | AU 2022287562 A1 | 02-02-2023 |
| | | BR 112021008524 A2 | 03-08-2021 |
| | | CA 3117927 A1 | 07-05-2020 |
| | | CL 2021001078 A1 | 29-10-2021 |
| | | CN 113272293 A | 17-08-2021 |
| | | CO 2021005553 A2 | 30-07-2021 |
| | | CR 20210209 A | 20-05-2021 |
| | | DO P2021000082 A | 22-07-2021 |
| | | EA 202191177 A1 | 28-07-2021 |
| | | EC SP21030066 A | 31-05-2021 |
| | | EP 3873893 A1 | 08-09-2021 |
| | | IL 282468 A | 31-05-2021 |
| | | JP 2022506146 A | 17-01-2022 |
| | | KR 20210086687 A | 08-07-2021 |
| | | MA 54076 A | 09-02-2022 |
| | | NI 202100029 A | 13-08-2021 |
| | | PE 20211693 A1 | 01-09-2021 |
| | | SG 11202104326T A | 28-05-2021 |
| | | TW 202031643 A | 01-09-2020 |
| | | US 2020140411 A1 | 07-05-2020 |
| | | US 2021387966 A1 | 16-12-2021 |
| | | US 2022289710 A1 | 15-09-2022 |
| | | WO 2020092667 A1 | 07-05-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020092187 A
- WO 2020092667 A
- WO 2009049180 A
- WO 2009049183 A
- WO 2009049181 A
- WO 2021113627 A


### Non-patent literature cited in the description


- **LUMPKIN ; CATERINA.** *Nature,* 2007, vol. 445, 858-865
- **CRAWFORD ; CATERINA.** *Toxicologic Pathology,* 2020, vol. 48 (1), 174
- **GOODWIN G ; MCMAHON S.B.** *Nature Reviews Neuroscience,* 2021, vol. 22, 263-274
- **BENNETT D.L. et al.** *Physiol Rev,* 2019, vol. 99, 1079-1151
- **AKOPIAN A.N. et al.** *Nature,* 1996, vol. 379, 257-261
- **SANGAMESWARAN L. et al.** *J. Biol. Chem.,* 1996, vol. 271, 5953-5956
- **PERSSON A.K. et al.** *Mol. Pain.,* 2010, vol. 6, 84
- **RUSH A.M. et al.** *Eur.J.Neurosci,* 2005, vol. 22, 39-49
- **AKOPIAN A.N. et al.** *Nat. Neurosci,* 1999, vol. 2, 541-548
- **NOVAKOVIC S.D. et al.** *J. Neurosci.,* 1998, vol. 18, 2184-2187
- **BLAIR N.T. et al.** *J. Neurosci.,* 2003, vol. 23, 10338-10350
- **RENGANATHAN M et al.** *J. Neurophysiol,* 2001, vol. 86, 629-640
- **CHOI J.S.** *J. Neurophysiol,* 2011, vol. 106, 3173-3184
- **TAN Z.Y. et al.** *J. Neurosci.,* 2014, vol. 34, 7190-7197
- **FABER C.G. et al.** *Ann. Neurol,* 2012, vol. 71, 26-39
- **HAN C et al.** *J. Neurol Neurosurg Psychiatry,* 2014, vol. 85, 499-505
- **KIST A.M. et al.** *PLoS One,* 2016, vol. 11, e0161789
- **EIJKENBOOM I. et al.** *J. Neurol Neurosurg Psychiatry,* 2019, vol. 90 (3), 342-352
- **LAIRD J.M. et al.** *J. Neurosci,* 2002, vol. 22, 8352-8356
- **JARVIS M.F. et al.** *Proc Natl Acad Sci USA,* 2007, vol. 104, 8520-8525
- **JOSHI S.K. et al.** *Pain,* 2006, vol. 123, 75-82
- **ROZA C. et al.** *J Physiol,* 2003, vol. 550, 921-926
- **KORT M.E. et al.** *Bioorg Med Chem Lett,* 2010, vol. 20, 6812-6815
- **SCANIO M.J. et al.** *Bioorg Med Chem,* 2010, vol. 18, 7816-7825
- **PAYNE C.E. et al.** *Br J Pharmacol,* 2015, vol. 172, 2654-2670
- **HILLE, B.** *J. Gen. Physiol.,* 1977, vol. 69, 497-515
- **HILLE. B.** Ion Channels of Excitable Membranes. Sinauer Associates, Inc, 1992, 391-421
- **HONDEGHEM L.M. ; KATZUNG B.G.** *Annu. Rev. Pharmacol. Toxicol.,* 1984, vol. 24, 387-423
- **CATTERALL W.A.** *Trends Pharmacol. Sci.,* 1987, vol. 8, 57-65
- *Chem. Rev.,* 2009, vol. 109 (6), 2551-2651
- March's Advanced Organic Chemistry. 2007, 1427-1474
- Current Organic Synthesis. 2011, vol. 8, 53
- *Chem. Rev.,* 2016, vol. 116, 12564
- *Chem. Soc. Rev.,* 2014, vol. 43, 3525
- *Chem. Sci.,* 2010, vol. 1, 13
- **T. W. GREEN ; P. G. M. WUTS.** Protective Groups in Organic Synthesis. Wiley-Interscience, 1999